# EUROPEAN PATENT SPECIFICATION

(11) **EP 2 504 451 B1**
(45) Date of publication and mention of the grant of the patent: **05.06.2019**
(21) Application number: 10832289.2
(22) Date of filing: 19.11.2010
(51) Int. Cl.: C12Q 1/68

(54) **METHODS TO PREDICT CLINICAL OUTCOME OF CANCER**
VERFAHREN ZUR VORHERSAGE DES KLINISCHEN VERLAUFS VON KREBS
PROCÉDÉS DESTINÉS À PRÉDIRE L'ISSUE CLINIQUE D'UN CANCER

(30) Priority: 23.11.2009 US 263763 P
(43) Date of publication of application: 03.10.2012
(73) Proprietor: Genomic Health, Inc., Redwood City, CA 94063 (US)
(72) Inventor: BAKER, Joffre B., Montara, CA 94037 (US); CRONIN, Maureen T., Los Altos, CA 94024 (US); COLLIN, Francois, Berkeley, CA 94702 (US); LIU, Mei-Lan, San Mateo, CA 94403 (US)
(74) Representative: Gowshall, Jonathan Vallance
(86) International application number: PCT/US2010/057490
(87) International publication number: WO 2011/063274

(56) References cited:
- WO-A2-2005/008213
- US-A1- 2005 048 542
- US-A1- 2007 172 487
- US-A1- 2008 300 208
- LAURA J VAN 'T VEER ET AL: "Gene Expression Profiling Predicts Clinical Outcome of Breast Cancer", NATURE, NATURE PUBLISHING GROUP, UNITED KINGDOM, vol. 415, no. 6871, 31 January 2002 (2002-01-31), pages 530-536, XP008138701, ISSN: 0028-0836, DOI: 10.1038/415530A
- ALDONA KARCZEWSKA ET AL: "Expression of interleukin-6, interleukin-6 receptor, and glycoprotein 130 correlates with good prognoses for patients with breast carcinoma", CANCER., vol. 88, no. 9, 1 May 2000 (2000-05-01), pages 2061-2071, XP055237631, US ISSN: 0008-543X, DOI: 10.1002/(SICI)1097-0142(20000501)88:9<2061 ::AID-CNCR12>3.0.CO;2-O
- J.R.A. SHERWIN ET AL: "Global gene analysis of late secretory phase, eutopic endometrium does not provide the basis for a minimally invasive test of endometriosis", HUMAN REPRODUCTION, vol. 23, no. 5, 1 May 2008 (2008-05-01), pages 1063-1068, XP055237908, GB ISSN: 0268-1161, DOI: 10.1093/humrep/den078
- SEMBA ET AL.: 'Coexpression of actin-related protein 2 and wiskott-aldrich syndrome family verproline-homologous protein 2 in adenocarcinoma of the lung.' CLINICAL CANCER RESEARCH vol. 12, no. 8, 2006, pages 2449 - 2454, XP002550702
- WANG ET AL.: 'Identification and testing of a gene expression signature of invasive carcinoma cells within primary mammary tumors.' CANCER RESEARCH vol. 64, 2004, pages 8585 - 8594, XP055069643

### SEQUENCE LISTING

<110> Genomic Health, Inc.
   BAKER, JOFFRE B.
   Cronin, Maureen T.
   Collin, Francois
   Liu, Mei-Lan
<120> Methods To Predict Clinical Outcome Of
   Cancer
<130> GHDX-040WO
<150> US 61/263,763
   <151> 2009-11-23
<160> 1536
<170> FastSEQ for Windows Version 4.0
<210> 1
   <211> 23
   <212> DNA
   <213> Artificial Sequence
<220>
   <223> Synthetic primer
<400> 1
   cgttccgatc ctctatactg cat 23
<210> 2
   <211> 20
   <212> DNA
   <213> Artificial Sequence
<220>
   <223> Synthetic primer
<400> 2
   gtgtggcagg tggacactaa 20
<210> 3
   <211> 20
   <212> DNA
   <213> Artificial Sequence
<220>
   <223> Synthetic primer
<400> 3
   aaacaccact ggagcattga 20
<210> 4
   <211> 18
   <212> DNA
   <213> Artificial Sequence
<220>
   <223> Synthetic primer
<400> 4
   accagtgcca caatgcag 18
<210> 5
   <211> 20
   <212> DNA
   <213> Artificial Sequence
<220>
   <223> Synthetic primer
<400> 5
   tgcagactgt accatgctga 20
<210> 6
   <211> 20
   <212> DNA
   <213> Artificial Sequence
<220>
   <223> Synthetic primer
<400> 6
   acacgtctgt caccatggaa 20
<210> 7
   <211> 18
   <212> DNA
   <213> Artificial Sequence
<220>
   <223> Synthetic primer
<400> 7
   atccgcattg aagaccca 18
<210> 8
   <211> 20
   <212> DNA
   <213> Artificial Sequence
<220>
   <223> Synthetic primer
<400> 8
   gactgtctcg tttccctggt 20
<210> 9
   <211> 21
   <212> DNA
   <213> Artificial Sequence
<220>
   <223> Synthetic primer
<400> 9
   tcaaaagtac ggacacctcc t 21
<210> 10
   <211> 19
   <212> DNA
   <213> Artificial Sequence
<220>
   <223> Synthetic primer
<400> 10
   gagcatgcgt ctactgcct 19
<210> 11
   <211> 20
   <212> DNA
   <213> Artificial Sequence
<220>
   <223> Synthetic primer
<400> 11
   gaagtgccag gaggcgatta 20
<210> 12
   <211> 19
<212> DNA
   <213> Artificial Sequence
<220>
   <223> Synthetic primer
<400> 12
   caaggcccca tctgaatca 19
<210> 13
   <211> 20
   <212> DNA
   <213> Artificial Sequence
<220>
   <223> Synthetic primer
<400> 13
   gcgagttcaa agtgttcgag 20
<210> 14
   <211> 18
   <212> DNA
   <213> Artificial Sequence
<220>
   <223> Synthetic primer
<400> 14
   taagccacaa gcacacgg 18
<210> 15
   <211> 21
   <212> DNA
   <213> Artificial Sequence
<220>
   <223> Synthetic primer
<400> 15
   acgagatgtc ctacggcttg a 21
<210> 16
   <211> 22
   <212> DNA
   <213> Artificial Sequence
<220>
   <223> Synthetic primer
<400> 16
   agccaacatg tgactaattg ga 22
<210> 17
   <211> 27
   <212> DNA
   <213> Artificial Sequence
<220>
   <223> Synthetic primer
<400> 17
   ctgcatgtga ttgaataaga aacaaga 27
<210> 18
   <211> 19
   <212> DNA
   <213> Artificial Sequence
<220>
   <223> Synthetic primer
<400> 18
   gtggaaacgg agctcttcc 19
<210> 19
   <211> 25
   <212> DNA
   <213> Artificial Sequence
<220>
   <223> Synthetic primer
<400> 19
   ttgtctctgc cttggactat ctaca 25
<210> 20
   <211> 21
   <212> DNA
   <213> Artificial Sequence
<220>
   <223> Synthetic primer
<400> 20
   gaggaatatg gaatccaagg g 21
<210> 21
   <211> 20
   <212> DNA
   <213> Artificial Sequence
<220>
   <223> Synthetic primer
<400> 21
   ggacagggta agaccgtgat 20
<210> 22
   <211> 20
   <212> DNA
   <213> Artificial Sequence
<220>
   <223> Synthetic primer
<400> 22
   ccgtgaaagc tgctctgtaa 20
<210> 23
   <211> 23
   <212> DNA
   <213> Artificial Sequence
<220>
   <223> Synthetic primer
<400> 23
   caagacacta agggcgacta cca 23
<210> 24
   <211> 20
   <212> DNA
   <213> Artificial Sequence
<220>
   <223> Synthetic primer
<400> 24
   gactgcaaag atggaaacga 20
<210> 25
   <211> 20
   <212> DNA
   <213> Artificial Sequence
<220>
   <223> Synthetic primer
<400> 25
   gatgaagcct ttcgcaagtt 20
<210> 26
   <211> 18
   <212> DNA
   <213> Artificial Sequence
<220>
   <223> Synthetic primer
<400> 26
   gtttatgcca tcggcacc 18
<210> 27
   <211> 20
   <212> DNA
   <213> Artificial Sequence
<220>
   <223> Synthetic primer
<400> 27
   cagtagagat ccccgcaact 20
<210> 28
   <211> 20
   <212> DNA
   <213> Artificial Sequence
<220>
   <223> Synthetic primer
<400> 28
   atcagagatt accgcgtcgt 20
<210> 29
   <211> 19
   <212> DNA
   <213> Artificial Sequence
<220>
   <223> Synthetic primer
<400> 29
   gactgggtca gtgatggca 19
<210> 30
   <211> 20
   <212> DNA
   <213> Artificial Sequence
<220>
   <223> Synthetic primer
<400> 30
   tccctgagaa cgaaaccact 20
<210> 31
   <211> 21
   <212> DNA
   <213> Artificial Sequence
<220>
   <223> Synthetic primer
<400> 31
   agctgcagaa gagctgcaca t 21
<210> 32
   <211> 21
   <212> DNA
   <213> Artificial Sequence
<220>
   <223> Synthetic primer
<400> 32
   cagcagatgt ggatcagcaa g 21
<210> 33
   <211> 22
   <212> DNA
   <213> Artificial Sequence
<220>
   <223> Synthetic primer
<400> 33
   ggctcttgtg cgtactgtcc tt 22
<210> 34
   <211> 19
   <212> DNA
   <213> Artificial Sequence
<220>
   <223> Synthetic primer
<400> 34
   gggtcaggtg cctcgagat 19
<210> 35
   <211> 23
   <212> DNA
   <213> Artificial Sequence
<220>
   <223> Synthetic primer
<400> 35
   cgttgtcagc acttggaata caa 23
<210> 36
   <211> 18
   <212> DNA
   <213> Artificial Sequence
<220>
   <223> Synthetic primer
<400> 36
   cctacggccg ctactacg 18
<210> 37
   <211> 23
   <212> DNA
   <213> Artificial Sequence
<220>
   <223> Synthetic primer
<400> 37
   gactcctcag ggcagacttt ctt 23
<210> 38
   <211> 18
   <212> DNA
   <213> Artificial Sequence
<220>
   <223> Synthetic primer
<400> 38
   ccgccgtgga cacagact 18
<210> 39
   <211> 20
   <212> DNA
   <213> Artificial Sequence
<220>
   <223> Synthetic primer
<400> 39
   cctggagggt cctgtacaat 20
<210> 40
   <211> 20
   <212> DNA
   <213> Artificial Sequence
<220>
   <223> Synthetic primer
<400> 40
   actgacaaga ccagcagcat 20
<210> 41
   <211> 26
   <212> DNA
   <213> Artificial Sequence
<220>
   <223> Synthetic primer
<400> 41
   tgacttccta gttcgtgact ctctgt 26
<210> 42
   <211> 20
   <212> DNA
   <213> Artificial Sequence
<220>
   <223> Synthetic primer
<400> 42
   ccccgagaca acggagataa 20
<210> 43
   <211> 25
   <212> DNA
   <213> Artificial Sequence
<220>
   <223> Synthetic primer
<400> 43
   cagatggacc tagtacccac tgaga 25
<210> 44
   <211> 18
   <212> DNA
   <213> Artificial Sequence
<220>
   <223> Synthetic primer
<400> 44
   aacccacccc tgtcttgg 18
<210> 45
   <211> 18
   <212> DNA
   <213> Artificial Sequence
<220>
   <223> Synthetic primer
<400> 45
   gagctccgca aggatgac 18
<210> 46
   <211> 23
   <212> DNA
   <213> Artificial Sequence
<220>
   <223> Synthetic primer
<400> 46
   attcctatgg ctctgcaatt gtc 23
<210> 47
   <211> 20
   <212> DNA
   <213> Artificial Sequence
<220>
   <223> Synthetic primer
<400> 47
   ctggacggag tagctccaag 20
<210> 48
   <211> 19
   <212> DNA
   <213> Artificial Sequence
<220>
   <223> Synthetic primer
<400> 48
   aattttatga gggccacgg 19
<210> 49
   <211> 20
   <212> DNA
   <213> Artificial Sequence
<220>
   <223> Synthetic primer
<400> 49
   gttgggacac agttggtctg 20
<210> 50
   <211> 21
   <212> DNA
   <213> Artificial Sequence
<220>
   <223> Synthetic primer
<400> 50
   ccgaggttaa tccagcacgt a 21
<210> 51
   <211> 24
   <212> DNA
   <213> Artificial Sequence
<220>
   <223> Synthetic primer
<400> 51
   tcaacagaag gctgaaccac taga 24
<210> 52
   <211> 22
   <212> DNA
   <213> Artificial Sequence
<220>
   <223> Synthetic primer
<400> 52
   ctgaagcaga tggttcatca tt 22
<210> 53
   <211> 25
   <212> DNA
   <213> Artificial Sequence
<220>
   <223> Synthetic primer
<400> 53
   gaggcaactg cttatggctt aatta 25
<210> 54
   <211> 18
   <212> DNA
   <213> Artificial Sequence
<220>
   <223> Synthetic primer
<400> 54
   caagagcaga gccaccgt 18
<210> 55
   <211> 20
   <212> DNA
   <213> Artificial Sequence
<220>
   <223> Synthetic primer
<400> 55
   gtgactgcac aggactctgg 20
<210> 56
   <211> 20
   <212> DNA
   <213> Artificial Sequence
<220>
   <223> Synthetic primer
<400> 56
   tcagctgtga gctgcggata 20
<210> 57
   <211> 22
   <212> DNA
   <213> Artificial Sequence
<220>
   <223> Synthetic primer
<400> 57
   gcggtatcag gaatttcaac ct 22
<210> 58
   <211> 20
   <212> DNA
   <213> Artificial Sequence
<220>
   <223> Synthetic primer
<400> 58
   ctacgagtca gcccatccat 20
<210> 59
   <211> 20
   <212> DNA
   <213> Artificial Sequence
<220>
   <223> Synthetic primer
<400> 59
   tgatgctgca gagaacttcc 20
<210> 60
   <211> 22
   <212> DNA
   <213> Artificial Sequence
<220>
   <223> Synthetic primer
<400> 60
   atccattcga tctcaccaag gt 22
<210> 61
   <211> 20
   <212> DNA
   <213> Artificial Sequence
<220>
   <223> Synthetic primer
<400> 61
   gtggctcaac attgtgttcc 20
<210> 62
   <211> 20
   <212> DNA
   <213> Artificial Sequence
<220>
   <223> Synthetic primer
<400> 62
   aggggatggt ctctgtcatt 20
<210> 63
   <211> 21
   <212> DNA
   <213> Artificial Sequence
<220>
   <223> Synthetic primer
<400> 63
   gaacgcatca tccagagact g 21
<210> 64
   <211> 21
   <212> DNA
   <213> Artificial Sequence
<220>
   <223> Synthetic primer
<400> 64
   agcagacagt ggtcagtcct t 21
<210> 65
   <211> 20
   <212> DNA
   <213> Artificial Sequence
<220>
   <223> Synthetic primer
<400> 65
   aggttctgag ctctggcttt 20
<210> 66
   <211> 20
   <212> DNA
   <213> Artificial Sequence
<220>
   <223> Synthetic primer
<400> 66
   ttcaggttgt tgcaggagac 20
<210> 67
   <211> 27
   <212> DNA
   <213> Artificial Sequence
<220>
   <223> Synthetic primer
<400> 67
   cctctgtgct acagattata cctttgc 27
<210> 68
   <211> 25
   <212> DNA
   <213> Artificial Sequence
<220>
   <223> Synthetic primer
<400> 68
   ggtcaccaag aaacatcagt atgaa 25
<210> 69
   <211> 18
   <212> DNA
   <213> Artificial Sequence
<220>
   <223> Synthetic primer
<400> 69
   cagactgaat gggggtgg 18
<210> 70
   <211> 20
   <212> DNA
   <213> Artificial Sequence
<220>
   <223> Synthetic primer
<400> 70
   ggatgacatg cactcagctc 20
<210> 71
   <211> 20
   <212> DNA
   <213> Artificial Sequence
<220>
   <223> Synthetic primer
<400> 71
   ggagtggaag gaactggaaa 20
<210> 72
   <211> 21
   <212> DNA
   <213> Artificial Sequence
<220>
   <223> Synthetic primer
<400> 72
   tccaactaat gccaccacca a 21
<210> 73
   <211> 20
   <212> DNA
   <213> Artificial Sequence
<220>
   <223> Synthetic primer
<400> 73
   gtgctggagt cgggactaac 20
<210> 74
   <211> 19
   <212> DNA
   <213> Artificial Sequence
<220>
   <223> Synthetic primer
<400> 74
   atcaccgaca gcacagaca 19
<210> 75
   <211> 20
   <212> DNA
   <213> Artificial Sequence
<220>
   <223> Synthetic primer
<400> 75
   gacgaagaca gtccctggat 20
<210> 76
   <211> 20
   <212> DNA
   <213> Artificial Sequence
<220>
   <223> Synthetic primer
<400> 76
   ctcataccag ccatccaatg 20
<210> 77
   <211> 18
   <212> DNA
   <213> Artificial Sequence
<220>
   <223> Synthetic primer
<400> 77
   tggttcccag ccctgtgt 18
<210> 78
   <211> 20
   <212> DNA
   <213> Artificial Sequence
<220>
   <223> Synthetic primer
<400> 78
   gtgcaggctc aggtgaagtg 20
<210> 79
   <211> 21
   <212> DNA
   <213> Artificial Sequence
<220>
   <223> Synthetic primer
<400> 79
   tggattggag ttctgggaat g 21
<210> 80
   <211> 20
   <212> DNA
   <213> Artificial Sequence
<220>
   <223> Synthetic primer
<400> 80
   tcttgctggc tacgcctctt 20
<210> 81
   <211> 20
   <212> DNA
   <213> Artificial Sequence
<220>
   <223> Synthetic primer
<400> 81
   ggtgagcaga agtggcctat 20
<210> 82
   <211> 18
   <212> DNA
   <213> Artificial Sequence
<220>
   <223> Synthetic primer
<400> 82
   gcagtccgct gtgttcaa 18
<210> 83
   <211> 20
   <212> DNA
   <213> Artificial Sequence
<220>
   <223> Synthetic primer
<400> 83
   gagctgaaag acgcacactg 20
<210> 84
   <211> 18
   <212> DNA
   <213> Artificial Sequence
<220>
   <223> Synthetic primer
<400> 84
   cggagaaggg caccagta 18
<210> 85
   <211> 18
   <212> DNA
   <213> Artificial Sequence
<220>
   <223> Synthetic primer
<400> 85
   gtcggcagaa gcaggact 18
<210> 86
   <211> 18
   <212> DNA
   <213> Artificial Sequence
<220>
   <223> Synthetic primer
<400> 86
   acccatgtac cgtcctcg 18
<210> 87
   <211> 20
   <212> DNA
   <213> Artificial Sequence
<220>
   <223> Synthetic primer
<400> 87
   ccttcccatc agcacagttc 20
<210> 88
   <211> 20
   <212> DNA
   <213> Artificial Sequence
<220>
   <223> Synthetic primer
<400> 88
   aagccctatc cgatgtaccc 20
<210> 89
   <211> 21
   <212> DNA
   <213> Artificial Sequence
<220>
   <223> Synthetic primer
<400> 89
   tggatctcta ccagcaatgt g 21
<210> 90
   <211> 22
   <212> DNA
   <213> Artificial Sequence
<220>
   <223> Synthetic primer
<400> 90
   acttgcctgt tcagagcact ca 22
<210> 91
   <211> 21
   <212> DNA
   <213> Artificial Sequence
<220>
   <223> Synthetic primer
<400> 91
   ttggttttgc tcggatactt g 21
<210> 92
   <211> 21
   <212> DNA
   <213> Artificial Sequence
<220>
   <223> Synthetic primer
<400> 92
   tgacaatcag cacacctgca t 21
<210> 93
   <211> 21
   <212> DNA
   <213> Artificial Sequence
<220>
   <223> Synthetic primer
<400> 93
   taaattcact cgtggtgtgg a 21
<210> 94
   <211> 20
   <212> DNA
   <213> Artificial Sequence
<220>
   <223> Synthetic primer
<400> 94
   ctgaaggagc tccaagacct 20
<210> 95
   <211> 21
   <212> DNA
   <213> Artificial Sequence
<220>
   <223> Synthetic primer
<400> 95
   ccagaatgca cgctacagga a 21
<210> 96
   <211> 18
   <212> DNA
   <213> Artificial Sequence
<220>
   <223> Synthetic primer
<400> 96
   ccaccatagg cagaggca 18
<210> 97
   <211> 20
   <212> DNA
   <213> Artificial Sequence
<220>
   <223> Synthetic primer
<400> 97
   gaggccagtg gtggaaacag 20
<210> 98
   <211> 20
   <212> DNA
   <213> Artificial Sequence
<220>
   <223> Synthetic primer
<400> 98
   aaggaagtgg tccctctgtg 20
<210> 99
   <211> 18
   <212> DNA
   <213> Artificial Sequence
<220>
   <223> Synthetic primer
<400> 99
   agaatgggtg tgaaggcg 18
<210> 100
   <211> 20
   <212> DNA
   <213> Artificial Sequence
<220>
   <223> Synthetic primer
<400> 100
   ggctggacgt ggttttgtct 20
<210> 101
   <211> 19
   <212> DNA
   <213> Artificial Sequence
<220>
   <223> Synthetic primer
<400> 101
   ggctgctttg ctgcaactg 19
<210> 102
   <211> 20
   <212> DNA
   <213> Artificial Sequence
<220>
   <223> Synthetic primer
<400> 102
   cggtacttga gcaatgccta 20
<210> 103
   <211> 23
   <212> DNA
   <213> Artificial Sequence
<220>
   <223> Synthetic primer
<400> 103
   ccccaggata cctaccacta cct 23
<210> 104
   <211> 21
   <212> DNA
   <213> Artificial Sequence
<220>
   <223> Synthetic primer
<400> 104
   aaatcgcagc ttatcacaag g 21
<210> 105
   <211> 18
   <212> DNA
   <213> Artificial Sequence
<220>
   <223> Synthetic primer
<400> 105
   gtggccatcc agctgacc 18
<210> 106
   <211> 24
   <212> DNA
   <213> Artificial Sequence
<220>
   <223> Synthetic primer
<400> 106
   cagccaagaa ctggtatagg agct 24
<210> 107
   <211> 21
   <212> DNA
   <213> Artificial Sequence
<220>
   <223> Synthetic primer
<400> 107
   ccttatcggc tggaacgagt t 21
<210> 108
   <211> 24
   <212> DNA
   <213> Artificial Sequence
<220>
   <223> Synthetic primer
<400> 108
   cgacagttgc gatgaaagtt ctaa 24
<210> 109
   <211> 20
   <212> DNA
   <213> Artificial Sequence
<220>
   <223> Synthetic primer
<400> 109
   atgcccagtg ttcctgactt 20
<210> 110
   <211> 20
   <212> DNA
   <213> Artificial Sequence
<220>
   <223> Synthetic primer
<400> 110
   gatgtgattg aggtgcatgg 20
<210> 111
   <211> 20
   <212> DNA
   <213> Artificial Sequence
<220>
   <223> Synthetic primer
<400> 111
   aagtcctgaa attgcgatca 20
<210> 112
   <211> 20
   <212> DNA
   <213> Artificial Sequence
<220>
   <223> Synthetic primer
<400> 112
   cagcaagaac tgcaacaaca 20
<210> 113
   <211> 18
   <212> DNA
   <213> Artificial Sequence
<220>
   <223> Synthetic primer
<400> 113
   tgcagcggct gattgaca 18
<210> 114
   <211> 20
   <212> DNA
   <213> Artificial Sequence
<220>
   <223> Synthetic primer
<400> 114
   gagcacaacc aaacctacga 20
<210> 115
   <211> 20
   <212> DNA
   <213> Artificial Sequence
<220>
   <223> Synthetic primer
<400> 115
   taccacaccc agcattcctc 20
<210> 116
   <211> 20
   <212> DNA
   <213> Artificial Sequence
<220>
   <223> Synthetic primer
<400> 116
   cctgaacatg aaggagctga 20
<210> 117
   <211> 19
   <212> DNA
   <213> Artificial Sequence
<220>
   <223> Synthetic primer
<400> 117
   gagttcaagt gccctgacg 19
<210> 118
   <211> 20
   <212> DNA
   <213> Artificial Sequence
<220>
   <223> Synthetic primer
<400> 118
   gctcacttcg gctaaaatgc 20
<210> 119
   <211> 24
   <212> DNA
   <213> Artificial Sequence
<220>
   <223> Synthetic primer
<400> 119
   gtacatgatc ccctgtgaga aggt 24
<210> 120
   <211> 21
   <212> DNA
   <213> Artificial Sequence
<220>
   <223> Synthetic primer
<400> 120
   tgtctcactg agcgagcaga a 21
<210> 121
   <211> 21
   <212> DNA
   <213> Artificial Sequence
<220>
   <223> Synthetic primer
<400> 121
   accaggcaat aacctaacag c 21
<210> 122
   <211> 19
   <212> DNA
   <213> Artificial Sequence
<220>
   <223> Synthetic primer
<400> 122
   ggagcaaaat cgatgcagt 19
<210> 123
   <211> 19
   <212> DNA
   <213> Artificial Sequence
<220>
   <223> Synthetic primer
<400> 123
   gagctacaga tgcccatgc 19
<210> 124
   <211> 18
   <212> DNA
   <213> Artificial Sequence
<220>
   <223> Synthetic primer
<400> 124
   tgcgcccttt cctctgta 18
<210> 125
   <211> 20
   <212> DNA
   <213> Artificial Sequence
<220>
   <223> Synthetic primer
<400> 125
   tgaccgcttc taccccaatg 20
<210> 126
   <211> 20
   <212> DNA
   <213> Artificial Sequence
<220>
   <223> Synthetic primer
<400> 126
   ccggagtgac tctatcacca 20
<210> 127
   <211> 26
   <212> DNA
   <213> Artificial Sequence
<220>
   <223> Synthetic primer
<400> 127
   tccttatagg tactttcagc catttg 26
<210> 128
   <211> 22
   <212> DNA
   <213> Artificial Sequence
<220>
   <223> Synthetic primer
<400> 128
   ccagctttgt gcctgtcact at 22
<210> 129
   <211> 20
   <212> DNA
   <213> Artificial Sequence
<220>
   <223> Synthetic primer
<400> 129
   tgctcattct tgaggagcat 20
<210> 130
   <211> 20
   <212> DNA
   <213> Artificial Sequence
<220>
   <223> Synthetic primer
<400> 130
   tggtgggtct aggtggtgta 20
<210> 131
   <211> 20
   <212> DNA
   <213> Artificial Sequence
<220>
   <223> Synthetic primer
<400> 131
   aaatgtcctc ctcgactgct 20
<210> 132
   <211> 20
   <212> DNA
   <213> Artificial Sequence
<220>
   <223> Synthetic primer
<400> 132
   ggtcaccgtt ggtgtcatca 20
<210> 133
   <211> 24
   <212> DNA
   <213> Artificial Sequence
<220>
   <223> Synthetic primer
<400> 133
   atggagatgt ggtcattcct agtg 24
<210> 134
   <211> 21
   <212> DNA
   <213> Artificial Sequence
<220>
   <223> Synthetic primer
<400> 134
   gccgccacaa gactaaggaa t 21
<210> 135
   <211> 20
   <212> DNA
   <213> Artificial Sequence
<220>
   <223> Synthetic primer
<400> 135
   tccaattcca gcatcactgt 20
<210> 136
   <211> 20
   <212> DNA
   <213> Artificial Sequence
<220>
   <223> Synthetic primer
<400> 136
   gattcagacg aggatgagcc 20
<210> 137
   <211> 21
   <212> DNA
   <213> Artificial Sequence
<220>
   <223> Synthetic primer
<400> 137
   cacggaggta taaggcagga g 21
<210> 138
   <211> 24
   <212> DNA
   <213> Artificial Sequence
<220>
   <223> Synthetic primer
<400> 138
   ctctgagaca gtgcttcgat gact 24
<210> 139
   <211> 22
   <212> DNA
   <213> Artificial Sequence
<220>
   <223> Synthetic primer
<400> 139
   tggcactact gcatgattga ca 22
<210> 140
   <211> 19
   <212> DNA
   <213> Artificial Sequence
<220>
   <223> Synthetic primer
<400> 140
   aaatcgctgg gaacaagtg 19
<210> 141
   <211> 20
   <212> DNA
   <213> Artificial Sequence
<220>
   <223> Synthetic primer
<400> 141
   agacatcagc tcctggttca 20
<210> 142
   <211> 18
   <212> DNA
   <213> Artificial Sequence
<220>
   <223> Synthetic primer
<400> 142
   tggtgacgat ggaggagc 18
<210> 143
   <211> 20
   <212> DNA
   <213> Artificial Sequence
<220>
   <223> Synthetic primer
<400> 143
   actccctcta cccttgagca 20
<210> 144
   <211> 19
   <212> DNA
   <213> Artificial Sequence
<220>
   <223> Synthetic primer
<400> 144
   ctgctggatg accttcctc 19
<210> 145
   <211> 20
   <212> DNA
   <213> Artificial Sequence
<220>
   <223> Synthetic primer
<400> 145
   tgccacctgg acatcatttg 20
<210> 146
   <211> 23
   <212> DNA
   <213> Artificial Sequence
<220>
   <223> Synthetic primer
<400> 146
   cgacaaggag tgcgtctact tct 23
<210> 147
   <211> 21
   <212> DNA
   <213> Artificial Sequence
<220>
   <223> Synthetic primer
<400> 147
   tttcctcaaa tttgcctcaa g 21
<210> 148
   <211> 19
   <212> DNA
   <213> Artificial Sequence
<220>
   <223> Synthetic primer
<400> 148
   actgtgaact gcctggtgc 19
<210> 149
   <211> 21
   <212> DNA
   <213> Artificial Sequence
<220>
   <223> Synthetic primer
<400> 149
   cgagtggaga ctggtgttct c 21
<210> 150
   <211> 18
   <212> DNA
   <213> Artificial Sequence
<220>
   <223> Synthetic primer
<400> 150
   atggactcca cagagccg 18
<210> 151
   <211> 20
   <212> DNA
   <213> Artificial Sequence
<220>
   <223> Synthetic primer
<400> 151
   ttgaacagag cctgaccaag 20
<210> 152
   <211> 19
   <212> DNA
   <213> Artificial Sequence
<220>
   <223> Synthetic primer
<400> 152
   gtccccgctg cagatctct 19
<210> 153
   <211> 20
   <212> DNA
   <213> Artificial Sequence
<220>
   <223> Synthetic primer
<400> 153
   ccatgtggat gaatgaggtg 20
<210> 154
   <211> 19
   <212> DNA
   <213> Artificial Sequence
<220>
   <223> Synthetic primer
<400> 154
   ggcggtgaag agtcacagt 19
<210> 155
   <211> 19
   <212> DNA
   <213> Artificial Sequence
<220>
   <223> Synthetic primer
<400> 155
   tcgagggcaa gaagagcaa 19
<210> 156
   <211> 25
   <212> DNA
   <213> Artificial Sequence
<220>
   <223> Synthetic primer
<400> 156
   gctagtactt tgatgctccc ttgat 25
<210> 157
   <211> 20
   <212> DNA
   <213> Artificial Sequence
<220>
   <223> Synthetic primer
<400> 157
   caaggccgtg aacgagaagt 20
<210> 158
   <211> 20
   <212> DNA
   <213> Artificial Sequence
<220>
   <223> Synthetic primer
<400> 158
   agccccagca actacagtct 20
<210> 159
   <211> 20
   <212> DNA
   <213> Artificial Sequence
<220>
   <223> Synthetic primer
<400> 159
   gggccctcca gaacaatgat 20
<210> 160
   <211> 21
   <212> DNA
   <213> Artificial Sequence
<220>
   <223> Synthetic primer
<400> 160
   cgcctgttca ccaagattga c 21
<210> 161
   <211> 18
   <212> DNA
   <213> Artificial Sequence
<220>
   <223> Synthetic primer
<400> 161
   caaccaggca gctccatc 18
<210> 162
   <211> 20
   <212> DNA
   <213> Artificial Sequence
<220>
   <223> Synthetic primer
<400> 162
   tgaacggggt atcctcctta 20
<210> 163
   <211> 20
   <212> DNA
   <213> Artificial Sequence
<220>
   <223> Synthetic primer
<400> 163
   tggtccatcg ccagttatca 20
<210> 164
   <211> 25
   <212> DNA
   <213> Artificial Sequence
<220>
   <223> Synthetic primer
<400> 164
   tggctcttaa tcagtttcgt tacct 25
<210> 165
   <211> 20
   <212> DNA
   <213> Artificial Sequence
<220>
   <223> Synthetic primer
<400> 165
   gtccaggtgg atgtgaaaga 20
<210> 166
   <211> 18
   <212> DNA
   <213> Artificial Sequence
<220>
   <223> Synthetic primer
<400> 166
   ccaacactag gctcccca 18
<210> 167
   <211> 22
   <212> DNA
   <213> Artificial Sequence
<220>
   <223> Synthetic primer
<400> 167
   ggcattgagc ctctctacat ca 22
<210> 168
   <211> 18
   <212> DNA
   <213> Artificial Sequence
<220>
   <223> Synthetic primer
<400> 168
   gtcactccgc caccgtag 18
<210> 169
   <211> 18
   <212> DNA
   <213> Artificial Sequence
<220>
   <223> Synthetic primer
<400> 169
   acccccagac cggatcag 18
<210> 170
   <211> 20
   <212> DNA
   <213> Artificial Sequence
<220>
   <223> Synthetic primer
<400> 170
   ccagcaccat tgttgaagat 20
<210> 171
   <211> 19
   <212> DNA
   <213> Artificial Sequence
<220>
   <223> Synthetic primer
<400> 171
   cgtggtgccc ctctatgac 19
<210> 172
   <211> 19
   <212> DNA
   <213> Artificial Sequence
<220>
   <223> Synthetic primer
<400> 172
   accatgtatc gagaggggc 19
<210> 173
   <211> 20
   <212> DNA
   <213> Artificial Sequence
<220>
   <223> Synthetic primer
<400> 173
   tggaaacagc gaaggataca 20
<210> 174
   <211> 23
   <212> DNA
   <213> Artificial Sequence
<220>
   <223> Synthetic primer
<400> 174
   gtgaaggatg tgaagcagac gta 23
<210> 175
   <211> 18
   <212> DNA
   <213> Artificial Sequence
<220>
   <223> Synthetic primer
<400> 175
   ctgaccagaa ccacggct 18
<210> 176
   <211> 18
   <212> DNA
   <213> Artificial Sequence
<220>
   <223> Synthetic primer
<400> 176
   gcctcttcct gttcgacg 18
<210> 177
   <211> 19
   <212> DNA
   <213> Artificial Sequence
<220>
   <223> Synthetic primer
<400> 177
   gagggactgt tggcatgca 19
<210> 178
   <211> 20
   <212> DNA
   <213> Artificial Sequence
<220>
   <223> Synthetic primer
<400> 178
   ctggcttaag gatggacagg 20
<210> 179
   <211> 18
   <212> DNA
   <213> Artificial Sequence
<220>
   <223> Synthetic primer
<400> 179
   ccagtggagc gcttccat 18
<210> 180
   <211> 18
   <212> DNA
   <213> Artificial Sequence
<220>
   <223> Synthetic primer
<400> 180
   gacatctgcg ctccatcc 18
<210> 181
   <211> 21
   <212> DNA
   <213> Artificial Sequence
<220>
   <223> Synthetic primer
<400> 181
   ggaagtgaca gacgtgaagg t 21
<210> 182
   <211> 21
   <212> DNA
   <213> Artificial Sequence
<220>
   <223> Synthetic primer
<400> 182
   cgagcccttt gatgacttcc t 21
<210> 183
   <211> 18
   <212> DNA
   <213> Artificial Sequence
<220>
   <223> Synthetic primer
<400> 183
   gagaacaagc agggctgg 18
<210> 184
   <211> 20
   <212> DNA
   <213> Artificial Sequence
<220>
   <223> Synthetic primer
<400> 184
   tgaagtccag gacgatgatg 20
<210> 185
   <211> 19
   <212> DNA
   <213> Artificial Sequence
<220>
   <223> Synthetic primer
<400> 185
   cgacagagct tgtgcacct 19
<210> 186
   <211> 18
   <212> DNA
   <213> Artificial Sequence
<220>
   <223> Synthetic primer
<400> 186
   ctgtttgctg tccggagg 18
<210> 187
   <211> 22
   <212> DNA
   <213> Artificial Sequence
<220>
   <223> Synthetic primer
<400> 187
   ccagctgcta ctttgacatc ga 22
<210> 188
   <211> 26
   <212> DNA
   <213> Artificial Sequence
<220>
   <223> Synthetic primer
<400> 188
   ggataattca gacaacaaca ccatct 26
<210> 189
   <211> 20
   <212> DNA
   <213> Artificial Sequence
<220>
   <223> Synthetic primer
<400> 189
   gaagcgcaga tcatgaagaa 20
<210> 190
   <211> 19
   <212> DNA
   <213> Artificial Sequence
<220>
   <223> Synthetic primer
<400> 190
   tcagcagcaa gggcatcat 19
<210> 191
   <211> 18
   <212> DNA
   <213> Artificial Sequence
<220>
   <223> Synthetic primer
<400> 191
   tgtttggagg gaagggct 18
<210> 192
   <211> 18
   <212> DNA
   <213> Artificial Sequence
<220>
   <223> Synthetic primer
<400> 192
   gtgctggtga cgaatcca 18
<210> 193
   <211> 20
   <212> DNA
   <213> Artificial Sequence
<220>
   <223> Synthetic primer
<400> 193
   accctcgaca agaccacact 20
<210> 194
   <211> 20
   <212> DNA
   <213> Artificial Sequence
<220>
   <223> Synthetic primer
<400> 194
   attccaccca tggcaaattc 20
<210> 195
   <211> 23
   <212> DNA
   <213> Artificial Sequence
<220>
   <223> Synthetic primer
<400> 195
   caaaggagct cactgtggtg tct 23
<210> 196
   <211> 20
   <212> DNA
   <213> Artificial Sequence
<220>
   <223> Synthetic primer
<400> 196
   ttgggaaata tttgggcatt 20
<210> 197
   <211> 19
   <212> DNA
   <213> Artificial Sequence
<220>
   <223> Synthetic primer
<400> 197
   gcatgggaac catcaacca 19
<210> 198
   <211> 30
   <212> DNA
   <213> Artificial Sequence
<220>
   <223> Synthetic primer
<400> 198
   tgtagaatca aactcttcat catcaactag 30
<210> 199
   <211> 21
   <212> DNA
   <213> Artificial Sequence
<220>
   <223> Synthetic primer
<400> 199
   cgctccagac ctatgatgac t 21
<210> 200
   <211> 18
   <212> DNA
   <213> Artificial Sequence
<220>
   <223> Synthetic primer
<400> 200
   gatcccaagg cccaactc 18
<210> 201
   <211> 19
   <212> DNA
   <213> Artificial Sequence
<220>
   <223> Synthetic primer
<400> 201
   gttcactggg ggtgtatgg 19
<210> 202
   <211> 20
   <212> DNA
   <213> Artificial Sequence
<220>
   <223> Synthetic primer
<400> 202
   tgtcatgtac gacggcttct 20
<210> 203
   <211> 20
   <212> DNA
   <213> Artificial Sequence
<220>
   <223> Synthetic primer
<400> 203
   gttcgctacg aggattgagc 20
<210> 204
   <211> 20
   <212> DNA
   <213> Artificial Sequence
<220>
   <223> Synthetic primer
<400> 204
   ttctggacct gggaccttag 20
<210> 205
   <211> 20
   <212> DNA
   <213> Artificial Sequence
<220>
   <223> Synthetic primer
<400> 205
   cgtgcctcta caccatcttc 20
<210> 206
   <211> 20
   <212> DNA
   <213> Artificial Sequence
<220>
   <223> Synthetic primer
<400> 206
   agtacaagca ggctgccaag 20
<210> 207
   <211> 18
   <212> DNA
   <213> Artificial Sequence
<220>
   <223> Synthetic primer
<400> 207
   gcttatgacc gaccccaa 18
<210> 208
   <211> 24
   <212> DNA
   <213> Artificial Sequence
<220>
   <223> Synthetic primer
<400> 208
   cacacagatc tcctactcca tcca 24
<210> 209
   <211> 19
   <212> DNA
   <213> Artificial Sequence
<220>
   <223> Synthetic primer
<400> 209
   ctgagtgtgg tttgcggat 19
<210> 210
   <211> 20
   <212> DNA
   <213> Artificial Sequence
<220>
   <223> Synthetic primer
<400> 210
   ccatctgcat ccatcttgtt 20
<210> 211
   <211> 20
   <212> DNA
   <213> Artificial Sequence
<220>
   <223> Synthetic primer
<400> 211
   cagatgacaa tggccacaat 20
<210> 212
   <211> 21
   <212> DNA
   <213> Artificial Sequence
<220>
   <223> Synthetic primer
<400> 212
   tgcttaggtg cggtaaaacc a 21
<210> 213
   <211> 18
   <212> DNA
   <213> Artificial Sequence
<220>
   <223> Synthetic primer
<400> 213
   ccccaggcac cagcttta 18
<210> 214
   <211> 19
   <212> DNA
   <213> Artificial Sequence
<220>
   <223> Synthetic primer
<400> 214
   tgcccccaag acactgtgt 19
<210> 215
   <211> 27
   <212> DNA
   <213> Artificial Sequence
<220>
   <223> Synthetic primer
<400> 215
   aagctatgag gaaaagaagt acacgat 27
<210> 216
   <211> 19
   <212> DNA
   <213> Artificial Sequence
<220>
   <223> Synthetic primer
<400> 216
   ctgggctgtg aggctgaga 19
<210> 217
   <211> 20
   <212> DNA
   <213> Artificial Sequence
<220>
   <223> Synthetic primer
<400> 217
   ctgcaggcac tccctgaaat 20
<210> 218
   <211> 21
   <212> DNA
   <213> Artificial Sequence
<220>
   <223> Synthetic primer
<400> 218
   caatgccatc ttgcgctaca t 21
<210> 219
   <211> 19
   <212> DNA
   <213> Artificial Sequence
<220>
   <223> Synthetic primer
<400> 219
   caccatcccc accctgtct 19
<210> 220
   <211> 20
   <212> DNA
   <213> Artificial Sequence
<220>
   <223> Synthetic primer
<400> 220
   cccactcagt agccaagtca 20
<210> 221
   <211> 22
   <212> DNA
   <213> Artificial Sequence
<220>
   <223> Synthetic primer
<400> 221
   ccaaacgtgt aacaattatg cc 22
<210> 222
   <211> 27
   <212> DNA
   <213> Artificial Sequence
<220>
   <223> Synthetic primer
<400> 222
   caagtaccac agcgatgact acattaa 27
<210> 223
   <211> 18
   <212> DNA
   <213> Artificial Sequence
<220>
   <223> Synthetic primer
<400> 223
   tcctgtgctc tggaagcc 18
<210> 224
   <211> 20
   <212> DNA
   <213> Artificial Sequence
<220>
   <223> Synthetic primer
<400> 224
   cggtgtgaga agtgcagcaa 20
<210> 225
   <211> 18
   <212> DNA
   <213> Artificial Sequence
<220>
   <223> Synthetic primer
<400> 225
   gaaagatagc tcgcggca 18
<210> 226
   <211> 20
   <212> DNA
   <213> Artificial Sequence
<220>
   <223> Synthetic primer
<400> 226
   caggacacaa gtgccagatt 20
<210> 227
   <211> 21
   <212> DNA
   <213> Artificial Sequence
<220>
   <223> Synthetic primer
<400> 227
   tccatgatgg ttctgcaggt t 21
<210> 228
   <211> 19
   <212> DNA
   <213> Artificial Sequence
<220>
   <223> Synthetic primer
<400> 228
   tggcctgtcc attggtgat 19
<210> 229
   <211> 24
   <212> DNA
   <213> Artificial Sequence
<220>
   <223> Synthetic primer
<400> 229
   tccaggatgt taggaactgt gaag 24
<210> 230
   <211> 19
   <212> DNA
   <213> Artificial Sequence
<220>
   <223> Synthetic primer
<400> 230
   agcaggagcg accaactga 19
<210> 231
   <211> 18
   <212> DNA
   <213> Artificial Sequence
<220>
   <223> Synthetic primer
<400> 231
   gcagcagtcg gcttctct 18
<210> 232
   <211> 23
   <212> DNA
   <213> Artificial Sequence
<220>
   <223> Synthetic primer
<400> 232
   agtgacagat ggacaatgca aga 23
<210> 233
   <211> 22
   <212> DNA
   <213> Artificial Sequence
<220>
   <223> Synthetic primer
<400> 233
   tcccttgtgt tccttctgtg aa 22
<210> 234
   <211> 21
   <212> DNA
   <213> Artificial Sequence
<220>
   <223> Synthetic primer
<400> 234
   cgtgccttat ggttactttg g 21
<210> 235
   <211> 20
   <212> DNA
   <213> Artificial Sequence
<220>
   <223> Synthetic primer
<400> 235
   cagcctcaag ttcggttttc 20
<210> 236
   <211> 18
   <212> DNA
   <213> Artificial Sequence
<220>
   <223> Synthetic primer
<400> 236
   ctggaccgca cggacatc 18
<210> 237
   <211> 20
   <212> DNA
   <213> Artificial Sequence
<220>
   <223> Synthetic primer
<400> 237
   gctttccaag tggggaatta 20
<210> 238
   <211> 20
   <212> DNA
   <213> Artificial Sequence
<220>
   <223> Synthetic primer
<400> 238
   cagtctcgcc atgttgaagt 20
<210> 239
   <211> 19
   <212> DNA
   <213> Artificial Sequence
<220>
   <223> Synthetic primer
<400> 239
   ctgctgcgac agtccacta 19
<210> 240
   <211> 19
   <212> DNA
   <213> Artificial Sequence
<220>
   <223> Synthetic primer
<400> 240
   ggtccgcttc gtctttcga 19
<210> 241
   <211> 20
   <212> DNA
   <213> Artificial Sequence
<220>
   <223> Synthetic primer
<400> 241
   ttcagtgtgt ccagtgcatc 20
<210> 242
   <211> 24
   <212> DNA
   <213> Artificial Sequence
<220>
   <223> Synthetic primer
<400> 242
   ggctagtaga actggatccc aaca 24
<210> 243
   <211> 19
   <212> DNA
   <213> Artificial Sequence
<220>
   <223> Synthetic primer
<400> 243
   cctccctctg gtggtgctt 19
<210> 244
   <211> 19
   <212> DNA
   <213> Artificial Sequence
<220>
   <223> Synthetic primer
<400> 244
   ccgactggag gagcataaa 19
<210> 245
   <211> 27
   <212> DNA
   <213> Artificial Sequence
<220>
   <223> Synthetic primer
<400> 245
   gaataccaca ctttctgcta caacact 27
<210> 246
   <211> 24
   <212> DNA
   <213> Artificial Sequence
<220>
   <223> Synthetic primer
<400> 246
   gcagacagtg accatctaca gctt 24
<210> 247
   <211> 19
   <212> DNA
   <213> Artificial Sequence
<220>
   <223> Synthetic primer
<400> 247
   agaaccgcaa ggtgagcaa 19
<210> 248
   <211> 19
   <212> DNA
   <213> Artificial Sequence
<220>
   <223> Synthetic primer
<400> 248
   tggcctggct cttaatttg 19
<210> 249
   <211> 20
   <212> DNA
   <213> Artificial Sequence
<220>
   <223> Synthetic primer
<400> 249
   ggtggagagt ggagccatga 20
<210> 250
   <211> 21
   <212> DNA
   <213> Artificial Sequence
<220>
   <223> Synthetic primer
<400> 250
   gcatggtagc cgaagatttc a 21
<210> 251
   <211> 19
   <212> DNA
   <213> Artificial Sequence
<220>
   <223> Synthetic primer
<400> 251
   ccgtgcttcc ggacaactt 19
<210> 252
   <211> 20
   <212> DNA
   <213> Artificial Sequence
<220>
   <223> Synthetic primer
<400> 252
   tgaaccgcag agaccaacag 20
<210> 253
   <211> 23
   <212> DNA
   <213> Artificial Sequence
<220>
   <223> Synthetic primer
<400> 253
   gggtcactat ggagttcaaa gga 23
<210> 254
   <211> 20
   <212> DNA
   <213> Artificial Sequence
<220>
   <223> Synthetic primer
<400> 254
   gcctcccata gctccttacc 20
<210> 255
   <211> 26
   <212> DNA
   <213> Artificial Sequence
<220>
   <223> Synthetic primer
<400> 255
   aaggaaccat ctcactgtgt gtaaac 26
<210> 256
   <211> 20
   <212> DNA
   <213> Artificial Sequence
<220>
   <223> Synthetic primer
<400> 256
   ggcgctgtca tcgatttctt 20
<210> 257
   <211> 20
   <212> DNA
   <213> Artificial Sequence
<220>
   <223> Synthetic primer
<400> 257
   tggaaggttc cacaagtcac 20
<210> 258
   <211> 20
   <212> DNA
   <213> Artificial Sequence
<220>
   <223> Synthetic primer
<400> 258
   accctctggt ggtaaatgga 20
<210> 259
   <211> 20
   <212> DNA
   <213> Artificial Sequence
<220>
   <223> Synthetic primer
<400> 259
   ggcctaatgt tccagatcct 20
<210> 260
   <211> 20
   <212> DNA
   <213> Artificial Sequence
<220>
   <223> Synthetic primer
<400> 260
   actttcctgc gaggtcagtc 20
<210> 261
   <211> 19
   <212> DNA
   <213> Artificial Sequence
<220>
   <223> Synthetic primer
<400> 261
   gtgcccgagc catatagca 19
<210> 262
   <211> 20
   <212> DNA
   <213> Artificial Sequence
<220>
   <223> Synthetic primer
<400> 262
   agtccagccg agatgctaag 20
<210> 263
   <211> 20
   <212> DNA
   <213> Artificial Sequence
<220>
   <223> Synthetic primer
<400> 263
   ccacagctca ccttctgtca 20
<210> 264
   <211> 20
   <212> DNA
   <213> Artificial Sequence
<220>
   <223> Synthetic primer
<400> 264
   ccatgatcct cactctgctg 20
<210> 265
   <211> 21
   <212> DNA
   <213> Artificial Sequence
<220>
   <223> Synthetic primer
<400> 265
   caacgcttca gtgatcaatc c 21
<210> 266
   <211> 20
   <212> DNA
   <213> Artificial Sequence
<220>
   <223> Synthetic primer
<400> 266
   aggccagccc tacattatca 20
<210> 267
   <211> 20
   <212> DNA
   <213> Artificial Sequence
<220>
   <223> Synthetic primer
<400> 267
   cagtgacaaa cagcccttcc 20
<210> 268
   <211> 21
   <212> DNA
   <213> Artificial Sequence
<220>
   <223> Synthetic primer
<400> 268
   actcggactg cacaagctat t 21
<210> 269
   <211> 20
   <212> DNA
   <213> Artificial Sequence
<220>
   <223> Synthetic primer
<400> 269
   tcagaattgg atttggctca 20
<210> 270
   <211> 19
   <212> DNA
   <213> Artificial Sequence
<220>
   <223> Synthetic primer
<400> 270
   accggggagc cctacatga 19
<210> 271
   <211> 18
   <212> DNA
   <213> Artificial Sequence
<220>
   <223> Synthetic primer
<400> 271
   caaggtgccc tcagtgga 18
<210> 272
   <211> 20
   <212> DNA
   <213> Artificial Sequence
<220>
   <223> Synthetic primer
<400> 272
   tcgtgaaaga tgaccaggag 20
<210> 273
   <211> 19
   <212> DNA
   <213> Artificial Sequence
<220>
   <223> Synthetic primer
<400> 273
   tggcttacac tggcaatgg 19
<210> 274
   <211> 18
   <212> DNA
   <213> Artificial Sequence
<220>
   <223> Synthetic primer
<400> 274
   ctgtcagctg ctgcttgg 18
<210> 275
   <211> 19
   <212> DNA
   <213> Artificial Sequence
<220>
   <223> Synthetic primer
<400> 275
   cggactttgg gtgcgactt 19
<210> 276
   <211> 18
   <212> DNA
   <213> Artificial Sequence
<220>
   <223> Synthetic primer
<400> 276
   aagcccgagg cactcatt 18
<210> 277
   <211> 18
   <212> DNA
   <213> Artificial Sequence
<220>
   <223> Synthetic primer
<400> 277
   gctgggaggc aggacttc 18
<210> 278
   <211> 18
   <212> DNA
   <213> Artificial Sequence
<220>
   <223> Synthetic primer
<400> 278
   gagctccatg gctcatcc 18
<210> 279
   <211> 19
   <212> DNA
   <213> Artificial Sequence
<220>
   <223> Synthetic primer
<400> 279
   tctcttgcag gaagccaga 19
<210> 280
   <211> 25
   <212> DNA
   <213> Artificial Sequence
<220>
   <223> Synthetic primer
<400> 280
   aattcctgct ccaaaagaaa gtctt 25
<210> 281
   <211> 18
   <212> DNA
   <213> Artificial Sequence
<220>
   <223> Synthetic primer
<400> 281
   caccccggct tcaacaac 18
<210> 282
   <211> 20
   <212> DNA
   <213> Artificial Sequence
<220>
   <223> Synthetic primer
<400> 282
   gacgtgaggg tcctgattct 20
<210> 283
   <211> 20
   <212> DNA
   <213> Artificial Sequence
<220>
   <223> Synthetic primer
<400> 283
   catcctcatg gattggtgtg 20
<210> 284
   <211> 20
   <212> DNA
   <213> Artificial Sequence
<220>
   <223> Synthetic primer
<400> 284
   ccacctcgcc atgatttttc 20
<210> 285
   <211> 20
   <212> DNA
   <213> Artificial Sequence
<220>
   <223> Synthetic primer
<400> 285
   atgtgccagt gagcttgagt 20
<210> 286
   <211> 20
   <212> DNA
   <213> Artificial Sequence
<220>
   <223> Synthetic primer
<400> 286
   tgatggtcca aatgaacgaa 20
<210> 287
   <211> 18
   <212> DNA
   <213> Artificial Sequence
<220>
   <223> Synthetic primer
<400> 287
   cttgctggcc aatgccta 18
<210> 288
   <211> 20
   <212> DNA
   <213> Artificial Sequence
<220>
   <223> Synthetic primer
<400> 288
   cagatgaggc acatggagac 20
<210> 289
   <211> 18
   <212> DNA
   <213> Artificial Sequence
<220>
   <223> Synthetic primer
<400> 289
   ctcctggcca acagcact 18
<210> 290
   <211> 20
   <212> DNA
   <213> Artificial Sequence
<220>
   <223> Synthetic primer
<400> 290
   caaggagact gggaggtgtc 20
<210> 291
   <211> 20
   <212> DNA
   <213> Artificial Sequence
<220>
   <223> Synthetic primer
<400> 291
   actgaccaag cctgagacct 20
<210> 292
   <211> 21
   <212> DNA
   <213> Artificial Sequence
<220>
   <223> Synthetic primer
<400> 292
   actcaagcgg aaattgaagc a 21
<210> 293
   <211> 18
   <212> DNA
   <213> Artificial Sequence
<220>
   <223> Synthetic primer
<400> 293
   agcgatgaag atggtcgc 18
<210> 294
   <211> 20
   <212> DNA
   <213> Artificial Sequence
<220>
   <223> Synthetic primer
<400> 294
   agcggaaaat ggcagacaat 20
<210> 295
   <211> 20
   <212> DNA
   <213> Artificial Sequence
<220>
   <223> Synthetic primer
<400> 295
   gcttcaggtg ttgtgactgc 20
<210> 296
   <211> 20
   <212> DNA
   <213> Artificial Sequence
<220>
   <223> Synthetic primer
<400> 296
   tgaacagtaa tggggagctg 20
<210> 297
   <211> 19
   <212> DNA
   <213> Artificial Sequence
<220>
   <223> Synthetic primer
<400> 297
   tgcaaacgct ggtgtcaca 19
<210> 298
   <211> 18
   <212> DNA
   <213> Artificial Sequence
<220>
   <223> Synthetic primer
<400> 298
   ccaatgggag aacaacgg 18
<210> 299
   <211> 19
   <212> DNA
   <213> Artificial Sequence
<220>
   <223> Synthetic primer
<400> 299
   ctgcaacacc gaagtggac 19
<210> 300
   <211> 20
   <212> DNA
   <213> Artificial Sequence
<220>
   <223> Synthetic primer
<400> 300
   agaccaagct ggaagcagag 20
<210> 301
   <211> 18
   <212> DNA
   <213> Artificial Sequence
<220>
   <223> Synthetic primer
<400> 301
   acatccaggg ctctgtgg 18
<210> 302
   <211> 18
   <212> DNA
   <213> Artificial Sequence
<220>
   <223> Synthetic primer
<400> 302
   gacctggcct tgctgaag 18
<210> 303
   <211> 20
   <212> DNA
   <213> Artificial Sequence
<220>
   <223> Synthetic primer
<400> 303
   agaagctgtc cctgcaagag 20
<210> 304
   <211> 21
   <212> DNA
   <213> Artificial Sequence
<220>
   <223> Synthetic primer
<400> 304
   gacttttgcc cgctaccttt c 21
<210> 305
   <211> 20
   <212> DNA
   <213> Artificial Sequence
<220>
   <223> Synthetic primer
<400> 305
   aggatcgcct gtcagaagag 20
<210> 306
   <211> 20
   <212> DNA
   <213> Artificial Sequence
<220>
   <223> Synthetic primer
<400> 306
   gtgaaatgaa acgcaccaca 20
<210> 307
   <211> 21
   <212> DNA
   <213> Artificial Sequence
<220>
   <223> Synthetic primer
<400> 307
   acggatctac cacaccattg c 21
<210> 308
   <211> 21
   <212> DNA
   <213> Artificial Sequence
<220>
   <223> Synthetic primer
<400> 308
   gggagatcat cgggacaact c 21
<210> 309
   <211> 19
   <212> DNA
   <213> Artificial Sequence
<220>
   <223> Synthetic primer
<400> 309
   ccaacgcttg ccaaatcct 19
<210> 310
   <211> 20
   <212> DNA
   <213> Artificial Sequence
<220>
   <223> Synthetic primer
<400> 310
   ccatgatgga gaggcagaca 20
<210> 311
   <211> 26
   <212> DNA
   <213> Artificial Sequence
<220>
   <223> Synthetic primer
<400> 311
   ggatggtagc agtctaggga ttaact 26
<210> 312
   <211> 23
   <212> DNA
   <213> Artificial Sequence
<220>
   <223> Synthetic primer
<400> 312
   tcacctctca tcttcaccag gat 23
<210> 313
   <211> 20
   <212> DNA
   <213> Artificial Sequence
<220>
   <223> Synthetic primer
<400> 313
   ccatacgtgc tgctacctgt 20
<210> 314
   <211> 23
   <212> DNA
   <213> Artificial Sequence
<220>
   <223> Synthetic primer
<400> 314
   cgagagtctg taggagggaa acc 23
<210> 315
   <211> 18
   <212> DNA
   <213> Artificial Sequence
<220>
   <223> Synthetic primer
<400> 315
   tcatggtgcc cgtcaatg 18
<210> 316
   <211> 22
   <212> DNA
   <213> Artificial Sequence
<220>
   <223> Synthetic primer
<400> 316
   tcatcctggc gatctacttc ct 22
<210> 317
   <211> 20
   <212> DNA
   <213> Artificial Sequence
<220>
   <223> Synthetic primer
<400> 317
   tgagaaacaa actgcaccca 20
<210> 318
   <211> 20
   <212> DNA
   <213> Artificial Sequence
<220>
   <223> Synthetic primer
<400> 318
   gatgcagaat tgaggcagac 20
<210> 319
   <211> 20
   <212> DNA
   <213> Artificial Sequence
<220>
   <223> Synthetic primer
<400> 319
   gctcgtggtt ctgtagtcca 20
<210> 320
   <211> 23
   <212> DNA
   <213> Artificial Sequence
<220>
   <223> Synthetic primer
<400> 320
   gaaggaatgg gaatcagtca tga 23
<210> 321
   <211> 18
   <212> DNA
   <213> Artificial Sequence
<220>
   <223> Synthetic primer
<400> 321
   gccgagatcg ccaagatg 18
<210> 322
   <211> 20
   <212> DNA
   <213> Artificial Sequence
<220>
   <223> Synthetic primer
<400> 322
   tggttttgag accacgatgt 20
<210> 323
   <211> 25
   <212> DNA
   <213> Artificial Sequence
<220>
   <223> Synthetic primer
<400> 323
   taactgacat tcttgagcac cagat 25
<210> 324
   <211> 27
   <212> DNA
   <213> Artificial Sequence
<220>
   <223> Synthetic primer
<400> 324
   cgagactctc ctcatagtga aaggtat 27
<210> 325
   <211> 23
   <212> DNA
   <213> Artificial Sequence
<220>
   <223> Synthetic primer
<400> 325
   caaccgaagt tttcactcca gtt 23
<210> 326
   <211> 19
   <212> DNA
   <213> Artificial Sequence
<220>
   <223> Synthetic primer
<400> 326
   gcggaaggtc cctcagaca 19
<210> 327
   <211> 19
   <212> DNA
   <213> Artificial Sequence
<220>
   <223> Synthetic primer
<400> 327
   ccgcaacgtg gttttctca 19
<210> 328
   <211> 18
   <212> DNA
   <213> Artificial Sequence
<220>
   <223> Synthetic primer
<400> 328
   gtggttttcc ctcggagc 18
<210> 329
   <211> 20
   <212> DNA
   <213> Artificial Sequence
<220>
   <223> Synthetic primer
<400> 329
   gcatcaggct gtcattatgg 20
<210> 330
   <211> 19
   <212> DNA
   <213> Artificial Sequence
<220>
   <223> Synthetic primer
<400> 330
   aggactggga cccatgaac 19
<210> 331
   <211> 25
   <212> DNA
   <213> Artificial Sequence
<220>
   <223> Synthetic primer
<400> 331
   tggctaagtg aagatgacaa tcatg 25
<210> 332
   <211> 20
   <212> DNA
   <213> Artificial Sequence
<220>
   <223> Synthetic primer
<400> 332
   aatatttgtg cggggtatgg 20
<210> 333
   <211> 21
   <212> DNA
   <213> Artificial Sequence
<220>
   <223> Synthetic primer
<400> 333
   aagcatgaac aggacttgac c 21
<210> 334
   <211> 22
   <212> DNA
   <213> Artificial Sequence
<220>
   <223> Synthetic primer
<400> 334
   gcaaggaaag ggtcttagtc ac 22
<210> 335
   <211> 18
   <212> DNA
   <213> Artificial Sequence
<220>
   <223> Synthetic primer
<400> 335
   gaaacctctg cgccatga 18
<210> 336
   <211> 24
   <212> DNA
   <213> Artificial Sequence
<220>
   <223> Synthetic primer
<400> 336
   ccattctatc atcaacgggt acaa 24
<210> 337
   <211> 20
   <212> DNA
   <213> Artificial Sequence
<220>
   <223> Synthetic primer
<400> 337
   gttctggttg ctggatttgg 20
<210> 338
   <211> 19
   <212> DNA
   <213> Artificial Sequence
<220>
   <223> Synthetic primer
<400> 338
   tcaccacggt ctttagcca 19
<210> 339
   <211> 20
   <212> DNA
   <213> Artificial Sequence
<220>
   <223> Synthetic primer
<400> 339
   gggctactgg cagctacatt 20
<210> 340
   <211> 20
   <212> DNA
   <213> Artificial Sequence
<220>
   <223> Synthetic primer
<400> 340
   cagcgggatt aaacagtcct 20
<210> 341
   <211> 20
   <212> DNA
   <213> Artificial Sequence
<220>
   <223> Synthetic primer
<400> 341
   aacagagaca ttgccaacca 20
<210> 342
   <211> 20
   <212> DNA
   <213> Artificial Sequence
<220>
   <223> Synthetic primer
<400> 342
   acaccaaaat gccatctcaa 20
<210> 343
   <211> 21
   <212> DNA
   <213> Artificial Sequence
<220>
   <223> Synthetic primer
<400> 343
   tggctgtgct ggtcactacc t 21
<210> 344
   <211> 18
   <212> DNA
   <213> Artificial Sequence
<220>
   <223> Synthetic primer
<400> 344
   gactgctgtc atggcgtg 18
<210> 345
   <211> 21
   <212> DNA
   <213> Artificial Sequence
<220>
   <223> Synthetic primer
<400> 345
   cctgctgacg atgatgaagg a 21
<210> 346
   <211> 22
   <212> DNA
   <213> Artificial Sequence
<220>
   <223> Synthetic primer
<400> 346
   actccctgat aaaggggaat tt 22
<210> 347
   <211> 19
   <212> DNA
   <213> Artificial Sequence
<220>
   <223> Synthetic primer
<400> 347
   caccctgcct ctacccaac 19
<210> 348
   <211> 20
   <212> DNA
   <213> Artificial Sequence
<220>
   <223> Synthetic primer
<400> 348
   catggccgtg tagaccctaa 20
<210> 349
   <211> 19
   <212> DNA
   <213> Artificial Sequence
<220>
   <223> Synthetic primer
<400> 349
   accctgagca ctggaggaa 19
<210> 350
   <211> 21
   <212> DNA
   <213> Artificial Sequence
<220>
   <223> Synthetic primer
<400> 350
   agacaaggat gccgtggata a 21
<210> 351
   <211> 22
   <212> DNA
   <213> Artificial Sequence
<220>
   <223> Synthetic primer
<400> 351
   gcagaactga agatgggaag at 22
<210> 352
   <211> 20
   <212> DNA
   <213> Artificial Sequence
<220>
   <223> Synthetic primer
<400> 352
   cgcgagcccc tcattataca 20
<210> 353
   <211> 19
   <212> DNA
   <213> Artificial Sequence
<220>
   <223> Synthetic primer
<400> 353
   caagctgaac ggtgtgtcc 19
<210> 354
   <211> 20
   <212> DNA
   <213> Artificial Sequence
<220>
   <223> Synthetic primer
<400> 354
   agctggggtg tctgtttcat 20
<210> 355
   <211> 21
   <212> DNA
   <213> Artificial Sequence
<220>
   <223> Synthetic primer
<400> 355
   agaggctgaa tatgcaggac a 21
<210> 356
   <211> 23
   <212> DNA
   <213> Artificial Sequence
<220>
   <223> Synthetic primer
<400> 356
   agttgcagaa tctaagcctg gaa 23
<210> 357
   <211> 19
   <212> DNA
   <213> Artificial Sequence
<220>
   <223> Synthetic primer
<400> 357
   atggccaatg tttgatgct 19
<210> 358
   <211> 20
   <212> DNA
   <213> Artificial Sequence
<220>
   <223> Synthetic primer
<400> 358
   cccaatcgga agcctaacta 20
<210> 359
   <211> 20
   <212> DNA
   <213> Artificial Sequence
<220>
   <223> Synthetic primer
<400> 359
   catcttccag gaggaccact 20
<210> 360
   <211> 20
   <212> DNA
   <213> Artificial Sequence
<220>
   <223> Synthetic primer
<400> 360
   aatacccaac gcacaaatga 20
<210> 361
   <211> 20
   <212> DNA
   <213> Artificial Sequence
<220>
   <223> Synthetic primer
<400> 361
   cctggaggct gcaacatacc 20
<210> 362
   <211> 20
   <212> DNA
   <213> Artificial Sequence
<220>
   <223> Synthetic primer
<400> 362
   tgttttgatt cccgggctta 20
<210> 363
   <211> 21
   <212> DNA
   <213> Artificial Sequence
<220>
   <223> Synthetic primer
<400> 363
   tctccagcaa aagcgatgtc t 21
<210> 364
   <211> 20
   <212> DNA
   <213> Artificial Sequence
<220>
   <223> Synthetic primer
<400> 364
   gatggagcag gtggctcagt 20
<210> 365
   <211> 18
   <212> DNA
   <213> Artificial Sequence
<220>
   <223> Synthetic primer
<400> 365
   cagccctgag gcaagaga 18
<210> 366
   <211> 20
   <212> DNA
   <213> Artificial Sequence
<220>
   <223> Synthetic primer
<400> 366
   gccaactgct ttcatttgtg 20
<210> 367
   <211> 20
   <212> DNA
   <213> Artificial Sequence
<220>
   <223> Synthetic primer
<400> 367
   accagtcccc cagaagacta 20
<210> 368
   <211> 22
   <212> DNA
   <213> Artificial Sequence
<220>
   <223> Synthetic primer
<400> 368
   ggatcgagct cttccagatc ct 22
<210> 369
   <211> 20
   <212> DNA
   <213> Artificial Sequence
<220>
   <223> Synthetic primer
<400> 369
   aacaccaatg ggttccatct 20
<210> 370
   <211> 20
   <212> DNA
   <213> Artificial Sequence
<220>
   <223> Synthetic primer
<400> 370
   ctacctgcct tgctttgtga 20
<210> 371
   <211> 19
   <212> DNA
   <213> Artificial Sequence
<220>
   <223> Synthetic primer
<400> 371
   ccagcccaca gaccagtta 19
<210> 372
   <211> 18
   <212> DNA
   <213> Artificial Sequence
<220>
   <223> Synthetic primer
<400> 372
   tggcgaccaa gacacctt 18
<210> 373
   <211> 21
   <212> DNA
   <213> Artificial Sequence
<220>
   <223> Synthetic primer
<400> 373
   catatcgttg gatcacagca c 21
<210> 374
   <211> 18
   <212> DNA
   <213> Artificial Sequence
<220>
   <223> Synthetic primer
<400> 374
   gcgctgcgga agatcatc 18
<210> 375
   <211> 18
   <212> DNA
   <213> Artificial Sequence
<220>
   <223> Synthetic primer
<400> 375
   gagtcgaccc tgcacctg 18
<210> 376
   <211> 21
   <212> DNA
   <213> Artificial Sequence
<220>
   <223> Synthetic primer
<400> 376
   tggcttcagg agctgaatac c 21
<210> 377
   <211> 21
   <212> DNA
   <213> Artificial Sequence
<220>
   <223> Synthetic primer
<400> 377
   tgcccttaaa ggaaccaatg a 21
<210> 378
   <211> 19
   <212> DNA
   <213> Artificial Sequence
<220>
   <223> Synthetic primer
<400> 378
   agtcaatctt cgcacacgg 19
<210> 379
   <211> 18
   <212> DNA
   <213> Artificial Sequence
<220>
   <223> Synthetic primer
<400> 379
   ctctccagtg tgggcacc 18
<210> 380
   <211> 22
   <212> DNA
   <213> Artificial Sequence
<220>
   <223> Synthetic primer
<400> 380
   agaggcatcc atgaacttca ca 22
<210> 381
   <211> 25
   <212> DNA
   <213> Artificial Sequence
<220>
   <223> Synthetic primer
<400> 381
   gtatcaggac cacatgcagt acatc 25
<210> 382
   <211> 20
   <212> DNA
   <213> Artificial Sequence
<220>
   <223> Synthetic primer
<400> 382
   tgtcttcagg gtcttgtcca 20
<210> 383
   <211> 20
   <212> DNA
   <213> Artificial Sequence
<220>
   <223> Synthetic primer
<400> 383
   atcgcagctg gtgggtgtac 20
<210> 384
   <211> 20
   <212> DNA
   <213> Artificial Sequence
<220>
   <223> Synthetic primer
<400> 384
   gtggacatcg gatacccaag 20
<210> 385
   <211> 22
   <212> DNA
   <213> Artificial Sequence
<220>
   <223> Synthetic reverse primer
<400> 385
   aggtccctgt tggccttata gg 22
<210> 386
   <211> 20
   <212> DNA
   <213> Artificial Sequence
<220>
   <223> Synthetic reverse primer
<400> 386
   ctccatccac tccaggtctc 20
<210> 387
   <211> 20
   <212> DNA
   <213> Artificial Sequence
<220>
   <223> Synthetic reverse primer
<400> 387
   caagcctgga acctatagcc 20
<210> 388
   <211> 18
   <212> DNA
   <213> Artificial Sequence
<220>
   <223> Synthetic reverse primer
<400> 388
   atagcgctga ccactgcc 18
<210> 389
   <211> 20
   <212> DNA
   <213> Artificial Sequence
<220>
   <223> Synthetic reverse primer
<400> 389
   ggccagcacc ataatcctat 20
<210> 390
   <211> 20
   <212> DNA
   <213> Artificial Sequence
<220>
   <223> Synthetic reverse primer
<400> 390
   actagggtgc tccgagtgac 20
<210> 391
   <211> 20
   <212> DNA
   <213> Artificial Sequence
<220>
   <223> Synthetic reverse primer
<400> 391
   atccgctaga actgcaccac 20
<210> 392
   <211> 20
   <212> DNA
   <213> Artificial Sequence
<220>
   <223> Synthetic reverse primer
<400> 392
   tgggcttaga tgcttgactc 20
<210> 393
   <211> 20
   <212> DNA
   <213> Artificial Sequence
<220>
   <223> Synthetic reverse primer
<400> 393
   tgcaaatgct ttgatggaat 20
<210> 394
   <211> 19
   <212> DNA
   <213> Artificial Sequence
<220>
   <223> Synthetic reverse primer
<400> 394
   ctggtcacgg tctccatgt 19
<210> 395
   <211> 21
   <212> DNA
   <213> Artificial Sequence
<220>
   <223> Synthetic reverse primer
<400> 395
   cgggcactca ctgctattac c 21
<210> 396
   <211> 21
   <212> DNA
   <213> Artificial Sequence
<220>
   <223> Synthetic reverse primer
<400> 396
   acccagaatc caacagtgca a 21
<210> 397
   <211> 20
   <212> DNA
   <213> Artificial Sequence
<220>
   <223> Synthetic reverse primer
<400> 397
   cacagatggc cagtgtttct 20
<210> 398
   <211> 18
   <212> DNA
   <213> Artificial Sequence
<220>
   <223> Synthetic reverse primer
<400> 398
   tgggcgccta aatcctaa 18
<210> 399
   <211> 19
   <212> DNA
   <213> Artificial Sequence
<220>
   <223> Synthetic reverse primer
<400> 399
   gggcacaaat cccgttcag 19
<210> 400
   <211> 20
   <212> DNA
   <213> Artificial Sequence
<220>
   <223> Synthetic reverse primer
<400> 400
   tctgatctcc atctgcctca 20
<210> 401
   <211> 22
   <212> DNA
   <213> Artificial Sequence
<220>
   <223> Synthetic reverse primer
<400> 401
   tgtggacctg atccctgtac ac 22
<210> 402
   <211> 20
   <212> DNA
   <213> Artificial Sequence
<220>
   <223> Synthetic reverse primer
<400> 402
   ccagagggtt gaaggcatag 20
<210> 403
   <211> 24
   <212> DNA
   <213> Artificial Sequence
<220>
   <223> Synthetic reverse primer
<400> 403
   ccagcattag attctccaac ttga 24
<210> 404
   <211> 18
   <212> DNA
   <213> Artificial Sequence
<220>
   <223> Synthetic reverse primer
<400> 404
   gtggcggaga tcaagagg 18
<210> 405
   <211> 20
   <212> DNA
   <213> Artificial Sequence
<220>
   <223> Synthetic reverse primer
<400> 405
   aaccggaaga agtcgatgag 20
<210> 406
   <211> 20
   <212> DNA
   <213> Artificial Sequence
<220>
   <223> Synthetic reverse primer
<400> 406
   ttgcagtggg aagaacagtc 20
<210> 407
   <211> 19
   <212> DNA
   <213> Artificial Sequence
<220>
   <223> Synthetic reverse primer
<400> 407
   cgtgtcgggc ttcagtcat 19
<210> 408
   <211> 20
   <212> DNA
   <213> Artificial Sequence
<220>
   <223> Synthetic reverse primer
<400> 408
   tagccataag gtccgctctc 20
<210> 409
   <211> 20
   <212> DNA
   <213> Artificial Sequence
<220>
   <223> Synthetic reverse primer
<400> 409
   aggtctccac acagcacaag 20
<210> 410
   <211> 22
   <212> DNA
   <213> Artificial Sequence
<220>
   <223> Synthetic reverse primer
<400> 410
   ggaatacacg agggcatagt tc 22
<210> 411
   <211> 20
   <212> DNA
   <213> Artificial Sequence
<220>
   <223> Synthetic reverse primer
<400> 411
   acaagcacat ggctatggaa 20
<210> 412
   <211> 20
   <212> DNA
   <213> Artificial Sequence
<220>
   <223> Synthetic reverse primer
<400> 412
   acttgtgcag cagcgtactt 20
<210> 413
   <211> 19
   <212> DNA
   <213> Artificial Sequence
<220>
   <223> Synthetic reverse primer
<400> 413
   ggagtgacgc atggacaga 19
<210> 414
   <211> 20
   <212> DNA
   <213> Artificial Sequence
<220>
   <223> Synthetic reverse primer
<400> 414
   tggtgccatt ttcctatgag 20
<210> 415
   <211> 20
   <212> DNA
   <213> Artificial Sequence
<220>
   <223> Synthetic reverse primer
<400> 415
   gcatctgcca actcctccat 20
<210> 416
   <211> 18
   <212> DNA
   <213> Artificial Sequence
<220>
   <223> Synthetic reverse primer
<400> 416
   gcatttgcgg tggacgat 18
<210> 417
   <211> 23
   <212> DNA
   <213> Artificial Sequence
<220>
   <223> Synthetic reverse primer
<400> 417
   tcagatgacg aagagcacag atg 23
<210> 418
   <211> 21
   <212> DNA
   <213> Artificial Sequence
<220>
   <223> Synthetic reverse primer
<400> 418
   ctgctcactc ggctcaaact c 21
<210> 419
   <211> 24
   <212> DNA
   <213> Artificial Sequence
<220>
   <223> Synthetic reverse primer
<400> 419
   gttcaacctc ttcctgtgga ctgt 24
<210> 420
   <211> 20
   <212> DNA
   <213> Artificial Sequence
<220>
   <223> Synthetic reverse primer
<400> 420
   gggcgaagag gatataaggg 20
<210> 421
   <211> 23
   <212> DNA
   <213> Artificial Sequence
<220>
   <223> Synthetic reverse primer
<400> 421
   cgaaggcact actcaatggt ttc 23
<210> 422
   <211> 21
   <212> DNA
   <213> Artificial Sequence
<220>
   <223> Synthetic reverse primer
<400> 422
   ttgccgtcag aaaacatgtc a 21
<210> 423
   <211> 19
   <212> DNA
   <213> Artificial Sequence
<220>
   <223> Synthetic reverse primer
<400> 423
   ctaattgggc tccatctcg 19
<210> 424
   <211> 20
   <212> DNA
   <213> Artificial Sequence
<220>
   <223> Synthetic reverse primer
<400> 424
   tcctgggagg tgaacttagg 20
<210> 425
   <211> 21
   <212> DNA
   <213> Artificial Sequence
<220>
   <223> Synthetic reverse primer
<400> 425
   tgagcgaggt tcttccactg a 21
<210> 426
   <211> 19
   <212> DNA
   <213> Artificial Sequence
<220>
   <223> Synthetic reverse primer
<400> 426
   ctcgggtttg gcctctttc 19
<210> 427
   <211> 24
   <212> DNA
   <213> Artificial Sequence
<220>
   <223> Synthetic reverse primer
<400> 427
   cctatgattt aagggcattt ttcc 24
<210> 428
   <211> 18
   <212> DNA
   <213> Artificial Sequence
<220>
   <223> Synthetic reverse primer
<400> 428
   ctcagctgac gggaaagg 18
<210> 429
   <211> 20
   <212> DNA
   <213> Artificial Sequence
<220>
   <223> Synthetic reverse primer
<400> 429
   cttgttgttc accaggacga 20
<210> 430
   <211> 21
   <212> DNA
   <213> Artificial Sequence
<220>
   <223> Synthetic reverse primer
<400> 430
   ggcaggagtg aatggctctt c 21
<210> 431
   <211> 20
   <212> DNA
   <213> Artificial Sequence
<220>
   <223> Synthetic reverse primer
<400> 431
   ggtatcttgt ggtgtctgcg 20
<210> 432
   <211> 20
   <212> DNA
   <213> Artificial Sequence
<220>
   <223> Synthetic reverse primer
<400> 432
   gtggccaaga ggtcagagtc 20
<210> 433
   <211> 20
   <212> DNA
   <213> Artificial Sequence
<220>
   <223> Synthetic reverse primer
<400> 433
   tgaagcagtc agttgtgctg 20
<210> 434
   <211> 21
   <212> DNA
   <213> Artificial Sequence
<220>
   <223> Synthetic reverse primer
<400> 434
   aagacatggc gctctcagtt c 21
<210> 435
   <211> 22
   <212> DNA
   <213> Artificial Sequence
<220>
   <223> Synthetic reverse primer
<400> 435
   caacagagtt tgccgagaca ct 22
<210> 436
   <211> 22
   <212> DNA
   <213> Artificial Sequence
<220>
   <223> Synthetic reverse primer
<400> 436
   gctgattccc aagagtctaa cc 22
<210> 437
   <211> 18
   <212> DNA
   <213> Artificial Sequence
<220>
   <223> Synthetic reverse primer
<400> 437
   ggcactcggc ttgagcat 18
<210> 438
   <211> 20
   <212> DNA
   <213> Artificial Sequence
<220>
   <223> Synthetic reverse primer
<400> 438
   tgagaccgtt ggattggatt 20
<210> 439
   <211> 19
   <212> DNA
   <213> Artificial Sequence
<220>
   <223> Synthetic reverse primer
<400> 439
   aggaccaaag ggagaccaa 19
<210> 440
   <211> 24
   <212> DNA
   <213> Artificial Sequence
<220>
   <223> Synthetic reverse primer
<400> 440
   acggtcctag gtttgaggtt aaga 24
<210> 441
   <211> 20
   <212> DNA
   <213> Artificial Sequence
<220>
   <223> Synthetic reverse primer
<400> 441
   gcgacagagg gcttcatctt 20
<210> 442
   <211> 18
   <212> DNA
   <213> Artificial Sequence
<220>
   <223> Synthetic reverse primer
<400> 442
   tgcccacggc tttcttac 18
<210> 443
   <211> 20
   <212> DNA
   <213> Artificial Sequence
<220>
   <223> Synthetic reverse primer
<400> 443
   cacgatgtct tcctccttga 20
<210> 444
   <211> 23
   <212> DNA
   <213> Artificial Sequence
<220>
   <223> Synthetic reverse primer
<400> 444
   tccggtttaa gaccagttta cca 23
<210> 445
   <211> 20
   <212> DNA
   <213> Artificial Sequence
<220>
   <223> Synthetic reverse primer
<400> 445
   caatggcctc cattttacag 20
<210> 446
   <211> 20
   <212> DNA
   <213> Artificial Sequence
<220>
   <223> Synthetic reverse primer
<400> 446
   aaaggggtgg gtagaaagga 20
<210> 447
   <211> 20
   <212> DNA
   <213> Artificial Sequence
<220>
   <223> Synthetic reverse primer
<400> 447
   cctctgcacg gtcataggtt 20
<210> 448
   <211> 20
   <212> DNA
   <213> Artificial Sequence
<220>
   <223> Synthetic reverse primer
<400> 448
   ctgcatgatt ctgagcaggt 20
<210> 449
   <211> 20
   <212> DNA
   <213> Artificial Sequence
<220>
   <223> Synthetic reverse primer
<400> 449
   atgctgactt ccttcctggt 20
<210> 450
   <211> 20
   <212> DNA
   <213> Artificial Sequence
<220>
   <223> Synthetic reverse primer
<400> 450
   catcttcttg ggcacacaat 20
<210> 451
   <211> 18
   <212> DNA
   <213> Artificial Sequence
<220>
   <223> Synthetic reverse primer
<400> 451
   cactgcagcc ccaatgct 18
<210> 452
   <211> 25
   <212> DNA
   <213> Artificial Sequence
<220>
   <223> Synthetic reverse primer
<400> 452
   ttcaatgata atgcaaggac tgatc 25
<210> 453
   <211> 20
   <212> DNA
   <213> Artificial Sequence
<220>
   <223> Synthetic reverse primer
<400> 453
   ctggtttgtc tggagaaggc 20
<210> 454
   <211> 20
   <212> DNA
   <213> Artificial Sequence
<220>
   <223> Synthetic reverse primer
<400> 454
   cctgacattt cccttgtcct 20
<210> 455
   <211> 20
   <212> DNA
   <213> Artificial Sequence
<220>
   <223> Synthetic reverse primer
<400> 455
   tcatgggcgt atctacgaat 20
<210> 456
   <211> 25
   <212> DNA
   <213> Artificial Sequence
<220>
   <223> Synthetic reverse primer
<400> 456
   gagagagtga gaccacgaag agact 25
<210> 457
   <211> 18
   <212> DNA
   <213> Artificial Sequence
<220>
   <223> Synthetic reverse primer
<400> 457
   tccctgcatt caagaggc 18
<210> 458
   <211> 20
   <212> DNA
   <213> Artificial Sequence
<220>
   <223> Synthetic reverse primer
<400> 458
   acctgtgttt ggatttgcag 20
<210> 459
   <211> 20
   <212> DNA
   <213> Artificial Sequence
<220>
   <223> Synthetic reverse primer
<400> 459
   actggggtgg aatgtgtctt 20
<210> 460
   <211> 21
   <212> DNA
   <213> Artificial Sequence
<220>
   <223> Synthetic reverse primer
<400> 460
   ttgggttgaa gaaatcagtc c 21
<210> 461
   <211> 19
   <212> DNA
   <213> Artificial Sequence
<220>
   <223> Synthetic reverse primer
<400> 461
   ctcctccacc ctgggttgt 19
<210> 462
   <211> 20
   <212> DNA
   <213> Artificial Sequence
<220>
   <223> Synthetic reverse primer
<400> 462
   gacctcaggg cgattcatga 20
<210> 463
   <211> 22
   <212> DNA
   <213> Artificial Sequence
<220>
   <223> Synthetic reverse primer
<400> 463
   gcttgcactc cacaggtaca ca 22
<210> 464
   <211> 21
   <212> DNA
   <213> Artificial Sequence
<220>
   <223> Synthetic reverse primer
<400> 464
   ctgcattgtg gcacagttct g 21
<210> 465
   <211> 20
   <212> DNA
   <213> Artificial Sequence
<220>
   <223> Synthetic reverse primer
<400> 465
   cttcagtctt ggcctgttca 20
<210> 466
   <211> 22
   <212> DNA
   <213> Artificial Sequence
<220>
   <223> Synthetic reverse primer
<400> 466
   ggatcccaca cctttaccat aa 22
<210> 467
   <211> 18
   <212> DNA
   <213> Artificial Sequence
<220>
   <223> Synthetic reverse primer
<400> 467
   gccgctcatt gatctcca 18
<210> 468
   <211> 18
   <212> DNA
   <213> Artificial Sequence
<220>
   <223> Synthetic reverse primer
<400> 468
   ccgtcattgg ccttcttc 18
<210> 469
   <211> 18
   <212> DNA
   <213> Artificial Sequence
<220>
   <223> Synthetic reverse primer
<400> 469
   ctactcatgg gcgggatg 18
<210> 470
   <211> 19
   <212> DNA
   <213> Artificial Sequence
<220>
   <223> Synthetic reverse primer
<400> 470
   ccgccttcag gttctcaat 19
<210> 471
   <211> 18
   <212> DNA
   <213> Artificial Sequence
<220>
   <223> Synthetic reverse primer
<400> 471
   ttgggatgct caaaagcc 18
<210> 472
   <211> 19
   <212> DNA
   <213> Artificial Sequence
<220>
   <223> Synthetic reverse primer
<400> 472
   ctgtggcatt gagtttggg 19
<210> 473
   <211> 20
   <212> DNA
   <213> Artificial Sequence
<220>
   <223> Synthetic reverse primer
<400> 473
   atgtcaggag tccctccatc 20
<210> 474
   <211> 22
   <212> DNA
   <213> Artificial Sequence
<220>
   <223> Synthetic reverse primer
<400> 474
   tggcaaatcc gaattagagt ga 22
<210> 475
   <211> 18
   <212> DNA
   <213> Artificial Sequence
<220>
   <223> Synthetic reverse primer
<400> 475
   gtctcagacc cttccccc 18
<210> 476
   <211> 23
   <212> DNA
   <213> Artificial Sequence
<220>
   <223> Synthetic reverse primer
<400> 476
   tgtgactaca gccgtgatcc tta 23
<210> 477
   <211> 20
   <212> DNA
   <213> Artificial Sequence
<220>
   <223> Synthetic reverse primer
<400> 477
   gcctcttgta gggccaatag 20
<210> 478
   <211> 20
   <212> DNA
   <213> Artificial Sequence
<220>
   <223> Synthetic reverse primer
<400> 478
   caaaaccgct gtgtttcttc 20
<210> 479
   <211> 21
   <212> DNA
   <213> Artificial Sequence
<220>
   <223> Synthetic reverse primer
<400> 479
   gcccatgcac tgaagtattg g 21
<210> 480
   <211> 20
   <212> DNA
   <213> Artificial Sequence
<220>
   <223> Synthetic reverse primer
<400> 480
   agtcgtcgag tgctagggac 20
<210> 481
   <211> 18
   <212> DNA
   <213> Artificial Sequence
<220>
   <223> Synthetic reverse primer
<400> 481
   tccgaggcca cagcaaac 18
<210> 482
   <211> 20
   <212> DNA
   <213> Artificial Sequence
<220>
   <223> Synthetic reverse primer
<400> 482
   gacgcagtct ttctgtctgg 20
<210> 483
   <211> 18
   <212> DNA
   <213> Artificial Sequence
<220>
   <223> Synthetic reverse primer
<400> 483
   tgcagagcag cactggag 18
<210> 484
   <211> 20
   <212> DNA
   <213> Artificial Sequence
<220>
   <223> Synthetic reverse primer
<400> 484
   cgctgcagaa aatgaaacga 20
<210> 485
   <211> 19
   <212> DNA
   <213> Artificial Sequence
<220>
   <223> Synthetic reverse primer
<400> 485
   cagagcgggc agcagaata 19
<210> 486
   <211> 20
   <212> DNA
   <213> Artificial Sequence
<220>
   <223> Synthetic reverse primer
<400> 486
   tcgatctcct catcatctgg 20
<210> 487
   <211> 18
   <212> DNA
   <213> Artificial Sequence
<220>
   <223> Synthetic reverse primer
<400> 487
   tgcgggactt gggaaaga 18
<210> 488
   <211> 20
   <212> DNA
   <213> Artificial Sequence
<220>
   <223> Synthetic reverse primer
<400> 488
   tgttggtacc cctgttgttg 20
<210> 489
   <211> 23
   <212> DNA
   <213> Artificial Sequence
<220>
   <223> Synthetic reverse primer
<400> 489
   cagtggtagg tgatgttctg gga 23
<210> 490
   <211> 19
   <212> DNA
   <213> Artificial Sequence
<220>
   <223> Synthetic reverse primer
<400> 490
   aaactggctg ccagcattg 19
<210> 491
   <211> 21
   <212> DNA
   <213> Artificial Sequence
<220>
   <223> Synthetic reverse primer
<400> 491
   ctccttggtg tcacccatga g 21
<210> 492
   <211> 22
   <212> DNA
   <213> Artificial Sequence
<220>
   <223> Synthetic reverse primer
<400> 492
   ggctgctaga gaccatggac at 22
<210> 493
   <211> 20
   <212> DNA
   <213> Artificial Sequence
<220>
   <223> Synthetic reverse primer
<400> 493
   ctccccatta caagtgctga 20
<210> 494
   <211> 21
   <212> DNA
   <213> Artificial Sequence
<220>
   <223> Synthetic reverse primer
<400> 494
   gaactccctg gagatgaaac c 21
<210> 495
   <211> 20
   <212> DNA
   <213> Artificial Sequence
<220>
   <223> Synthetic reverse primer
<400> 495
   cacatgcatg gaccttgatt 20
<210> 496
   <211> 18
   <212> DNA
   <213> Artificial Sequence
<220>
   <223> Synthetic reverse primer
<400> 496
   atccctcgga ctgcctct 18
<210> 497
   <211> 25
   <212> DNA
   <213> Artificial Sequence
<220>
   <223> Synthetic reverse primer
<400> 497
   caactgttcc tggtctacaa actca 25
<210> 498
   <211> 20
   <212> DNA
   <213> Artificial Sequence
<220>
   <223> Synthetic reverse primer
<400> 498
   cctgcagaga tgggtatgaa 20
<210> 499
   <211> 20
   <212> DNA
   <213> Artificial Sequence
<220>
   <223> Synthetic reverse primer
<400> 499
   ctagaggctg gtgccactgt 20
<210> 500
   <211> 19
   <212> DNA
   <213> Artificial Sequence
<220>
   <223> Synthetic reverse primer
<400> 500
   catcacgtct ccgaactcc 19
<210> 501
   <211> 20
   <212> DNA
   <213> Artificial Sequence
<220>
   <223> Synthetic reverse primer
<400> 501
   agttgtaatg gcaggcacag 20
<210> 502
   <211> 21
   <212> DNA
   <213> Artificial Sequence
<220>
   <223> Synthetic reverse primer
<400> 502
   tcagctccat tgaatgtgaa a 21
<210> 503
   <211> 21
   <212> DNA
   <213> Artificial Sequence
<220>
   <223> Synthetic reverse primer
<400> 503
   gggacagctt gtagcctttg c 21
<210> 504
   <211> 19
   <212> DNA
   <213> Artificial Sequence
<220>
   <223> Synthetic reverse primer
<400> 504
   accattgcag ccctgattg 19
<210> 505
   <211> 20
   <212> DNA
   <213> Artificial Sequence
<220>
   <223> Synthetic reverse primer
<400> 505
   ctgttctcca agccaagaca 20
<210> 506
   <211> 20
   <212> DNA
   <213> Artificial Sequence
<220>
   <223> Synthetic reverse primer
<400> 506
   tagggaagtg atgggagagg 20
<210> 507
   <211> 20
   <212> DNA
   <213> Artificial Sequence
<220>
   <223> Synthetic reverse primer
<400> 507
   tttgagatgc ttgacgttgg 20
<210> 508
   <211> 19
   <212> DNA
   <213> Artificial Sequence
<220>
   <223> Synthetic reverse primer
<400> 508
   caatgcggca tatactggg 19
<210> 509
   <211> 22
   <212> DNA
   <213> Artificial Sequence
<220>
   <223> Synthetic reverse primer
<400> 509
   aggataaggc caaccatgat gt 22
<210> 510
   <211> 19
   <212> DNA
   <213> Artificial Sequence
<220>
   <223> Synthetic reverse primer
<400> 510
   gccagcattg ccattatct 19
<210> 511
   <211> 21
   <212> DNA
   <213> Artificial Sequence
<220>
   <223> Synthetic reverse primer
<400> 511
   caccatggcg atgtactttc c 21
<210> 512
   <211> 20
   <212> DNA
   <213> Artificial Sequence
<220>
   <223> Synthetic reverse primer
<400> 512
   gggaatgtgg tagcccaaga 20
<210> 513
   <211> 20
   <212> DNA
   <213> Artificial Sequence
<220>
   <223> Synthetic reverse primer
<400> 513
   gtggctgcat tagtgtccat 20
<210> 514
   <211> 20
   <212> DNA
   <213> Artificial Sequence
<220>
   <223> Synthetic reverse primer
<400> 514
   accaaagatg ctgtgttcca 20
<210> 515
   <211> 20
   <212> DNA
   <213> Artificial Sequence
<220>
   <223> Synthetic reverse primer
<400> 515
   tgaatgccat ctttcttcca 20
<210> 516
   <211> 18
   <212> DNA
   <213> Artificial Sequence
<220>
   <223> Synthetic reverse primer
<400> 516
   gagcgtcggg tgcaaatc 18
<210> 517
   <211> 22
   <212> DNA
   <213> Artificial Sequence
<220>
   <223> Synthetic reverse primer
<400> 517
   caccacccca agtatccgta ag 22
<210> 518
   <211> 20
   <212> DNA
   <213> Artificial Sequence
<220>
   <223> Synthetic reverse primer
<400> 518
   cgatgttccc ttcgatggag 20
<210> 519
   <211> 20
   <212> DNA
   <213> Artificial Sequence
<220>
   <223> Synthetic reverse primer
<400> 519
   ggcagtgaag gcgataaagt 20
<210> 520
   <211> 20
   <212> DNA
   <213> Artificial Sequence
<220>
   <223> Synthetic reverse primer
<400> 520
   cacctcttgc tgtccctttg 20
<210> 521
   <211> 18
   <212> DNA
   <213> Artificial Sequence
<220>
   <223> Synthetic reverse primer
<400> 521
   agaaggaagg tccagccg 18
<210> 522
   <211> 19
   <212> DNA
   <213> Artificial Sequence
<220>
   <223> Synthetic reverse primer
<400> 522
   ccatgaggcc caacttcct 19
<210> 523
   <211> 19
   <212> DNA
   <213> Artificial Sequence
<220>
   <223> Synthetic reverse primer
<400> 523
   cctgccgcat tgttttcag 19
<210> 524
   <211> 20
   <212> DNA
   <213> Artificial Sequence
<220>
   <223> Synthetic reverse primer
<400> 524
   aatgcgtatc tgtccacgac 20
<210> 525
   <211> 20
   <212> DNA
   <213> Artificial Sequence
<220>
   <223> Synthetic reverse primer
<400> 525
   gacaaacacc cttcctccag 20
<210> 526
   <211> 18
   <212> DNA
   <213> Artificial Sequence
<220>
   <223> Synthetic reverse primer
<400> 526
   ctcgtcccgg ttcatcag 18
<210> 527
   <211> 20
   <212> DNA
   <213> Artificial Sequence
<220>
   <223> Synthetic reverse primer
<400> 527
   caggcctcag ttccttcagt 20
<210> 528
   <211> 20
   <212> DNA
   <213> Artificial Sequence
<220>
   <223> Synthetic reverse primer
<400> 528
   ccaacagtac agccagttgc 20
<210> 529
   <211> 21
   <212> DNA
   <213> Artificial Sequence
<220>
   <223> Synthetic reverse primer
<400> 529
   tggacctagg gcttccaagt c 21
<210> 530
   <211> 20
   <212> DNA
   <213> Artificial Sequence
<220>
   <223> Synthetic reverse primer
<400> 530
   caggccgtaa ggagctgtct 20
<210> 531
   <211> 20
   <212> DNA
   <213> Artificial Sequence
<220>
   <223> Synthetic reverse primer
<400> 531
   ttacacatcc aaccagtgcc 20
<210> 532
   <211> 19
   <212> DNA
   <213> Artificial Sequence
<220>
   <223> Synthetic reverse primer
<400> 532
   accacagcat gggtgagag 19
<210> 533
   <211> 20
   <212> DNA
   <213> Artificial Sequence
<220>
   <223> Synthetic reverse primer
<400> 533
   ccgttgtaac gttgactgga 20
<210> 534
   <211> 18
   <212> DNA
   <213> Artificial Sequence
<220>
   <223> Synthetic reverse primer
<400> 534
   ggcgctgact tccttgac 18
<210> 535
   <211> 20
   <212> DNA
   <213> Artificial Sequence
<220>
   <223> Synthetic reverse primer
<400> 535
   tgttgagatt cctcgcagtt 20
<210> 536
   <211> 24
   <212> DNA
   <213> Artificial Sequence
<220>
   <223> Synthetic reverse primer
<400> 536
   ctccagctta gggtagttgt ccat 24
<210> 537
   <211> 20
   <212> DNA
   <213> Artificial Sequence
<220>
   <223> Synthetic reverse primer
<400> 537
   tgcctgagaa gaggtgaggt 20
<210> 538
   <211> 20
   <212> DNA
   <213> Artificial Sequence
<220>
   <223> Synthetic reverse primer
<400> 538
   ttggtagtgc tccacacgat 20
<210> 539
   <211> 20
   <212> DNA
   <213> Artificial Sequence
<220>
   <223> Synthetic reverse primer
<400> 539
   gatgaggatg tcccggatga 20
<210> 540
   <211> 20
   <212> DNA
   <213> Artificial Sequence
<220>
   <223> Synthetic reverse primer
<400> 540
   gaacagctgg aggccaagtc 20
<210> 541
   <211> 18
   <212> DNA
   <213> Artificial Sequence
<220>
   <223> Synthetic reverse primer
<400> 541
   cggtcacgga gccaatct 18
<210> 542
   <211> 20
   <212> DNA
   <213> Artificial Sequence
<220>
   <223> Synthetic reverse primer
<400> 542
   tgttcaaagg ttgaccatgc 20
<210> 543
   <211> 21
   <212> DNA
   <213> Artificial Sequence
<220>
   <223> Synthetic reverse primer
<400> 543
   tgcactgctt ggccttaaag a 21
<210> 544
   <211> 18
   <212> DNA
   <213> Artificial Sequence
<220>
   <223> Synthetic reverse primer
<400> 544
   gtggcgtgcc tcgaagtc 18
<210> 545
   <211> 19
   <212> DNA
   <213> Artificial Sequence
<220>
   <223> Synthetic reverse primer
<400> 545
   gtaatgctgt ccacggtgc 19
<210> 546
   <211> 20
   <212> DNA
   <213> Artificial Sequence
<220>
   <223> Synthetic reverse primer
<400> 546
   aggtacctct cggtcagtgg 20
<210> 547
   <211> 23
   <212> DNA
   <213> Artificial Sequence
<220>
   <223> Synthetic reverse primer
<400> 547
   tgttctagcg atcttgcttc aca 23
<210> 548
   <211> 25
   <212> DNA
   <213> Artificial Sequence
<220>
   <223> Synthetic reverse primer
<400> 548
   caaggcatat cgatcctcat aaagt 25
<210> 549
   <211> 20
   <212> DNA
   <213> Artificial Sequence
<220>
   <223> Synthetic reverse primer
<400> 549
   cggccaggat acacatctta 20
<210> 550
   <211> 19
   <212> DNA
   <213> Artificial Sequence
<220>
   <223> Synthetic reverse primer
<400> 550
   cctccgccag gtctttagt 19
<210> 551
   <211> 19
   <212> DNA
   <213> Artificial Sequence
<220>
   <223> Synthetic reverse primer
<400> 551
   tctccgagga accctttgg 19
<210> 552
   <211> 21
   <212> DNA
   <213> Artificial Sequence
<220>
   <223> Synthetic reverse primer
<400> 552
   ctgtccaatt gctgattgct t 21
<210> 553
   <211> 23
   <212> DNA
   <213> Artificial Sequence
<220>
   <223> Synthetic reverse primer
<400> 553
   tgtagggcag acttcctcaa aca 23
<210> 554
   <211> 20
   <212> DNA
   <213> Artificial Sequence
<220>
   <223> Synthetic reverse primer
<400> 554
   agtctcttgg gcatcgagtt 20
<210> 555
   <211> 19
   <212> DNA
   <213> Artificial Sequence
<220>
   <223> Synthetic reverse primer
<400> 555
   ggctagtggg cgcatgtag 19
<210> 556
   <211> 19
   <212> DNA
   <213> Artificial Sequence
<220>
   <223> Synthetic reverse primer
<400> 556
   tgaccaggaa ctgccacag 19
<210> 557
   <211> 20
   <212> DNA
   <213> Artificial Sequence
<220>
   <223> Synthetic reverse primer
<400> 557
   caccgaacac tccctagtcc 20
<210> 558
   <211> 20
   <212> DNA
   <213> Artificial Sequence
<220>
   <223> Synthetic reverse primer
<400> 558
   aaccggtgct ctccacattc 20
<210> 559
   <211> 18
   <212> DNA
   <213> Artificial Sequence
<220>
   <223> Synthetic reverse primer
<400> 559
   ggaagtgggt catgtggg 18
<210> 560
   <211> 18
   <212> DNA
   <213> Artificial Sequence
<220>
   <223> Synthetic reverse primer
<400> 560
   gctttgcccg gtagctct 18
<210> 561
   <211> 25
   <212> DNA
   <213> Artificial Sequence
<220>
   <223> Synthetic reverse primer
<400> 561
   gagtgagaat tcgatccaag tcttc 25
<210> 562
   <211> 20
   <212> DNA
   <213> Artificial Sequence
<220>
   <223> Synthetic reverse primer
<400> 562
   acgagactcc agtgctgatg 20
<210> 563
   <211> 22
   <212> DNA
   <213> Artificial Sequence
<220>
   <223> Synthetic reverse primer
<400> 563
   ctctctgggt cgtctgaaac aa 22
<210> 564
   <211> 18
   <212> DNA
   <213> Artificial Sequence
<220>
   <223> Synthetic reverse primer
<400> 564
   caaactggtc ccggtcct 18
<210> 565
   <211> 18
   <212> DNA
   <213> Artificial Sequence
<220>
   <223> Synthetic reverse primer
<400> 565
   acacggtagc cggtcact 18
<210> 566
   <211> 18
   <212> DNA
   <213> Artificial Sequence
<220>
   <223> Synthetic reverse primer
<400> 566
   ggagcgggct gtctcaga 18
<210> 567
   <211> 20
   <212> DNA
   <213> Artificial Sequence
<220>
   <223> Synthetic reverse primer
<400> 567
   cttgacgaag cactcgttga 20
<210> 568
   <211> 20
   <212> DNA
   <213> Artificial Sequence
<220>
   <223> Synthetic reverse primer
<400> 568
   acggcttgct tactgaaggt 20
<210> 569
   <211> 19
   <212> DNA
   <213> Artificial Sequence
<220>
   <223> Synthetic reverse primer
<400> 569
   ggtcgtccat tggaatcct 19
<210> 570
   <211> 20
   <212> DNA
   <213> Artificial Sequence
<220>
   <223> Synthetic reverse primer
<400> 570
   gtggaggaac tctgggaatg 20
<210> 571
   <211> 21
   <212> DNA
   <213> Artificial Sequence
<220>
   <223> Synthetic reverse primer
<400> 571
   ggtcacaaac ttgccattgg a 21
<210> 572
   <211> 25
   <212> DNA
   <213> Artificial Sequence
<220>
   <223> Synthetic reverse primer
<400> 572
   tgaagtaatc agccacagac tcaat 25
<210> 573
   <211> 20
   <212> DNA
   <213> Artificial Sequence
<220>
   <223> Synthetic reverse primer
<400> 573
   ctcctcagac accactgcat 20
<210> 574
   <211> 23
   <212> DNA
   <213> Artificial Sequence
<220>
   <223> Synthetic reverse primer
<400> 574
   ggtggttttc ttgagcgtgt act 23
<210> 575
   <211> 22
   <212> DNA
   <213> Artificial Sequence
<220>
   <223> Synthetic reverse primer
<400> 575
   gaagatagct gagggctgtg ac 22
<210> 576
   <211> 20
   <212> DNA
   <213> Artificial Sequence
<220>
   <223> Synthetic reverse primer
<400> 576
   cccggcaaaa acaaataagt 20
<210> 577
   <211> 20
   <212> DNA
   <213> Artificial Sequence
<220>
   <223> Synthetic reverse primer
<400> 577
   tgggagttca tgggtacaga 20
<210> 578
   <211> 22
   <212> DNA
   <213> Artificial Sequence
<220>
   <223> Synthetic reverse primer
<400> 578
   gatgggattt ccattgatga ca 22
<210> 579
   <211> 26
   <212> DNA
   <213> Artificial Sequence
<220>
   <223> Synthetic reverse primer
<400> 579
   gagtcagaat ggcttattca cagatg 26
<210> 580
   <211> 20
   <212> DNA
   <213> Artificial Sequence
<220>
   <223> Synthetic reverse primer
<400> 580
   agaagctagg gtggttgtcc 20
<210> 581
   <211> 21
   <212> DNA
   <213> Artificial Sequence
<220>
   <223> Synthetic reverse primer
<400> 581
   tgaggagttt gccttgattc g 21
<210> 582
   <211> 22
   <212> DNA
   <213> Artificial Sequence
<220>
   <223> Synthetic reverse primer
<400> 582
   cacagaatcc agctgtgcaa ct 22
<210> 583
   <211> 20
   <212> DNA
   <213> Artificial Sequence
<220>
   <223> Synthetic reverse primer
<400> 583
   acagtggaag gaccaggact 20
<210> 584
   <211> 20
   <212> DNA
   <213> Artificial Sequence
<220>
   <223> Synthetic reverse primer
<400> 584
   agccattgca gctaggtgag 20
<210> 585
   <211> 23
   <212> DNA
   <213> Artificial Sequence
<220>
   <223> Synthetic reverse primer
<400> 585
   aaataccaac atgcacctct ctt 23
<210> 586
   <211> 20
   <212> DNA
   <213> Artificial Sequence
<220>
   <223> Synthetic reverse primer
<400> 586
   agtccacagt gttgggacaa 20
<210> 587
   <211> 18
   <212> DNA
   <213> Artificial Sequence
<220>
   <223> Synthetic reverse primer
<400> 587
   tgcgtaccca cttcctgc 18
<210> 588
   <211> 20
   <212> DNA
   <213> Artificial Sequence
<220>
   <223> Synthetic reverse primer
<400> 588
   aaagagctgt gagtggctgg 20
<210> 589
   <211> 20
   <212> DNA
   <213> Artificial Sequence
<220>
   <223> Synthetic reverse primer
<400> 589
   atgttcacca ccaggatcag 20
<210> 590
   <211> 19
   <212> DNA
   <213> Artificial Sequence
<220>
   <223> Synthetic reverse primer
<400> 590
   gcagctcagg gaagtcaca 19
<210> 591
   <211> 20
   <212> DNA
   <213> Artificial Sequence
<220>
   <223> Synthetic reverse primer
<400> 591
   aaagttccag gcaacatcgt 20
<210> 592
   <211> 21
   <212> DNA
   <213> Artificial Sequence
<220>
   <223> Synthetic reverse primer
<400> 592
   ggtccagcag tgtctcctga a 21
<210> 593
   <211> 19
   <212> DNA
   <213> Artificial Sequence
<220>
   <223> Synthetic reverse primer
<400> 593
   tactccctgg ctcctgctt 19
<210> 594
   <211> 20
   <212> DNA
   <213> Artificial Sequence
<220>
   <223> Synthetic reverse primer
<400> 594
   ggccaccagg gtattatctg 20
<210> 595
   <211> 20
   <212> DNA
   <213> Artificial Sequence
<220>
   <223> Synthetic reverse primer
<400> 595
   gaagcctttc tttccacagc 20
<210> 596
   <211> 21
   <212> DNA
   <213> Artificial Sequence
<220>
   <223> Synthetic reverse primer
<400> 596
   caagagcctg aatgcgtcag t 21
<210> 597
   <211> 23
   <212> DNA
   <213> Artificial Sequence
<220>
   <223> Synthetic reverse primer
<400> 597
   acttcggctg tgtgttatat gca 23
<210> 598
   <211> 21
   <212> DNA
   <213> Artificial Sequence
<220>
   <223> Synthetic reverse primer
<400> 598
   gaggtccgtg gtagcgttct c 21
<210> 599
   <211> 20
   <212> DNA
   <213> Artificial Sequence
<220>
   <223> Synthetic reverse primer
<400> 599
   ggcccagctt gaatttttca 20
<210> 600
   <211> 21
   <212> DNA
   <213> Artificial Sequence
<220>
   <223> Synthetic reverse primer
<400> 600
   gcgaatctgc tccttttctg a 21
<210> 601
   <211> 20
   <212> DNA
   <213> Artificial Sequence
<220>
   <223> Synthetic reverse primer
<400> 601
   ccaagaaacc atggctgctt 20
<210> 602
   <211> 25
   <212> DNA
   <213> Artificial Sequence
<220>
   <223> Synthetic reverse primer
<400> 602
   gtccactcga atcttttctt cttca 25
<210> 603
   <211> 21
   <212> DNA
   <213> Artificial Sequence
<220>
   <223> Synthetic reverse primer
<400> 603
   ggcctcagtg tgcatcattc t 21
<210> 604
   <211> 20
   <212> DNA
   <213> Artificial Sequence
<220>
   <223> Synthetic reverse primer
<400> 604
   cacgcaggtg gtatcagtct 20
<210> 605
   <211> 20
   <212> DNA
   <213> Artificial Sequence
<220>
   <223> Synthetic reverse primer
<400> 605
   tcttaagcac gttctccacg 20
<210> 606
   <211> 22
   <212> DNA
   <213> Artificial Sequence
<220>
   <223> Synthetic reverse primer
<400> 606
   gcttgctgta ctccgacatg tt 22
<210> 607
   <211> 21
   <212> DNA
   <213> Artificial Sequence
<220>
   <223> Synthetic reverse primer
<400> 607
   ctccacggtc tcagttgatc t 21
<210> 608
   <211> 19
   <212> DNA
   <213> Artificial Sequence
<220>
   <223> Synthetic reverse primer
<400> 608
   cctctcgcaa gtgctccat 19
<210> 609
   <211> 21
   <212> DNA
   <213> Artificial Sequence
<220>
   <223> Synthetic reverse primer
<400> 609
   ggaggtgctt cactgtcatt t 21
<210> 610
   <211> 18
   <212> DNA
   <213> Artificial Sequence
<220>
   <223> Synthetic reverse primer
<400> 610
   gcagggagct ggagtagc 18
<210> 611
   <211> 21
   <212> DNA
   <213> Artificial Sequence
<220>
   <223> Synthetic reverse primer
<400> 611
   tgagcagcac catcagtaac g 21
<210> 612
   <211> 22
   <212> DNA
   <213> Artificial Sequence
<220>
   <223> Synthetic reverse primer
<400> 612
   gcttgtcatc tgcagcagtg tt 22
<210> 613
   <211> 22
   <212> DNA
   <213> Artificial Sequence
<220>
   <223> Synthetic reverse primer
<400> 613
   gcgtgtctgc gtagtagctg tt 22
<210> 614
   <211> 28
   <212> DNA
   <213> Artificial Sequence
<220>
   <223> Synthetic reverse primer
<400> 614
   gtttgccaag ttaaatttgg tacataat 28
<210> 615
   <211> 21
   <212> DNA
   <213> Artificial Sequence
<220>
   <223> Synthetic reverse primer
<400> 615
   gggagggaga agagattcga t 21
<210> 616
   <211> 20
   <212> DNA
   <213> Artificial Sequence
<220>
   <223> Synthetic reverse primer
<400> 616
   ccgagtcgcc actgctaagt 20
<210> 617
   <211> 24
   <212> DNA
   <213> Artificial Sequence
<220>
   <223> Synthetic reverse primer
<400> 617
   ggcaataaac aggctcatga ttaa 24
<210> 618
   <211> 18
   <212> DNA
   <213> Artificial Sequence
<220>
   <223> Synthetic reverse primer
<400> 618
   cacagggttt cagcgagc 18
<210> 619
   <211> 18
   <212> DNA
   <213> Artificial Sequence
<220>
   <223> Synthetic reverse primer
<400> 619
   gttggaagca aacgcaca 18
<210> 620
   <211> 18
   <212> DNA
   <213> Artificial Sequence
<220>
   <223> Synthetic reverse primer
<400> 620
   cgcctcgcga aagacttg 18
<210> 621
   <211> 20
   <212> DNA
   <213> Artificial Sequence
<220>
   <223> Synthetic reverse primer
<400> 621
   tgcctgcgat atttgttagg 20
<210> 622
   <211> 24
   <212> DNA
   <213> Artificial Sequence
<220>
   <223> Synthetic reverse primer
<400> 622
   ataaacgctt caaatttctc tctg 24
<210> 623
   <211> 18
   <212> DNA
   <213> Artificial Sequence
<220>
   <223> Synthetic reverse primer
<400> 623
   caggttcgct ctgggaag 18
<210> 624
   <211> 19
   <212> DNA
   <213> Artificial Sequence
<220>
   <223> Synthetic reverse primer
<400> 624
   gcacaggttc gctctggaa 19
<210> 625
   <211> 20
   <212> DNA
   <213> Artificial Sequence
<220>
   <223> Synthetic reverse primer
<400> 625
   atctgtttcc attggctcct 20
<210> 626
   <211> 20
   <212> DNA
   <213> Artificial Sequence
<220>
   <223> Synthetic reverse primer
<400> 626
   ggtctgccca aatgcttttc 20
<210> 627
   <211> 26
   <212> DNA
   <213> Artificial Sequence
<220>
   <223> Synthetic reverse primer
<400> 627
   gctacatcta cacttggttg gcttaa 26
<210> 628
   <211> 19
   <212> DNA
   <213> Artificial Sequence
<220>
   <223> Synthetic reverse primer
<400> 628
   atgctggctg actctgctc 19
<210> 629
   <211> 24
   <212> DNA
   <213> Artificial Sequence
<220>
   <223> Synthetic reverse primer
<400> 629
   ggattgcagc taaccctgta tacc 24
<210> 630
   <211> 22
   <212> DNA
   <213> Artificial Sequence
<220>
   <223> Synthetic reverse primer
<400> 630
   cttctgagac ctctggcttc gt 22
<210> 631
   <211> 21
   <212> DNA
   <213> Artificial Sequence
<220>
   <223> Synthetic reverse primer
<400> 631
   tccaactgaa ggtccctgat g 21
<210> 632
   <211> 19
   <212> DNA
   <213> Artificial Sequence
<220>
   <223> Synthetic reverse primer
<400> 632
   tgcaatcatg caagaccac 19
<210> 633
   <211> 21
   <212> DNA
   <213> Artificial Sequence
<220>
   <223> Synthetic reverse primer
<400> 633
   gctcgttcag cttcacattg c 21
<210> 634
   <211> 30
   <212> DNA
   <213> Artificial Sequence
<220>
   <223> Synthetic reverse primer
<400> 634
   tttccggtaa tagtctgtct catagatatc 30
<210> 635
   <211> 20
   <212> DNA
   <213> Artificial Sequence
<220>
   <223> Synthetic reverse primer
<400> 635
   tggactgctt ccaggtgtca 20
<210> 636
   <211> 20
   <212> DNA
   <213> Artificial Sequence
<220>
   <223> Synthetic reverse primer
<400> 636
   gtcttggaca cccgcagaat 20
<210> 637
   <211> 19
   <212> DNA
   <213> Artificial Sequence
<220>
   <223> Synthetic reverse primer
<400> 637
   gggtctgaat ggccaggtt 19
<210> 638
   <211> 20
   <212> DNA
   <213> Artificial Sequence
<220>
   <223> Synthetic reverse primer
<400> 638
   cagagctctt gcatgtggag 20
<210> 639
   <211> 20
   <212> DNA
   <213> Artificial Sequence
<220>
   <223> Synthetic reverse primer
<400> 639
   atcaggaagg ctgccaagag 20
<210> 640
   <211> 25
   <212> DNA
   <213> Artificial Sequence
<220>
   <223> Synthetic reverse primer
<400> 640
   tggagcttat taaaggcatt cttca 25
<210> 641
   <211> 20
   <212> DNA
   <213> Artificial Sequence
<220>
   <223> Synthetic reverse primer
<400> 641
   tcttgacctt gcagctttgt 20
<210> 642
   <211> 20
   <212> DNA
   <213> Artificial Sequence
<220>
   <223> Synthetic reverse primer
<400> 642
   ggccccaatg aaatagactg 20
<210> 643
   <211> 20
   <212> DNA
   <213> Artificial Sequence
<220>
   <223> Synthetic reverse primer
<400> 643
   aaaattgtgc cttggaggag 20
<210> 644
   <211> 19
   <212> DNA
   <213> Artificial Sequence
<220>
   <223> Synthetic reverse primer
<400> 644
   aactccgagt ggtgatcca 19
<210> 645
   <211> 23
   <212> DNA
   <213> Artificial Sequence
<220>
   <223> Synthetic reverse primer
<400> 645
   cggtagtggt tgatgactgt tga 23
<210> 646
   <211> 20
   <212> DNA
   <213> Artificial Sequence
<220>
   <223> Synthetic reverse primer
<400> 646
   agaaggtatc agggctggaa 20
<210> 647
   <211> 20
   <212> DNA
   <213> Artificial Sequence
<220>
   <223> Synthetic reverse primer
<400> 647
   cctcagtgcc agtctcttcc 20
<210> 648
   <211> 20
   <212> DNA
   <213> Artificial Sequence
<220>
   <223> Synthetic reverse primer
<400> 648
   gaagctttgt agccggtgat 20
<210> 649
   <211> 18
   <212> DNA
   <213> Artificial Sequence
<220>
   <223> Synthetic reverse primer
<400> 649
   gtctggccgg gattcttt 18
<210> 650
   <211> 20
   <212> DNA
   <213> Artificial Sequence
<220>
   <223> Synthetic reverse primer
<400> 650
   gtcttctcca cagtccagca 20
<210> 651
   <211> 19
   <212> DNA
   <213> Artificial Sequence
<220>
   <223> Synthetic reverse primer
<400> 651
   gtttagcctc atgggcgtc 19
<210> 652
   <211> 20
   <212> DNA
   <213> Artificial Sequence
<220>
   <223> Synthetic reverse primer
<400> 652
   tgccatcacc attgaaatct 20
<210> 653
   <211> 20
   <212> DNA
   <213> Artificial Sequence
<220>
   <223> Synthetic reverse primer
<400> 653
   cctgagctta gctggtgttg 20
<210> 654
   <211> 27
   <212> DNA
   <213> Artificial Sequence
<220>
   <223> Synthetic reverse primer
<400> 654
   ccttaagctc tttcactgac tcaatct 27
<210> 655
   <211> 19
   <212> DNA
   <213> Artificial Sequence
<220>
   <223> Synthetic reverse primer
<400> 655
   gcgcacacct tcatctcat 19
<210> 656
   <211> 20
   <212> DNA
   <213> Artificial Sequence
<220>
   <223> Synthetic reverse primer
<400> 656
   ggtgaacatc atgacgcagt 20
<210> 657
   <211> 19
   <212> DNA
   <213> Artificial Sequence
<220>
   <223> Synthetic reverse primer
<400> 657
   gcatagctgt gagatgcgg 19
<210> 658
   <211> 18
   <212> DNA
   <213> Artificial Sequence
<220>
   <223> Synthetic reverse primer
<400> 658
   agggggtgtc cgtaaagg 18
<210> 659
   <211> 24
   <212> DNA
   <213> Artificial Sequence
<220>
   <223> Synthetic reverse primer
<400> 659
   ttacaactct tccactggga cgat 24
<210> 660
   <211> 20
   <212> DNA
   <213> Artificial Sequence
<220>
   <223> Synthetic reverse primer
<400> 660
   tgtctgtgag cttggtcctg 20
<210> 661
   <211> 20
   <212> DNA
   <213> Artificial Sequence
<220>
   <223> Synthetic reverse primer
<400> 661
   gaagcaggtc agagtgagcc 20
<210> 662
   <211> 22
   <212> DNA
   <213> Artificial Sequence
<220>
   <223> Synthetic reverse primer
<400> 662
   tcacacccac tgaatcctac tg 22
<210> 663
   <211> 19
   <212> DNA
   <213> Artificial Sequence
<220>
   <223> Synthetic reverse primer
<400> 663
   ccgtagggcc aattcagac 19
<210> 664
   <211> 20
   <212> DNA
   <213> Artificial Sequence
<220>
   <223> Synthetic reverse primer
<400> 664
   cgtgatgcga agctctgaga 20
<210> 665
   <211> 22
   <212> DNA
   <213> Artificial Sequence
<220>
   <223> Synthetic reverse primer
<400> 665
   catcttcacc agcatgatgt ca 22
<210> 666
   <211> 20
   <212> DNA
   <213> Artificial Sequence
<220>
   <223> Synthetic reverse primer
<400> 666
   tcctcactca tcacgtcctc 20
<210> 667
   <211> 20
   <212> DNA
   <213> Artificial Sequence
<220>
   <223> Synthetic reverse primer
<400> 667
   gccaaaccat tcattgtcac 20
<210> 668
   <211> 25
   <212> DNA
   <213> Artificial Sequence
<220>
   <223> Synthetic reverse primer
<400> 668
   gcaatctctt caaacacttc atcct 25
<210> 669
   <211> 20
   <212> DNA
   <213> Artificial Sequence
<220>
   <223> Synthetic reverse primer
<400> 669
   tgagcccctg gttaacagta 20
<210> 670
   <211> 19
   <212> DNA
   <213> Artificial Sequence
<220>
   <223> Synthetic reverse primer
<400> 670
   aagcttcaca agttggggc 19
<210> 671
   <211> 18
   <212> DNA
   <213> Artificial Sequence
<220>
   <223> Synthetic reverse primer
<400> 671
   tgattgtccg cagtcagg 18
<210> 672
   <211> 20
   <212> DNA
   <213> Artificial Sequence
<220>
   <223> Synthetic reverse primer
<400> 672
   ttgaaatggc agaacggtag 20
<210> 673
   <211> 18
   <212> DNA
   <213> Artificial Sequence
<220>
   <223> Synthetic reverse primer
<400> 673
   acacaaggcc cagcctct 18
<210> 674
   <211> 20
   <212> DNA
   <213> Artificial Sequence
<220>
   <223> Synthetic reverse primer
<400> 674
   cggcagaact gacagtgttc 20
<210> 675
   <211> 20
   <212> DNA
   <213> Artificial Sequence
<220>
   <223> Synthetic reverse primer
<400> 675
   gtcacacttg cagcatttca 20
<210> 676
   <211> 21
   <212> DNA
   <213> Artificial Sequence
<220>
   <223> Synthetic reverse primer
<400> 676
   actccctgaa gccgagacac t 21
<210> 677
   <211> 18
   <212> DNA
   <213> Artificial Sequence
<220>
   <223> Synthetic reverse primer
<400> 677
   gacatggcag cacaagca 18
<210> 678
   <211> 20
   <212> DNA
   <213> Artificial Sequence
<220>
   <223> Synthetic reverse primer
<400> 678
   cttgagggtt tgggtttcca 20
<210> 679
   <211> 20
   <212> DNA
   <213> Artificial Sequence
<220>
   <223> Synthetic reverse primer
<400> 679
   aagagctgcc catccttctc 20
<210> 680
   <211> 19
   <212> DNA
   <213> Artificial Sequence
<220>
   <223> Synthetic reverse primer
<400> 680
   ttctgggaac tgctggaag 19
<210> 681
   <211> 21
   <212> DNA
   <213> Artificial Sequence
<220>
   <223> Synthetic reverse primer
<400> 681
   ccccacgagt tctggttctt c 21
<210> 682
   <211> 19
   <212> DNA
   <213> Artificial Sequence
<220>
   <223> Synthetic reverse primer
<400> 682
   cgctgaggct ggtactgtg 19
<210> 683
   <211> 19
   <212> DNA
   <213> Artificial Sequence
<220>
   <223> Synthetic reverse primer
<400> 683
   gtctctggac acaggctgg 19
<210> 684
   <211> 20
   <212> DNA
   <213> Artificial Sequence
<220>
   <223> Synthetic reverse primer
<400> 684
   gaggaatgga aagacctcgg 20
<210> 685
   <211> 21
   <212> DNA
   <213> Artificial Sequence
<220>
   <223> Synthetic reverse primer
<400> 685
   gcagacacaa tggaaagaac c 21
<210> 686
   <211> 19
   <212> DNA
   <213> Artificial Sequence
<220>
   <223> Synthetic reverse primer
<400> 686
   cggacagttt cttccggtt 19
<210> 687
   <211> 20
   <212> DNA
   <213> Artificial Sequence
<220>
   <223> Synthetic reverse primer
<400> 687
   agccgtacca gctcagactt 20
<210> 688
   <211> 26
   <212> DNA
   <213> Artificial Sequence
<220>
   <223> Synthetic reverse primer
<400> 688
   gccactaact gcttcagtat gaagag 26
<210> 689
   <211> 20
   <212> DNA
   <213> Artificial Sequence
<220>
   <223> Synthetic reverse primer
<400> 689
   tgcacataag caacagcaga 20
<210> 690
   <211> 20
   <212> DNA
   <213> Artificial Sequence
<220>
   <223> Synthetic reverse primer
<400> 690
   gaccctgctc acaaccagac 20
<210> 691
   <211> 26
   <212> DNA
   <213> Artificial Sequence
<220>
   <223> Synthetic reverse primer
<400> 691
   tccatatcca acaaaaaaac tcaaag 26
<210> 692
   <211> 19
   <212> DNA
   <213> Artificial Sequence
<220>
   <223> Synthetic reverse primer
<400> 692
   gggcctggtt gaaaagcat 19
<210> 693
   <211> 24
   <212> DNA
   <213> Artificial Sequence
<220>
   <223> Synthetic reverse primer
<400> 693
   acggtagtga cagcatcaaa actc 24
<210> 694
   <211> 21
   <212> DNA
   <213> Artificial Sequence
<220>
   <223> Synthetic reverse primer
<400> 694
   ggagtccgtc cttaccgtca a 21
<210> 695
   <211> 22
   <212> DNA
   <213> Artificial Sequence
<220>
   <223> Synthetic reverse primer
<400> 695
   ggaatgtccc atacccaaag aa 22
<210> 696
   <211> 23
   <212> DNA
   <213> Artificial Sequence
<220>
   <223> Synthetic reverse primer
<400> 696
   tgtcaccgtg atctctttgg taa 23
<210> 697
   <211> 21
   <212> DNA
   <213> Artificial Sequence
<220>
   <223> Synthetic reverse primer
<400> 697
   cctaaaggtt tgaatggcag a 21
<210> 698
   <211> 24
   <212> DNA
   <213> Artificial Sequence
<220>
   <223> Synthetic reverse primer
<400> 698
   ggttccgata tttggtggtc ttac 24
<210> 699
   <211> 23
   <212> DNA
   <213> Artificial Sequence
<220>
   <223> Synthetic reverse primer
<400> 699
   cgattgtctt tgctcttcat gtg 23
<210> 700
   <211> 20
   <212> DNA
   <213> Artificial Sequence
<220>
   <223> Synthetic reverse primer
<400> 700
   ccgttgagtg gaatcagcaa 20
<210> 701
   <211> 20
   <212> DNA
   <213> Artificial Sequence
<220>
   <223> Synthetic reverse primer
<400> 701
   caaggcctca aatctcaagg 20
<210> 702
   <211> 20
   <212> DNA
   <213> Artificial Sequence
<220>
   <223> Synthetic reverse primer
<400> 702
   tcttggcaag tcggttaaga 20
<210> 703
   <211> 20
   <212> DNA
   <213> Artificial Sequence
<220>
   <223> Synthetic reverse primer
<400> 703
   acaaagggag agcgtgaagt 20
<210> 704
   <211> 26
   <212> DNA
   <213> Artificial Sequence
<220>
   <223> Synthetic reverse primer
<400> 704
   gtctattaga gtcagatccg ggacat 26
<210> 705
   <211> 27
   <212> DNA
   <213> Artificial Sequence
<220>
   <223> Synthetic reverse primer
<400> 705
   cttttgatgg tagagttcca gtgattc 27
<210> 706
   <211> 20
   <212> DNA
   <213> Artificial Sequence
<220>
   <223> Synthetic reverse primer
<400> 706
   tgaatcatgc cagtgctgta 20
<210> 707
   <211> 18
   <212> DNA
   <213> Artificial Sequence
<220>
   <223> Synthetic reverse primer
<400> 707
   atggcttgcc cacaatgc 18
<210> 708
   <211> 22
   <212> DNA
   <213> Artificial Sequence
<220>
   <223> Synthetic reverse primer
<400> 708
   cttggcgtgt ggaaatctac ag 22
<210> 709
   <211> 25
   <212> DNA
   <213> Artificial Sequence
<220>
   <223> Synthetic reverse primer
<400> 709
   cctcagtcca taaaccacac tatca 25
<210> 710
   <211> 24
   <212> DNA
   <213> Artificial Sequence
<220>
   <223> Synthetic reverse primer
<400> 710
   tgatgatcta agtttcccga ggtt 24
<210> 711
   <211> 21
   <212> DNA
   <213> Artificial Sequence
<220>
   <223> Synthetic reverse primer
<400> 711
   tgctgggttt ctcctcctgt t 21
<210> 712
   <211> 19
   <212> DNA
   <213> Artificial Sequence
<220>
   <223> Synthetic reverse primer
<400> 712
   agcaagggaa cagcctcat 19
<210> 713
   <211> 20
   <212> DNA
   <213> Artificial Sequence
<220>
   <223> Synthetic reverse primer
<400> 713
   agtagttgtg ctgcccttcc 20
<210> 714
   <211> 20
   <212> DNA
   <213> Artificial Sequence
<220>
   <223> Synthetic reverse primer
<400> 714
   cccataatcc tgagcaatgg 20
<210> 715
   <211> 25
   <212> DNA
   <213> Artificial Sequence
<220>
   <223> Synthetic reverse primer
<400> 715
   tgcacatatc attacaccag ttcgt 25
<210> 716
   <211> 20
   <212> DNA
   <213> Artificial Sequence
<220>
   <223> Synthetic reverse primer
<400> 716
   aacgagatcc ctgtgcttgt 20
<210> 717
   <211> 18
   <212> DNA
   <213> Artificial Sequence
<220>
   <223> Synthetic reverse primer
<400> 717
   cctccccaag tcagttgc 18
<210> 718
   <211> 19
   <212> DNA
   <213> Artificial Sequence
<220>
   <223> Synthetic reverse primer
<400> 718
   acacctgcac aattctccg 19
<210> 719
   <211> 18
   <212> DNA
   <213> Artificial Sequence
<220>
   <223> Synthetic reverse primer
<400> 719
   ttcttttgcg cttcagcc 18
<210> 720
   <211> 23
   <212> DNA
   <213> Artificial Sequence
<220>
   <223> Synthetic reverse primer
<400> 720
   tcagcaagtg ggaaggtgta atc 23
<210> 721
   <211> 19
   <212> DNA
   <213> Artificial Sequence
<220>
   <223> Synthetic reverse primer
<400> 721
   ccttaaagcg gactccagg 19
<210> 722
   <211> 18
   <212> DNA
   <213> Artificial Sequence
<220>
   <223> Synthetic reverse primer
<400> 722
   tcctcctgta ggctggca 18
<210> 723
   <211> 20
   <212> DNA
   <213> Artificial Sequence
<220>
   <223> Synthetic reverse primer
<400> 723
   ctctcagcat cggtacaagg 20
<210> 724
   <211> 20
   <212> DNA
   <213> Artificial Sequence
<220>
   <223> Synthetic reverse primer
<400> 724
   atctgcgttg aagcagtgag 20
<210> 725
   <211> 21
   <212> DNA
   <213> Artificial Sequence
<220>
   <223> Synthetic reverse primer
<400> 725
   gtgatttgcc caggaaagtt t 21
<210> 726
   <211> 20
   <212> DNA
   <213> Artificial Sequence
<220>
   <223> Synthetic reverse primer
<400> 726
   tttatcccca gcgaatttgt 20
<210> 727
   <211> 22
   <212> DNA
   <213> Artificial Sequence
<220>
   <223> Synthetic reverse primer
<400> 727
   tcccccttac tcagcttgaa ct 22
<210> 728
   <211> 22
   <212> DNA
   <213> Artificial Sequence
<220>
   <223> Synthetic reverse primer
<400> 728
   cgagtacttg tggaaggtgg ac 22
<210> 729
   <211> 21
   <212> DNA
   <213> Artificial Sequence
<220>
   <223> Synthetic reverse primer
<400> 729
   gcgaggtaat ttgtgccctt t 21
<210> 730
   <211> 21
   <212> DNA
   <213> Artificial Sequence
<220>
   <223> Synthetic reverse primer
<400> 730
   tgaggacact cggtctctag c 21
<210> 731
   <211> 18
   <212> DNA
   <213> Artificial Sequence
<220>
   <223> Synthetic reverse primer
<400> 731
   ctagccccac agccaaga 18
<210> 732
   <211> 18
   <212> DNA
   <213> Artificial Sequence
<220>
   <223> Synthetic reverse primer
<400> 732
   agttttaagg gtgccccg 18
<210> 733
   <211> 19
   <212> DNA
   <213> Artificial Sequence
<220>
   <223> Synthetic reverse primer
<400> 733
   gagactttgg gggattcca 19
<210> 734
   <211> 20
   <212> DNA
   <213> Artificial Sequence
<220>
   <223> Synthetic reverse primer
<400> 734
   gaagtccacc tgggcatctc 20
<210> 735
   <211> 19
   <212> DNA
   <213> Artificial Sequence
<220>
   <223> Synthetic reverse primer
<400> 735
   ccctttccaa acttgaggc 19
<210> 736
   <211> 19
   <212> DNA
   <213> Artificial Sequence
<220>
   <223> Synthetic reverse primer
<400> 736
   cactcgccgt tgacatcct 19
<210> 737
   <211> 19
   <212> DNA
   <213> Artificial Sequence
<220>
   <223> Synthetic reverse primer
<400> 737
   tgcaagctgt ctttgagcc 19
<210> 738
   <211> 20
   <212> DNA
   <213> Artificial Sequence
<220>
   <223> Synthetic reverse primer
<400> 738
   acagcaaggc gagcataaat 20
<210> 739
   <211> 18
   <212> DNA
   <213> Artificial Sequence
<220>
   <223> Synthetic reverse primer
<400> 739
   ctatcggcct cagcatgg 18
<210> 740
   <211> 25
   <212> DNA
   <213> Artificial Sequence
<220>
   <223> Synthetic reverse primer
<400> 740
   tgagtttttt gcgagagtat tgaca 25
<210> 741
   <211> 20
   <212> DNA
   <213> Artificial Sequence
<220>
   <223> Synthetic reverse primer
<400> 741
   acacttcaag tcacgcttgc 20
<210> 742
   <211> 20
   <212> DNA
   <213> Artificial Sequence
<220>
   <223> Synthetic reverse primer
<400> 742
   gtagggctgc tggaaggtaa 20
<210> 743
   <211> 20
   <212> DNA
   <213> Artificial Sequence
<220>
   <223> Synthetic reverse primer
<400> 743
   tccgaccttc aatcatttca 20
<210> 744
   <211> 20
   <212> DNA
   <213> Artificial Sequence
<220>
   <223> Synthetic reverse primer
<400> 744
   ggagacaatg caaaccacac 20
<210> 745
   <211> 23
   <212> DNA
   <213> Artificial Sequence
<220>
   <223> Synthetic reverse primer
<400> 745
   tacaatggct ttggaggata gca 23
<210> 746
   <211> 24
   <212> DNA
   <213> Artificial Sequence
<220>
   <223> Synthetic reverse primer
<400> 746
   caaagctgtc agctctagca aaag 24
<210> 747
   <211> 22
   <212> DNA
   <213> Artificial Sequence
<220>
   <223> Synthetic reverse primer
<400> 747
   ttcatccctc gatatggctt ct 22
<210> 748
   <211> 25
   <212> DNA
   <213> Artificial Sequence
<220>
   <223> Synthetic reverse primer
<400> 748
   agtctggaac atgtcagtct tgatg 25
<210> 749
   <211> 21
   <212> DNA
   <213> Artificial Sequence
<220>
   <223> Synthetic reverse primer
<400> 749
   cgagcatttg tctcatcctt t 21
<210> 750
   <211> 20
   <212> DNA
   <213> Artificial Sequence
<220>
   <223> Synthetic reverse primer
<400> 750
   actcaggccc atttccttta 20
<210> 751
   <211> 20
   <212> DNA
   <213> Artificial Sequence
<220>
   <223> Synthetic reverse primer
<400> 751
   cctggtgctg ttgtagatgg 20
<210> 752
   <211> 20
   <212> DNA
   <213> Artificial Sequence
<220>
   <223> Synthetic reverse primer
<400> 752
   gccaccgata tagcgctgtt 20
<210> 753
   <211> 20
   <212> DNA
   <213> Artificial Sequence
<220>
   <223> Synthetic reverse primer
<400> 753
   cctcttcatc aggccaaact 20
<210> 754
   <211> 20
   <212> DNA
   <213> Artificial Sequence
<220>
   <223> Synthetic reverse primer
<400> 754
   accgaaattg gagagcatgt 20
<210> 755
   <211> 21
   <212> DNA
   <213> Artificial Sequence
<220>
   <223> Synthetic reverse primer
<400> 755
   ttcagagaaa ggaggtgtgg a 21
<210> 756
   <211> 22
   <212> DNA
   <213> Artificial Sequence
<220>
   <223> Synthetic reverse primer
<400> 756
   gggaaagtgg tacgtctttg ag 22
<210> 757
   <211> 20
   <212> DNA
   <213> Artificial Sequence
<220>
   <223> Synthetic reverse primer
<400> 757
   ttggccagat ctaaccatga 20
<210> 758
   <211> 22
   <212> DNA
   <213> Artificial Sequence
<220>
   <223> Synthetic reverse primer
<400> 758
   gcttgagggt ctgaatcttg ct 22
<210> 759
   <211> 18
   <212> DNA
   <213> Artificial Sequence
<220>
   <223> Synthetic reverse primer
<400> 759
   gcgaatgcca tgactgaa 18
<210> 760
   <211> 24
   <212> DNA
   <213> Artificial Sequence
<220>
   <223> Synthetic reverse primer
<400> 760
   tgctgtcgtg atgagaaaat agtg 24
<210> 761
   <211> 22
   <212> DNA
   <213> Artificial Sequence
<220>
   <223> Synthetic reverse primer
<400> 761
   gcttcaacgg caaagttctc tt 22
<210> 762
   <211> 19
   <212> DNA
   <213> Artificial Sequence
<220>
   <223> Synthetic reverse primer
<400> 762
   gtactgagcg atggagcgt 19
<210> 763
   <211> 19
   <212> DNA
   <213> Artificial Sequence
<220>
   <223> Synthetic reverse primer
<400> 763
   gcggtgtagc tcccagagt 19
<210> 764
   <211> 24
   <212> DNA
   <213> Artificial Sequence
<220>
   <223> Synthetic reverse primer
<400> 764
   caaactccac agtacttggg ttga 24
<210> 765
   <211> 22
   <212> DNA
   <213> Artificial Sequence
<220>
   <223> Synthetic reverse primer
<400> 765
   tgtcggaatt gatactggca tt 22
<210> 766
   <211> 20
   <212> DNA
   <213> Artificial Sequence
<220>
   <223> Synthetic reverse primer
<400> 766
   gtgcacgtgg atgaaagagt 20
<210> 767
   <211> 21
   <212> DNA
   <213> Artificial Sequence
<220>
   <223> Synthetic reverse primer
<400> 767
   agctccctgt tgcatggact t 21
<210> 768
   <211> 20
   <212> DNA
   <213> Artificial Sequence
<220>
   <223> Synthetic reverse primer
<400> 768
   gcagacaaaa gttggaaggc 20
<210> 769
   <211> 25
   <212> DNA
   <213> Artificial Sequence
<220>
   <223> Synthetic probe
<400> 769
   atgcctacag caccctgatg tcgca 25
<210> 770
   <211> 24
   <212> DNA
   <213> Artificial Sequence
<220>
   <223> Synthetic probe
<400> 770
   cgcttcaaag gaccagacct cctc 24
<210> 771
   <211> 24
   <212> DNA
   <213> Artificial Sequence
<220>
   <223> Synthetic probe
<400> 771
   ctcgccaatg atgctgctca agtt 24
<210> 772
   <211> 24
   <212> DNA
   <213> Artificial Sequence
<220>
   <223> Synthetic probe
<400> 772
   ccatgagctg tagccgaatg tcca 24
<210> 773
   <211> 22
   <212> DNA
   <213> Artificial Sequence
<220>
   <223> Synthetic probe
<400> 773
   ctgcacacgg ttctaggctc cg 22
<210> 774
   <211> 24
   <212> DNA
   <213> Artificial Sequence
<220>
   <223> Synthetic probe
<400> 774
   tctgctctac aagcccattg accg 24
<210> 775
   <211> 24
   <212> DNA
   <213> Artificial Sequence
<220>
   <223> Synthetic probe
<400> 775
   cccgcagaaa gcacatggta ttcc 24
<210> 776
   <211> 24
   <212> DNA
   <213> Artificial Sequence
<220>
   <223> Synthetic probe
<400> 776
   ctctgtcacc aatgtggacc tgcc 24
<210> 777
   <211> 29
   <212> DNA
   <213> Artificial Sequence
<220>
   <223> Synthetic probe
<400> 777
   tgtaggtatc tcttagtccc gccatctga 29
<210> 778
   <211> 24
   <212> DNA
   <213> Artificial Sequence
<220>
   <223> Synthetic probe
<400> 778
   ctgacactca tctgagccct ccca 24
<210> 779
   <211> 28
   <212> DNA
   <213> Artificial Sequence
<220>
   <223> Synthetic probe
<400> 779
   tgctacttgc aaaggcgtgt cctactgc 28
<210> 780
   <211> 20
   <212> DNA
   <213> Artificial Sequence
<220>
   <223> Synthetic probe
<400> 780
   ctgcgctgga tggacaccgc 20
<210> 781
   <211> 24
   <212> DNA
   <213> Artificial Sequence
<220>
   <223> Synthetic probe
<400> 781
   cacacagggt gccatcaatc acct 24
<210> 782
   <211> 24
   <212> DNA
   <213> Artificial Sequence
<220>
   <223> Synthetic probe
<400> 782
   cgagtggaag tgctccccac tttc 24
<210> 783
   <211> 28
   <212> DNA
   <213> Artificial Sequence
<220>
   <223> Synthetic probe
<400> 783
   cgatctcagc ctgtttgtgc atctcgat 28
<210> 784
   <211> 24
   <212> DNA
   <213> Artificial Sequence
<220>
   <223> Synthetic probe
<400> 784
   caacacgtca ccaccctttg ctct 24
<210> 785
   <211> 23
   <212> DNA
   <213> Artificial Sequence
<220>
   <223> Synthetic probe
<400> 785
   tgaccacacc aaagcctccc tgg 23
<210> 786
   <211> 24
   <212> DNA
   <213> Artificial Sequence
<220>
   <223> Synthetic probe
<400> 786
   acctcagtcc aaagtgcctg aggc 24
<210> 787
   <211> 23
   <212> DNA
   <213> Artificial Sequence
<220>
   <223> Synthetic probe
<400> 787
   tcacggtaca caatctttcc gga 23
<210> 788
   <211> 24
   <212> DNA
   <213> Artificial Sequence
<220>
   <223> Synthetic probe
<400> 788
   ccagttcctg ccgtctgctc ttct 24
<210> 789
   <211> 22
   <212> DNA
   <213> Artificial Sequence
<220>
   <223> Synthetic probe
<400> 789
   ctgcagcgtc aatctccgct tg 22
<210> 790
   <211> 24
   <212> DNA
   <213> Artificial Sequence
<220>
   <223> Synthetic probe
<400> 790
   aagctgacac agccctccca agtg 24
<210> 791
   <211> 24
   <212> DNA
   <213> Artificial Sequence
<220>
   <223> Synthetic probe
<400> 791
   ccaccacaca ggtacagcag cgct 24
<210> 792
   <211> 24
   <212> DNA
   <213> Artificial Sequence
<220>
   <223> Synthetic probe
<400> 792
   ctatgacgat gccctcaacg cctc 24
<210> 793
   <211> 21
   <212> DNA
   <213> Artificial Sequence
<220>
   <223> Synthetic probe
<400> 793
   ctttcgggaa gccaggccct t 21
<210> 794
   <211> 24
   <212> DNA
   <213> Artificial Sequence
<220>
   <223> Synthetic probe
<400> 794
   actggatcct ggccaccgac tatg 24
<210> 795
   <211> 22
   <212> DNA
   <213> Artificial Sequence
<220>
   <223> Synthetic probe
<400> 795
   cttgtccttg ggtcaccctg ca 22
<210> 796
   <211> 21
   <212> DNA
   <213> Artificial Sequence
<220>
   <223> Synthetic probe
<400> 796
   acaccagcgg tgccgactac c 21
<210> 797
   <211> 24
   <212> DNA
   <213> Artificial Sequence
<220>
   <223> Synthetic probe
<400> 797
   ctagagccat ccttggccat cctg 24
<210> 798
   <211> 24
   <212> DNA
   <213> Artificial Sequence
<220>
   <223> Synthetic probe
<400> 798
   caagaatctt gcagcagcat ggct 24
<210> 799
   <211> 22
   <212> DNA
   <213> Artificial Sequence
<220>
   <223> Synthetic probe
<400> 799
   tgacgagcag cgaacagcca cg 22
<210> 800
   <211> 23
   <212> DNA
   <213> Artificial Sequence
<220>
   <223> Synthetic probe
<400> 800
   aggagtatga cgagtccggc ccc 23
<210> 801
   <211> 29
   <212> DNA
   <213> Artificial Sequence
<220>
   <223> Synthetic probe
<400> 801
   aggctcagtg atgtcttccc tgtcaccag 29
<210> 802
   <211> 24
   <212> DNA
   <213> Artificial Sequence
<220>
   <223> Synthetic probe
<400> 802
   tgggcccaga gcatgttcca gate 24
<210> 803
   <211> 26
   <212> DNA
   <213> Artificial Sequence
<220>
   <223> Synthetic probe
<400> 803
   cccaattaac atgacccggc aaccat 26
<210> 804
   <211> 23
   <212> DNA
   <213> Artificial Sequence
<220>
   <223> Synthetic probe
<400> 804
   agatgtgccg gtacacccac ctc 23
<210> 805
   <211> 23
   <212> DNA
   <213> Artificial Sequence
<220>
   <223> Synthetic probe
<400> 805
   ccagcctgca gacaactggc ctc 23
<210> 806
   <211> 25
   <212> DNA
   <213> Artificial Sequence
<220>
   <223> Synthetic probe
<400> 806
   tgccactcgg aaaaagacct ctcgg 25
<210> 807
   <211> 24
   <212> DNA
   <213> Artificial Sequence
<220>
   <223> Synthetic probe
<400> 807
   catcatggga ctcctgccct tacc 24
<210> 808
   <211> 21
   <212> DNA
   <213> Artificial Sequence
<220>
   <223> Synthetic probe
<400> 808
   agtcacgacc cctgccctca c 21
<210> 809
   <211> 25
   <212> DNA
   <213> Artificial Sequence
<220>
   <223> Synthetic probe
<400> 809
   cagccctggg aactttgtcc tgacc 25
<210> 810
   <211> 23
   <212> DNA
   <213> Artificial Sequence
<220>
   <223> Synthetic probe
<400> 810
   ctttccgttg gcatccgcaa cag 23
<210> 811
   <211> 22
   <212> DNA
   <213> Artificial Sequence
<220>
   <223> Synthetic probe
<400> 811
   ttccacgccg aaggacagcg at 22
<210> 812
   <211> 24
   <212> DNA
   <213> Artificial Sequence
<220>
   <223> Synthetic probe
<400> 812
   tccgggtagc tctcaaactc gagg 24
<210> 813
   <211> 24
   <212> DNA
   <213> Artificial Sequence
<220>
   <223> Synthetic probe
<400> 813
   caagggtctc cagcacctct acgc 24
<210> 814
   <211> 23
   <212> DNA
   <213> Artificial Sequence
<220>
   <223> Synthetic probe
<400> 814
   ccggttaact gtggcctgtg ccc 23
<210> 815
   <211> 24
   <212> DNA
   <213> Artificial Sequence
<220>
   <223> Synthetic probe
<400> 815
   ctctcactgt gacagcccac ctcg 24
<210> 816
   <211> 24
   <212> DNA
   <213> Artificial Sequence
<220>
   <223> Synthetic probe
<400> 816
   ctgtgttcga ctcagcctca ggga 24
<210> 817
   <211> 23
   <212> DNA
   <213> Artificial Sequence
<220>
   <223> Synthetic probe
<400> 817
   cagtcggccc aggacggtct act 23
<210> 818
   <211> 23
   <212> DNA
   <213> Artificial Sequence
<220>
   <223> Synthetic probe
<400> 818
   tgctgggagc ctacacttgg ccc 23
<210> 819
   <211> 24
   <212> DNA
   <213> Artificial Sequence
<220>
   <223> Synthetic probe
<400> 819
   tacagtccca gcaccgacaa ttcc 24
<210> 820
   <211> 24
   <212> DNA
   <213> Artificial Sequence
<220>
   <223> Synthetic probe
<400> 820
   cctcgctttg tttaacagcc cagg 24
<210> 821
   <211> 24
   <212> DNA
   <213> Artificial Sequence
<220>
   <223> Synthetic probe
<400> 821
   ttacagcgac agtcatggcc gcat 24
<210> 822
   <211> 24
   <212> DNA
   <213> Artificial Sequence
<220>
   <223> Synthetic probe
<400> 822
   ccggagtcct agcctcccaa attc 24
<210> 823
   <211> 24
   <212> DNA
   <213> Artificial Sequence
<220>
   <223> Synthetic probe
<400> 823
   cctgctctgt tctggggtcc aaac 24
<210> 824
   <211> 19
   <212> DNA
   <213> Artificial Sequence
<220>
   <223> Synthetic probe
<400> 824
   caggtcccat tgccgggcg 19
<210> 825
   <211> 24
   <212> DNA
   <213> Artificial Sequence
<220>
   <223> Synthetic probe
<400> 825
   tgatttcccg ttccgctcgg ttct 24
<210> 826
   <211> 24
   <212> DNA
   <213> Artificial Sequence
<220>
   <223> Synthetic probe
<400> 826
   catggctacc acttcgacac agcc 24
<210> 827
   <211> 21
   <212> DNA
   <213> Artificial Sequence
<220>
   <223> Synthetic probe
<400> 827
   aaagcacacc gctggcagga c 21
<210> 828
   <211> 28
   <212> DNA
   <213> Artificial Sequence
<220>
   <223> Synthetic probe
<400> 828
   tggcctcaca aggactaccc tctcatcc 28
<210> 829
   <211> 24
   <212> DNA
   <213> Artificial Sequence
<220>
   <223> Synthetic probe
<400> 829
   atttcagctg atcagtgggc ctcc 24
<210> 830
   <211> 28
   <212> DNA
   <213> Artificial Sequence
<220>
   <223> Synthetic probe
<400> 830
   cataatacat tcacctccct gcctcctc 28
<210> 831
   <211> 24
   <212> DNA
   <213> Artificial Sequence
<220>
   <223> Synthetic probe
<400> 831
   cgcttcatct tggctgaggt cctc 24
<210> 832
   <211> 24
   <212> DNA
   <213> Artificial Sequence
<220>
   <223> Synthetic probe
<400> 832
   ctctgctgac actcgagccc acat 24
<210> 833
   <211> 22
   <212> DNA
   <213> Artificial Sequence
<220>
   <223> Synthetic probe
<400> 833
   acagagccct ggcaaagcca ag 22
<210> 834
   <211> 27
   <212> DNA
   <213> Artificial Sequence
<220>
   <223> Synthetic probe
<400> 834
   tgtctccatt attgatcggt tcatgca 27
<210> 835
   <211> 22
   <212> DNA
   <213> Artificial Sequence
<220>
   <223> Synthetic probe
<400> 835
   tacccgccat ccatgatcgc ca 22
<210> 836
   <211> 30
   <212> DNA
   <213> Artificial Sequence
<220>
   <223> Synthetic probe
<400> 836
   cccagataat acaggtggcc aacaattcct 30
<210> 837
   <211> 25
   <212> DNA
   <213> Artificial Sequence
<220>
   <223> Synthetic probe
<400> 837
   tggaataagt acctaaggcg ccccc 25
<210> 838
   <211> 21
   <212> DNA
   <213> Artificial Sequence
<220>
   <223> Synthetic probe
<400> 838
   ctcccatccc agtggagcca a 21
<210> 839
   <211> 22
   <212> DNA
   <213> Artificial Sequence
<220>
   <223> Synthetic probe
<400> 839
   cgcaccattc ggtcatttga gg 22
<210> 840
   <211> 23
   <212> DNA
   <213> Artificial Sequence
<220>
   <223> Synthetic probe
<400> 840
   ctgttgactg cagggcacca cca 23
<210> 841
   <211> 24
   <212> DNA
   <213> Artificial Sequence
<220>
   <223> Synthetic probe
<400> 841
   caggtccctt gtcccaagtt ccac 24
<210> 842
   <211> 27
   <212> DNA
   <213> Artificial Sequence
<220>
   <223> Synthetic probe
<400> 842
   ccctgctacc aatatggact ccagtca 27
<210> 843
   <211> 24
   <212> DNA
   <213> Artificial Sequence
<220>
   <223> Synthetic probe
<400> 843
   caccgacagc acagacagaa tccc 24
<210> 844
   <211> 24
   <212> DNA
   <213> Artificial Sequence
<220>
   <223> Synthetic probe
<400> 844
   caccaagccc agaggacagt tcct 24
<210> 845
   <211> 28
   <212> DNA
   <213> Artificial Sequence
<220>
   <223> Synthetic probe
<400> 845
   ctccaagccc agattcagat tcgagtca 28
<210> 846
   <211> 30
   <212> DNA
   <213> Artificial Sequence
<220>
   <223> Synthetic probe
<400> 846
   tcagcttcta caactggaca gacaacgctg 30
<210> 847
   <211> 23
   <212> DNA
   <213> Artificial Sequence
<220>
   <223> Synthetic probe
<400> 847
   actggccgtg gcactggaca aca 23
<210> 848
   <211> 28
   <212> DNA
   <213> Artificial Sequence
<220>
   <223> Synthetic probe
<400> 848
   tgtccctgtt agacgtcctc cgtccata 28
<210> 849
   <211> 23
   <212> DNA
   <213> Artificial Sequence
<220>
   <223> Synthetic probe
<400> 849
   ctccccgtcg atgccagaga act 23
<210> 850
   <211> 24
   <212> DNA
   <213> Artificial Sequence
<220>
   <223> Synthetic probe
<400> 850
   tgctccacta acaaccctcc tgcc 24
<210> 851
   <211> 24
   <212> DNA
   <213> Artificial Sequence
<220>
   <223> Synthetic probe
<400> 851
   aattcctgca tggccagttt cctc 24
<210> 852
   <211> 23
   <212> DNA
   <213> Artificial Sequence
<220>
   <223> Synthetic probe
<400> 852
   ctgcccaaga gcctgtcatc cag 23
<210> 853
   <211> 24
   <212> DNA
   <213> Artificial Sequence
<220>
   <223> Synthetic probe
<400> 853
   ccttctgccc atagtgatca gcga 24
<210> 854
   <211> 24
   <212> DNA
   <213> Artificial Sequence
<220>
   <223> Synthetic probe
<400> 854
   ccaacccaga tgaaatcggc aact 24
<210> 855
   <211> 24
   <212> DNA
   <213> Artificial Sequence
<220>
   <223> Synthetic probe
<400> 855
   ccagtcgcct cagtaaagcc acct 24
<210> 856
   <211> 24
   <212> DNA
   <213> Artificial Sequence
<220>
   <223> Synthetic probe
<400> 856
   cacaacatcc ctggtgaacg tcgt 24
<210> 857
   <211> 24
   <212> DNA
   <213> Artificial Sequence
<220>
   <223> Synthetic probe
<400> 857
   atcacccatc atcatccaat cgca 24
<210> 858
   <211> 23
   <212> DNA
   <213> Artificial Sequence
<220>
   <223> Synthetic probe
<400> 858
   tccttcccac ccccagtcct gtc 23
<210> 859
   <211> 24
   <212> DNA
   <213> Artificial Sequence
<220>
   <223> Synthetic probe
<400> 859
   aaaatgagac tctccgtcgg cagc 24
<210> 860
   <211> 20
   <212> DNA
   <213> Artificial Sequence
<220>
   <223> Synthetic probe
<400> 860
   caggccctct tccgagcggt 20
<210> 861
   <211> 21
   <212> DNA
   <213> Artificial Sequence
<220>
   <223> Synthetic probe
<400> 861
   cttcaattgg caagcccagg c 21
<210> 862
   <211> 22
   <212> DNA
   <213> Artificial Sequence
<220>
   <223> Synthetic probe
<400> 862
   tgctgatgtg ccctctcctt gg 22
<210> 863
   <211> 22
   <212> DNA
   <213> Artificial Sequence
<220>
   <223> Synthetic probe
<400> 863
   acccattctt ctcccagccg gg 22
<210> 864
   <211> 24
   <212> DNA
   <213> Artificial Sequence
<220>
   <223> Synthetic probe
<400> 864
   acaccctact ccctgtgcct ccag 24
<210> 865
   <211> 26
   <212> DNA
   <213> Artificial Sequence
<220>
   <223> Synthetic probe
<400> 865
   agctgatgag tctgccctac cgcctg 26
<210> 866
   <211> 24
   <212> DNA
   <213> Artificial Sequence
<220>
   <223> Synthetic probe
<400> 866
   tgaagtctcc agctttgcct cagc 24
<210> 867
   <211> 24
   <212> DNA
   <213> Artificial Sequence
<220>
   <223> Synthetic probe
<400> 867
   caccaggacc acaaagcctg tttg 24
<210> 868
   <211> 18
   <212> DNA
   <213> Artificial Sequence
<220>
   <223> Synthetic probe
<400> 868
   ctgcgcccgc tcttcgcg 18
<210> 869
   <211> 24
   <212> DNA
   <213> Artificial Sequence
<220>
   <223> Synthetic probe
<400> 869
   cgcacagaca agccttactc cgcc 24
<210> 870
   <211> 23
   <212> DNA
   <213> Artificial Sequence
<220>
   <223> Synthetic probe
<400> 870
   cgggaagaat tcgcttccac ctg 23
<210> 871
   <211> 20
   <212> DNA
   <213> Artificial Sequence
<220>
   <223> Synthetic probe
<400> 871
   cccttcagcc tgccccaccg 20
<210> 872
   <211> 24
   <212> DNA
   <213> Artificial Sequence
<220>
   <223> Synthetic probe
<400> 872
   actcagttac cgagccacgt cacg 24
<210> 873
   <211> 21
   <212> DNA
   <213> Artificial Sequence
<220>
   <223> Synthetic probe
<400> 873
   tcctgcgcct gatgtccacc g 21
<210> 874
   <211> 30
   <212> DNA
   <213> Artificial Sequence
<220>
   <223> Synthetic probe
<400> 874
   tctcctagcc agacgtgttt cttgtccttg 30
<210> 875
   <211> 21
   <212> DNA
   <213> Artificial Sequence
<220>
   <223> Synthetic probe
<400> 875
   cctgcagccc atccacaacc t 21
<210> 876
   <211> 27
   <212> DNA
   <213> Artificial Sequence
<220>
   <223> Synthetic probe
<400> 876
   cctcctcctg ttgctgccac taatgct 27
<210> 877
   <211> 26
   <212> DNA
   <213> Artificial Sequence
<220>
   <223> Synthetic probe
<400> 877
   cgaaacgcta ttctcacagg ttcagc 26
<210> 878
   <211> 24
   <212> DNA
   <213> Artificial Sequence
<220>
   <223> Synthetic probe
<400> 878
   tgttcatcct ggcgctcttc atgt 24
<210> 879
   <211> 26
   <212> DNA
   <213> Artificial Sequence
<220>
   <223> Synthetic probe
<400> 879
   ccgattccaa aagaccatca ggttct 26
<210> 880
   <211> 23
   <212> DNA
   <213> Artificial Sequence
<220>
   <223> Synthetic probe
<400> 880
   tttgctgaat gctccagcca agg 23
<210> 881
   <211> 28
   <212> DNA
   <213> Artificial Sequence
<220>
   <223> Synthetic probe
<400> 881
   tcagatggag acctcgtgcc aaattaca 28
<210> 882
   <211> 21
   <212> DNA
   <213> Artificial Sequence
<220>
   <223> Synthetic probe
<400> 882
   agccactccc cacgctgttg t 21
<210> 883
   <211> 24
   <212> DNA
   <213> Artificial Sequence
<220>
   <223> Synthetic probe
<400> 883
   cgcagatccg atttctctgg gatc 24
<210> 884
   <211> 21
   <212> DNA
   <213> Artificial Sequence
<220>
   <223> Synthetic probe
<400> 884
   tcccgatggt ctgcagcagc t 21
<210> 885
   <211> 28
   <212> DNA
   <213> Artificial Sequence
<220>
   <223> Synthetic probe
<400> 885
   aacatcatgt tcttcttcat gacctcgc 28
<210> 886
   <211> 24
   <212> DNA
   <213> Artificial Sequence
<220>
   <223> Synthetic probe
<400> 886
   accaacgctg acagcatgca tttc 24
<210> 887
   <211> 22
   <212> DNA
   <213> Artificial Sequence
<220>
   <223> Synthetic probe
<400> 887
   accctgcccg cgatcacact ga 22
<210> 888
   <211> 24
   <212> DNA
   <213> Artificial Sequence
<220>
   <223> Synthetic probe
<400> 888
   cttgaggacg cgaacagtcc acca 24
<210> 889
   <211> 29
   <212> DNA
   <213> Artificial Sequence
<220>
   <223> Synthetic probe
<400> 889
   aggtgcaata tgggcatata tctccattg 29
<210> 890
   <211> 24
   <212> DNA
   <213> Artificial Sequence
<220>
   <223> Synthetic probe
<400> 890
   tctgtgtggt ccatccttgg aagc 4
<210> 891
   <211> 24
   <212> DNA
   <213> Artificial Sequence
<220>
   <223> Synthetic probe
<400> 891
   ttcttcgaaa gccatgttgc caga 24
<210> 892
   <211> 24
   <212> DNA
   <213> Artificial Sequence
<220>
   <223> Synthetic probe
<400> 892
   tacccttaag aacgccccct ccac 24
<210> 893
   <211> 28
   <212> DNA
   <213> Artificial Sequence
<220>
   <223> Synthetic probe
<400> 893
   ctgaaactgg aacacaacca cccacaag 28
<210> 894
   <211> 24
   <212> DNA
   <213> Artificial Sequence
<220>
   <223> Synthetic probe
<400> 894
   tggacacact gatgcaagcc aaga 24
<210> 895
   <211> 19
   <212> DNA
   <213> Artificial Sequence
<220>
   <223> Synthetic probe
<400> 895
   cacagccacg gggcccaaa 19
<210> 896
   <211> 25
   <212> DNA
   <213> Artificial Sequence
<220>
   <223> Synthetic probe
<400> 896
   ctcatgccac cactgccaac acctc 25
<210> 897
   <211> 24
   <212> DNA
   <213> Artificial Sequence
<220>
   <223> Synthetic probe
<400> 897
   cagcaccctt ggcagtttcg aaat 24
<210> 898
   <211> 24
   <212> DNA
   <213> Artificial Sequence
<220>
   <223> Synthetic probe
<400> 898
   ctgtcacact ccctcaggca ggac 24
<210> 899
   <211> 24
   <212> DNA
   <213> Artificial Sequence
<220>
   <223> Synthetic probe
<400> 899
   atcactggaa ctcctcggtc ggac 24
<210> 900
   <211> 26
   <212> DNA
   <213> Artificial Sequence
<220>
   <223> Synthetic probe
<400> 900
   cagccacgat gaccactacc agcact 26
<210> 901
   <211> 26
   <212> DNA
   <213> Artificial Sequence
<220>
   <223> Synthetic probe
<400> 901
   tgcttcctcc cactatctga aaataa 26
<210> 902
   <211> 24
   <212> DNA
   <213> Artificial Sequence
<220>
   <223> Synthetic probe
<400> 902
   agctgcccgt ctttctcagc cagc 24
<210> 903
   <211> 25
   <212> DNA
   <213> Artificial Sequence
<220>
   <223> Synthetic probe
<400> 903
   agaaaagctg tttgtctccc cagca 25
<210> 904
   <211> 24
   <212> DNA
   <213> Artificial Sequence
<220>
   <223> Synthetic probe
<400> 904
   aaagtccatt tgccactgat ggca 24
<210> 905
   <211> 24
   <212> DNA
   <213> Artificial Sequence
<220>
   <223> Synthetic probe
<400> 905
   ctacctggac atccctgctc agcc 24
<210> 906
   <211> 23
   <212> DNA
   <213> Artificial Sequence
<220>
   <223> Synthetic probe
<400> 906
   cagacttggt gccctttgac tcc 23
<210> 907
   <211> 21
   <212> DNA
   <213> Artificial Sequence
<220>
   <223> Synthetic probe
<400> 907
   cagggccatg acaatcgcca a 21
<210> 908
   <211> 24
   <212> DNA
   <213> Artificial Sequence
<220>
   <223> Synthetic probe
<400> 908
   cgccctggct caacttttcc ttaa 24
<210> 909
   <211> 26
   <212> DNA
   <213> Artificial Sequence
<220>
   <223> Synthetic probe
<400> 909
   cgaggccatt gacttcatag actcca 26
<210> 910
   <211> 24
   <212> DNA
   <213> Artificial Sequence
<220>
   <223> Synthetic probe
<400> 910
   ttgagcacac tgcagtccat ctcc 24
<210> 911
   <211> 24
   <212> DNA
   <213> Artificial Sequence
<220>
   <223> Synthetic probe
<400> 911
   cagaagaaca gctcagggac ccct 24
<210> 912
   <211> 24
   <212> DNA
   <213> Artificial Sequence
<220>
   <223> Synthetic probe
<400> 912
   ctcaccagaa gccccaacct caac 24
<210> 913
   <211> 23
   <212> DNA
   <213> Artificial Sequence
<220>
   <223> Synthetic probe
<400> 913
   cactcccgag cacgttgttc cgt 23
<210> 914
   <211> 29
   <212> DNA
   <213> Artificial Sequence
<220>
   <223> Synthetic probe
<400> 914
   ccacttggac atcatctggg tgaacactc 29
<210> 915
   <211> 23
   <212> DNA
   <213> Artificial Sequence
<220>
   <223> Synthetic probe
<400> 915
   cctttgcctc agggcatcct ttt 23
<210> 916
   <211> 24
   <212> DNA
   <213> Artificial Sequence
<220>
   <223> Synthetic probe
<400> 916
   tgctacctgc ccctttgtca tgtg 24
<210> 917
   <211> 24
   <212> DNA
   <213> Artificial Sequence
<220>
   <223> Synthetic probe
<400> 917
   caaaggtgac caccataccg ggtt 24
<210> 918
   <211> 24
   <212> DNA
   <213> Artificial Sequence
<220>
   <223> Synthetic probe
<400> 918
   ctcgtcgtag cgcttctcgc tgta 24
<210> 919
   <211> 28
   <212> DNA
   <213> Artificial Sequence
<220>
   <223> Synthetic probe
<400> 919
   tgatgctttc tccagaaact cgaactca 28
<210> 920
   <211> 23
   <212> DNA
   <213> Artificial Sequence
<220>
   <223> Synthetic probe
<400> 920
   cggatccttt cctcactcgc cca 23
<210> 921
   <211> 24
   <212> DNA
   <213> Artificial Sequence
<220>
   <223> Synthetic probe
<400> 921
   acccagtctc accttctccc cacc 24
<210> 922
   <211> 26
   <212> DNA
   <213> Artificial Sequence
<220>
   <223> Synthetic probe
<400> 922
   tgagatggac atttaaagca ccagcc 26
<210> 923
   <211> 20
   <212> DNA
   <213> Artificial Sequence
<220>
   <223> Synthetic probe
<400> 923
   cgcccagagg cacccacctg 20
<210> 924
   <211> 22
   <212> DNA
   <213> Artificial Sequence
<220>
   <223> Synthetic probe
<400> 924
   ccagagagcc tccctgcagc ca 22
<210> 925
   <211> 26
   <212> DNA
   <213> Artificial Sequence
<220>
   <223> Synthetic probe
<400> 925
   ctgcaactgc ctcctgctca aagtca 26
<210> 926
   <211> 23
   <212> DNA
   <213> Artificial Sequence
<220>
   <223> Synthetic probe
<400> 926
   cactgacatc atggctggcc ttg 23
<210> 927
   <211> 25
   <212> DNA
   <213> Artificial Sequence
<220>
   <223> Synthetic probe
<400> 927
   ccgctctcat cgcagtcagg atcat 25
<210> 928
   <211> 20
   <212> DNA
   <213> Artificial Sequence
<220>
   <223> Synthetic probe
<400> 928
   tgcgcccgat gagatcaccg 20
<210> 929
   <211> 24
   <212> DNA
   <213> Artificial Sequence
<220>
   <223> Synthetic probe
<400> 929
   cacctgatgc atgatggaca ctgc 24
<210> 930
   <211> 24
   <212> DNA
   <213> Artificial Sequence
<220>
   <223> Synthetic probe
<400> 930
   cgtcccattt gagcctgtca atgt 24
<210> 931
   <211> 30
   <212> DNA
   <213> Artificial Sequence
<220>
   <223> Synthetic probe
<400> 931
   atctgtatgc ggaacctcaa aagagtccct 30
<210> 932
   <211> 30
   <212> DNA
   <213> Artificial Sequence
<220>
   <223> Synthetic probe
<400> 932
   tgtcccacga ataatgcgta aattctccag 30
<210> 933
   <211> 21
   <212> DNA
   <213> Artificial Sequence
<220>
   <223> Synthetic probe
<400> 933
   cagcaggccc tcaaggagct g 21
<210> 934
   <211> 24
   <212> DNA
   <213> Artificial Sequence
<220>
   <223> Synthetic probe
<400> 934
   agccatatgc cttctcatct gggc 24
<210> 935
   <211> 21
   <212> DNA
   <213> Artificial Sequence
<220>
   <223> Synthetic probe
<400> 935
   agagccggcc agccctgaca g 21
<210> 936
   <211> 24
   <212> DNA
   <213> Artificial Sequence
<220>
   <223> Synthetic probe
<400> 936
   tcgcctacca tttggtgcaa gcaa 24
<210> 937
   <211> 23
   <212> DNA
   <213> Artificial Sequence
<220>
   <223> Synthetic probe
<400> 937
   ctggccctca tgtccccttc acg 23
<210> 938
   <211> 26
   <212> DNA
   <213> Artificial Sequence
<220>
   <223> Synthetic probe
<400> 938
   ccccagacca agtgtgaata catgct 26
<210> 939
   <211> 19
   <212> DNA
   <213> Artificial Sequence
<220>
   <223> Synthetic probe
<400> 939
   ctggagatgc tggacgccc 19
<210> 940
   <211> 24
   <212> DNA
   <213> Artificial Sequence
<220>
   <223> Synthetic probe
<400> 940
   ttaccagagg cgaggttccc ttca 24
<210> 941
   <211> 25
   <212> DNA
   <213> Artificial Sequence
<220>
   <223> Synthetic probe
<400> 941
   tcctgacttc tgtgagctca ttgcg 25
<210> 942
   <211> 21
   <212> DNA
   <213> Artificial Sequence
<220>
   <223> Synthetic probe
<400> 942
   tggcacgggt cttctcctac c 21
<210> 943
   <211> 24
   <212> DNA
   <213> Artificial Sequence
<220>
   <223> Synthetic probe
<400> 943
   cggcctgtcc acgaaccact tata 24
<210> 944
   <211> 24
   <212> DNA
   <213> Artificial Sequence
<220>
   <223> Synthetic probe
<400> 944
   tcgcccacct acgtactggc ctac 24
<210> 945
   <211> 27
   <212> DNA
   <213> Artificial Sequence
<220>
   <223> Synthetic probe
<400> 945
   tcccagagac caacgttcaa gcagttg 27
<210> 946
   <211> 23
   <212> DNA
   <213> Artificial Sequence
<220>
   <223> Synthetic probe
<400> 946
   cctttcatgg ggagaaccgc att 23
<210> 947
   <211> 23
   <212> DNA
   <213> Artificial Sequence
<220>
   <223> Synthetic probe
<400> 947
   tcggccactt catcaggacg cag 23
<210> 948
   <211> 24
   <212> DNA
   <213> Artificial Sequence
<220>
   <223> Synthetic probe
<400> 948
   aatctgctcc actgtcaggg tccc 24
<210> 949
   <211> 24
   <212> DNA
   <213> Artificial Sequence
<220>
   <223> Synthetic probe
<400> 949
   actctcaggc ggtgtccaca tgat 24
<210> 950
   <211> 22
   <212> DNA
   <213> Artificial Sequence
<220>
   <223> Synthetic probe
<400> 950
   tcccagcatc atccaggccc ag 22
<210> 951
   <211> 24
   <212> DNA
   <213> Artificial Sequence
<220>
   <223> Synthetic probe
<400> 951
   agaacagcat ccgccacaac ctct 24
<210> 952
   <211> 24
   <212> DNA
   <213> Artificial Sequence
<220>
   <223> Synthetic probe
<400> 952
   ctctacagca gctcagccag cctg 24
<210> 953
   <211> 24
   <212> DNA
   <213> Artificial Sequence
<220>
   <223> Synthetic probe
<400> 953
   cagaccaagc ctttgcccag aatt 24
<210> 954
   <211> 24
   <212> DNA
   <213> Artificial Sequence
<220>
   <223> Synthetic probe
<400> 954
   tgtttccatg gctaccccac aggt 24
<210> 955
   <211> 22
   <212> DNA
   <213> Artificial Sequence
<220>
   <223> Synthetic probe
<400> 955
   tgaccggcgc atcacactga gg 22
<210> 956
   <211> 29
   <212> DNA
   <213> Artificial Sequence
<220>
   <223> Synthetic probe
<400> 956
   tcaattgtaa cattctcacc caggccttg 29
<210> 957
   <211> 24
   <212> DNA
   <213> Artificial Sequence
<220>
   <223> Synthetic probe
<400> 957
   ctgaagcacg acaagctggt ccag 24
<210> 958
   <211> 19
   <212> DNA
   <213> Artificial Sequence
<220>
   <223> Synthetic probe
<400> 958
   cgcccgcagg cctcatcct 19
<210> 959
   <211> 24
   <212> DNA
   <213> Artificial Sequence
<220>
   <223> Synthetic probe
<400> 959
   tgagccagat tccacacctc acgt 24
<210> 960
   <211> 25
   <212> DNA
   <213> Artificial Sequence
<220>
   <223> Synthetic probe
<400> 960
   ttcatctcaa tggaaggatc ctgcc 25
<210> 961
   <211> 24
   <212> DNA
   <213> Artificial Sequence
<220>
   <223> Synthetic probe
<400> 961
   aacttcagcc ccagctccca agtc 24
<210> 962
   <211> 22
   <212> DNA
   <213> Artificial Sequence
<220>
   <223> Synthetic probe
<400> 962
   ccgttctcag ccttgacggt gc 22
<210> 963
   <211> 24
   <212> DNA
   <213> Artificial Sequence
<220>
   <223> Synthetic probe
<400> 963
   tgttccaacc actgaatctg gacc 24
<210> 964
   <211> 26
   <212> DNA
   <213> Artificial Sequence
<220>
   <223> Synthetic probe
<400> 964
   ttgggacatt gtagacttgg ccagac 26
<210> 965
   <211> 30
   <212> DNA
   <213> Artificial Sequence
<220>
   <223> Synthetic probe
<400> 965
   ccatggacca acttcactat gtgacagagc 30
<210> 966
   <211> 26
   <212> DNA
   <213> Artificial Sequence
<220>
   <223> Synthetic probe
<400> 966
   tgcagttgac atggcctgtt cagtcc 26
<210> 967
   <211> 24
   <212> DNA
   <213> Artificial Sequence
<220>
   <223> Synthetic probe
<400> 967
   tgttagccaa agactgccac tgca 24
<210> 968
   <211> 24
   <212> DNA
   <213> Artificial Sequence
<220>
   <223> Synthetic probe
<400> 968
   tgtccacagg accctgagtg gttc 24
<210> 969
   <211> 24
   <212> DNA
   <213> Artificial Sequence
<220>
   <223> Synthetic probe
<400> 969
   atcccctccc tctccaccca tcta 24
<210> 970
   <211> 21
   <212> DNA
   <213> Artificial Sequence
<220>
   <223> Synthetic probe
<400> 970
   aggcgttgca cttcaccagc c 21
<210> 971
   <211> 24
   <212> DNA
   <213> Artificial Sequence
<220>
   <223> Synthetic probe
<400> 971
   cctcttgccc acttactggg tgga 24
<210> 972
   <211> 24
   <212> DNA
   <213> Artificial Sequence
<220>
   <223> Synthetic probe
<400> 972
   ccgggtgaca gcactaacca gacc 24
<210> 973
   <211> 22
   <212> DNA
   <213> Artificial Sequence
<220>
   <223> Synthetic probe
<400> 973
   ctcttcccca tcggctttgt gg 22
<210> 974
   <211> 24
   <212> DNA
   <213> Artificial Sequence
<220>
   <223> Synthetic probe
<400> 974
   cctcctgctg gcttcctttg atca 24
<210> 975
   <211> 24
   <212> DNA
   <213> Artificial Sequence
<220>
   <223> Synthetic probe
<400> 975
   ctcatcacct ggtctccggt gtgt 24
<210> 976
   <211> 26
   <212> DNA
   <213> Artificial Sequence
<220>
   <223> Synthetic probe
<400> 976
   catgctgcat cctaaggctc ctcagg 26
<210> 977
   <211> 24
   <212> DNA
   <213> Artificial Sequence
<220>
   <223> Synthetic probe
<400> 977
   ctggccttcc cgtgtaacca gttc 24
<210> 978
   <211> 23
   <212> DNA
   <213> Artificial Sequence
<220>
   <223> Synthetic probe
<400> 978
   ctccccaccc ttgagaagtg cct 23
<210> 979
   <211> 28
   <212> DNA
   <213> Artificial Sequence
<220>
   <223> Synthetic probe
<400> 979
   cacaattccc agagaaacca agaagagc 28
<210> 980
   <211> 20
   <212> DNA
   <213> Artificial Sequence
<220>
   <223> Synthetic probe
<400> 980
   accacgcgaa cggtgcatcg 20
<210> 981
   <211> 20
   <212> DNA
   <213> Artificial Sequence
<220>
   <223> Synthetic probe
<400> 981
   tccccgagcc cagcaggaca 20
<210> 982
   <211> 29
   <212> DNA
   <213> Artificial Sequence
<220>
   <223> Synthetic probe
<400> 982
   tgacctgatc cagagtaagt gcctctcca 29
<210> 983
   <211> 30
   <212> DNA
   <213> Artificial Sequence
<220>
   <223> Synthetic probe
<400> 983
   tcagccactg gcttctgtca taatcaggag 30
<210> 984
   <211> 27
   <212> DNA
   <213> Artificial Sequence
<220>
   <223> Synthetic probe
<400> 984
   cccgcctacc ctcgtaaagc agattca 27
<210> 985
   <211> 26
   <212> DNA
   <213> Artificial Sequence
<220>
   <223> Synthetic probe
<400> 985
   ctgaagctct actcacagtt tctggg 26
<210> 986
   <211> 27
   <212> DNA
   <213> Artificial Sequence
<220>
   <223> Synthetic probe
<400> 986
   ctcgcaagca caacatgtgt ggtgaga 27
<210> 987
   <211> 23
   <212> DNA
   <213> Artificial Sequence
<220>
   <223> Synthetic probe
<400> 987
   cacagccgcc tgaaagccac aat 23
<210> 988
   <211> 27
   <212> DNA
   <213> Artificial Sequence
<220>
   <223> Synthetic probe
<400> 988
   tcaagtaaac gggctgtttt ccaaaca 27
<210> 989
   <211> 23
   <212> DNA
   <213> Artificial Sequence
<220>
   <223> Synthetic probe
<400> 989
   aaagacatcc agctagcacg ccg 23
<210> 990
   <211> 23
   <212> DNA
   <213> Artificial Sequence
<220>
   <223> Synthetic probe
<400> 990
   ttcttgcgct ccatccgtcc aga 23
<210> 991
   <211> 24
   <212> DNA
   <213> Artificial Sequence
<220>
   <223> Synthetic probe
<400> 991
   caagaacctc ccagaagggc tcaa 24
<210> 992
   <211> 24
   <212> DNA
   <213> Artificial Sequence
<220>
   <223> Synthetic probe
<400> 992
   ccagaccata gcacactcgg gcac 24
<210> 993
   <211> 24
   <212> DNA
   <213> Artificial Sequence
<220>
   <223> Synthetic probe
<400> 993
   cagaatgtcc gccttctcca gctt 24
<210> 994
   <211> 23
   <212> DNA
   <213> Artificial Sequence
<220>
   <223> Synthetic probe
<400> 994
   cgctcacgtt ctcatccaag tgg 23
<210> 995
   <211> 21
   <212> DNA
   <213> Artificial Sequence
<220>
   <223> Synthetic probe
<400> 995
   ccccggacag tggctctgac g 21
<210> 996
   <211> 28
   <212> DNA
   <213> Artificial Sequence
<220>
   <223> Synthetic probe
<400> 996
   ttccacatct ctcccagttt cttcgcaa 28
<210> 997
   <211> 24
   <212> DNA
   <213> Artificial Sequence
<220>
   <223> Synthetic probe
<400> 997
   agtcgctggt ttcatgccct tcca 24
<210> 998
   <211> 28
   <212> DNA
   <213> Artificial Sequence
<220>
   <223> Synthetic probe
<400> 998
   ctccatatcc aaacaaagca tgtgtgcg 28
<210> 999
   <211> 24
   <212> DNA
   <213> Artificial Sequence
<220>
   <223> Synthetic probe
<400> 999
   agtctcctac tcccgggttc tgcg 24
<210> 1000
   <211> 24
   <212> DNA
   <213> Artificial Sequence
<220>
   <223> Synthetic probe
<400> 1000
   tgaactcctt cctggaatac ccca 24
<210> 1001
   <211> 28
   <212> DNA
   <213> Artificial Sequence
<220>
   <223> Synthetic probe
<400> 1001
   agccctgttc tcgttgccct aattcatc 28
<210> 1002
   <211> 24
   <212> DNA
   <213> Artificial Sequence
<220>
   <223> Synthetic probe
<400> 1002
   acactcggca ggagtagtac ccgc 24
<210> 1003
   <211> 24
   <212> DNA
   <213> Artificial Sequence
<220>
   <223> Synthetic probe
<400> 1003
   accggagcct tcccagaaca aact 24
<210> 1004
   <211> 25
   <212> DNA
   <213> Artificial Sequence
<220>
   <223> Synthetic probe
<400> 1004
   accgcttcta ccaatacctc gccca 25
<210> 1005
   <211> 24
   <212> DNA
   <213> Artificial Sequence
<220>
   <223> Synthetic probe
<400> 1005
   agttgcttcc atccaacctg gagg 24
<210> 1006
   <211> 30
   <212> DNA
   <213> Artificial Sequence
<220>
   <223> Synthetic probe
<400> 1006
   cagaatggcc tgtattcact atcttcgaga 30
<210> 1007
   <211> 24
   <212> DNA
   <213> Artificial Sequence
<220>
   <223> Synthetic probe
<400> 1007
   agagtgactc ccgttgtccc aagg 24
<210> 1008
   <211> 20
   <212> DNA
   <213> Artificial Sequence
<220>
   <223> Synthetic probe
<400> 1008
   tgactcccgc ggtcccaagg 20
<210> 1009
   <211> 29
   <212> DNA
   <213> Artificial Sequence
<220>
   <223> Synthetic probe
<400> 1009
   cattttcctc agacttgtga acctccact 29
<210> 1010
   <211> 30
   <212> DNA
   <213> Artificial Sequence
<220>
   <223> Synthetic probe
<400> 1010
   taattagacc taggcctcag ctgcactgcc 30
<210> 1011
   <211> 26
   <212> DNA
   <213> Artificial Sequence
<220>
   <223> Synthetic probe
<400> 1011
   ctcagggccc accattgaag aggttg 26
<210> 1012
   <211> 24
   <212> DNA
   <213> Artificial Sequence
<220>
   <223> Synthetic probe
<400> 1012
   cgcacttttc tgagcagacg tcca 24
<210> 1013
   <211> 23
   <212> DNA
   <213> Artificial Sequence
<220>
   <223> Synthetic probe
<400> 1013
   ccaggcgtgg cgtcctctcc ata 23
<210> 1014
   <211> 20
   <212> DNA
   <213> Artificial Sequence
<220>
   <223> Synthetic probe
<400> 1014
   ccggcgccca acgtgattct 20
<210> 1015
   <211> 26
   <212> DNA
   <213> Artificial Sequence
<220>
   <223> Synthetic probe
<400> 1015
   tggagattct ccagcacgtc atcgac 26
<210> 1016
   <211> 30
   <212> DNA
   <213> Artificial Sequence
<220>
   <223> Synthetic probe
<400> 1016
   cttttgtttt gcccagtata gactcggaag 30
<210> 1017
   <211> 20
   <212> DNA
   <213> Artificial Sequence
<220>
   <223> Synthetic probe
<400> 1017
   ccgtgaatgc agcccgccag 20
<210> 1018
   <211> 28
   <212> DNA
   <213> Artificial Sequence
<220>
   <223> Synthetic probe
<400> 1018
   cgcgtcatac caaaatctcc gattttga 28
<210> 1019
   <211> 24
   <212> DNA
   <213> Artificial Sequence
<220>
   <223> Synthetic probe
<400> 1019
   taccccgtgg gcaagttctt ccaa 24
<210> 1020
   <211> 25
   <212> DNA
   <213> Artificial Sequence
<220>
   <223> Synthetic probe
<400> 1020
   atccaggcac ctctaccacg ccctc 25
<210> 1021
   <211> 23
   <212> DNA
   <213> Artificial Sequence
<220>
   <223> Synthetic probe
<400> 1021
   cccggtcacc aggcaggagt tct 23
<210> 1022
   <211> 24
   <212> DNA
   <213> Artificial Sequence
<220>
   <223> Synthetic probe
<400> 1022
   cagccctaca cgaaaggacc tgct 24
<210> 1023
   <211> 22
   <212> DNA
   <213> Artificial Sequence
<220>
   <223> Synthetic probe
<400> 1023
   tgacttccaa gctggccgtg gc 22
<210> 1024
   <211> 22
   <212> DNA
   <213> Artificial Sequence
<220>
   <223> Synthetic probe
<400> 1024
   ctgctccacg gccttgctct tg 22
<210> 1025
   <211> 26
   <212> DNA
   <213> Artificial Sequence
<220>
   <223> Synthetic probe
<400> 1025
   cctgtgatca acagtacccg tatggg 26
<210> 1026
   <211> 24
   <212> DNA
   <213> Artificial Sequence
<220>
   <223> Synthetic probe
<400> 1026
   tcggcttccc tgtagagctg aaca 24
<210> 1027
   <211> 25
   <212> DNA
   <213> Artificial Sequence
<220>
   <223> Synthetic probe
<400> 1027
   catattgccc agtggtcacc tcaca 25
<210> 1028
   <211> 24
   <212> DNA
   <213> Artificial Sequence
<220>
   <223> Synthetic probe
<400> 1028
   attcaaaaca gagcccccaa agcc 24
<210> 1029
   <211> 25
   <212> DNA
   <213> Artificial Sequence
<220>
   <223> Synthetic probe
<400> 1029
   acgtccgggt cctcactgtc cttcc 25
<210> 1030
   <211> 24
   <212> DNA
   <213> Artificial Sequence
<220>
   <223> Synthetic probe
<400> 1030
   cccacatgac ttcctcttgg cctt 24
<210> 1031
   <211> 20
   <212> DNA
   <213> Artificial Sequence
<220>
   <223> Synthetic probe
<400> 1031
   tccatcccag ctccagccag 20
<210> 1032
   <211> 24
   <212> DNA
   <213> Artificial Sequence
<220>
   <223> Synthetic probe
<400> 1032
   cactccagac ctcgcttagc atgg 24
<210> 1033
   <211> 24
   <212> DNA
   <213> Artificial Sequence
<220>
   <223> Synthetic probe
<400> 1033
   cgatcctgca tctgtaaatc gccc 24
<210> 1034
   <211> 24
   <212> DNA
   <213> Artificial Sequence
<220>
   <223> Synthetic probe
<400> 1034
   tctgagcctt gtcctctatc cggc 24
<210> 1035
   <211> 24
   <212> DNA
   <213> Artificial Sequence
<220>
   <223> Synthetic probe
<400> 1035
   tcgccatctt ttgtgggatt cctt 24
<210> 1036
   <211> 24
   <212> DNA
   <213> Artificial Sequence
<220>
   <223> Synthetic probe
<400> 1036
   ccgacagcca cagaataacc caaa 24
<210> 1037
   <211> 30
   <212> DNA
   <213> Artificial Sequence
<220>
   <223> Synthetic probe
<400> 1037
   tgctaatgta aggcatcaca gtcttttcca 30
<210> 1038
   <211> 28
   <212> DNA
   <213> Artificial Sequence
<220>
   <223> Synthetic probe
<400> 1038
   aaatacctgc aaccgttact gccgtgac 28
<210> 1039
   <211> 24
   <212> DNA
   <213> Artificial Sequence
<220>
   <223> Synthetic probe
<400> 1039
   caccaacctg tacccgtatt gcga 24
<210> 1040
   <211> 27
   <212> DNA
   <213> Artificial Sequence
<220>
   <223> Synthetic probe
<400> 1040
   tgctatgttt ctacaaaacc gccaagg 27
<210> 1041
   <211> 24
   <212> DNA
   <213> Artificial Sequence
<220>
   <223> Synthetic probe
<400> 1041
   actcgatttc ccagccaacc acag 24
<210> 1042
   <211> 23
   <212> DNA
   <213> Artificial Sequence
<220>
   <223> Synthetic probe
<400> 1042
   caagggacac gccttctgaa cgt 23
<210> 1043
   <211> 23
   <212> DNA
   <213> Artificial Sequence
<220>
   <223> Synthetic probe
<400> 1043
   ccacttgtcg aaccaccgct cgt 23
<210> 1044
   <211> 24
   <212> DNA
   <213> Artificial Sequence
<220>
   <223> Synthetic probe
<400> 1044
   cccttcaagc tgccaatgaa gacc 24
<210> 1045
   <211> 24
   <212> DNA
   <213> Artificial Sequence
<220>
   <223> Synthetic probe
<400> 1045
   cttcaaggcc atgctgacca tcag 24
<210> 1046
   <211> 24
   <212> DNA
   <213> Artificial Sequence
<220>
   <223> Synthetic probe
<400> 1046
   tgcattcctc tgagctcact gctg 24
<210> 1047
   <211> 24
   <212> DNA
   <213> Artificial Sequence
<220>
   <223> Synthetic probe
<400> 1047
   agtcagtggc ccatcagcaa tcag 24
<210> 1048
   <211> 22
   <212> DNA
   <213> Artificial Sequence
<220>
   <223> Synthetic probe
<400> 1048
   aagccgctcc actcgcatgt cc 22
<210> 1049
   <211> 23
   <212> DNA
   <213> Artificial Sequence
<220>
   <223> Synthetic probe
<400> 1049
   cctccccaac aaagaccacc gca 23
<210> 1050
   <211> 24
   <212> DNA
   <213> Artificial Sequence
<220>
   <223> Synthetic probe
<400> 1050
   ttaccccagc tccatccttg catc 24
<210> 1051
   <211> 26
   <212> DNA
   <213> Artificial Sequence
<220>
   <223> Synthetic probe
<400> 1051
   ttcgtaacag cagtcatcat ccatgg 26
<210> 1052
   <211> 25
   <212> DNA
   <213> Artificial Sequence
<220>
   <223> Synthetic probe
<400> 1052
   tttgaccggg tattcccacc aggaa 25
<210> 1053
   <211> 24
   <212> DNA
   <213> Artificial Sequence
<220>
   <223> Synthetic probe
<400> 1053
   ccttggagaa acacaagcac ctgc 24
<210> 1054
   <211> 24
   <212> DNA
   <213> Artificial Sequence
<220>
   <223> Synthetic probe
<400> 1054
   actcctgttt tcaccaccat gcca 24
<210> 1055
   <211> 24
   <212> DNA
   <213> Artificial Sequence
<220>
   <223> Synthetic probe
<400> 1055
   atctacgttg tccagctgcc agcc 24
<210> 1056
   <211> 24
   <212> DNA
   <213> Artificial Sequence
<220>
   <223> Synthetic probe
<400> 1056
   ctgattcctc aggtccttgg cctg 24
<210> 1057
   <211> 24
   <212> DNA
   <213> Artificial Sequence
<220>
   <223> Synthetic probe
<400> 1057
   ctgttcctgg agcatggcct cttc 24
<210> 1058
   <211> 24
   <212> DNA
   <213> Artificial Sequence
<220>
   <223> Synthetic probe
<400> 1058
   caagtgcctg taccacacgg aagg 24
<210> 1059
   <211> 23
   <212> DNA
   <213> Artificial Sequence
<220>
   <223> Synthetic probe
<400> 1059
   ccactcgcca tactgggtgc agt 23
<210> 1060
   <211> 28
   <212> DNA
   <213> Artificial Sequence
<220>
   <223> Synthetic probe
<400> 1060
   aggtcttatc agcacagtct ccgcctcc 28
<210> 1061
   <211> 24
   <212> DNA
   <213> Artificial Sequence
<220>
   <223> Synthetic probe
<400> 1061
   ctggacgcgg ttctactcca acag 24
<210> 1062
   <211> 25
   <212> DNA
   <213> Artificial Sequence
<220>
   <223> Synthetic probe
<400> 1062
   acccagataa cgcatcatgg agcga 25
<210> 1063
   <211> 24
   <212> DNA
   <213> Artificial Sequence
<220>
   <223> Synthetic probe
<400> 1063
   tgcctccctg tcgcaccagt acta 24
<210> 1064
   <211> 24
   <212> DNA
   <213> Artificial Sequence
<220>
   <223> Synthetic probe
<400> 1064
   actgagcgca cacgaaacac tgct 24
<210> 1065
   <211> 22
   <212> DNA
   <213> Artificial Sequence
<220>
   <223> Synthetic probe
<400> 1065
   cagcccccca actgacctca tc 22
<210> 1066
   <211> 24
   <212> DNA
   <213> Artificial Sequence
<220>
   <223> Synthetic probe
<400> 1066
   caggctcagc aagctgaaca cctg 24
<210> 1067
   <211> 24
   <212> DNA
   <213> Artificial Sequence
<220>
   <223> Synthetic probe
<400> 1067
   ttactccagg ggacaagcct tcca 24
<210> 1068
   <211> 24
   <212> DNA
   <213> Artificial Sequence
<220>
   <223> Synthetic probe
<400> 1068
   ccttcagggc ctgcactttc aact 24
<210> 1069
   <211> 24
   <212> DNA
   <213> Artificial Sequence
<220>
   <223> Synthetic probe
<400> 1069
   ctgtgtttag gcactcccct tgcg 24
<210> 1070
   <211> 24
   <212> DNA
   <213> Artificial Sequence
<220>
   <223> Synthetic probe
<400> 1070
   ttcttcttct gttcctcgct ccgg 24
<210> 1071
   <211> 25
   <212> DNA
   <213> Artificial Sequence
<220>
   <223> Synthetic probe
<400> 1071
   catgttcttc acaatcgctg catcc 25
<210> 1072
   <211> 24
   <212> DNA
   <213> Artificial Sequence
<220>
   <223> Synthetic probe
<400> 1072
   acagctcatt gttgtcacgc cgga 24
<210> 1073
   <211> 24
   <212> DNA
   <213> Artificial Sequence
<220>
   <223> Synthetic probe
<400> 1073
   cccgggttgt cttccgtcag atag 24
<210> 1074
   <211> 20
   <212> DNA
   <213> Artificial Sequence
<220>
   <223> Synthetic probe
<400> 1074
   cagccctttg gggaagctgg 20
<210> 1075
   <211> 22
   <212> DNA
   <213> Artificial Sequence
<220>
   <223> Synthetic probe
<400> 1075
   tttgacaccc cttccccagc ca 22
<210> 1076
   <211> 30
   <212> DNA
   <213> Artificial Sequence
<220>
   <223> Synthetic probe
<400> 1076
   agcaagattt cctccaggtc catcaaaagg 30
<210> 1077
   <211> 24
   <212> DNA
   <213> Artificial Sequence
<220>
   <223> Synthetic probe
<400> 1077
   aaccagctct ctgtgacccc aatt 24
<210> 1078
   <211> 24
   <212> DNA
   <213> Artificial Sequence
<220>
   <223> Synthetic probe
<400> 1078
   ctgggagcat ggcgatggat accc 24
<210> 1079
   <211> 28
   <212> DNA
   <213> Artificial Sequence
<220>
   <223> Synthetic probe
<400> 1079
   cctgtatgct gcaactcatg aacttggc 28
<210> 1080
   <211> 30
   <212> DNA
   <213> Artificial Sequence
<220>
   <223> Synthetic probe
<400> 1080
   aagcaatgtt gatatctgcc tctccctgtg 30
<210> 1081
   <211> 29
   <212> DNA
   <213> Artificial Sequence
<220>
   <223> Synthetic probe
<400> 1081
   tcatcaaacc atggttcatc accaatatc 29
<210> 1082
   <211> 22
   <212> DNA
   <213> Artificial Sequence
<220>
   <223> Synthetic probe
<400> 1082
   cgagggcaac cctgatcgtc ca 22
<210> 1083
   <211> 29
   <212> DNA
   <213> Artificial Sequence
<220>
   <223> Synthetic probe
<400> 1083
   acctgatacg tcttggtctt catcgccat 29
<210> 1084
   <211> 26
   <212> DNA
   <213> Artificial Sequence
<220>
   <223> Synthetic probe
<400> 1084
   tctgtcctgg ctggagtcgc tttcat 26
<210> 1085
   <211> 24
   <212> DNA
   <213> Artificial Sequence
<220>
   <223> Synthetic probe
<400> 1085
   tgaactccgc agctagcatc caaa 24
<210> 1086
   <211> 29
   <212> DNA
   <213> Artificial Sequence
<220>
   <223> Synthetic probe
<400> 1086
   caagaagatt tacttcgtcg attcccaga 29
<210> 1087
   <211> 27
   <212> DNA
   <213> Artificial Sequence
<220>
   <223> Synthetic probe
<400> 1087
   tcagtcaaca tcaccctcct aggatga 27
<210> 1088
   <211> 23
   <212> DNA
   <213> Artificial Sequence
<220>
   <223> Synthetic probe
<400> 1088
   tcaccctgga gatcagctcc cga 23
<210> 1089
   <211> 24
   <212> DNA
   <213> Artificial Sequence
<220>
   <223> Synthetic probe
<400> 1089
   cagcattgtc tgtcctccct ggca 24
<210> 1090
   <211> 26
   <212> DNA
   <213> Artificial Sequence
<220>
   <223> Synthetic probe
<400> 1090
   tggagtgctg taaacatacc ctccca 26
<210> 1091
   <211> 28
   <212> DNA
   <213> Artificial Sequence
<220>
   <223> Synthetic probe
<400> 1091
   cgggctgttc cctttgagaa ccttaaca 28
<210> 1092
   <211> 24
   <212> DNA
   <213> Artificial Sequence
<220>
   <223> Synthetic probe
<400> 1092
   atgaccaccc cggctcgtat gtca 24
<210> 1093
   <211> 28
   <212> DNA
   <213> Artificial Sequence
<220>
   <223> Synthetic probe
<400> 1093
   tccccacagt agacacatat gatggccg 28
<210> 1094
   <211> 29
   <212> DNA
   <213> Artificial Sequence
<220>
   <223> Synthetic probe
<400> 1094
   ctcagagcct ctctggttct ttcaatcgg 29
<210> 1095
   <211> 22
   <212> DNA
   <213> Artificial Sequence
<220>
   <223> Synthetic probe
<400> 1095
   ctcggtgttg gccatgctcc ag 22
<210> 1096
   <211> 24
   <212> DNA
   <213> Artificial Sequence
<220>
   <223> Synthetic probe
<400> 1096
   atcttctcag acgtcccgag ccag 24
<210> 1097
   <211> 28
   <212> DNA
   <213> Artificial Sequence
<220>
   <223> Synthetic probe
<400> 1097
   tgtccttacc tgtgggagct gtaaggtc 28
<210> 1098
   <211> 24
   <212> DNA
   <213> Artificial Sequence
<220>
   <223> Synthetic probe
<400> 1098
   tcctttggta tcagacccga agcg 24
<210> 1099
   <211> 29
   <212> DNA
   <213> Artificial Sequence
<220>
   <223> Synthetic probe
<400> 1099
   cctttccagc tttacagtga attgctgca 29
<210> 1100
   <211> 29
   <212> DNA
   <213> Artificial Sequence
<220>
   <223> Synthetic probe
<400> 1100
   ccaagagaaa cgagatttaa aaacccacc 29
<210> 1101
   <211> 24
   <212> DNA
   <213> Artificial Sequence
<220>
   <223> Synthetic probe
<400> 1101
   ctttccaacc cctggggaag acat 24
<210> 1102
   <211> 24
   <212> DNA
   <213> Artificial Sequence
<220>
   <223> Synthetic probe
<400> 1102
   cctcccgaag ttgcttgaaa gcac 24
<210> 1103
   <211> 24
   <212> DNA
   <213> Artificial Sequence
<220>
   <223> Synthetic probe
<400> 1103
   cattcgcttc ttcctccact tggc 24
<210> 1104
   <211> 25
   <212> DNA
   <213> Artificial Sequence
<220>
   <223> Synthetic probe
<400> 1104
   tctccacaga caaggccagg actcg 25
<210> 1105
   <211> 24
   <212> DNA
   <213> Artificial Sequence
<220>
   <223> Synthetic probe
<400> 1105
   atcaccaaca gcatgacctt tgcg 24
<210> 1106
   <211> 24
   <212> DNA
   <213> Artificial Sequence
<220>
   <223> Synthetic probe
<400> 1106
   aggacagtgg agcagccaac acac 24
<210> 1107
   <211> 25
   <212> DNA
   <213> Artificial Sequence
<220>
   <223> Synthetic probe
<400> 1107
   cattggaatt gccattagtc ccagc 25
<210> 1108
   <211> 25
   <212> DNA
   <213> Artificial Sequence
<220>
   <223> Synthetic probe
<400> 1108
   ccagcacagc cagttaaaag atgca 25
<210> 1109
   <211> 24
   <212> DNA
   <213> Artificial Sequence
<220>
   <223> Synthetic probe
<400> 1109
   ttggatctgc ttgctgtcca aacc 24
<210> 1110
   <211> 24
   <212> DNA
   <213> Artificial Sequence
<220>
   <223> Synthetic probe
<400> 1110
   cacgccatgg aaaccatgat gttt 24
<210> 1111
   <211> 27
   <212> DNA
   <213> Artificial Sequence
<220>
   <223> Synthetic probe
<400> 1111
   cacaagtact cctgccaaga gggcgac 27
<210> 1112
   <211> 24
   <212> DNA
   <213> Artificial Sequence
<220>
   <223> Synthetic probe
<400> 1112
   atcacatcca gggccttctc caga 24
<210> 1113
   <211> 28
   <212> DNA
   <213> Artificial Sequence
<220>
   <223> Synthetic probe
<400> 1113
   ttccccaact tccttagtgc ctgtgaca 28
<210> 1114
   <211> 24
   <212> DNA
   <213> Artificial Sequence
<220>
   <223> Synthetic probe
<400> 1114
   catgccgtct acagggatga cctg 24
<210> 1115
   <211> 23
   <212> DNA
   <213> Artificial Sequence
<220>
   <223> Synthetic probe
<400> 1115
   cccggggcct gttatgtcaa act 23
<210> 1116
   <211> 24
   <212> DNA
   <213> Artificial Sequence
<220>
   <223> Synthetic probe
<400> 1116
   ccggagggaa ccctgactac agaa 24
<210> 1117
   <211> 24
   <212> DNA
   <213> Artificial Sequence
<220>
   <223> Synthetic probe
<400> 1117
   aggataagac cacagcacag gcgc 24
<210> 1118
   <211> 23
   <212> DNA
   <213> Artificial Sequence
<220>
   <223> Synthetic probe
<400> 1118
   ttgctcaagg acctggacgc caa 23
<210> 1119
   <211> 24
   <212> DNA
   <213> Artificial Sequence
<220>
   <223> Synthetic probe
<400> 1119
   ctgtccacca aatgcacgct gata 24
<210> 1120
   <211> 22
   <212> DNA
   <213> Artificial Sequence
<220>
   <223> Synthetic probe
<400> 1120
   ctccccacag cgcatcgagg aa 22
<210> 1121
   <211> 24
   <212> DNA
   <213> Artificial Sequence
<220>
   <223> Synthetic probe
<400> 1121
   cagcaccgat ttcttcaggt ccct 24
<210> 1122
   <211> 23
   <212> DNA
   <213> Artificial Sequence
<220>
   <223> Synthetic probe
<400> 1122
   cctgacttca ggtcaaggga tgg 23
<210> 1123
   <211> 24
   <212> DNA
   <213> Artificial Sequence
<220>
   <223> Synthetic probe
<400> 1123
   ccaatctctg cctcagttct gcca 24
<210> 1124
   <211> 20
   <212> DNA
   <213> Artificial Sequence
<220>
   <223> Synthetic probe
<400> 1124
   cctgcggctt tcggatccca 20
<210> 1125
   <211> 25
   <212> DNA
   <213> Artificial Sequence
<220>
   <223> Synthetic probe
<400> 1125
   tggccatcca tctcacagaa attgg 25
<210> 1126
   <211> 25
   <212> DNA
   <213> Artificial Sequence
<220>
   <223> Synthetic probe
<400> 1126
   tctggattag agtcctgcag ctcgc 25
<210> 1127
   <211> 24
   <212> DNA
   <213> Artificial Sequence
<220>
   <223> Synthetic probe
<400> 1127
   ctctgtggca ccctggacta cctg 24
<210> 1128
   <211> 23
   <212> DNA
   <213> Artificial Sequence
<220>
   <223> Synthetic probe
<400> 1128
   cacgttctct gccccgtttc ttg 23
<210> 1129
   <211> 25
   <212> DNA
   <213> Artificial Sequence
<220>
   <223> Synthetic probe
<400> 1129
   atcctcctga agcccttttc gcagc 25
<210> 1130
   <211> 28
   <212> DNA
   <213> Artificial Sequence
<220>
   <223> Synthetic probe
<400> 1130
   tgccttcttc ctccctcact tctcacct 28
<210> 1131
   <211> 30
   <212> DNA
   <213> Artificial Sequence
<220>
   <223> Synthetic probe
<400> 1131
   ccataggaga atgcttccca catcaacact 30
<210> 1132
   <211> 24
   <212> DNA
   <213> Artificial Sequence
<220>
   <223> Synthetic probe
<400> 1132
   cccatagtcc tcagccgcct tcag 24
<210> 1133
   <211> 24
   <212> DNA
   <213> Artificial Sequence
<220>
   <223> Synthetic probe
<400> 1133
   cttccagcgg caatgtaagc aaca 24
<210> 1134
   <211> 28
   <212> DNA
   <213> Artificial Sequence
<220>
   <223> Synthetic probe
<400> 1134
   agggatctga accaatacag agcagaca 28
<210> 1135
   <211> 21
   <212> DNA
   <213> Artificial Sequence
<220>
   <223> Synthetic probe
<400> 1135
   tcctgagccc gaggaagtcc c 21
<210> 1136
   <211> 21
   <212> DNA
   <213> Artificial Sequence
<220>
   <223> Synthetic probe
<400> 1136
   cggccagatg agcacattgc c 21
<210> 1137
   <211> 24
   <212> DNA
   <213> Artificial Sequence
<220>
   <223> Synthetic probe
<400> 1137
   ttctgggctc ctgattgctc aagc 24
<210> 1138
   <211> 24
   <212> DNA
   <213> Artificial Sequence
<220>
   <223> Synthetic probe
<400> 1138
   ccaagaacga gtgtctctgg accg 24
<210> 1139
   <211> 24
   <212> DNA
   <213> Artificial Sequence
<220>
   <223> Synthetic probe
<400> 1139
   tgttcctcac tgagcctgga agca 24
<210> 1140
   <211> 24
   <212> DNA
   <213> Artificial Sequence
<220>
   <223> Synthetic probe
<400> 1140
   agggcctaat gcacgcacta aagc 24
<210> 1141
   <211> 24
   <212> DNA
   <213> Artificial Sequence
<220>
   <223> Synthetic probe
<400> 1141
   tccaccatcg ctttctctgc tctg 24
<210> 1142
   <211> 21
   <212> DNA
   <213> Artificial Sequence
<220>
   <223> Synthetic probe
<400> 1142
   ccacgctgcc ctcggacaag c 21
<210> 1143
   <211> 23
   <212> DNA
   <213> Artificial Sequence
<220>
   <223> Synthetic probe
<400> 1143
   aattaacagc cacccctcag gcg 23
<210> 1144
   <211> 26
   <212> DNA
   <213> Artificial Sequence
<220>
   <223> Synthetic probe
<400> 1144
   caggcacaca caggtgggac acaaat 26
<210> 1145
   <211> 22
   <212> DNA
   <213> Artificial Sequence
<220>
   <223> Synthetic probe
<400> 1145
   atttcacgca tctggcgttc ca 22
<210> 1146
   <211> 24
   <212> DNA
   <213> Artificial Sequence
<220>
   <223> Synthetic probe
<400> 1146
   tggacactgt ggaccctccc tacc 24
<210> 1147
   <211> 24
   <212> DNA
   <213> Artificial Sequence
<220>
   <223> Synthetic probe
<400> 1147
   ccagaacaga tgcgagcagt ccat 24
<210> 1148
   <211> 20
   <212> DNA
   <213> Artificial Sequence
<220>
   <223> Synthetic probe
<400> 1148
   cgggctgcat cagcacacgc 20
<210> 1149
   <211> 24
   <212> DNA
   <213> Artificial Sequence
<220>
   <223> Synthetic probe
<400> 1149
   ttgatgcctg tcttcgcgcc ttct 24
<210> 1150
   <211> 24
   <212> DNA
   <213> Artificial Sequence
<220>
   <223> Synthetic probe
<400> 1150
   ttccgtaaga ggcctggtgc tctc 24
<210> 1151
   <211> 27
   <212> DNA
   <213> Artificial Sequence
<220>
   <223> Synthetic probe
<400> 1151
   ctgctgttta ccttggcgag gcctttc 27
<210> 1152
   <211> 24
   <212> DNA
   <213> Artificial Sequence
<220>
   <223> Synthetic probe
<400> 1152
   cccctccttc tcctgcttca gctt 24
<210> 1153
   <211> 78
   <212> DNA
   <213> Artificial Sequence
<220>
   <223> Synthetic oligonucleotide
<400> 1153
   cgttccgatc ctctatactg catcccaggc atgcctacag caccctgatg tcgcagccta 60
   taaggccaac agggacct                                               78
<210> 1154
   <211> 66
   <212> DNA
   <213> Artificial Sequence
<220>
   <223> Synthetic oligonucleotide
<400> 1154
   gtgtggcagg tggacactaa ggaggaggtc tggtcctttg aagcgggaga cctggagtgg 60
   atggag                                                            66
<210> 1155
   <211> 77
   <212> DNA
   <213> Artificial Sequence
<220>
   <223> Synthetic oligonucleotide
<400> 1155
   aaacaccact ggagcattga ctaccaggct cgccaatgat gctgctcaag ttaaaggggc 60
   tataggttcc aggcttg                                                77
<210> 1156
   <211> 68
   <212> DNA
   <213> Artificial Sequence
<220>
   <223> Synthetic oligonucleotide
<400> 1156
   accagtgcca caatgcagtg gctggacatt cggctacagc tcatgggggc ggcagtggtc 60
   agcgctat                                                          68
<210> 1157
   <211> 76
   <212> DNA
   <213> Artificial Sequence
<220>
   <223> Synthetic oligonucleotide
<400> 1157
   tgcagactgt accatgctga ccattgccca tcgcctgcac acggttctag gctccgatag 60
   gattatggtg ctggcc                                                 76
<210> 1158
   <211> 67
   <212> DNA
   <213> Artificial Sequence
<220>
   <223> Synthetic oligonucleotide
<400> 1158
   acacgtctgt caccatggaa gctctgctct acaagcccat tgaccgggtc actcggagca 60
   ccctagt                                                           67
<210> 1159
   <211> 66
   <212> DNA
   <213> Artificial Sequence
<220>
   <223> Synthetic oligonucleotide
<400> 1159
   atccgcattg aagacccacc ccgcagaaag cacatggtat tcctgggtgg tgcagttcta 60
   gcggat                                                            66
<210> 1160
   <211> 74
   <212> DNA
   <213> Artificial Sequence
<220>
   <223> Synthetic oligonucleotide
<400> 1160
   gactgtctcg tttccctggt gacctctgtc accaatgtgg acctgccccc taaagagtca 60
   agcatctaag ccca                                                   74
<210> 1161
   <211> 74
   <212> DNA
   <213> Artificial Sequence
<220>
   <223> Synthetic oligonucleotide
<400> 1161
   tcaaaagtac ggacacctcc tgtcagatgg cgggactaag agatacctac aaggattcca 60
   tcaaagcatt tgca                                                   74
<210> 1162
   <211> 66
   <212> DNA
   <213> Artificial Sequence
<220>
   <223> Synthetic oligonucleotide
<400> 1162
   gagcatgcgt ctactgcctc actgacactc atctgagccc tcccatgaca tggagaccgt 60
   gaccag                                                            66
<210> 1163
   <211> 73
   <212> DNA
   <213> Artificial Sequence
<220>
   <223> Synthetic oligonucleotide
<400> 1163
   gaagtgccag gaggcgatta atgctacttg caaaggcgtg tcctactgca caggtaatag 60
   cagtgagtgc ccg                                                    73
<210> 1164
   <211> 62
   <212> DNA
   <213> Artificial Sequence
<220>
   <223> Synthetic oligonucleotide
<400> 1164
   caaggcccca tctgaatcag ctgcgctgga tggacaccgc cttgcactgt tggattctgg 60
   gt                                                                62
<210> 1165
   <211> 72
   <212> DNA
   <213> Artificial Sequence
<220>
   <223> Synthetic oligonucleotide
<400> 1165
   gcgagttcaa agtgttcgag gccaaggtga ttgatggcac cctgtgtggg ccagaaacac 60
   tggccatctg tg                                                     72
<210> 1166
   <211> 75
   <212> DNA
   <213> Artificial Sequence
<220>
   <223> Synthetic oligonucleotide
<400> 1166
   taagccacaa gcacacgggg ctccagcccc cccgagtgga agtgctcccc actttcttta 60
   ggatttaggc gccca                                                  75
<210> 1167
   <211> 78
   <212> DNA
   <213> Artificial Sequence
<220>
   <223> Synthetic oligonucleotide
<400> 1167
   acgagatgtc ctacggcttg aacatcgaga tgcacaaaca ggctgagatc gtcaaaaggc 60
   tgaacgggat ttgtgccc                                               78
<210> 1168
   <211> 70
   <212> DNA
   <213> Artificial Sequence
<220>
   <223> Synthetic oligonucleotide
<400> 1168
   agccaacatg tgactaattg gaagaagagc aaagggtggt gacgtgttga tgaggcagat 60
   ggagatcaga 70
<210> 1169
   <211> 78
   <212> DNA
   <213> Artificial Sequence
<220>
   <223> Synthetic oligonucleotide
<400> 1169
   ctgcatgtga ttgaataaga aacaagaaag tgaccacacc aaagcctccc tggctggtgt 60
   acagggatca ggtccaca 78
<210> 1170
   <211> 67
   <212> DNA
   <213> Artificial Sequence
<220>
   <223> Synthetic oligonucleotide
<400> 1170
   gtggaaacgg agctcttccc ctgcctcagg cactttggac tgaggttcta tgccttcaac 60
   cctctgg 67
<210> 1171
   <211> 75
   <212> DNA
   <213> Artificial Sequence
<220>
   <223> Synthetic oligonucleotide
<400> 1171
   ttgtctctgc cttggactat ctacattccg gaaagattgt gtaccgtgat ctcaagttgg 60
   agaatctaat gctgg 75
<210> 1172
   <211> 66
   <212> DNA
   <213> Artificial Sequence
<220>
   <223> Synthetic oligonucleotide
<400> 1172
   gaggaatatg gaatccaagg gggccagttc ctgccgtctg ctcttctgcc tcttgatctc 60
   cgccac 66
<210> 1173
   <211> 68
   <212> DNA
   <213> Artificial Sequence
<220>
   <223> Synthetic oligonucleotide
<400> 1173
   ggacagggta agaccgtgat ccaagcggag attgacgctg cagcggaact catcgacttc 60
   ttccggtt 68
<210> 1174
   <211> 69
   <212> DNA
   <213> Artificial Sequence
<220>
   <223> Synthetic oligonucleotide
<400> 1174
   ccgtgaaagc tgctctgtaa aagctgacac agccctccca agtgagcagg actgttcttc 60
   ccactgcaa 69
<210> 1175
   <211> 71
   <212> DNA
   <213> Artificial Sequence
<220>
   <223> Synthetic oligonucleotide
<400> 1175
   caagacacta agggcgacta ccagaaagcg ctgctgtacc tgtgtggtgg agatgactga 60
   agcccgacac g 71
<210> 1176
   <211> 81
   <212> DNA
   <213> Artificial Sequence
<220>
   <223> Synthetic oligonucleotide
<400> 1176
   gactgcaaag atggaaacga ccttctatga cgatgccctc aacgcctcgt tcctcccgtc 60
   cgagagcgga ccttatggct a                                            81
<210> 1177
   <211> 68
   <212> DNA
   <213> Artificial Sequence
<220>
   <223> Synthetic oligonucleotide
<400> 1177
   gatgaagcct ttcgcaagtt cctgaagggc ctggcttccc gaaagcccct tgtgctgtgt 60
   ggagacct                                                           68
<210> 1178
   <211> 67
   <212> DNA
   <213> Artificial Sequence
<220>
   <223> Synthetic oligonucleotide
<400> 1178
   gtttatgcca tcggcaccgt actggatcct ggccaccgac tatgagaact atgccctcgt 60
   gtattcc                                                            67
<210> 1179
   <211> 64
   <212> DNA
   <213> Artificial Sequence
<220>
   <223> Synthetic oligonucleotide
<400> 1179
   cagtagagat ccccgcaact cgcttgtcct tgggtcaccc tgcattccat agccatgtgc 60
   ttgt                                                               64
<210> 1180
   <211> 67
   <212> DNA
   <213> Artificial Sequence
<220>
   <223> Synthetic oligonucleotide
<400> 1180
   atcagagatt accgcgtcgt ggtagtcggc accgctggtg tggggaaaag tacgctgctg 60
   cacaagt 67
<210> 1181
   <211> 68
   <212> DNA
   <213> Artificial Sequence
<220>
   <223> Synthetic oligonucleotide
<400> 1181
   gactgggtca gtgatggcaa caggatggcc aaggatggct ctagaacact ctgtccatgc 60
   gtcactcc 68
<210> 1182
   <211> 79
   <212> DNA
   <213> Artificial Sequence
<220>
   <223> Synthetic oligonucleotide
<400> 1182
   tccctgagaa cgaaaccact tttgcaagaa tcttgcagca gcatggctat gcaaccggcc 60
   tcataggaaa atggcacca 79
<210> 1183
   <211> 67
   <212> DNA
   <213> Artificial Sequence
<220>
   <223> Synthetic oligonucleotide
<400> 1183
   agctgcagaa gagctgcaca tttgacgagc agcgaacagc cacgatcatg gaggagttgg 60
   cagatgc 67
<210> 1184
   <211> 66
   <212> DNA
   <213> Artificial Sequence
<220>
   <223> Synthetic oligonucleotide
<400> 1184
   cagcagatgt ggatcagcaa gcaggagtat gacgagtccg gcccctccat cgtccaccgc 60
   aaatgc 66
<210> 1185
   <211> 80
   <212> DNA
   <213> Artificial Sequence
<220>
   <223> Synthetic oligonucleotide
<400> 1185
   ggctcttgtg cgtactgtcc ttcgggctgg tgacagggaa gacatcactg agcctgccat 60
   ctgtgctctt cgtcatctga 80
<210> 1186
   <211> 73
   <212> DNA
   <213> Artificial Sequence
<220>
   <223> Synthetic oligonucleotide
<400> 1186
   gggtcaggtg cctcgagatc gggcttgggc ccagagcatg ttccagatcc cagagtttga 60
   gccgagtgag cag 73
<210> 1187
   <211> 81
   <212> DNA
   <213> Artificial Sequence
<220>
   <223> Synthetic oligonucleotide
<400> 1187
   cgttgtcagc acttggaata caagatggtt gccgggtcat gttaattggg aaaaagaaca 60
   gtccacagga agaggttgaa c 81
<210> 1188
   <211> 76
   <212> DNA
   <213> Artificial Sequence
<220>
   <223> Synthetic oligonucleotide
<400> 1188
   cctacggccg ctactacggg cctgggggtg gagatgtgcc ggtacaccca cctccaccct 60
   tatatcctct tcgccc 76
<210> 1189
   <211> 72
   <212> DNA
   <213> Artificial Sequence
<220>
   <223> Synthetic oligonucleotide
<400> 1189
   gactcctcag ggcagacttt cttcccagcc tgcagacaac tggcctccag aaaccattga 60
   gtagtgcctt cg 72
<210> 1190
   <211> 70
   <212> DNA
   <213> Artificial Sequence
<220>
   <223> Synthetic oligonucleotide
<400> 1190
   ccgccgtgga cacagactcc ccccgagagg tctttttccg agtggcagct gacatgtttt 60
   ctgacggcaa 70
<210> 1191
   <211> 83
   <212> DNA
   <213> Artificial Sequence
<220>
   <223> Synthetic oligonucleotide
<400> 1191
   cctggagggt cctgtacaat ctcatcatgg gactcctgcc cttacccagg ggccacagag 60
   cccccgagat ggagcccaat tag 83
<210> 1192
   <211> 65
   <212> DNA
   <213> Artificial Sequence
<220>
   <223> Synthetic oligonucleotide
<400> 1192
   actgacaaga ccagcagcat ccagtcacga cccctgccct caccccctaa gttcacctcc 60
   cagga                                                             65
<210> 1193
   <211> 75
   <212> DNA
   <213> Artificial Sequence
<220>
   <223> Synthetic oligonucleotide
<400> 1193
   tgacttccta gttcgtgact ctctgtccag ccctgggaac tttgtcctga cctgtcagtg 60
   gaagaacctc gctca                                                  75
<210> 1194
   <211> 73
   <212> DNA
   <213> Artificial Sequence
<220>
   <223> Synthetic oligonucleotide
<400> 1194
   ccccgagaca acggagataa gtgctgttgc ggatgccaac ggaaagaatc ttgggaaaga 60
   ggccaaaccc gag                                                    73
<210> 1195
   <211> 73
   <212> DNA
   <213> Artificial Sequence
<220>
   <223> Synthetic oligonucleotide
<400> 1195
   cagatggacc tagtacccac tgagatttcc acgccgaagg acagcgatgg gaaaaatgcc 60
   cttaaatcat agg                                                    73
<210> 1196
   <211> 73
   <212> DNA
   <213> Artificial Sequence
<220>
   <223> Synthetic oligonucleotide
<400> 1196
   aacccacccc tgtcttggag ctccgggtag ctctcaaact cgaggctgcg cacccccttt 60
   cccgtcagct gag                                                    73
<210> 1197
   <211> 66
   <212> DNA
   <213> Artificial Sequence
<220>
   <223> Synthetic oligonucleotide
<400> 1197
   gagctccgca aggatgactt caagggtctc cagcacctct acgccctcgt cctggtgaac 60
   aacaag                                                            66
<210> 1198
   <211> 70
   <212> DNA
   <213> Artificial Sequence
<220>
   <223> Synthetic oligonucleotide
<400> 1198
   attcctatgg ctctgcaatt gtcaccggtt aactgtggcc tgtgcccagg aagagccatt 60
   cactcctgcc                                                        70
<210> 1199
   <211> 68
   <212> DNA
   <213> Artificial Sequence
<220>
   <223> Synthetic oligonucleotide
<400> 1199
   ctggacggag tagctccaag agctctcact gtgacagccc acctcgctcg cagacaccac 60
   aagatacc                                                          68
<210> 1200
   <211> 66
   <212> DNA
   <213> Artificial Sequence
<220>
   <223> Synthetic oligonucleotide
<400> 1200
   aattttatga gggccacggg tctgtgttcg actcagcctc agggacgact ctgacctctt 60
   ggccac 66
<210> 1201
   <211> 63
   <212> DNA
   <213> Artificial Sequence
<220>
   <223> Synthetic oligonucleotide
<400> 1201
   gttgggacac agttggtctg cagtcggccc aggacggtct actcagcaca actgactgct 60
   tca 63
<210> 1202
   <211> 68
   <212> DNA
   <213> Artificial Sequence
<220>
   <223> Synthetic oligonucleotide
<400> 1202
   ccgaggttaa tccagcacgt atggggccaa gtgtaggctc ccagcaggaa ctgagagcgc 60
   catgtctt 68
<210> 1203
   <211> 82
   <212> DNA
   <213> Artificial Sequence
<220>
   <223> Synthetic oligonucleotide
<400> 1203
   tcaacagaag gctgaaccac tagaaagact acagtcccag caccgacaat tccaagctcg 60
   agtgtctcgg caaactctgt tg 82
<210> 1204
   <211> 73
   <212> DNA
   <213> Artificial Sequence
<220>
   <223> Synthetic oligonucleotide
<400> 1204
   ctgaagcaga tggttcatca tttcctgggc tgttaaacaa agcgaggtta aggttagact 60
   cttgggaatc agc 73
<210> 1205
   <211> 75
   <212> DNA
   <213> Artificial Sequence
<220>
   <223> Synthetic oligonucleotide
<400> 1205
   gaggcaactg cttatggctt aattaagtca gatgcggcca tgactgtcgc tgtaaagatg 60
   ctcaagccga gtgcc                                                  75
<210> 1206
   <211> 67
   <212> DNA
   <213> Artificial Sequence
<220>
   <223> Synthetic oligonucleotide
<400> 1206
   caagagcaga gccaccgtag ccggagtcct agcctcccaa attcggaaat ccaatccaac 60
   ggtctca                                                           67
<210> 1207
   <211> 67
   <212> DNA
   <213> Artificial Sequence
<220>
   <223> Synthetic oligonucleotide
<400> 1207
   gtgactgcac aggactctgg gttcctgctc tgttctgggg tccaaacctt ggtctccctt 60
   tggtcct                                                           67
<210> 1208
   <211> 65
   <212> DNA
   <213> Artificial Sequence
<220>
   <223> Synthetic oligonucleotide
<400> 1208
   tcagctgtga gctgcggata ccgcccggca atgggacctg ctcttaacct caaacctagg 60
   accgt                                                             65
<210> 1209
   <211> 70
   <212> DNA
   <213> Artificial Sequence
<220>
   <223> Synthetic oligonucleotide
<400> 1209
   gcggtatcag gaatttcaac ctagagaacc gagcggaacg ggaaatcagc aagatgaagc 60
   cctctgtcgc 70
<210> 1210
   <211> 67
   <212> DNA
   <213> Artificial Sequence
<220>
   <223> Synthetic oligonucleotide
<400> 1210
   ctacgagtca gcccatccat ccatggctac cacttcgaca cagcctctcg taagaaagcc 60
   gtgggca 67
<210> 1211
   <211> 67
   <212> DNA
   <213> Artificial Sequence
<220>
   <223> Synthetic oligonucleotide
<400> 1211
   tgatgctgca gagaacttcc agaaagcaca ccgctggcag gacaacatca aggaggaaga 60
   catcgtg 67
<210> 1212
   <211> 78
   <212> DNA
   <213> Artificial Sequence
<220>
   <223> Synthetic oligonucleotide
<400> 1212
   atccattcga tctcaccaag gtttggcctc acaaggacta ccctctcatc ccagttggta 60
   aactggtctt aaaccgga 78
<210> 1213
   <211> 74
   <212> DNA
   <213> Artificial Sequence
<220>
   <223> Synthetic oligonucleotide
<400> 1213
   gtggctcaac attgtgttcc catttcagct gatcagtggg cctccaagga ggggctgtaa 60
   aatggaggcc attg                                                   74
<210> 1214
   <211> 78
   <212> DNA
   <213> Artificial Sequence
<220>
   <223> Synthetic oligonucleotide
<400> 1214
   aggggatggt ctctgtcatt tctctttgta cataatacat tcacctccct gcctcctctc 60
   ctttctaccc accccttt                                              78
<210> 1215
   <211> 78
   <212> DNA
   <213> Artificial Sequence
<220>
   <223> Synthetic oligonucleotide
<400> 1215
   gaacgcatca tccagagact gcagaggacc tcagccaaga tgaagcgccg cagcagttaa 60
   cctatgaccg tgcagagg                                               78
<210> 1216
   <211> 77
   <212> DNA
   <213> Artificial Sequence
<220>
   <223> Synthetic oligonucleotide
<400> 1216
   agcagacagt ggtcagtcct ttcttggctc tgctgacact cgagcccaca ttccgtcacc 60
   tgctcagaat catgcag                                                77
<210> 1217
   <211> 65
   <212> DNA
   <213> Artificial Sequence
<220>
   <223> Synthetic oligonucleotide
<400> 1217
   aggttctgag ctctggcttt gccttggctt tgccagggct ctgtgaccag gaaggaagtc 60
   agcat                                                             65
<210> 1218
   <211> 84
   <212> DNA
   <213> Artificial Sequence
<220>
   <223> Synthetic oligonucleotide
<400> 1218
   ttcaggttgt tgcaggagac catgtacatg actgtctcca ttattgatcg gttcatgcag 60
   aataattgtg tgcccaagaa gatg                                        84
<210> 1219
   <211> 76
   <212> DNA
   <213> Artificial Sequence
<220>
   <223> Synthetic oligonucleotide
<400> 1219
   cctctgtgct acagattata cctttgccat gtacccgcca tccatgatcg ccacgggcag 60
   cattggggct gcagtg                                                 76
<210> 1220
   <211> 85
   <212> DNA
   <213> Artificial Sequence
<220>
   <223> Synthetic oligonucleotide
<400> 1220
   ggtcaccaag aaacatcagt atgaaattag gaattgttgg ccacctgtat tatctggggg 60
   gatcagtcct tgcattatca ttgaa                                       85
<210> 1221
   <211> 67
   <212> DNA
   <213> Artificial Sequence
<220>
   <223> Synthetic oligonucleotide
<400> 1221
   cagactgaat gggggtgggg ggggcgcctt aggtacttat tccagatgcc ttctccagac 60
   aaaccag 67
<210> 1222
   <211> 64
   <212> DNA
   <213> Artificial Sequence
<220>
   <223> Synthetic oligonucleotide
<400> 1222
   ggatgacatg cactcagctc ttggctccac tgggatggga ggagaggaca agggaaatgt 60
   cagg 64
<210> 1223
   <211> 78
   <212> DNA
   <213> Artificial Sequence
<220>
   <223> Synthetic oligonucleotide
<400> 1223
   ggagtggaag gaactggaaa cattattccg tatacgcacc attcggtcat ttgagggaat 60
   tcgtagatac gcccatga 78
<210> 1224
   <211> 77
   <212> DNA
   <213> Artificial Sequence
<220>
   <223> Synthetic oligonucleotide
<400> 1224
   tccaactaat gccaccacca aggcggctgg tggtgccctg cagtcaacag ccagtctctt 60
   cgtggtctca ctctctc 77
<210> 1225
   <211> 67
   <212> DNA
   <213> Artificial Sequence
<220>
   <223> Synthetic oligonucleotide
<400> 1225
   gtgctggagt cgggactaac ccaggtccct tgtcccaagt tccactgctg cctcttgaat 60
   gcaggga 67
<210> 1226
   <211> 90
   <212> DNA
   <213> Artificial Sequence
<220>
   <223> Synthetic oligonucleotide
<400> 1226
   atcaccgaca gcacagacag aatccctgct accaatatgg actccagtca tagtacaacg 60
   cttcagccta ctgcaaatcc aaacacaggt 90
<210> 1227
   <211> 78
   <212> DNA
   <213> Artificial Sequence
<220>
   <223> Synthetic oligonucleotide
<400> 1227
   gacgaagaca gtccctggat caccgacagc acagacagaa tccctgctac cagagaccaa 60
   gacacattcc accccagt 78
<210> 1228
   <211> 78
   <212> DNA
   <213> Artificial Sequence
<220>
   <223> Synthetic oligonucleotide
<400> 1228
   ctcataccag ccatccaatg caaggaagga caacaccaag cccagaggac agttcctgga 60
   ctgatttctt caacccaa 78
<210> 1229
   <211> 74
   <212> DNA
   <213> Artificial Sequence
<220>
   <223> Synthetic oligonucleotide
<400> 1229
   tggttcccag ccctgtgtcc acctccaagc ccagattcag attcgagtca tgtacacaac 60
   ccagggtgga ggag 74
<210> 1230
   <211> 84
   <212> DNA
   <213> Artificial Sequence
<220>
   <223> Synthetic oligonucleotide
<400> 1230
   gtgcaggctc aggtgaagtg ctgcggctgg gtcagcttct acaactggac agacaacgct 60
   gagctcatga atcgccctga ggtc 84
<210> 1231
   <211> 68
   <212> DNA
   <213> Artificial Sequence
<220>
   <223> Synthetic oligonucleotide
<400> 1231
   tggattggag ttctgggaat gtactggccg tggcactgga caacagtgtg tacctgtgga 60
   gtgcaagc 68
<210> 1232
   <211> 71
   <212> DNA
   <213> Artificial Sequence
<220>
   <223> Synthetic oligonucleotide
<400> 1232
   tcttgctggc tacgcctctt ctgtccctgt tagacgtcct ccgtccatat cagaactgtg 60
   ccacaatgca g 71
<210> 1233
   <211> 67
   <212> DNA
   <213> Artificial Sequence
<220>
   <223> Synthetic oligonucleotide
<400> 1233
   ggtgagcaga agtggcctat atcgctcccc gtcgatgcca gagaacttga acaggccaag 60
   actgaag 67
<210> 1234
   <211> 77
   <212> DNA
   <213> Artificial Sequence
<220>
   <223> Synthetic oligonucleotide
<400> 1234
   gcagtccgct gtgttcaata tgatggcagg agggttgtta gtggagcata tgattttatg 60
   gtaaaggtgt gggatcc 77
<210> 1235
   <211> 67
   <212> DNA
   <213> Artificial Sequence
<220>
   <223> Synthetic oligonucleotide
<400> 1235
   gagctgaaag acgcacactg tcagaggaaa ctggccatgc aggaattcat ggagatcaat 60
   gagcggc 67
<210> 1236
   <211> 67
   <212> DNA
   <213> Artificial Sequence
<220>
   <223> Synthetic oligonucleotide
<400> 1236
   cggagaaggg caccagtaag ctgcccaaga gcctgtcatc cagccccgtg aagaaggcca 60
   atgacgg 67
<210> 1237
   <211> 70
   <212> DNA
   <213> Artificial Sequence
<220>
   <223> Synthetic oligonucleotide
<400> 1237
   gtcggcagaa gcaggacttg taccttctgc ccatagtgat cagcgatggc ggcatcccgc 60
   ccatgagtag                                                        70
<210> 1238
   <211> 71
   <212> DNA
   <213> Artificial Sequence
<220>
   <223> Synthetic oligonucleotide
<400> 1238
   acccatgtac cgtcctcggc cagccaaccc agatgaaatc ggcaacttta taattgagaa 60
   cctgaaggcg g                                                      71
<210> 1239
   <211> 66
   <212> DNA
   <213> Artificial Sequence
<220>
   <223> Synthetic oligonucleotide
<400> 1239
   ccttcccatc agcacagttc gtgaggtggc tttactgagg cgactggagg cttttgagca 60
   tcccaa                                                            66
<210> 1240
   <211> 67
   <212> DNA
   <213> Artificial Sequence
<220>
   <223> Synthetic oligonucleotide
<400> 1240
   aagccctatc cgatgtaccc ggccacaaca tccctggtga acgtcgtgcc caaactcaat 60
   gccacag                                                           67
<210> 1241
   <211> 70
   <212> DNA
   <213> Artificial Sequence
<220>
   <223> Synthetic oligonucleotide
<400> 1241
   tggatctcta ccagcaatgt ggaattatca cccatcatca tccaatcgca gatggaggga 60
   ctcctgacat                                                        70
<210> 1242
   <211> 71
   <212> DNA
   <213> Artificial Sequence
<220>
   <223> Synthetic oligonucleotide
<400> 1242
   acttgcctgt tcagagcact cattccttcc cacccccagt cctgtcctat cactctaatt 60
   cggatttgcc a                                                      71
<210> 1243
   <211> 66
   <212> DNA
   <213> Artificial Sequence
<220>
   <223> Synthetic oligonucleotide
<400> 1243
   ttggttttgc tcggatactt gccaaaatga gactctccgt cggcagctgg gggaagggtc 60
   tgagac                                                            66
<210> 1244
   <211> 77
   <212> DNA
   <213> Artificial Sequence
<220>
   <223> Synthetic oligonucleotide
<400> 1244
   tgacaatcag cacacctgca ttcaccgctc ggaagagggc ctgagctgca tgaataagga 60
   tcacggctgt agtcaca                                                77
<210> 1245
   <211> 63
   <212> DNA
   <213> Artificial Sequence
<220>
   <223> Synthetic oligonucleotide
<400> 1245
   taaattcact cgtggtgtgg acttcaattg gcaagcccag gccctattgg ccctacaaga 60
   ggc                                                               63
<210> 1246
   <211> 76
   <212> DNA
   <213> Artificial Sequence
<220>
   <223> Synthetic oligonucleotide
<400> 1246
   ctgaaggagc tccaagacct cgctctccaa ggcgccaagg agagggcaca tcagcagaag 60
   aaacacagcg gttttg                                                 76
<210> 1247
   <211> 69
   <212> DNA
   <213> Artificial Sequence
<220>
   <223> Synthetic oligonucleotide
<400> 1247
   ccagaatgca cgctacagga aaacccattc ttctcccagc cgggtgcccc aatacttcag 60
   tgcatgggc                                                         69
<210> 1248
   <211> 80
   <212> DNA
   <213> Artificial Sequence
<220>
   <223> Synthetic oligonucleotide
<400> 1248
   ccaccatagg cagaggcagg ccttcctaca ccctactccc tgtgcctcca gcctcgacta 60
   gtccctagca ctcgacgact                                             80
<210> 1249
   <211> 72
   <212> DNA
   <213> Artificial Sequence
<220>
   <223> Synthetic oligonucleotide
<400> 1249
   gaggccagtg gtggaaacag gtgtggagct gatgagtctg ccctaccgcc tggtgtttgc 60
   tgtggcctcg ga                                                     72
<210> 1250
   <211> 76
   <212> DNA
   <213> Artificial Sequence
<220>
   <223> Synthetic oligonucleotide
<400> 1250
   aaggaagtgg tccctctgtg gcaagtgatg aagtctccag ctttgcctca gctctcccag 60
   acagaaagac tgcgtc 76
<210> 1251
   <211> 66
   <212> DNA
   <213> Artificial Sequence
<220>
   <223> Synthetic oligonucleotide
<400> 1251
   agaatgggtg tgaaggcgtc tcaaacaggc tttgtggtcc tggtgctgct ccagtgctgc 60
   tctgca 66
<210> 1252
   <211> 62
   <212> DNA
   <213> Artificial Sequence
<220>
   <223> Synthetic oligonucleotide
<400> 1252
   ggctggacgt ggttttgtct gctgcgcccg ctcttcgcgc tctcgtttca ttttctgcag 60
   cg 62
<210> 1253
   <211> 72
   <212> DNA
   <213> Artificial Sequence
<220>
   <223> Synthetic oligonucleotide
<400> 1253
   ggctgctttg ctgcaactgt ccaccccgca cagacaagcc ttactccgcc aagtattctg 60
   ctgcccgctc tg 72
<210> 1254
   <211> 68
   <212> DNA
   <213> Artificial Sequence
<220>
   <223> Synthetic oligonucleotide
<400> 1254
   cggtacttga gcaatgccta cgcccgggaa gaattcgctt ccacctgtcc agatgatgag 60
   gagatcga 68
<210> 1255
   <211> 76
   <212> DNA
   <213> Artificial Sequence
<220>
   <223> Synthetic oligonucleotide
<400> 1255
   ccccaggata cctaccacta cctgcccttc agcctgcccc accggaggcc tcacttcttc 60
   tttcccaagt cccgca 76
<210> 1256
   <211> 67
   <212> DNA
   <213> Artificial Sequence
<220>
   <223> Synthetic oligonucleotide
<400> 1256
   aaatcgcagc ttatcacaag gcactcagtt accgagccac gtcacgccaa caacaggggt 60
   accaaca 67
<210> 1257
   <211> 68
   <212> DNA
   <213> Artificial Sequence
<220>
   <223> Synthetic oligonucleotide
<400> 1257
   gtggccatcc agctgacctt cctgcgcctg atgtccaccg aggcctccca gaacatcacc 60
   taccactg 68
<210> 1258
   <211> 80
   <212> DNA
   <213> Artificial Sequence
<220>
   <223> Synthetic oligonucleotide
<400> 1258
   cagccaagaa ctggtatagg agctccaagg acaagaaaca cgtctggcta ggagaaacta 60
   tcaatgctgg cagccagttt                                              80
<210> 1259
   <211> 67
   <212> DNA
   <213> Artificial Sequence
<220>
   <223> Synthetic oligonucleotide
<400> 1259
   ccttatcggc tggaacgagt tcatcctgca gcccatccac aacctgctca tgggtgacac 60
   caaggag                                                            67
<210> 1260
   <211> 81
   <212> DNA
   <213> Artificial Sequence
<220>
   <223> Synthetic oligonucleotide
<400> 1260
   cgacagttgc gatgaaagtt ctaatctctt ccctcctcct gttgctgcca ctaatgctga 60
   tgtccatggt ctctagcagc c                                            81
<210> 1261
   <211> 72
   <212> DNA
   <213> Artificial Sequence
<220>
   <223> Synthetic oligonucleotide
<400> 1261
   atgcccagtg ttcctgactt cgaaacgcta ttctcacagg ttcagctctt catcagcact 60
   tgtaatgggg ag                                                      72
<210> 1262
   <211> 69
   <212> DNA
   <213> Artificial Sequence
<220>
   <223> Synthetic oligonucleotide
<400> 1262
   gatgtgattg aggtgcatgg aaaacatgaa gagcgccagg atgaacatgg tttcatctcc 60
   agggagttc                                                         69
<210> 1263
   <211> 78
   <212> DNA
   <213> Artificial Sequence
<220>
   <223> Synthetic oligonucleotide
<400> 1263
   aagtcctgaa attgcgatca gatattgcag taccgattcc aaaagaccat caggttctaa 60
   tcaaggtcca tgcatgtg                                               78
<210> 1264
   <211> 68
   <212> DNA
   <213> Artificial Sequence
<220>
   <223> Synthetic oligonucleotide
<400> 1264
   cagcaagaac tgcaacaaca gctttgctga atgctccagc caaggccatg agaggcagtc 60
   cgagggat                                                          68
<210> 1265
   <211> 74
   <212> DNA
   <213> Artificial Sequence
<220>
   <223> Synthetic oligonucleotide
<400> 1265
   tgcagcggct gattgacagt cagatggaga cctcgtgcca aattacattt gagtttgtag 60
   accaggaaca gttg                                                   74
<210> 1266
   <211> 80
   <212> DNA
   <213> Artificial Sequence
<220>
   <223> Synthetic oligonucleotide
<400> 1266
   gagcacaacc aaacctacga gtgcagggcc cacaacagcg tggggagtgg ctcctgggcc 60
   ttcataccca tctctgcagg                                             80
<210> 1267
   <211> 67
   <212> DNA
   <213> Artificial Sequence
<220>
   <223> Synthetic oligonucleotide
<400> 1267
   taccacaccc agcattcctc ctgatcccag agaaatcgga tctgcgaaca gtggcaccag 60
   cctctag                                                           67
<210> 1268
   <211> 64
   <212> DNA
   <213> Artificial Sequence
<220>
   <223> Synthetic oligonucleotide
<400> 1268
   cctgaacatg aaggagctga agctgctgca gaccatcggg aagggggagt tcggagacgt 60
   gatg                                                              64
<210> 1269
   <211> 76
   <212> DNA
   <213> Artificial Sequence
<220>
   <223> Synthetic oligonucleotide
<400> 1269
   gagttcaagt gccctgacgg cgaggtcatg aagaagaaca tgatgttcat caagacctgt 60
   gcctgccatt acaact                                                 76
<210> 1270
   <211> 67
   <212> DNA
   <213> Artificial Sequence
<220>
   <223> Synthetic oligonucleotide
<400> 1270
   gctcacttcg gctaaaatgc agaaatgcat gctgtcagcg ttggtatttc acattcaatg 60
   gagctga                                                           67
<210> 1271
   <211> 80
   <212> DNA
   <213> Artificial Sequence
<220>
   <223> Synthetic oligonucleotide
<400> 1271
   gtacatgatc ccctgtgaga aggtgtccac cctgcccgcg atcacactga agctgggagg 60
   caaaggctac aagctgtccc                                             80
<210> 1272
   <211> 67
   <212> DNA
   <213> Artificial Sequence
<220>
   <223> Synthetic oligonucleotide
<400> 1272
   tgtctcactg agcgagcaga atctggtgga ctgttcgcgt cctcaaggca atcagggctg 60
   caatggt                                                           67
<210> 1273
   <211> 79
   <212> DNA
   <213> Artificial Sequence
<220>
   <223> Synthetic oligonucleotide
<400> 1273
   accaggcaat aacctaacag cacccattat aggtgcaata tgggcatata tctccattgt 60
   gtcttggctt ggagaacag                                              79
<210> 1274
   <211> 68
   <212> DNA
   <213> Artificial Sequence
<220>
   <223> Synthetic oligonucleotide
<400> 1274
   ggagcaaaat cgatgcagtg cttccaagga tggaccacac agaggctgcc tctcccatca 60
   cttcccta                                                          68
<210> 1275
   <211> 67
   <212> DNA
   <213> Artificial Sequence
<220>
   <223> Synthetic oligonucleotide
<400> 1275
   gagctacaga tgcccatgcc gattcttcga aagccatgtt gccagagcca acgtcaagca 60
   tctcaaa                                                           67
<210> 1276
   <211> 74
   <212> DNA
   <213> Artificial Sequence
<220>
   <223> Synthetic oligonucleotide
<400> 1276
   tgcgcccttt cctctgtaca tataccctta agaacgcccc ctccacacac tgccccccag 60
   tatatgccgc attg                                                   74
<210> 1277
   <211> 72
   <212> DNA
   <213> Artificial Sequence
<220>
   <223> Synthetic oligonucleotide
<400> 1277
   tgaccgcttc taccccaatg acttgtgggt ggttgtgttc cagtttcagc acatcatggt 60
   tggccttatc ct                                                     72
<210> 1278
   <211> 76
   <212> DNA
   <213> Artificial Sequence
<220>
   <223> Synthetic oligonucleotide
<400> 1278
   ccggagtgac tctatcacca acatgctgga cacactgatg caagccaaga tgaactcaga 60
   taatggcaat gctggc                                                 76
<210> 1279
   <211> 70
   <212> DNA
   <213> Artificial Sequence
<220>
   <223> Synthetic oligonucleotide
<400> 1279
   tccttatagg tactttcagc catttggctt tgggccccgt ggctgtgcag gaaagtacat 60
   cgccatggtg                                                        70
<210> 1280
   <211> 71
   <212> DNA
   <213> Artificial Sequence
<220>
   <223> Synthetic oligonucleotide
<400> 1280
   ccagctttgt gcctgtcact attcctcatg ccaccactgc caacacctct gtcttgggct 60
   accacattcc c                                                       71
<210> 1281
   <211> 76
   <212> DNA
   <213> Artificial Sequence
<220>
   <223> Synthetic oligonucleotide
<400> 1281
   tgctcattct tgaggagcat taaggtattt cgaaactgcc aagggtgctg gtgcggatgg 60
   acactaatgc agccac                                                  76
<210> 1282
   <211> 67
   <212> DNA
   <213> Artificial Sequence
<220>
   <223> Synthetic oligonucleotide
<400> 1282
   tggtgggtct aggtggtgta actgtcacac tccctcaggc aggaccatgg aacacagcat 60
   ctttggt                                                            67
<210> 1283
   <211> 75
   <212> DNA
   <213> Artificial Sequence
<220>
   <223> Synthetic oligonucleotide
<400> 1283
   aaatgtcctc ctcgactgct ccgcggagtc cgaccgagga gttccagtga tcaagtggaa 60
   gaaagatggc attca                                                  75
<210> 1284
   <211> 66
   <212> DNA
   <213> Artificial Sequence
<220>
   <223> Synthetic oligonucleotide
<400> 1284
   ggtcaccgtt ggtgtcatca cagtgctggt agtggtcatc gtggctgtga tttgcacccg 60
   acgctc                                                            66
<210> 1285
   <211> 74
   <212> DNA
   <213> Artificial Sequence
<220>
   <223> Synthetic oligonucleotide
<400> 1285
   atggagatgt ggtcattcct agtgattatt ttcagatagt gggaggaagc aacttacgga 60
   tacttggggt ggtg                                                   74
<210> 1286
   <211> 110
   <212> DNA
   <213> Artificial Sequence
<220>
   <223> Synthetic oligonucleotide
<400> 1286
   gccgccacaa gactaaggaa tggccacccc gcccaagaga agctgcccgt ctttctcagc 60
   cagctctgag gggacccgca tcaagaaaat ctccatcgaa gggaacatcg           110
<210> 1287
   <211> 68
   <212> DNA
   <213> Artificial Sequence
<220>
   <223> Synthetic oligonucleotide
<400> 1287
   tccaattcca gcatcactgt ggagaaaagc tgtttgtctc cccagcatac tttatcgcct 60
   tcactgcc                                                          68
<210> 1288
   <211> 68
   <212> DNA
   <213> Artificial Sequence
<220>
   <223> Synthetic oligonucleotide
<400> 1288
   gattcagacg aggatgagcc ttgtgccatc agtggcaaat ggactttcca aagggacagc 60
   aagaggtg                                                          68
<210> 1289
   <211> 67
   <212> DNA
   <213> Artificial Sequence
<220>
   <223> Synthetic oligonucleotide
<400> 1289
   cacggaggta taaggcagga gcctacctgg acatccctgc tcagccccgc ggctggacct 60
   tccttct                                                           67
<210> 1290
   <211> 84
   <212> DNA
   <213> Artificial Sequence
<220>
   <223> Synthetic oligonucleotide
<400> 1290
   ctctgagaca gtgcttcgat gactttgcag acttggtgcc ctttgactcc tgggagccgc 60
   tcatgaggaa gttgggcctc atgg                                        84
<210> 1291
   <211> 73
   <212> DNA
   <213> Artificial Sequence
<220>
   <223> Synthetic oligonucleotide
<400> 1291
   tggcactact gcatgattga catagagaag atcagggcca tgacaatcgc caagctgaaa 60
   acaatgcggc agg                                                    73
<210> 1292
   <211> 78
   <212> DNA
   <213> Artificial Sequence
<220>
   <223> Synthetic oligonucleotide
<400> 1292
   aaatcgctgg gaacaagtgc cgttaattaa ggaaaagttg agccagggcg tgaccctcgt 60
   cgtggacaga tacgcatt                                               78
<210> 1293
   <211> 76
   <212> DNA
   <213> Artificial Sequence
<220>
   <223> Synthetic oligonucleotide
<400> 1293
   agacatcagc tcctggttca acgaggccat tgacttcata gactccatca agaatgctgg 60
   aggaagggtg tttgtc                                                 76
<210> 1294
   <211> 68
   <212> DNA
   <213> Artificial Sequence
<220>
   <223> Synthetic oligonucleotide
<400> 1294
   tggtgacgat ggaggagctg cgggagatgg actgcagtgt gctcaaaagg ctgatgaacc 60
   gggacgag                                                          68
<210> 1295
   <211> 75
   <212> DNA
   <213> Artificial Sequence
<220>
   <223> Synthetic oligonucleotide
<400> 1295
   actccctcta cccttgagca agggcagggg tccctgagct gttcttctgc cccatactga 60
   aggaactgag gcctg 75
<210> 1296
   <211> 76
   <212> DNA
   <213> Artificial Sequence
<220>
   <223> Synthetic oligonucleotide
<400> 1296
   ctgctggatg accttcctcc cagagtggct caccagaagc cccaacctca acaccagcaa 60
   ctggctgtac tgttgg 76
<210> 1297
   <211> 73
   <212> DNA
   <213> Artificial Sequence
<220>
   <223> Synthetic oligonucleotide
<400> 1297
   tgccacctgg acatcatttg ggtcaacact cccgagcacg ttgttccgta tggacttgga 60
   agccctaggt cca 73
<210> 1298
   <211> 79
   <212> DNA
   <213> Artificial Sequence
<220>
   <223> Synthetic oligonucleotide
<400> 1298
   cgacaaggag tgcgtctact tctgccactt ggacatcatc tgggtgaaca ctcctgaaca 60
   gacagctcct tacggcctg 79
<210> 1299
   <211> 76
   <212> DNA
   <213> Artificial Sequence
<220>
   <223> Synthetic oligonucleotide
<400> 1299
   tttcctcaaa tttgcctcaa gatggaaacc ctttgcctca gggcatcctt ttggctggca 60
   ctggttggat gtgtaa                                                 76
<210> 1300
   <211> 72
   <212> DNA
   <213> Artificial Sequence
<220>
   <223> Synthetic oligonucleotide
<400> 1300
   actgtgaact gcctggtgca gtgtccacat gacaaagggg caggtagcac cctctctcac 60
   ccatgctgtg gt                                                     72
<210> 1301
   <211> 67
   <212> DNA
   <213> Artificial Sequence
<220>
   <223> Synthetic oligonucleotide
<400> 1301
   cgagtggaga ctggtgttct caaacccggt atggtggtca cctttgctcc agtcaacgtt 60
   acaacgg                                                           67
<210> 1302
   <211> 66
   <212> DNA
   <213> Artificial Sequence
<220>
   <223> Synthetic oligonucleotide
<400> 1302
   atggactcca cagagccggc ctacagcgag aagcgctacg acgagatcgt caaggaagtc 60
   agcgcc                                                            66
<210> 1303
   <211> 74
   <212> DNA
   <213> Artificial Sequence
<220>
   <223> Synthetic oligonucleotide
<400> 1303
   ttgaacagag cctgaccaag agggatgagt tcgagtttct ggagaaagca tcaaaactgc 60
   gaggaatctc aaca                                                    74
<210> 1304
   <211> 76
   <212> DNA
   <213> Artificial Sequence
<220>
   <223> Synthetic oligonucleotide
<400> 1304
   gtccccgctg cagatctctg acccgttcgg atcctttcct cactcgccca ccatggacaa 60
   ctaccctaag ctggag                                                  76
<210> 1305
   <211> 78
   <212> DNA
   <213> Artificial Sequence
<220>
   <223> Synthetic oligonucleotide
<400> 1305
   ccatgtggat gaatgaggtg tctcctttcc atacccagtc tcaccttctc cccaccctac 60
   ctcacctctt ctcaggca                                                78
<210> 1306
   <211> 66
   <212> DNA
   <213> Artificial Sequence
<220>
   <223> Synthetic oligonucleotide
<400> 1306
   ggcggtgaag agtcacagtt tgagatggac atttaaagca ccagccatcg tgtggagcac 60
   taccaa                                                             66
<210> 1307
   <211> 71
   <212> DNA
   <213> Artificial Sequence
<220>
   <223> Synthetic oligonucleotide
<400> 1307
   tcgagggcaa gaagagcaag cacgcgccca gaggcaccca cctgtgggag ttcatccggg 60
   acatcctcat c                                                       71
<210> 1308
   <211> 75
   <212> DNA
   <213> Artificial Sequence
<220>
   <223> Synthetic oligonucleotide
<400> 1308
   gctagtactt tgatgctccc ttgatggggt ccagagagcc tccctgcagc caccagactt 60
   ggcctccagc tgttc                                                  75
<210> 1309
   <211> 68
   <212> DNA
   <213> Artificial Sequence
<220>
   <223> Synthetic oligonucleotide
<400> 1309
   caaggccgtg aacgagaagt cctgcaactg cctcctgctc aaagtcaacc agattggctc 60
   cgtgaccg                                                          68
<210> 1310
   <211> 75
   <212> DNA
   <213> Artificial Sequence
<220>
   <223> Synthetic oligonucleotide
<400> 1310
   agccccagca actacagtct gggatgccaa ggccagccat gatgtcagtg gcccagcatg 60
   gtcaaccttt gaaca                                                  75
<210> 1311
   <211> 75
   <212> DNA
   <213> Artificial Sequence
<220>
   <223> Synthetic oligonucleotide
<400> 1311
   gggccctcca gaacaatgat gggctttatg atcctgactg cgatgagagc gggctcttta 60
   aggccaagca gtgca                                                  75
<210> 1312
   <211> 72
   <212> DNA
   <213> Artificial Sequence
<220>
   <223> Synthetic oligonucleotide
<400> 1312
   cgcctgttca ccaagattga caccattgcg cccgatgaga tcaccgtcag cagcgacttc 60
   gaggcacgcc ac 72
<210> 1313
   <211> 66
   <212> DNA
   <213> Artificial Sequence
<220>
   <223> Synthetic oligonucleotide
<400> 1313
   caaccaggca gctccatcgg cagtgtccat catgcatcag gtgagccgca ccgtggacag 60
   cattac 66
<210> 1314
   <211> 77
   <212> DNA
   <213> Artificial Sequence
<220>
   <223> Synthetic oligonucleotide
<400> 1314
   tgaacggggt atcctcctta gccacggggc ccgtcccatt tgagcctgtc aatgtcacca 60
   ctgaccgaga ggtacct 77
<210> 1315
   <211> 76
   <212> DNA
   <213> Artificial Sequence
<220>
   <223> Synthetic oligonucleotide
<400> 1315
   tggtccatcg ccagttatca catctgtatg cggaacctca aaagagtccc tggtgtgaag 60
   caagatcgct agaaca 76
<210> 1316
   <211> 86
   <212> DNA
   <213> Artificial Sequence
<220>
   <223> Synthetic oligonucleotide
<400> 1316
   tggctcttaa tcagtttcgt tacctgcctc tggagaattt acgcattatt cgtgggacaa 60
   aactttatga ggatcgatat gccttg 86
<210> 1317
   <211> 67
   <212> DNA
   <213> Artificial Sequence
<220>
   <223> Synthetic oligonucleotide
<400> 1317
   gtccaggtgg atgtgaaaga tccccagcag gccctcaagg agctggctaa gatgtgtatc 60
   ctggccg 67
<210> 1318
   <211> 70
   <212> DNA
   <213> Artificial Sequence
<220>
   <223> Synthetic oligonucleotide
<400> 1318
   ccaacactag gctccccacc agccatatgc cttctcatct gggcacttac tactaaagac 60
   ctggcggagg 70
<210> 1319
   <211> 67
   <212> DNA
   <213> Artificial Sequence
<220>
   <223> Synthetic oligonucleotide
<400> 1319
   ggcattgagc ctctctacat caaggcagag ccggccagcc ctgacagtcc aaagggttcc 60
   tcggaga 67
<210> 1320
   <211> 66
   <212> DNA
   <213> Artificial Sequence
<220>
   <223> Synthetic oligonucleotide
<400> 1320
   gtcactccgc caccgtagaa tcgcctacca tttggtgcaa gcaaaaagca atcagcaatt 60
   ggacag 66
<210> 1321
   <211> 70
   <212> DNA
   <213> Artificial Sequence
<220>
   <223> Synthetic oligonucleotide
<400> 1321
   acccccagac cggatcaggc aagctggccc tcatgtcccc ttcacggtgt ttgaggaagt 60
   ctgccctaca 70
<210> 1322
   <211> 67
   <212> DNA
   <213> Artificial Sequence
<220>
   <223> Synthetic oligonucleotide
<400> 1322
   ccagcaccat tgttgaagat ccccagacca agtgtgaata catgctcaac tcgatgccca 60
   agagact 67
<210> 1323
   <211> 68
   <212> DNA
   <213> Artificial Sequence
<220>
   <223> Synthetic oligonucleotide
<400> 1323
   cgtggtgccc ctctatgacc tgctgctgga gatgctggac gcccaccgcc tacatgcgcc 60
   cactagcc 68
<210> 1324
   <211> 67
   <212> DNA
   <213> Artificial Sequence
<220>
   <223> Synthetic oligonucleotide
<400> 1324
   accatgtatc gagaggggcc cccttaccag aggcgaggtt cccttcagct gtggcagttc 60
   ctggtca 67
<210> 1325
   <211> 78
   <212> DNA
   <213> Artificial Sequence
<220>
   <223> Synthetic oligonucleotide
<400> 1325
   tggaaacagc gaaggataca gcctgtgcac atcctgactt ctgtgagctc attgcgcggg 60
   actagggagt gttcggtg 78
<210> 1326
   <211> 73
   <212> DNA
   <213> Artificial Sequence
<220>
   <223> Synthetic oligonucleotide
<400> 1326
   gtgaaggatg tgaagcagac gtacttggca cgggtcttct cctacccggc agggaatgtg 60
   gagagcaccg gtt 73
<210> 1327
   <211> 66
   <212> DNA
   <213> Artificial Sequence
<220>
   <223> Synthetic oligonucleotide
<400> 1327
   ctgaccagaa ccacggctta tccggcctgt ccacgaacca cttatacacc cacatgaccc 60
   acttcc 66
<210> 1328
   <211> 66
   <212> DNA
   <213> Artificial Sequence
<220>
   <223> Synthetic oligonucleotide
<400> 1328
   gcctcttcct gttcgacggc tcgcccacct acgtactggc ctacacccag agctaccggg 60
   caaagc 66
<210> 1329
   <211> 80
   <212> DNA
   <213> Artificial Sequence
<220>
   <223> Synthetic oligonucleotide
<400> 1329
   gagggactgt tggcatgcag tgccctccca gagaccaacg ttcaagcagt tggtagaaga 60
   cttggatcga attctcactc 80
<210> 1330
   <211> 81
   <212> DNA
   <213> Artificial Sequence
<220>
   <223> Synthetic oligonucleotide
<400> 1330
   ctggcttaag gatggacagg cctttcatgg ggagaaccgc attggaggca ttcggctgcg 60
   ccatcagcac tggagtctcg t 81
<210> 1331
   <211> 67
   <212> DNA
   <213> Artificial Sequence
<220>
   <223> Synthetic oligonucleotide
<400> 1331
   ccagtggagc gcttccatga cctgcgtcct gatgaagtgg ccgatttgtt tcagacgacc 60
   cagagag 67
<210> 1332
   <211> 66
   <212> DNA
   <213> Artificial Sequence
<220>
   <223> Synthetic oligonucleotide
<400> 1332
   gacatctgcg ctccatcctc gggaccctga cagtggagca gatttatcag gaccgggacc 60
   agtttg 66
<210> 1333
   <211> 69
   <212> DNA
   <213> Artificial Sequence
<220>
   <223> Synthetic oligonucleotide
<400> 1333
   ggaagtgaca gacgtgaagg tcaccatcat gtggacaccg cctgagagtg cagtgaccgg 60
   ctaccgtgt 69
<210> 1334
   <211> 67
   <212> DNA
   <213> Artificial Sequence
<220>
   <223> Synthetic oligonucleotide
<400> 1334
   cgagcccttt gatgacttcc tgttcccagc atcatccagg cccagtggct ctgagacagc 60
   ccgctcc 67
<210> 1335
   <211> 66
   <212> DNA
   <213> Artificial Sequence
<220>
   <223> Synthetic oligonucleotide
<400> 1335
   gagaacaagc agggctggca gaacagcatc cgccacaacc tctcgctcaa cgagtgcttc 60
   gtcaag 66
<210> 1336
   <211> 83
   <212> DNA
   <213> Artificial Sequence
<220>
   <223> Synthetic oligonucleotide
<400> 1336
   tgaagtccag gacgatgatg cgcctctctc gcccatgctc tacagcagct cagccagcct 60
   gtcaccttca gtaagcaagc cgt 83
<210> 1337
   <211> 70
   <212> DNA
   <213> Artificial Sequence
<220>
   <223> Synthetic oligonucleotide
<400> 1337
   cgacagagct tgtgcaccta agctgcagac caagcctttg cccagaattt aaggattcca 60
   atggacgacc 70
<210> 1338
   <211> 66
   <212> DNA
   <213> Artificial Sequence
<220>
   <223> Synthetic oligonucleotide
<400> 1338
   ctgtttgctg tccggaggca cctgtggggt agccatggaa acagcacatt cccagagttc 60
   ctccac 66
<210> 1339
   <211> 74
   <212> DNA
   <213> Artificial Sequence
<220>
   <223> Synthetic oligonucleotide
<400> 1339
   ccagctgcta ctttgacatc gagtggcgtg accggcgcat cacactgagg gcgtccaatg 60
   gcaagtttgt gacc 74
<210> 1340
   <211> 80
   <212> DNA
   <213> Artificial Sequence
<220>
   <223> Synthetic oligonucleotide
<400> 1340
   ggataattca gacaacaaca ccatctttgt gcaaggcctg ggtgagaatg ttacaattga 60
   gtctgtggct gattacttca 80
<210> 1341
   <211> 69
   <212> DNA
   <213> Artificial Sequence
<220>
   <223> Synthetic oligonucleotide
<400> 1341
   gaagcgcaga tcatgaagaa gctgaagcac gacaagctgg tccagctcta tgcagtggtg 60
   tctgaggag                                                         69
<210> 1342
   <211> 68
   <212> DNA
   <213> Artificial Sequence
<220>
   <223> Synthetic oligonucleotide
<400> 1342
   tcagcagcaa gggcatcatg gaggaggatg aggcctgcgg gcgccagtac acgctcaaga 60
   aaaccacc                                                          68
<210> 1343
   <211> 74
   <212> DNA
   <213> Artificial Sequence
<220>
   <223> Synthetic oligonucleotide
<400> 1343
   tgtttggagg gaagggctgg ggctctgagc cagattccac acctcacgtt cagtcacagc 60
   cctcagctat cttc                                                   74
<210> 1344
   <211> 73
   <212> DNA
   <213> Artificial Sequence
<220>
   <223> Synthetic oligonucleotide
<400> 1344
   gtgctggtga cgaatccaca ttcatctcaa tggaaggatc ctgccttaag tcaacttatt 60
   tgtttttgcc ggg                                                    73
<210> 1345
   <211> 70
   <212> DNA
   <213> Artificial Sequence
<220>
   <223> Synthetic oligonucleotide
<400> 1345
   accctcgaca agaccacact ttgggacttg ggagctgggg ctgaagttgc tctgtaccca 60
   tgaactccca                                                        70
<210> 1346
   <211> 74
   <212> DNA
   <213> Artificial Sequence
<220>
   <223> Synthetic oligonucleotide
<400> 1346
   attccaccca tggcaaattc catggcaccg tcaaggctga gaacgggaag cttgtcatca 60
   atggaaatcc catc                                                   74
<210> 1347
   <211> 75
   <212> DNA
   <213> Artificial Sequence
<220>
   <223> Synthetic oligonucleotide
<400> 1347
   caaaggagct cactgtggtg tctgtgttcc aaccactgaa tctggacccc atctgtgaat 60
   aagccattct gactc                                                  75
<210> 1348
   <211> 73
   <212> DNA
   <213> Artificial Sequence
<220>
   <223> Synthetic oligonucleotide
<400> 1348
   ttgggaaata tttgggcatt ggtctggcca agtctacaat gtcccaatat caaggacaac 60
   caccctagct tct                                                    73
<210> 1349
   <211> 83
   <212> DNA
   <213> Artificial Sequence
<220>
   <223> Synthetic oligonucleotide
<400> 1349
   gcatgggaac catcaaccag caggccatgg accaacttca ctatgtgaca gagctgacag 60
   atcgaatcaa ggcaaactcc tca                                         83
<210> 1350
   <211> 85
   <212> DNA
   <213> Artificial Sequence
<220>
   <223> Synthetic oligonucleotide
<400> 1350
   tgtagaatca aactcttcat catcaactag aagtgcagtt gacatggcct gttcagtcct 60
   tggagttgca cagctggatt ctgtg                                       85
<210> 1351
   <211> 72
   <212> DNA
   <213> Artificial Sequence
<220>
   <223> Synthetic oligonucleotide
<400> 1351
   cgctccagac ctatgatgac ttgttagcca aagactgcca ctgcatatga gcagtcctgg 60
   tccttccact gt                                                     72
<210> 1352
   <211> 66
   <212> DNA
   <213> Artificial Sequence
<220>
   <223> Synthetic oligonucleotide
<400> 1352
   gatcccaagg cccaactccc cgaaccactc agggtcctgt ggacagctca cctagctgca 60
   atggct                                                            66
<210> 1353
   <211> 68
   <212> DNA
   <213> Artificial Sequence
<220>
   <223> Synthetic oligonucleotide
<400> 1353
   gttcactggg ggtgtatggg gtagatgggt ggagagggag gggataagag aggtgcatgt 60
   tggtattt                                                          68
<210> 1354
   <211> 74
   <212> DNA
   <213> Artificial Sequence
<220>
   <223> Synthetic oligonucleotide
<400> 1354
   tgtcatgtac gacggcttct ccatgcagcg gctggtgaag tgcaacgcct ggccttgtcc 60
   caacactgtg gact                                                   74
<210> 1355
   <211> 67
   <212> DNA
   <213> Artificial Sequence
<220>
   <223> Synthetic oligonucleotide
<400> 1355
   gttcgctacg aggattgagc gtctccaccc agtaagtggg caagaggcgg caggaagtgg 60
   gtacgca                                                           67
<210> 1356
   <211> 68
   <212> DNA
   <213> Artificial Sequence
<220>
   <223> Synthetic oligonucleotide
<400> 1356
   ttctggacct gggaccttag gagccgggtg acagcactaa ccagacctcc agccactcac 60
   agctcttt                                                          68
<210> 1357
   <211> 70
   <212> DNA
   <213> Artificial Sequence
<220>
   <223> Synthetic oligonucleotide
<400> 1357
   cgtgcctcta caccatcttc ctcttcccca tcggctttgt gggcaacatc ctgatcctgg 60
   tggtgaacat                                                        70
<210> 1358
   <211> 66
   <212> DNA
   <213> Artificial Sequence
<220>
   <223> Synthetic oligonucleotide
<400> 1358
   agtacaagca ggctgccaag tgcctcctgc tggcttcctt tgatcactgt gacttccctg 60
   agctgc                                                            66
<210> 1359
   <211> 67
   <212> DNA
   <213> Artificial Sequence
<220>
   <223> Synthetic oligonucleotide
<400> 1359
   gcttatgacc gaccccaagc tcatcacctg gtctccggtg tgtcgcaacg atgttgcctg 60
   gaacttt                                                           67
<210> 1360
   <211> 75
   <212> DNA
   <213> Artificial Sequence
<220>
   <223> Synthetic oligonucleotide
<400> 1360
   cacacagatc tcctactcca tccagtcctg aggagcctta ggatgcagca tgccttcagg 60
   agacactgct ggacc                                                  75
<210> 1361
   <211> 66
   <212> DNA
   <213> Artificial Sequence
<220>
   <223> Synthetic oligonucleotide
<400> 1361
   ctgagtgtgg tttgcggatc ctggccttcc cgtgtaacca gttcgggaag caggagccag 60
   ggagta 66
<210> 1362
   <211> 67
   <212> DNA
   <213> Artificial Sequence
<220>
   <223> Synthetic oligonucleotide
<400> 1362
   ccatctgcat ccatcttgtt tgggctcccc acccttgaga agtgcctcag ataataccct 60
   ggtggcc 67
<210> 1363
   <211> 71
   <212> DNA
   <213> Artificial Sequence
<220>
   <223> Synthetic oligonucleotide
<400> 1363
   cagatgacaa tggccacaat gctcttcttg gtttctctgg gaattgtgtt ggctgtggaa 60
   agaaaggctt c 71
<210> 1364
   <211> 73
   <212> DNA
   <213> Artificial Sequence
<220>
   <223> Synthetic oligonucleotide
<400> 1364
   tgcttaggtg cggtaaaacc agcgcttgtc cgatgcaccg ttcgcgtggt aaactgacgc 60
   attcaggctc ttg 73
<210> 1365
   <211> 64
   <212> DNA
   <213> Artificial Sequence
<220>
   <223> Synthetic oligonucleotide
<400> 1365
   ccccaggcac cagctttact ccccgagccc agcaggacat ctgcatataa cacacagccg 60
   aagt 64
<210> 1366
   <211> 72
   <212> DNA
   <213> Artificial Sequence
<220>
   <223> Synthetic oligonucleotide
<400> 1366
   tgcccccaag acactgtgtg tgacctgatc cagagtaagt gcctctccaa ggagaacgct 60
   accacggacc tc 72
<210> 1367
   <211> 86
   <212> DNA
   <213> Artificial Sequence
<220>
   <223> Synthetic oligonucleotide
<400> 1367
   aagctatgag gaaaagaagt acacgatggg ggacgctcct gattatgaca gaagccagtg 60
   gctgaatgaa aaattcaagc tgggcc 86
<210> 1368
   <211> 71
   <212> DNA
   <213> Artificial Sequence
<220>
   <223> Synthetic oligonucleotide
<400> 1368
   ctgggctgtg aggctgagag tgaatctgct ttacgagggt aggcggggaa tcagaaaagg 60
   agcagattcg c 71
<210> 1369
   <211> 68
   <212> DNA
   <213> Artificial Sequence
<220>
   <223> Synthetic oligonucleotide
<400> 1369
   ctgcaggcac tccctgaaat gctgaagctc tactcacagt ttctggggaa gcagccatgg 60
   tttcttgg 68
<210> 1370
   <211> 76
   <212> DNA
   <213> Artificial Sequence
<220>
   <223> Synthetic oligonucleotide
<400> 1370
   caatgccatc ttgcgctaca tcgctcgcaa gcacaacatg tgtggtgaga ctgaagaaga 60
   aaagattcga gtggac                                                 76
<210> 1371
   <211> 66
   <212> DNA
   <213> Artificial Sequence
<220>
   <223> Synthetic oligonucleotide
<400> 1371
   caccatcccc accctgtctt ccacagccgc ctgaaagcca caatgagaat gatgcacact 60
   gaggcc                                                            66
<210> 1372
   <211> 73
   <212> DNA
   <213> Artificial Sequence
<220>
   <223> Synthetic oligonucleotide
<400> 1372
   cccactcagt agccaagtca caatgtttgg aaaacagccc gtttacttga gcaagactga 60
   taccacctgc gtg                                                    73
<210> 1373
   <211> 70
   <212> DNA
   <213> Artificial Sequence
<220>
   <223> Synthetic oligonucleotide
<400> 1373
   ccaaacgtgt aacaattatg ccaaaagaca tccagctagc acgccgcata cgtggagaac 60
   gtgcttaaga                                                        70
<210> 1374
   <211> 74
   <212> DNA
   <213> Artificial Sequence
<220>
   <223> Synthetic oligonucleotide
<400> 1374
   caagtaccac agcgatgact acattaaatt cttgcgctcc atccgtccag ataacatgtc 60
   ggagtacagc aagc 74
<210> 1375
   <211> 66
   <212> DNA
   <213> Artificial Sequence
<220>
   <223> Synthetic oligonucleotide
<400> 1375
   tcctgtgctc tggaagccct tgagcccttc tgggaggttc ttgtgagatc aactgagacc 60
   gtggag 66
<210> 1376
   <211> 70
   <212> DNA
   <213> Artificial Sequence
<220>
   <223> Synthetic oligonucleotide
<400> 1376
   cggtgtgaga agtgcagcaa gccctgtgcc cgagtgtgct atggtctggg catggagcac 60
   ttgcgagagg 70
<210> 1377
   <211> 68
   <212> DNA
   <213> Artificial Sequence
<220>
   <223> Synthetic oligonucleotide
<400> 1377
   gaaagatagc tcgcggcatt ccaagctgga gaaggcggac attctggaaa tgacagtgaa 60
   gcacctcc 68
<210> 1378
   <211> 72
   <212> DNA
   <213> Artificial Sequence
<220>
   <223> Synthetic oligonucleotide
<400> 1378
   caggacacaa gtgccagatt gcgggctggg gccacttgga tgagaacgtg agcggctact 60
   ccagctccct gc 72
<210> 1379
   <211> 73
   <212> DNA
   <213> Artificial Sequence
<220>
   <223> Synthetic oligonucleotide
<400> 1379
   tccatgatgg ttctgcaggt ttctgcggcc ccccggacag tggctctgac ggcgttactg 60
   atggtgctgc tca 73
<210> 1380
   <211> 71
   <212> DNA
   <213> Artificial Sequence
<220>
   <223> Synthetic oligonucleotide
<400> 1380
   tggcctgtcc attggtgatg ttgcgaagaa actgggagag atgtggaata acactgctgc 60
   agatgacaag c 71
<210> 1381
   <211> 73
   <212> DNA
   <213> Artificial Sequence
<220>
   <223> Synthetic oligonucleotide
<400> 1381
   tccaggatgt taggaactgt gaagatggaa gggcatgaaa ccagcgactg gaacagctac 60
   tacgcagaca cgc 73
<210> 1382
   <211> 84
   <212> DNA
   <213> Artificial Sequence
<220>
   <223> Synthetic oligonucleotide
<400> 1382
   agcaggagcg accaactgat cgcacacatg ctttgtttgg atatggagtg aacacaatta 60
   tgtaccaaat ttaacttggc aaac                                        84
<210> 1383
   <211> 68
   <212> DNA
   <213> Artificial Sequence
<220>
   <223> Synthetic oligonucleotide
<400> 1383
   gcagcagtcg gcttctctac gcagaacccg ggagtaggag actcagaatc gaatctcttc 60
   tccctccc                                                          68
<210> 1384
   <211> 69
   <212> DNA
   <213> Artificial Sequence
<220>
   <223> Synthetic oligonucleotide
<400> 1384
   agtgacagat ggacaatgca agaatgaact ccttcctgga ataccccata cttagcagtg 60
   gcgactcgg                                                         69
<210> 1385
   <211> 78
   <212> DNA
   <213> Artificial Sequence
<220>
   <223> Synthetic oligonucleotide
<400> 1385
   tcccttgtgt tccttctgtg aagaagccct gttctcgttg ccctaattca tcttttaatc 60
   atgagcctgt ttattgcc                                               78
<210> 1386
   <211> 71
   <212> DNA
   <213> Artificial Sequence
<220>
   <223> Synthetic oligonucleotide
<400> 1386
   cgtgccttat ggttactttg gaggcgggta ctactcctgc cgagtgtccc ggagctcgct 60
   gaaaccctgt g 71
<210> 1387
   <211> 68
   <212> DNA
   <213> Artificial Sequence
<220>
   <223> Synthetic oligonucleotide
<400> 1387
   cagcctcaag ttcggttttc gctaccggag ccttcccaga acaaacttct tgtgcgtttg 60
   cttccaac 68
<210> 1388
   <211> 78
   <212> DNA
   <213> Artificial Sequence
<220>
   <223> Synthetic oligonucleotide
<400> 1388
   ctggaccgca cggacatcca caccttccac cgcttctacc aatacctcgc ccacagcaag 60
   caagtctttc gcgaggcg 78
<210> 1389
   <211> 68
   <212> DNA
   <213> Artificial Sequence
<220>
   <223> Synthetic oligonucleotide
<400> 1389
   gctttccaag tggggaatta aagttgcttc catccaacct ggaggcttcc taacaaatat 60
   cgcaggca 68
<210> 1390
   <211> 77
   <212> DNA
   <213> Artificial Sequence
<220>
   <223> Synthetic oligonucleotide
<400> 1390
   cagtctcgcc atgttgaagt cagaatggcc tgtattcact atcttcgaga gaacagagag 60
   aaatttgaag cgtttat 77
<210> 1391
   <211> 70
   <212> DNA
   <213> Artificial Sequence
<220>
   <223> Synthetic oligonucleotide
<400> 1391
   ctgctgcgac agtccactac ctttttcgag agtgactccc gttgtcccaa ggcttcccag 60
   agcgaacctg                                                        70
<210> 1392
   <211> 63
   <212> DNA
   <213> Artificial Sequence
<220>
   <223> Synthetic oligonucleotide
<400> 1392
   ggtccgcttc gtctttcgag agtgactccc gcggtcccaa ggctttccag agcgaacctg 60
   tgc                                                               63
<210> 1393
   <211> 72
   <212> DNA
   <213> Artificial Sequence
<220>
   <223> Synthetic oligonucleotide
<400> 1393
   ttcagtgtgt ccagtgcatc tttagtggag gttcacaagt ctgaggaaaa tgaggagcca 60
   atggaaacag at                                                     72
<210> 1394
   <211> 84
   <212> DNA
   <213> Artificial Sequence
<220>
   <223> Synthetic oligonucleotide
<400> 1394
   ggctagtaga actggatccc aacaccaaac tcttaattag acctaggcct cagctgcact 60
   gcccgaaaag catttgggca gacc                                        84
<210> 1395
   <211> 73
   <212> DNA
   <213> Artificial Sequence
<220>
   <223> Synthetic oligonucleotide
<400> 1395
   cctccctctg gtggtgcttc ctcagggccc accattgaag aggttgatta agccaaccaa 60
   gtgtagatgt agc                                                    73
<210> 1396
   <211> 84
   <212> DNA
   <213> Artificial Sequence
<220>
   <223> Synthetic oligonucleotide
<400> 1396
   ccgactggag gagcataaaa gcgcagccga gcccagcgcc ccgcactttt ctgagcagac 60
   gtccagagca gagtcagcca gcat                                        84
<210> 1397
   <211> 83
   <212> DNA
   <213> Artificial Sequence
<220>
   <223> Synthetic oligonucleotide
<400> 1397
   gaataccaca ctttctgcta caacactggg ctatggagag gacgccacgc ctggcacagg 60
   gtatacaggg ttagctgcaa tcc                                         83
<210> 1398
   <211> 68
   <212> DNA
   <213> Artificial Sequence
<220>
   <223> Synthetic oligonucleotide
<400> 1398
   gcagacagtg accatctaca gctttccggc gcccaacgtg attctgacga agccagaggt 60
   ctcagaag                                                          68
<210> 1399
   <211> 70
   <212> DNA
   <213> Artificial Sequence
<220>
   <223> Synthetic oligonucleotide
<400> 1399
   agaaccgcaa ggtgagcaag gtggagattc tccagcacgt catcgactac atcagggacc 60
   ttcagttgga 70
<210> 1400
   <211> 83
   <212> DNA
   <213> Artificial Sequence
<220>
   <223> Synthetic oligonucleotide
<400> 1400
   tggcctggct cttaatttgc ttttgttttg cccagtatag actcggaagt aacagttata 60
   gctagtggtc ttgcatgatt gca 83
<210> 1401
   <211> 74
   <212> DNA
   <213> Artificial Sequence
<220>
   <223> Synthetic oligonucleotide
<400> 1401
   ggtggagagt ggagccatga ccaaggacct ggcgggctgc attcacggcc tcagcaatgt 60
   gaagctgaac gagc 74
<210> 1402
   <211> 83
   <212> DNA
   <213> Artificial Sequence
<220>
   <223> Synthetic oligonucleotide
<400> 1402
   gcatggtagc cgaagatttc acagtcaaaa tcggagattt tggtatgacg cgagatatct 60
   atgagacaga ctattaccgg aaa 83
<210> 1403
   <211> 72
   <212> DNA
   <213> Artificial Sequence
<220>
   <223> Synthetic oligonucleotide
<400> 1403
   ccgtgcttcc ggacaacttc cccagatacc ccgtgggcaa gttcttccaa tatgacacct 60
   ggaagcagtc ca 72
<210> 1404
   <211> 77
   <212> DNA
   <213> Artificial Sequence
<220>
   <223> Synthetic oligonucleotide
<400> 1404
   tgaaccgcag agaccaacag aggaatccag gcacctctac cacgccctcc cagcccaatt 60
   ctgcgggtgt ccaagac 77
<210> 1405
   <211> 68
   <212> DNA
   <213> Artificial Sequence
<220>
   <223> Synthetic oligonucleotide
<400> 1405
   gggtcactat ggagttcaaa ggacagaact cctgcctggt gaccgggaca acctggccat 60
   tcagaccc 68
<210> 1406
   <211> 66
   <212> DNA
   <213> Artificial Sequence
<220>
   <223> Synthetic oligonucleotide
<400> 1406
   gcctcccata gctccttacc ccagccctac acgaaaggac ctgcttctcc acatgcaaga 60
   gctctg 66
<210> 1407
   <211> 70
   <212> DNA
   <213> Artificial Sequence
<220>
   <223> Synthetic oligonucleotide
<400> 1407
   aaggaaccat ctcactgtgt gtaaacatga cttccaagct ggccgtggct ctcttggcag 60
   ccttcctgat                                                        70
<210> 1408
   <211> 79
   <212> DNA
   <213> Artificial Sequence
<220>
   <223> Synthetic oligonucleotide
<400> 1408
   ggcgctgtca tcgatttctt ccctgtgaaa acaagagcaa ggccgtggag caggtgaaga 60
   atgcctttaa taagctcca                                              79
<210> 1409
   <211> 66
   <212> DNA
   <213> Artificial Sequence
<220>
   <223> Synthetic oligonucleotide
<400> 1409
   tggaaggttc cacaagtcac cctgtgatca acagtacccg tatgggacaa agctgcaagg 60
   tcaaga                                                            66
<210> 1410
   <211> 76
   <212> DNA
   <213> Artificial Sequence
<220>
   <223> Synthetic oligonucleotide
<400> 1410
   accctctggt ggtaaatgga cattttccta catcggcttc cctgtagagc tgaacacagt 60
   ctatttcatt ggggcc                                                 76
<210> 1411
   <211> 74
   <212> DNA
   <213> Artificial Sequence
<220>
   <223> Synthetic oligonucleotide
<400> 1411
   ggcctaatgt tccagatcct tcaaagagtc atattgccca gtggtcacct cacactcctc 60
   caaggcacaa tttt 74
<210> 1412
   <211> 66
   <212> DNA
   <213> Artificial Sequence
<220>
   <223> Synthetic oligonucleotide
<400> 1412
   actttcctgc gaggtcagtc aaggctttgg gggctctgtt ttgaatgtgg atcaccactc 60
   ggagtt 66
<210> 1413
   <211> 72
   <212> DNA
   <213> Artificial Sequence
<220>
   <223> Synthetic oligonucleotide
<400> 1413
   gtgcccgagc catatagcag gcacgtccgg gtcctcactg tccttccact caacagtcat 60
   caaccactac cg 72
<210> 1414
   <211> 69
   <212> DNA
   <213> Artificial Sequence
<220>
   <223> Synthetic oligonucleotide
<400> 1414
   agtccagccg agatgctaag agcaaggcca agaggaagtc atgtggggat tccagccctg 60
   ataccttct 69
<210> 1415
   <211> 74
   <212> DNA
   <213> Artificial Sequence
<220>
   <223> Synthetic oligonucleotide
<400> 1415
   ccacagctca ccttctgtca ggtgtccatc ccagctccag ccagctccca gagaggaaga 60
   gactggcact gagg                                                   74
<210> 1416
   <211> 77
   <212> DNA
   <213> Artificial Sequence
<220>
   <223> Synthetic oligonucleotide
<400> 1416
   ccatgatcct cactctgctg gtggactata cactccagac ctcgcttagc atggtaaatc 60
   accggctaca aagcttc                                                77
<210> 1417
   <211> 66
   <212> DNA
   <213> Artificial Sequence
<220>
   <223> Synthetic oligonucleotide
<400> 1417
   caacgcttca gtgatcaatc ccggggcgat ttacagatgc aggatcggaa agaatcccgg 60
   ccagac                                                            66
<210> 1418
   <211> 75
   <212> DNA
   <213> Artificial Sequence
<220>
   <223> Synthetic oligonucleotide
<400> 1418
   aggccagccc tacattatca gagcaagagc cggatagagg acaaggctca gatcttgctg 60
   gactgtggag aagac                                                  75
<210> 1419
   <211> 69
   <212> DNA
   <213> Artificial Sequence
<220>
   <223> Synthetic oligonucleotide
<400> 1419
   cagtgacaaa cagcccttcc aacccaagga atcccacaaa agatggcgat gacgcccatg 60
   aggctaaac 69
<210> 1420
   <211> 79
   <212> DNA
   <213> Artificial Sequence
<220>
   <223> Synthetic oligonucleotide
<400> 1420
   actcggactg cacaagctat ttttgatgac agctatttgg gttattctgt ggctgtcgga 60
   gatttcaatg gtgatggca 79
<210> 1421
   <211> 74
   <212> DNA
   <213> Artificial Sequence
<220>
   <223> Synthetic oligonucleotide
<400> 1421
   tcagaattgg atttggctca tttgtggaaa agactgtgat gccttacatt agcacaacac 60
   cagctaagct cagg 74
<210> 1422
   <211> 78
   <212> DNA
   <213> Artificial Sequence
<220>
   <223> Synthetic oligonucleotide
<400> 1422
   accggggagc cctacatgac gaaaatacct gcaaccgtta ctgccgtgac gagattgagt 60
   cagtgaaaga gcttaagg 78
<210> 1423
   <211> 66
   <212> DNA
   <213> Artificial Sequence
<220>
   <223> Synthetic oligonucleotide
<400> 1423
   caaggtgccc tcagtggagc tcaccaacct gtacccgtat tgcgactatg agatgaaggt 60
   gtgcgc 66
<210> 1424
   <211> 71
   <212> DNA
   <213> Artificial Sequence
<220>
   <223> Synthetic oligonucleotide
<400> 1424
   tcgtgaaaga tgaccaggag gctgtgctat gtttctacaa aaccgccaag gactgcgtca 60
   tgatgttcac c 71
<210> 1425
   <211> 69
   <212> DNA
   <213> Artificial Sequence
<220>
   <223> Synthetic oligonucleotide
<400> 1425
   tggcttacac tggcaatggt agtttctgtg gttggctggg aaatcgagtg ccgcatctca 60
   cagctatgc 69
<210> 1426
   <211> 70
   <212> DNA
   <213> Artificial Sequence
<220>
   <223> Synthetic oligonucleotide
<400> 1426
   ctgtcagctg ctgcttgggg tcaagggaca cgccttctga acgtcccctg cccctttacg 60
   gacaccccct 70
<210> 1427
   <211> 80
   <212> DNA
   <213> Artificial Sequence
<220>
   <223> Synthetic oligonucleotide
<400> 1427
   cggactttgg gtgcgacttg acgagcggtg gttcgacaag tggccttgcg ggccggatcg 60
   tcccagtgga agagttgtaa 80
<210> 1428
   <211> 67
   <212> DNA
   <213> Artificial Sequence
<220>
   <223> Synthetic oligonucleotide
<400> 1428
   aagcccgagg cactcattgt tgcccttcaa gctgccaatg aagacctcag gaccaagctc 60
   acagaca 67
<210> 1429
   <211> 66
   <212> DNA
   <213> Artificial Sequence
<220>
   <223> Synthetic oligonucleotide
<400> 1429
   gctgggaggc aggacttcct cttcaaggcc atgctgacca tcagctggct cactctgacc 60
   tgcttc 66
<210> 1430
   <211> 69
   <212> DNA
   <213> Artificial Sequence
<220>
   <223> Synthetic oligonucleotide
<400> 1430
   gagctccatg gctcatcccc agcagtgagc tcagaggaat gcacacccag taggattcag 60
   tgggtgtga 69
<210> 1431
   <211> 67
   <212> DNA
   <213> Artificial Sequence
<220>
   <223> Synthetic oligonucleotide
<400> 1431
   tctcttgcag gaagccagac aacagtcagt ggcccatcag caatcagggt ctgaattggc 60
   cctacgg                                                           67
<210> 1432
   <211> 73
   <212> DNA
   <213> Artificial Sequence
<220>
   <223> Synthetic oligonucleotide
<400> 1432
   aattcctgct ccaaaagaaa gtcttcgaag ccgctccact cgcatgtcca ctgtctcaga 60
   gcttcgcatc acg                                                    73
<210> 1433
   <211> 66
   <212> DNA
   <213> Artificial Sequence
<220>
   <223> Synthetic oligonucleotide
<400> 1433
   caccccggct tcaacaacag cctccccaac aaagaccacc gcaatgacat catgctggtg 60
   aagatg                                                            66
<210> 1434
   <211> 78
   <212> DNA
   <213> Artificial Sequence
<220>
   <223> Synthetic oligonucleotide
<400> 1434
   gacgtgaggg tcctgattct ccctggtttt accccagctc catccttgca tcactgggga 60
   ggacgtgatg agtgagga                                               78
<210> 1435
   <211> 67
   <212> DNA
   <213> Artificial Sequence
<220>
   <223> Synthetic oligonucleotide
<400> 1435
   catcctcatg gattggtgtg tttcgtaaca gcagtcatca tccatgggtg acaatgaatg 60
   gtttggc 67
<210> 1436
   <211> 77
   <212> DNA
   <213> Artificial Sequence
<220>
   <223> Synthetic oligonucleotide
<400> 1436
   ccacctcgcc atgatttttc ctttgaccgg gtattcccac caggaagtgg acaggatgaa 60
   gtgtttgaag agattgc 77
<210> 1437
   <211> 71
   <212> DNA
   <213> Artificial Sequence
<220>
   <223> Synthetic oligonucleotide
<400> 1437
   atgtgccagt gagcttgagt ccttggagaa acacaagcac ctgctagaaa gtactgttaa 60
   ccaggggctc a 71
<210> 1438
   <211> 67
   <212> DNA
   <213> Artificial Sequence
<220>
   <223> Synthetic oligonucleotide
<400> 1438
   tgatggtcca aatgaacgaa ttggcatggt ggtgaaaaca ggagttgtgc cccaacttgt 60
   gaagctt 67
<210> 1439
   <211> 66
   <212> DNA
   <213> Artificial Sequence
<220>
   <223> Synthetic oligonucleotide
<400> 1439
   cttgctggcc aatgcctaca tctacgttgt ccagctgcca gccaagatcc tgactgcgga 60
   caatca 66
<210> 1440
   <211> 73
   <212> DNA
   <213> Artificial Sequence
<220>
   <223> Synthetic oligonucleotide
<400> 1440
   cagatgaggc acatggagac ccaggccaag gacctgagga atcagttgct caactaccgt 60
   tctgccattt caa 73
<210> 1441
   <211> 67
   <212> DNA
   <213> Artificial Sequence
<220>
   <223> Synthetic oligonucleotide
<400> 1441
   ctcctggcca acagcactgc actagaagag gccatgctcc aggaacagca gaggctgggc 60
   cttgtgt 67
<210> 1442
   <211> 66
   <212> DNA
   <213> Artificial Sequence
<220>
   <223> Synthetic oligonucleotide
<400> 1442
   caaggagact gggaggtgtc tcaagtgcct gtaccacacg gaaggggaac actgtcagtt 60
   ctgccg 66
<210> 1443
   <211> 67
   <212> DNA
   <213> Artificial Sequence
<220>
   <223> Synthetic oligonucleotide
<400> 1443
   actgaccaag cctgagacct actgcaccca gtatggcgag tggcagatga aatgctgcaa 60
   gtgtgac 67
<210> 1444
   <211> 80
   <212> DNA
   <213> Artificial Sequence
<220>
   <223> Synthetic oligonucleotide
<400> 1444
   actcaagcgg aaattgaagc agataggtct tatcagcaca gtctccgcct cctggattca 60
   gtgtctcggc ttcagggagt 80
<210> 1445
   <211> 67
   <212> DNA
   <213> Artificial Sequence
<220>
   <223> Synthetic oligonucleotide
<400> 1445
   agcgatgaag atggtcgcgc cctggacgcg gttctactcc aacagctgct gcttgtgctg 60
   ccatgtc 67
<210> 1446
   <211> 69
   <212> DNA
   <213> Artificial Sequence
<220>
   <223> Synthetic oligonucleotide
<400> 1446
   agcggaaaat ggcagacaat ttttcgctcc atgatgcgtt atctgggtct ggaaacccaa 60
   accctcaag 69
<210> 1447
   <211> 67
   <212> DNA
   <213> Artificial Sequence
<220>
   <223> Synthetic oligonucleotide
<400> 1447
   gcttcaggtg ttgtgactgc agtgcctccc tgtcgcacca gtactatgag aaggatgggc 60
   agctctt 67
<210> 1448
   <211> 71
   <212> DNA
   <213> Artificial Sequence
<220>
   <223> Synthetic oligonucleotide
<400> 1448
   tgaacagtaa tggggagctg taccatgagc agtgtttcgt gtgcgctcag tgcttccagc 60
   agttcccaga a 71
<210> 1449
   <211> 66
   <212> DNA
   <213> Artificial Sequence
<220>
   <223> Synthetic oligonucleotide
<400> 1449
   tgcaaacgct ggtgtcacag ccagcccccc aactgacctc atctggaaga accagaactc 60
   gtgggg 66
<210> 1450
   <211> 66
   <212> DNA
   <213> Artificial Sequence
<220>
   <223> Synthetic oligonucleotide
<400> 1450
   ccaatgggag aacaacgggc aggtgttcag cttgctgagc ctgggctcac agtaccagcc 60
   tcagcg 66
<210> 1451
   <211> 67
   <212> DNA
   <213> Artificial Sequence
<220>
   <223> Synthetic oligonucleotide
<400> 1451
   ctgcaacacc gaagtggact gttactccag gggacaagcc ttccaccccc agcctgtgtc 60
   cagagac 67
<210> 1452
   <211> 66
   <212> DNA
   <213> Artificial Sequence
<220>
   <223> Synthetic oligonucleotide
<400> 1452
   agaccaagct ggaagcagag aagttgaaag tgcaggccct gaaggaccga ggtctttcca 60
   ttcctc 66
<210> 1453
   <211> 67
   <212> DNA
   <213> Artificial Sequence
<220>
   <223> Synthetic oligonucleotide
<400> 1453
   acatccaggg ctctgtggtc cgcaagggga gtgcctaaac acagagggtt ctttccattg 60
   tgtctgc 67
<210> 1454
   <211> 67
   <212> DNA
   <213> Artificial Sequence
<220>
   <223> Synthetic oligonucleotide
<400> 1454
   gacctggcct tgctgaagaa tctccggagc gaggaacaga agaagaagaa ccggaagaaa 60
   ctgtccg 67
<210> 1455
   <211> 67
   <212> DNA
   <213> Artificial Sequence
<220>
   <223> Synthetic oligonucleotide
<400> 1455
   agaagctgtc cctgcaagag caggatgcag cgattgtgaa gaacatgaag tctgagctgg 60
   tacggct 67
<210> 1456
   <211> 75
   <212> DNA
   <213> Artificial Sequence
<220>
   <223> Synthetic oligonucleotide
<400> 1456
   gacttttgcc cgctaccttt cattccggcg tgacaacaat gagctgttgc tcttcatact 60
   gaagcagtta gtggc                                                  75
<210> 1457
   <211> 70
   <212> DNA
   <213> Artificial Sequence
<220>
   <223> Synthetic oligonucleotide
<400> 1457
   aggatcgcct gtcagaagag gagacccggg ttgtcttccg tcagatagta tctgctgttg 60
   cttatgtgca                                                        70
<210> 1458
   <211> 69
   <212> DNA
   <213> Artificial Sequence
<220>
   <223> Synthetic oligonucleotide
<400> 1458
   gtgaaatgaa acgcaccaca ctggacagcc ctttggggaa gctggagctg tctggttgtg 60
   agcagggtc                                                         69
<210> 1459
   <211> 79
   <212> DNA
   <213> Artificial Sequence
<220>
   <223> Synthetic oligonucleotide
<400> 1459
   acggatctac cacaccattg catatttgac accccttccc cagccaaata gagctttgag 60
   tttttttgtt ggatatgga                                             79
<210> 1460
   <211> 72
   <212> DNA
   <213> Artificial Sequence
<220>
   <223> Synthetic oligonucleotide
<400> 1460
   gggagatcat cgggacaact ctccttttga tggacctgga ggaaatcttg ctcatgcttt 60
   tcaaccaggc cc                                                     72
<210> 1461
   <211> 78
   <212> DNA
   <213> Artificial Sequence
<220>
   <223> Synthetic oligonucleotide
<400> 1461
   ccaacgcttg ccaaatcctg acaattcaga accagctctc tgtgacccca atttgagttt 60
   tgatgctgtc actaccgt                                               78
<210> 1462
   <211> 86
   <212> DNA
   <213> Artificial Sequence
<220>
   <223> Synthetic oligonucleotide
<400> 1462
   ccatgatgga gaggcagaca tcatgatcaa ctttggccgc tgggagcatg gcgatggata 60
   cccctttgac ggtaaggacg gactcc                                      86
<210> 1463
   <211> 79
   <212> DNA
   <213> Artificial Sequence
<220>
   <223> Synthetic oligonucleotide
<400> 1463
   ggatggtagc agtctaggga ttaacttcct gtatgctgca actcatgaac ttggccattc 60
   tttgggtatg ggacattcc                                              79
<210> 1464
   <211> 79
   <212> DNA
   <213> Artificial Sequence
<220>
   <223> Synthetic oligonucleotide
<400> 1464
   tcacctctca tcttcaccag gatctcacag ggagaggcag atatcaacat tgctttttac 60
   caaagagatc acggtgaca                                              79
<210> 1465
   <211> 72
   <212> DNA
   <213> Artificial Sequence
<220>
   <223> Synthetic oligonucleotide
<400> 1465
   ccatacgtgc tgctacctgt agatattggt gatgaaccat ggtttgatga ttctgccatt 60
   caaaccttta gg                                                     72
<210> 1466
   <211> 75
   <212> DNA
   <213> Artificial Sequence
<220>
   <223> Synthetic oligonucleotide
<400> 1466
   cgagagtctg taggagggaa accgccatgg acgatcaggg ttgccctcgg tgtaagacca 60
   ccaaatatcg gaacc                                                  75
<210> 1467
   <211> 79
   <212> DNA
   <213> Artificial Sequence
<220>
   <223> Synthetic oligonucleotide
<400> 1467
   tcatggtgcc cgtcaatgct gtgatggcga tgaagaccaa gacgtatcag gtggcccaca 60
   tgaagagcaa agacaatcg                                              79
<210> 1468
   <211> 91
   <212> DNA
   <213> Artificial Sequence
<220>
   <223> Synthetic oligonucleotide
<400> 1468
   tcatcctggc gatctacttc ctctggcaga acctaggtcc ctctgtcctg gctggagtcg 60
   ctttcatggt cttgctgatt ccactcaacg g                                91
<210> 1469
   <211> 70
   <212> DNA
   <213> Artificial Sequence
<220>
   <223> Synthetic oligonucleotide
<400> 1469
   tgagaaacaa actgcaccca ctgaactccg cagctagcat ccaaatcagc ccttgagatt 60
   tgaggccttg                                                        70
<210> 1470
   <211> 73
   <212> DNA
   <213> Artificial Sequence
<220>
   <223> Synthetic oligonucleotide
<400> 1470
   gatgcagaat tgaggcagac tttacaagaa gatttacttc gtcgattccc agatcttaac 60
   cgacttgcca aga                                                    73
<210> 1471
   <211> 69
   <212> DNA
   <213> Artificial Sequence
<220>
   <223> Synthetic oligonucleotide
<400> 1471
   gctcgtggtt ctgtagtcca gtcatcctag gagggtgatg ttgactgaga cttcacgctc 60
   tccctttgt                                                         69
<210> 1472
   <211> 78
   <212> DNA
   <213> Artificial Sequence
<220>
   <223> Synthetic oligonucleotide
<400> 1472
   gaaggaatgg gaatcagtca tgagctaatc accctggaga tcagctcccg agatgtcccg 60
   gatctgactc taatagac                                                78
<210> 1473
   <211> 74
   <212> DNA
   <213> Artificial Sequence
<220>
   <223> Synthetic oligonucleotide
<400> 1473
   gccgagatcg ccaagatgtt gccagggagg acagacaatg ctgtgaagaa tcactggaac 60
   tctaccatca aaag                                                    74
<210> 1474
   <211> 75
   <212> DNA
   <213> Artificial Sequence
<220>
   <223> Synthetic oligonucleotide
<400> 1474
   tggttttgag accacgatgt tgggagggta tgtttacagc actccagcca aaaaatacag 60
   cactggcatg attca                                                   75
<210> 1475
   <211> 73
   <212> DNA
   <213> Artificial Sequence
<220>
   <223> Synthetic oligonucleotide
<400> 1475
   taactgacat tcttgagcac cagatccggg ctgttccctt tgagaacctt aacatgcatt 60
   gtgggcaagc cat                                                     73
<210> 1476
   <211> 83
   <212> DNA
   <213> Artificial Sequence
<220>
   <223> Synthetic oligonucleotide
<400> 1476
   cgagactctc ctcatagtga aaggtatgtg tcagccatga ccaccccggc tcgtatgtca 60
   cctgtagatt tccacacgcc aag 83
<210> 1477
   <211> 80
   <212> DNA
   <213> Artificial Sequence
<220>
   <223> Synthetic oligonucleotide
<400> 1477
   caaccgaagt tttcactcca gttgtcccca cagtagacac atatgatggc cgaggtgata 60
   gtgtggttta tggactgagg 80
<210> 1478
   <211> 76
   <212> DNA
   <213> Artificial Sequence
<220>
   <223> Synthetic oligonucleotide
<400> 1478
   gcggaaggtc cctcagacat ccccgattga aagaaccaga gaggctctga gaaacctcgg 60
   gaaacttaga tcatca 76
<210> 1479
   <211> 81
   <212> DNA
   <213> Artificial Sequence
<220>
   <223> Synthetic oligonucleotide
<400> 1479
   ccgcaacgtg gttttctcac cctatggggt ggcctcggtg ttggccatgc tccagctgac 60
   aacaggagga gaaacccagc a 81
<210> 1480
   <211> 71
   <212> DNA
   <213> Artificial Sequence
<220>
   <223> Synthetic oligonucleotide
<400> 1480
   gtggttttcc ctcggagccc cctggctcgg gacgtctgag aagatgccgg tcatgaggct 60
   gttcccttgc t                                                      71
<210> 1481
   <211> 85
   <212> DNA
   <213> Artificial Sequence
<220>
   <223> Synthetic oligonucleotide
<400> 1481
   gcatcaggct gtcattatgg tgtccttacc tgtgggagct gtaaggtctt ctttaagagg 60
   gcaatggaag ggcagcacaa ctact                                       85
<210> 1482
   <211> 67
   <212> DNA
   <213> Artificial Sequence
<220>
   <223> Synthetic oligonucleotide
<400> 1482
   aggactggga cccatgaaca ttcctttggt atcagacccg aagcgcacca ttgctcagga 60
   ttatggg                                                           67
<210> 1483
   <211> 81
   <212> DNA
   <213> Artificial Sequence
<220>
   <223> Synthetic oligonucleotide
<400> 1483
   tggctaagtg aagatgacaa tcatgttgca gcaattcact gtaaagctgg aaagggacga 60
   actggtgtaa tgatatgtgc a                                           81
<210> 1484
   <211> 70
   <212> DNA
   <213> Artificial Sequence
<220>
   <223> Synthetic oligonucleotide
<400> 1484
   aatatttgtg cggggtatgg gggtgggttt ttaaatctcg tttctcttgg acaagcacag 60
   ggatctcgtt                                                        70
<210> 1485
   <211> 67
   <212> DNA
   <213> Artificial Sequence
<220>
   <223> Synthetic oligonucleotide
<400> 1485
   aagcatgaac aggacttgac catctttcca acccctgggg aagacatttg caactgactt 60
   ggggagg                                                           67
<210> 1486
   <211> 68
   <212> DNA
   <213> Artificial Sequence
<220>
   <223> Synthetic oligonucleotide
<400> 1486
   gcaaggaaag ggtcttagtc actgcctccc gaagttgctt gaaagcactc ggagaattgt 60
   gcaggtgt                                                          68
<210> 1487
   <211> 66
   <212> DNA
   <213> Artificial Sequence
<220>
   <223> Synthetic oligonucleotide
<400> 1487
   gaaacctctg cgccatgaga gccaagtgga ggaagaagcg aatgcgcagg ctgaagcgca 60
   aaagaa                                                            66
<210> 1488
   <211> 75
   <212> DNA
   <213> Artificial Sequence
<220>
   <223> Synthetic oligonucleotide
<400> 1488
   ccattctatc atcaacgggt acaaacgagt cctggccttg tctgtggaga cggattacac 60
   cttcccactt gctga 75
<210> 1489
   <211> 67
   <212> DNA
   <213> Artificial Sequence
<220>
   <223> Synthetic oligonucleotide
<400> 1489
   gttctggttg ctggatttgg tcgcaaaggt catgctgttg gtgatattcc tggagtccgc 60
   tttaagg 67
<210> 1490
   <211> 80
   <212> DNA
   <213> Artificial Sequence
<220>
   <223> Synthetic oligonucleotide
<400> 1490
   tcaccacggt ctttagccat gcacaaacgg tagttttgtg tgttggctgc tccactgtcc 60
   tctgccagcc tacaggagga 80
<210> 1491
   <211> 66
   <212> DNA
   <213> Artificial Sequence
<220>
   <223> Synthetic oligonucleotide
<400> 1491
   gggctactgg cagctacatt gctgggacta atggcaattc caatggcctt gtaccgatgc 60
   tgagag 66
<210> 1492
   <211> 71
   <212> DNA
   <213> Artificial Sequence
<220>
   <223> Synthetic oligonucleotide
<400> 1492
   cagcgggatt aaacagtcct ttaaccagca cagccagtta aaagatgcag cctcactgct 60
   tcaacgcaga t 71
<210> 1493
   <211> 69
   <212> DNA
   <213> Artificial Sequence
<220>
   <223> Synthetic oligonucleotide
<400> 1493
   aacagagaca ttgccaacca tattggatct gcttgctgtc caaaccagca aacttcctgg 60
   gcaaatcac 69
<210> 1494
   <211> 77
   <212> DNA
   <213> Artificial Sequence
<220>
   <223> Synthetic oligonucleotide
<400> 1494
   acaccaaaat gccatctcaa atggaacacg ccatggaaac catgatgttt acatttcaca 60
   aattcgctgg ggataaa 77
<210> 1495
   <211> 73
   <212> DNA
   <213> Artificial Sequence
<220>
   <223> Synthetic oligonucleotide
<400> 1495
   tggctgtgct ggtcactacc ttccacaagt actcctgcca agagggcgac aagttcaagc 60
   tgagtaaggg gga 73
<210> 1496
   <211> 70
   <212> DNA
   <213> Artificial Sequence
<220>
   <223> Synthetic oligonucleotide
<400> 1496
   gactgctgtc atggcgtgcc ctctggagaa ggccctggat gtgatggtgt ccaccttcca 60
   caagtactcg 70
<210> 1497
   <211> 75
   <212> DNA
   <213> Artificial Sequence
<220>
   <223> Synthetic oligonucleotide
<400> 1497
   cctgctgacg atgatgaagg agaacttccc caacttcctt agtgcctgtg acaaaaaggg 60
   cacaaattac ctcgc 75
<210> 1498
   <211> 76
   <212> DNA
   <213> Artificial Sequence
<220>
   <223> Synthetic oligonucleotide
<400> 1498
   actccctgat aaaggggaat ttccatgccg tctacaggga tgacctgaag aaattgctag 60
   agaccgagtg tcctca 76
<210> 1499
   <211> 67
   <212> DNA
   <213> Artificial Sequence
<220>
   <223> Synthetic oligonucleotide
<400> 1499
   caccctgcct ctacccaacc agggccccgg ggcctgttat gtcaaactgt cttggctgtg 60
   gggctag 67
<210> 1500
   <211> 70
   <212> DNA
   <213> Artificial Sequence
<220>
   <223> Synthetic oligonucleotide
<400> 1500
   catggccgtg tagaccctaa cccggaggga accctgacta cagaaattac cccggggcac 60
   ccttaaaact 70
<210> 1501
   <211> 67
   <212> DNA
   <213> Artificial Sequence
<220>
   <223> Synthetic oligonucleotide
<400> 1501
   accctgagca ctggaggaag agcgcctgtg ctgtggtctt atcctatgtg gaatccccca 60
   aagtctc                                                            67
<210> 1502
   <211> 67
   <212> DNA
   <213> Artificial Sequence
<220>
   <223> Synthetic oligonucleotide
<400> 1502
   agacaaggat gccgtggata aattgctcaa ggacctggac gccaatggag atgcccaggt 60
   ggacttc                                                           67
<210> 1503
   <211> 67
   <212> DNA
   <213> Artificial Sequence
<220>
   <223> Synthetic oligonucleotide
<400> 1503
   gcagaactga agatgggaag atttatcagc gtgcatttgg tggacagagc ctcaagtttg 60
   gaaaggg                                                           67
<210> 1504
   <211> 86
   <212> DNA
   <213> Artificial Sequence
<220>
   <223> Synthetic oligonucleotide
<400> 1504
   cgcgagcccc tcattataca ctgggcagcc tccccacagc gcatcgagga atgcgtgctc 60
   tcaggcaagg atgtcaacgg cgagtg                                      86
<210> 1505
   <211> 66
   <212> DNA
   <213> Artificial Sequence
<220>
   <223> Synthetic oligonucleotide
<400> 1505
   caagctgaac ggtgtgtccg aaagggacct gaagaaatcg gtgctgtggc tcaaagacag 60
   cttgca 66
<210> 1506
   <211> 72
   <212> DNA
   <213> Artificial Sequence
<220>
   <223> Synthetic oligonucleotide
<400> 1506
   agctggggtg tctgtttcat gtggaatacc tgacttcagg tcaagggatg gtatttatgc 60
   tcgccttgct gt 72
<210> 1507
   <211> 66
   <212> DNA
   <213> Artificial Sequence
<220>
   <223> Synthetic oligonucleotide
<400> 1507
   agaggctgaa tatgcaggac agttggcaga actgaggcag agattggacc atgctgaggc 60
   cgatag 66
<210> 1508
   <211> 71
   <212> DNA
   <213> Artificial Sequence
<220>
   <223> Synthetic oligonucleotide
<400> 1508
   agttgcagaa tctaagcctg gaaggcctgc ggctttcgga tcccattgtc aatactctcg 60
   caaaaaactc a 71
<210> 1509
   <211> 74
   <212> DNA
   <213> Artificial Sequence
<220>
   <223> Synthetic oligonucleotide
<400> 1509
   atggccaatg tttgatgctt aaccccccca atttctgtga gatggatggc cagtgcaagc 60
   gtgacttgaa gtgt 74
<210> 1510
   <211> 69
   <212> DNA
   <213> Artificial Sequence
<220>
   <223> Synthetic oligonucleotide
<400> 1510
   cccaatcgga agcctaacta cagcgagctg caggactcta atccagagtt taccttccag 60
   cagccctac 69
<210> 1511
   <211> 69
   <212> DNA
   <213> Artificial Sequence
<220>
   <223> Synthetic oligonucleotide
<400> 1511
   catcttccag gaggaccact ctctgtggca ccctggacta cctgccccct gaaatgattg 60
   aaggtcgga 69
<210> 1512
   <211> 71
   <212> DNA
   <213> Artificial Sequence
<220>
   <223> Synthetic oligonucleotide
<400> 1512
   aatacccaac gcacaaatga ccgcacgttc tctgccccgt ttcttgcccc agtgtggttt 60
   gcattgtctc c 71
<210> 1513
   <211> 90
   <212> DNA
   <213> Artificial Sequence
<220>
   <223> Synthetic oligonucleotide
<400> 1513
   cctggaggct gcaacatacc tcaatcctgt cccaggccgg atcctcctga agcccttttc 60
   gcagcactgc tatcctccaa agccattgta                                  90
<210> 1514
   <211> 80
   <212> DNA
   <213> Artificial Sequence
<220>
   <223> Synthetic oligonucleotide
<400> 1514
   tgttttgatt cccgggctta ccaggtgaga agtgagggag gaagaaggca gtgtcccttt 60
   tgctagagct gacagctttg                                             80
<210> 1515
   <211> 85
   <212> DNA
   <213> Artificial Sequence
<220>
   <223> Synthetic oligonucleotide
<400> 1515
   tctccagcaa aagcgatgtc tggagctttg gagtgttgat gtgggaagca ttctcctatg 60
   ggcagaagcc atatcgaggg atgaa                                       85
<210> 1516
   <211> 73
   <212> DNA
   <213> Artificial Sequence
<220>
   <223> Synthetic oligonucleotide
<400> 1516
   gatggagcag gtggctcagt tcctgaaggc ggctgaggac tctggggtca tcaagactga 60
   catgttccag act                                                    73
<210> 1517
   <211> 72
   <212> DNA
   <213> Artificial Sequence
<220>
   <223> Synthetic oligonucleotide
<400> 1517
   cagccctgag gcaagagaag aaagtacttc cagcggcaat gtaagcaaca gaaaggatga 60
   gacaaatgct cg                                                     72
<210> 1518
   <211> 68
   <212> DNA
   <213> Artificial Sequence
<220>
   <223> Synthetic oligonucleotide
<400> 1518
   gccaactgct ttcatttgtg agggatctga accaatacag agcagacata aaggaaatgg 60
   gcctgagt                                                          68
<210> 1519
   <211> 75
   <212> DNA
   <213> Artificial Sequence
<220>
   <223> Synthetic oligonucleotide
<400> 1519
   accagtcccc cagaagacta tcctgagccc gaggaagtcc ccccggaggt gatttccatc 60
   tacaacagca ccagg                                                  75
<210> 1520
   <211> 65
   <212> DNA
   <213> Artificial Sequence
<220>
   <223> Synthetic oligonucleotide
<400> 1520
   ggatcgagct cttccagatc cttcggccag atgagcacat tgccaaacag cgctatatcg 60
   gtggc                                                             65
<210> 1521
   <211> 66
   <212> DNA
   <213> Artificial Sequence
<220>
   <223> Synthetic oligonucleotide
<400> 1521
   aacaccaatg ggttccatct ttctgggctc ctgattgctc aagcacagtt tggcctgatg 60
   aagagg                                                            66
<210> 1522
   <211> 67
   <212> DNA
   <213> Artificial Sequence
<220>
   <223> Synthetic oligonucleotide
<400> 1522
   ctacctgcct tgctttgtga cttccaagaa cgagtgtctc tggaccgaca tgctctccaa 60
   tttcggt                                                           67
<210> 1523
   <211> 67
   <212> DNA
   <213> Artificial Sequence
<220>
   <223> Synthetic oligonucleotide
<400> 1523
   ccagcccaca gaccagttac tgttcctcac tgagcctgga agcaaatcca cacctccttt 60
   ctctgaa                                                           67
<210> 1524
   <211> 67
   <212> DNA
   <213> Artificial Sequence
<220>
   <223> Synthetic oligonucleotide
<400> 1524
   tggcgaccaa gacaccttga agggcctaat gcacgcacta aagcactcaa agacgtacca 60
   ctttccc                                                           67
<210> 1525
   <211> 71
   <212> DNA
   <213> Artificial Sequence
<220>
   <223> Synthetic oligonucleotide
<400> 1525
   catatcgttg gatcacagca catcagagca gagaaagcga tggtggatgg ctcatggtta 60
   gatctggcca a                                                      71
<210> 1526
   <211> 64
   <212> DNA
   <213> Artificial Sequence
<220>
   <223> Synthetic oligonucleotide
<400> 1526
   gcgctgcgga agatcatccc cacgctgccc tcggacaagc tgagcaagat tcagaccctc 60
   aagc                                                              64
<210> 1527
   <211> 522
   <212> DNA
   <213> Artificial Sequence
<220>
   <223> Synthetic oligonucleotide
<400> 1527
   gagtcgaccc tgcacctggt cctgcgtctg agaggtggta tgcagatctt cgtgaagacc 60
   ctgaccggca agaccatcac cctggaagtg gagcccagtg acaccatcga aaatgtgaag 120
   gccaagatcc aggataaaga aggcatccct cccgaccagc agaggctcat ctttgcaggc 180
   aagcagctgg aagatggccg cactctttct gactacaaca tccagaagga gtcgaccctg 240
   cacctggtcc tgcgtctgag aggtggtatg cagatcttcg tgaagaccct gaccggcaag 300
   accatcactc tggaagtgga gcccagtgac accatcgaaa atgtgaaggc caagatccaa 360
   gataaagaag gcatccctcc cgaccagcag aggctcatct ttgcaggcaa gcagctggaa 420
   gatggccgca ctctttctga ctacaacatc cagaaggagt cgaccctgca cctggtcctg 480
   cgcctgaggg gtggctgtta attcttcagt catggcattc gc                     522
<210> 1528
   <211> 89
   <212> DNA
   <213> Artificial Sequence
<220>
   <223> Synthetic oligonucleotide
<400> 1528
   tggcttcagg agctgaatac cctcccaggc acacacaggt gggacacaaa taagggtttt 60
   ggaaccacta ttttctcatc acgacagca                                   89
<210> 1529
   <211> 72
   <212> DNA
   <213> Artificial Sequence
<220>
   <223> Synthetic oligonucleotide
<400> 1529
   tgcccttaaa ggaaccaatg agtccctgga acgccagatg cgtgaaatgg aagagaactt 60
   tgccgttgaa gc                                                     72
<210> 1530
   <211> 67
   <212> DNA
   <213> Artificial Sequence
<220>
   <223> Synthetic oligonucleotide
<400> 1530
   agtcaatctt cgcacacggc gagtggacac tgtggaccct ccctacccac gctccatcgc 60
   tcagtac                                                           67
<210> 1531
   <211> 68
   <212> DNA
   <213> Artificial Sequence
<220>
   <223> Synthetic oligonucleotide
<400> 1531
   ctctccagtg tgggcaccag ccggccagaa cagatgcgag cagtccatga ctctgggagc 60
   tacaccgc                                                          68
<210> 1532
   <211> 75
   <212> DNA
   <213> Artificial Sequence
<220>
   <223> Synthetic oligonucleotide
<400> 1532
   agaggcatcc atgaacttca cacttgcggg ctgcatcagc acacgctcct atcaacccaa 60
   gtactgtgga gtttg                                                  75
<210> 1533
   <211> 75
   <212> DNA
   <213> Artificial Sequence
<220>
   <223> Synthetic oligonucleotide
<400> 1533
   gtatcaggac cacatgcagt acatcggaga aggcgcgaag acaggcatca aagaatgcca 60
   gtatcaattc cgaca                                                  75
<210> 1534
   <211> 79
   <212> DNA
   <213> Artificial Sequence
<220>
   <223> Synthetic oligonucleotide
<400> 1534
   tgtcttcagg gtcttgtcca gaatgtagat gggttccgta agaggcctgg tgctctctta 60
   ctctttcatc cacgtgcac                                              79
<210> 1535
   <211> 74
   <212> DNA
   <213> Artificial Sequence
<220>
   <223> Synthetic oligonucleotide
<400> 1535
   atcgcagctg gtgggtgtac acactgctgt ttaccttggc gaggcctttc accaagtcca 60
   tgcaacaggg agct                                                   74
<210> 1536
   <211> 81
   <212> DNA
   <213> Artificial Sequence
<220>
   <223> Synthetic oligonucleotide
<400> 1536
   gtggacatcg gatacccaag gagacgaagc tgaagcagga gaaggagggg aaaattaacc 60
   ggccttccaa cttttgtctg c                                           81

## Description

### INTRODUCTION

Oncologists have a number of treatment options available to them, including different combinations of therapeutic regimens that are characterized as "standard of care." The absolute benefit from adjuvant treatment is larger for patients with poor prognostic features, and this has resulted in the policy to select only these so-called 'high-risk' patients for adjuvant chemotherapy. See, e.g., S. Paik, et al., J Clin Oncol. 24(23):3726-34 (2006). Therefore, the best likelihood of good treatment outcome requires that patients be assigned to optimal available cancer treatment, and that this assignment be made as quickly as possible following diagnosis.

Today our healthcare system is riddled with inefficiency and wasteful spending - one example of this is that the efficacy rate of many oncology therapeutics working only about 25% of the time. Many of those cancer patients are experiencing toxic side effects for costly therapies that may not be working. This imbalance between high treatment costs and low therapeutic efficacy is often a result of treating a specific diagnosis one way across a diverse patient population. But with the advent of gene profiling tools, genomic testing, and advanced diagnostics, this is beginning to change.

In particular, once a patient is diagnosed with breast cancer there is a strong need for methods that allow the physician to predict the expected course of disease, including the likelihood of cancer recurrence, long-term survival of the patient, and the like, and select the most appropriate treatment option accordingly. Accepted prognostic and predictive factors in breast cancer include age, tumor size, axillary lymph node status, histological tumor type, pathological grade and hormone receptor status. Molecular diagnostics, however, have been demonstrated to identify more patients with a low risk of breast cancer than was possible with standard prognostic indicators. S. Paik, The Oncologist 12(6):631-635 (2007). Despite recent advances, the challenge of breast cancer treatment remains to target specific treatment regimens to pathogenically distinct tumor types, and ultimately personalize tumor treatment in order to maximize outcome. Accurate prediction of prognosis
and clinical outcome would allow the oncologist to tailor the administration of adjuvant chemotherapy such that women with a higher risk of a recurrence or poor prognosis would receive more aggressive treatment. Furthermore, accurately stratifying patients based on risk would greatly advance the understanding of expected absolute benefit from treatment, thereby increasing success rates for clinical trials for new breast cancer therapies.

Currently, most diagnostic tests used in clinical practice are frequently not quantitative, relying on immunohistochemistry (IHC). This method often yields different results in different laboratories, in part because the reagents are not standardized, and in part because the interpretations are subjective and cannot be easily quantified. Other RNA-based molecular diagnostics require fresh-frozen tissues, which presents a myriad of challenges including incompatibilities with current clinical practices and sample transport regulations. Fixed paraffin-embedded tissue is more readily available and methods have been established to detect RNA in fixed tissue. However, these methods typically do not allow for the study of large numbers of genes (DNA or RNA) from small amounts of material. Thus, traditionally fixed tissue has been rarely used other than for IHC detection of proteins. Karczewska et al., Cancer, v 88, no 9, p 2061-2071, 2000, investigates expression of IL6, IL6R and IL6ST in breast cancer.

### SUMMARY

The present invention is defined in the claims.

The present invention provides a gene, IL6ST, the expression level of which is associated with a good prognosis in breast cancer. The good prognosis assumes the patient receives the standard of care. The clinical outcome may be defined by clinical endpoints, such as disease or recurrence free survival, metastasis free survival, overall survival, etc.

The present invention accommodates the use of archived paraffin-embedded biopsy material for assay of the gene, and therefore is compatible with the most widely available type of biopsy material. It is also compatible with several different methods of tumor tissue harvest, for example, via core biopsy or fine needle aspiration. The tissue sample may comprise cancer cells.

In one aspect, the present disclosure concerns a method of predicting a clinical outcome of a cancer patient, comprising (a) obtaining an expression level of an expression product (e.g., an RNA transcript) of at least one prognostic gene listed in Tables 1-12 from a tissue sample obtained from a tumor of the patient; (b) normalizing the expression level of the expression product of the at least one prognostic gene, to obtain a normalized expression level; and (c) calculating a risk score based on the normalized expression value, wherein increased expression of prognostic genes in Tables 1, 3, 5, and 7 are positively correlated
with good prognosis, and wherein increased expression of prognostic genes in Tables 2, 4, 6, and 8 are negatively associated with good prognosis. In some embodiments, the tumor is estrogen receptor-positive. In other embodiments, the tumor is estrogen receptor negative.

In one aspect, the present disclosure provides a method of predicting a clinical outcome of a cancer patient, comprising (a) obtaining an expression level of an expression product (e.g., an RNA transcript) of at least one prognostic gene from a tissue sample obtained from a tumor of the patient, where the at least one prognostic gene is selected from GSTM2, IL6ST, GSTM3, C8orf4, TNFRSF11B, NAT1, RUNX1, CSF1, ACTR2, LMNB1, TFRC, LAPTM4B, ENO1, CDC20, and IDH2; (b) normalizing the expression level of the expression product of the at least one prognostic gene, to obtain a normalized expression level; and (c) calculating a risk score based on the normalized expression value, wherein increased expression of a prognostic gene selected from GSTM2, IL6ST, GSTM3, C8orf4, TNFRSF11B, NAT1, RUNX1, and CSF1 is positively correlated with good prognosis, and wherein increased expression of a prognostic gene selected from ACTR2, LMNB1, TFRC, LAPTM4B, ENO1, CDC20, and IDH2 is negatively associated with good prognosis. In some embodiments, the tumor is estrogen receptor-positive. In other embodiments, the tumor is estrogen receptor negative.

In various embodiments, the normalized expression level of at least 2, or at least 5, or at least 10, or at least 15, or at least 20, or a least 25 prognostic genes (as determined by assaying a level of an expression product of the gene) is determined. In alternative embodiments, the normalized expression levels of at least one of the genes that co-expresses with prognostic genes in Tables 16-18 is obtained.

In another embodiment, the risk score is determined using normalized expression levels of at least one a stromal or transferrin receptor group gene, or a gene that co-expresses with a stromal or transferrin receptor group gene.

In another embodiment, the cancer is breast cancer. In another embodiment, the patient is a human patient.

In yet another embodiment, the cancer is ER-positive breast cancer.

In yet another embodiment, the cancer is ER-negative breast cancer.

In a further embodiment, the expression product comprises RNA. For example, the RNA could be exonic RNA, intronic RNA, or short RNA (e.g., microRNA, siRNA, promoter-associated small RNA, shRNA, etc.). In various embodiments, the RNA is fragmented RNA.

In a different aspect, the invention concerns an array comprising polynucleotides hybridizing to an RNA transcription of at least one of the prognostic genes listed in Tables 1-12.

In a still further aspect, the invention concerns a method of preparing a personalized genomics profile for a patient, comprising (a) obtaining an expression level of an expression product (e.g., an RNA transcript) of at least one prognostic gene listed in Tables 1-12 from a tissue sample obtained from a tumor of the patient; (b) normalizing the expression level of the expression product of the at least one prognostic gene to obtain a normalized expression level; and (c) calculating a risk score based on the normalized expression value, wherein increased expression of prognostic genes in Tables 1, 3, 5, and 7 are positively correlated with good prognosis, and wherein increased expression of prognostic genes in Tables 2, 4, 6, and 8 are negatively associated with good prognosis. In some embodiments, the tumor is estrogen receptor-positive, and in other embodiments the tumor is estrogen receptor negative.

In various embodiments, a subject method can further include providing a report. The report may include prediction of the likelihood of risk that said patient will have a particular clinical outcome.

The invention further provides a computer-implemented method for classifying a cancer patient based on risk of cancer recurrence, comprising (a) classifying, on a computer, said patient as having a good prognosis or a poor prognosis based on an expression profile comprising measurements of expression levels of expression products of a plurality of prognostic genes in a tumor tissue sample taken from the patient, said plurality of genes comprising at least three different prognostic genes listed in any of Tables 1-12, wherein a good prognosis predicts no recurrence or metastasis within a predetermined period after initial diagnosis, and wherein a poor prognosis predicts recurrence or metastasis within said predetermined period after initial diagnosis; and (b) calculating a risk score based on said expression levels.

### DETAILED DESCRIPTION

### DEFINITIONS

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, NY 1994), and March, Advanced Organic Chemistry Reactions, Mechanisms and Structure 4th ed., John Wiley & Sons (New York, NY 1992), provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

"Prognostic factors" are those variables related to the natural history of cancer, which influence the recurrence rates and outcome of patients once they have developed cancer. Clinical parameters that have been associated with a worse prognosis include, for example, lymph node involvement, and high grade tumors. Prognostic factors are frequently used to categorize patients into subgroups with different baseline relapse risks.

The term "prognosis" is used herein to refer to the prediction of the likelihood of cancer-attributable death or progression, including recurrence, metastatic spread, and drug resistance, of a neoplastic disease, such as breast cancer. The term "good prognosis" means a desired or "positive" clinical outcome. For example, in the context of breast cancer, a good prognosis may be an expectation of no recurrences or metastasis within two, three, four, five or more years of the initial diagnosis of breast cancer. The terms "bad prognosis" or "poor prognosis" are used herein interchangeably herein to mean an undesired clinical outcome. For example, in the context of breast cancer, a bad prognosis may be an expectation of a recurrence or metastasis within two, three, four, five or more years of the initial diagnosis of breast cancer.

The term "prognostic gene" is used herein to refer to a gene, the expression of which is correlated, positively or negatively, with a good prognosis for a cancer patient treated with the standard of care. A gene may be both a prognostic and predictive gene, depending on the correlation of the gene expression level with the corresponding endpoint. For example, using a Cox proportional hazards model, if a gene is only prognostic, its hazard ratio (HR) does not change when measured in patients treated with the standard of care or in patients treated with a new intervention.

The term "predictive gene" is used herein to refer to a gene, the expression of which is correlated, positively or negatively, with response to a beneficial response to treatment. For example, treatment could include chemotherapy.

The terms "risk score" or "risk classification" are used interchangeably herein to describe a level of risk (or likelihood) that a patient will experience a particular clinical outcome. A patient may be classified into a risk group or classified at a level of risk based on the methods of the present disclosure, e.g. high, medium, or low risk. A "risk group" is a group of subjects or individuals with a similar level of risk for a particular clinical outcome.

A clinical outcome can be defined using different endpoints. The term "long-term" survival is used herein to refer to survival for a particular time period, e.g., for at least 3 years, more preferably for at least 5 years. The term "Recurrence-Free Survival" (RFS) is used herein to refer to survival for a time period (usually in years) from randomization to first cancer recurrence or death due to recurrence of cancer. The term "Overall Survival" (OS) is used herein to refer to the time (in years) from randomization to death from any cause. The term "Disease-Free Survival" (DFS) is used herein to refer to survival for a time period (usually in years) from randomization to first cancer recurrence or death from any cause.

The calculation of the measures listed above in practice may vary from study to study depending on the definition of events to be either censored or not considered.

The term "biomarker" as used herein refers to a gene, the expression level of which, as measured using a gene product.

The term "microarray" refers to an ordered arrangement of hybridizable array elements, preferably polynucleotide probes, on a substrate.

As used herein, the term "normalized expression level" as applied to a gene refers to the normalized level of a gene product, e.g. the normalized value determined for the RNA expression level of a gene or for the polypeptide expression level of a gene.

The term "Cₜ" as used herein refers to threshold cycle, the cycle number in quantitative polymerase chain reaction (qPCR) at which the fluorescence generated within a reaction well exceeds the defined threshold, i.e. the point during the reaction at which a sufficient number of amplicons have accumulated to meet the defined threshold.

The term "gene product" or "expression product" are used herein to refer to the RNA transcription products (transcripts) of the gene, including mRNA, and the polypeptide translation products of such RNA transcripts. A gene product can be, for example, an unspliced RNA, an mRNA, a splice variant mRNA, a microRNA, a fragmented RNA, a polypeptide, a post-translationally modified polypeptide, a splice variant polypeptide, etc.

The term "RNA transcript" as used herein refers to the RNA transcription products of a gene, including, for example, mRNA, an unspliced RNA, a splice variant mRNA, a microRNA, and a fragmented RNA. "Fragmented RNA" as used herein refers to RNA a mixture of intact RNA and RNA that has been degraded as a result of the sample processing (e.g., fixation, slicing tissue blocks, etc.).

Unless indicated otherwise, each gene name used herein corresponds to the Official Symbol assigned to the gene and provided by Entrez Gene (URL: www.ncbi.nlm.nih.gov/sites/entrez) as of the filing date of this application.

The terms "correlated" and "associated" are used interchangeably herein to refer to a strength of association between two measurements (or measured entities). The disclosure provides genes and gene subsets, the expression levels of which are associated with a particular outcome measure. For example, the increased expression level of a gene may be positively correlated (positively associated) with an increased likelihood of good clinical outcome for the patient, such as an increased likelihood of long-term survival without recurrence of the cancer and/or metastasis-free survival. Such a positive correlation may be demonstrated statistically in various ways, e.g. by a low hazard ratio (e.g. HR < 1.0). In another example, the increased expression level of a gene may be negatively correlated (negatively associated) with an increased likelihood of good clinical outcome for the patient. In that case, for example, the patient may have a decreased likelihood of long-term survival without recurrence of the cancer and/or cancer metastasis, and the like. Such a negative correlation indicates that the patient likely has a poor prognosis, e.g., a high hazard ratio (e.g., HR > 1.0). "Correlated" is also used herein to refer to a strength of association between the expression levels of two different genes, such that expression level of a first gene can be substituted with an expression level of a second gene in a given algorithm in view of their correlation of expression. Such "correlated expression" of two genes that are substitutable in an algorithm usually gene expression levels that are positively correlated with one another, e.g., if increased expression of a first gene is positively correlated with an outcome (e.g., increased likelihood of good clinical outcome), then the second gene that is co-expressed and exhibits correlated expression with the first gene is also positively correlated with the same outcome

The term "recurrence," as used herein, refers to local or distant (metastasis) recurrence of cancer. For example, breast cancer can come back as a local recurrence (in the treated breast or near the tumor surgical site) or as a distant recurrence in the body. The most common sites of breast cancer recurrence include the lymph nodes, bones, liver, or lungs.

The term "polynucleotide," when used in singular or plural, generally refers to any polyribonucleotide or polydeoxribonucleotide, which may be unmodified RNA or DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. The term "polynucleotide" specifically includes cDNAs. The term includes DNAs (including cDNAs) and RNAs that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of viruses and cells, including simple and complex cells.

The term "oligonucleotide" refers to a relatively short polynucleotide, including, without limitation, single-stranded deoxyribonucleotides, single- or double-stranded ribonucleotides, RNA:DNA hybrids and double-stranded DNAs. Oligonucleotides, such as single-stranded DNA probe oligonucleotides, are often synthesized by chemical methods, for example using automated oligonucleotide synthesizers that are commercially available. However, oligonucleotides can be made by a variety of other methods, including *in vitro* recombinant DNA-mediated techniques and by expression of DNAs in cells and organisms.

The phrase "amplification" refers to a process by which multiple copies of a gene or RNA transcript are formed in a particular sample or cell line. The duplicated region (a stretch of amplified polynucleotide) is often referred to as "amplicon." Usually, the amount of the messenger RNA (mRNA) produced, *i*.*e*., the level of gene expression, also increases in the proportion of the number of copies made of the particular gene expressed.

The term "estrogen receptor (ER)" designates the estrogen receptor status of a cancer patient. A tumor is ER-positive if there is a significant number of estrogen receptors present in the cancer cells, while ER-negative indicates that the cells do not have a significant number of receptors present. The definition of "significant" varies amongst testing sites and methods (e.g., immunohistochemistry, PCR). The ER status of a cancer patient can be evaluated by various known means. For example, the ER level of breast cancer is determined by measuring an expression level of a gene encoding the estrogen receptor in a breast tumor sample obtained from a patient.

The term "tumor," as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Examples of cancer include, but are not limited to, breast cancer, ovarian cancer, colon cancer, lung cancer, prostate cancer, hepatocellular cancer, gastric cancer, pancreatic cancer, cervical cancer, liver cancer, bladder cancer, cancer of the urinary tract, thyroid cancer, renal cancer, carcinoma, melanoma, and brain cancer.

The gene subset identified herein as the "stromal group" includes genes that are synthesized predominantly by stromal cells and are involved in stromal response and genes that co-express with stromal group genes. "Stromal cells" are defined herein as connective tissue cells that make up the support structure of biological tissues. Stromal cells include fibroblasts, immune cells, pericytes, endothelial cells, and inflammatory cells. "Stromal response" refers to a desmoplastic response of the host tissues at the site of a primary tumor or invasion. See, e.g., E. Rubin, J. Farber, Pathology, 985-986 (2nd Ed. 1994). The stromal group includes, for example, CDH11, TAGLN, ITGA4, INHBA, COLIA1, COLIA2, FN1, CXCL14, TNFRSF1, CXCL12, C10ORF116, RUNX1, GSTM2, TGFB3, CAV1, DLC1, TNFRSF10, F3, and DICER1, and co-expressed genes identified in Tables 16-18.

The gene subset identified herein as the "metabolic group" includes genes that are associated with cellular metabolism, including genes associated with carrying proteins for transferring iron, the cellular iron homeostasis pathway, and homeostatic biochemical metabolic pathways, and genes that co-express with metabolic group genes. The metabolic group includes, for example, TFRC, ENO1, IDH2, ARF1, CLDN4, PRDX1, and GBP1, and co-expressed genes identified in Tables 16-18.

The gene subset identified herein as the "immune group" includes genes that are involved in cellular immunoregulatory functions, such as T and B cell trafficking, lymphocyte-associated or lymphocyte markers, and interferon regulation genes, and genes that co-express with immune group genes. The immune group includes, for example, CCL19 and IRF1, and co-expressed genes identified in Tables 16-18.

The gene subset identified herein as the "proliferation group" includes genes that are associated with cellular development and division, cell cycle and mitotic regulation, angiogenesis, cell replication, nuclear transport/stability, wnt signaling, apoptosis, and genes that co-express with proliferation group genes. The proliferation group includes, for example, PGF, SPC25, AURKA, BIRC5, BUB1, CCNB1, CENPA, KPNA, LMNB1, MCM2, MELK, NDC80, TPX2M, and WISP1, and co-expressed genes identified in Tables 16-18.

The term "co-expressed", as used herein, refers to a statistical correlation between the expression level of one gene and the expression level of another gene. Pairwise co-expression may be calculated by various methods known in the art, e.g., by calculating Pearson correlation coefficients or Spearman correlation coefficients. Co-expressed gene cliques may also be identified using a graph theory.

As used herein, the terms "gene clique" and "clique" refer to a subgraph of a graph in which every vertex is connected by an edge to every other vertex of the subgraph.

As used herein, a "maximal clique" is a clique in which no other vertex can be added and still be a clique.

The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, neoplasia, premalignancy, malignancy, invasion of surrounding or distant tissues or organs, such as lymph nodes, etc.

A "computer-based system" refers to a system of hardware, software, and data storage medium used to analyze information. The minimum hardware of a patient computer-based system comprises a central processing unit (CPU), and hardware for data input, data output (e.g., display), and data storage. An ordinarily skilled artisan can readily appreciate that any currently available computer-based systems and/or components thereof are suitable for use in connection with the methods of the present disclosure. The data storage medium may comprise any manufacture comprising a recording of the present information as described above, or a memory access device that can access such a manufacture.

To "record" data, programming or other information on a computer readable medium refers to a process for storing information, using any such methods as known in the art. Any convenient data storage structure may be chosen, based on the means used to access the stored information. A variety of data processor programs and formats can be used for storage, e.g. word processing text file, database format, etc.

A "processor" or "computing means" references any hardware and/or software combination that will perform the functions required of it. For example, a suitable processor may be a programmable digital microprocessor such as available in the form of an electronic controller, mainframe, server or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic medium or optical disk may carry the programming, and can be read by a suitable reader communicating with each processor at its corresponding station.

As used herein, "graph theory" refers to a field of study in Computer Science and Mathematics in which situations are represented by a diagram containing a set of points and lines connecting some of those points. The diagram is referred to as a "graph", and the points and lines referred to as "vertices" and "edges" of the graph. In terms of gene co-expression analysis, a gene (or its equivalent identifier, e.g. an array probe) may be represented as a node or vertex in the graph. If the measures of similarity (e.g., correlation coefficient, mutual information, and alternating conditional expectation) between two genes are higher than a significant threshold, the two genes are said to be co-expressed and an edge will be drawn in the graph. When co-expressed edges for all possible gene pairs for a given study have been drawn, all maximal cliques are computed. The resulting maximal clique is defined as a gene clique. A gene clique is a computed co-expressed gene group that meets predefined criteria.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1 % Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

In the context of the present invention, reference to "at least one," "at least two," "at least five," etc. of the genes listed in any particular gene set means any one or any and all combinations of the genes listed.

The term "node negative" cancer, such as "node negative" breast cancer, is used herein to refer to cancer that has not spread to the lymph nodes.

The terms "splicing" and "RNA splicing" are used interchangeably and refer to RNA processing that removes introns and joins exons to produce mature mRNA with continuous coding sequence that moves into the cytoplasm of a eukaryotic cell.

In theory, the term "exon" refers to any segment of an interrupted gene that is represented in the mature RNA product (B. Lewin. Genes IV Cell Press, Cambridge Mass. 1990). In theory the term "intron" refers to any segment of DNA that is transcribed but removed from within the transcript by splicing together the exons on either side of it. Operationally, exon sequences occur in the mRNA sequence of a gene as defined by Ref. SEQ ID numbers. Operationally, intron sequences are the intervening sequences within the genomic DNA of a gene, bracketed by exon sequences and having GT and AG splice consensus sequences at their 5' and 3' boundaries.

### GENE EXPRESSION ASSAY

The present disclosure provides methods that employ, unless otherwise indicated, conventional techniques of molecular biology (including recombinant techniques), microbiology, cell biology, and biochemistry, which are within the skill of the art. Such techniques are explained fully in the literature, such as, "Molecular Cloning: A Laboratory Manual", 2nd edition (Sambrook et al., 1989); "Oligonucleotide Synthesis" (M.J. Gait, ed., 1984); "Animal Cell Culture" (R.I. Freshney, ed., 1987); "Methods in Enzymology" (Academic Press, Inc.); "Handbook of Experimental Immunology", 4th edition (D.M. Weir & C.C. Blackwell, eds., Blackwell Science Inc., 1987); "Gene Transfer Vectors for Mammalian Cells" (J.M. Miller & M.P. Calos, eds., 1987); "Current Protocols in Molecular Biology" (F.M. Ausubel et al., eds., 1987); and "PCR: The Polymerase Chain Reaction", (Mullis et al., eds., 1994).

### 1. Gene Expression Profiling

Methods of gene expression profiling include methods based on hybridization analysis of polynucleotides, methods based on sequencing of polynucleotides, and proteomics-based methods. The most commonly used methods known in the art for the quantification of mRNA expression in a sample include northern blotting and in situ hybridization (Parker & Barnes, Methods in Molecular Biology 106:247-283 (1999)); RNAse protection assays (Hod, Biotechniques 13:852-854 (1992)); and PCR-based methods, such as reverse transcription polymerase chain reaction (RT-PCR) (Weis et al., Trends in Genetics 8:263-264 (1992)). Alternatively, antibodies may be employed that can recognize specific duplexes, including DNA duplexes, RNA duplexes, and DNA-RNA hybrid duplexes or DNA-protein duplexes.

### 2. PCR-based Gene Expression Profiling Methods

### a. Reverse Transcriptase PCR (RT-PCR)

Of the techniques listed above, the most sensitive and most flexible quantitative method is RT-PCR, which can be used to compare mRNA levels in different sample populations, in normal and tumor tissues, with or without drug treatment, to characterize patterns of gene expression, to discriminate between closely related mRNAs, and to analyze RNA structure.

The first step is the isolation of mRNA from a target sample. The starting material is typically total RNA isolated from human tumors or tumor cell lines, and corresponding normal tissues or cell lines, respectively. Thus RNA can be isolated from a variety of primary tumors, including breast, lung, colon, prostate, brain, liver, kidney, pancreas, spleen, thymus, testis, ovary, uterus, etc., tumor, or tumor cell lines, with pooled DNA from healthy donors. If the source of mRNA is a primary tumor, mRNA can be extracted, for example, from frozen or archived paraffin-embedded and fixed (e.g. formalin-fixed) tissue samples.

General methods for mRNA extraction are well known in the art and are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56:A67 (1987), and De Andrés et al., BioTechniques 18:42044 (1995). In particular, RNA isolation can be performed using purification kit, buffer set and protease from commercial manufacturers, such as Qiagen, according to the manufacturer's instructions. For example, total RNA from cells in culture can be isolated using Qiagen RNeasy mini-columns. Other commercially available RNA isolation kits include MasterPure™ Complete DNA and RNA Purification Kit (EPICENTRE®, Madison, WI), and Paraffin Block RNA Isolation Kit (Ambion, Inc.). Total RNA from tissue samples can be isolated using RNA Stat-60 (Tel-Test). RNA prepared from tumor can be isolated, for example, by cesium chloride density gradient centrifugation.

In some cases, it may be appropriate to amplify RNA prior to initiating expression profiling. It is often the case that only very limited amounts of valuable clinical specimens are available for molecular analysis. This may be due to the fact that the tissues have already be used for other laboratory analyses or may be due to the fact that the original specimen is very small as in the case of needle biopsy or very small primary tumors. When tissue is limiting in quantity it is generally also the case that only small amounts of total RNA can be recovered from the specimen and as a result only a limited number of genomic markers can be analyzed in the specimen. RNA amplification compensates for this limitation by faithfully reproducing the original RNA sample as a much larger amount of RNA of the same relative composition. Using this amplified copy of the original RNA specimen, unlimited genomic analysis can be done to discovery biomarkers associated with the clinical characteristics of the original biological sample. This effectively immortalizes clinical study specimens for the purposes of genomic analysis and biomarker discovery.

As RNA cannot serve as a template for PCR, the first step in gene expression profiling by real-time RT-PCR (RT-PCR) is the reverse transcription of the RNA template into cDNA, followed by its exponential amplification in a PCR reaction. The two most commonly used reverse transcriptases are avian myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, CA, USA), following the manufacturer's instructions. The derived cDNA can then be used as a template in the subsequent PCR reaction. For further details see, e.g. Held et al., Genome Research 6:986-994 (1996).

Although the PCR step can use a variety of thermostable DNA-dependent DNA polymerases, it typically employs the Taq DNA polymerase, which has a 5'-3' nuclease activity but lacks a 3'-5' proofreading endonuclease activity. Thus, TaqMan® PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TaqMan® RT-PCR can be performed using commercially available equipment, such as, for example, ABI PRISM 7900® Sequence Detection System™ (Perkin-Elmer-Applied Biosystems, Foster City, CA, USA), or LightCycler® 480 Real-Time PCR System (Roche Diagnostics, GmbH, Penzberg, Germany). In a preferred embodiment, the 5' nuclease procedure is run on a real-time quantitative PCR device such as the ABI PRISM 7900® Sequence Detection System™. The system consists of a thermocycler, laser, charge-coupled device (CCD), camera and computer. The system amplifies samples in a 384-well format on a thermocycler. During amplification, laser-induced fluorescent signal is collected in real-time through fiber optics cables for all 384 wells, and detected at the CCD. The system includes software for running the instrument and for analyzing the data.

5'-Nuclease assay data are initially expressed as Ct, or the threshold cycle. As discussed above, fluorescence values are recorded during every cycle and represent the amount of product amplified to that point in the amplification reaction. The point when the fluorescent signal is first recorded as statistically significant is the threshold cycle (Cₜ).

To minimize errors and the effect of sample-to-sample variation, RT-PCR is usually performed using an internal standard. The ideal internal standard is expressed at a constant level among different tissues, and is unaffected by the experimental treatment. RNAs most frequently used to normalize patterns of gene expression are mRNAs for the housekeeping genes glyceraldehyde-3-phosphate-dehydrogenase (GAPDH) and β-actin.

The steps of a representative protocol for profiling gene expression using fixed, paraffin-embedded tissues as the RNA source, including mRNA isolation, purification, primer extension and amplification are given in various published journal articles. M. Cronin, Am J Pathol 164(1):35-42 (2004). Briefly, a representative process starts with cutting about 10 µm thick sections of paraffin-embedded tumor tissue samples. The RNA is then extracted, and protein and DNA are removed. After analysis of the RNA concentration, RNA repair and/or amplification steps may be included, if necessary, and RNA is reverse transcribed using gene specific primers followed by RT-PCR.

### b. Design of Intron-Based PCR Primers and Probes

PCR primers and probes can be designed based upon exon or intron sequences present in the mRNA transcript of the gene of interest. Prior to carrying out primer/probe design, it is necessary to map the target gene sequence to the human genome assembly in order to identify intron-exon boundaries and overall gene structure. This can be performed using publicly available software, such as Primer3 (Whitehead Inst.) and Primer Express® (Applied Biosystems).

Where necessary or desired, repetitive sequences of the target sequence can be masked to mitigate non-specific signals. Exemplary tools to accomplish this include the Repeat Masker program available on-line through the Baylor College of Medicine, which screens DNA sequences against a library of repetitive elements and returns a query sequence in which the repetitive elements are masked. The masked intron and exon sequences can then be used to design primer and probe sequences for the desired target sites using any commercially or otherwise publicly available primer/probe design packages, such as Primer Express (Applied Biosystems); MGB assay-by-design (Applied Biosystems); Primer3 (Steve Rozen and Helen J. Skaletsky (2000) Primer3 on the WWW for general users and for biologist programmers. In: Rrawetz S, Misener S (eds) Bioinformatics Methods and Protocols: Methods in Molecular Biology. Humana Press, Totowa, NJ, pp 365-386).

Other factors that can influence PCR primer design include primer length, melting temperature (Tm), and G/C content, specificity, complementary primer sequences, and 3 '-end sequence. In general, optimal PCR primers are generally 17-30 bases in length, and contain about 20-80%, such as, for example, about 50-60% G+C bases, and exhibit Tm's between 50 and 80 ⁰C, e.g. about 50 to 70 ⁰C.

For further guidelines for PCR primer and probe design see, e.g. Dieffenbach, CW. et al, "General Concepts for PCR Primer Design" in: PCR Primer, A Laboratory Manual, Cold Spring Harbor Laboratory Press,. New York, 1995, pp. 133-155; Innis and Gelfand, "Optimization of PCRs" in: PCR Protocols, A Guide to Methods and Applications, CRC Press, London, 1994, pp. 5-11; and Plasterer, T.N. Primerselect: Primer and probe design. Methods Mol. Biol. 70:520-527 (1997).

Table A provides further information concerning the primer, probe, and amplicon sequences associated with the Examples disclosed herein.

### c. MassARRAY System

In the MassARRAY-based gene expression profiling method, developed by Sequenom, Inc. (San Diego, CA) following the isolation of RNA and reverse transcription, the obtained cDNA is spiked with a synthetic DNA molecule (competitor), which matches the targeted cDNA region in all positions, except a single base, and serves as an internal standard. The cDNA/competitor mixture is PCR amplified and is subjected to a post-PCR shrimp alkaline phosphatase (SAP) enzyme treatment, which results in the dephosphorylation of the remaining nucleotides. After inactivation of the alkaline phosphatase, the PCR products from the competitor and cDNA are subjected to primer extension, which generates distinct mass signals for the competitor- and cDNA-derives PCR products. After purification, these products are dispensed on a chip array, which is preloaded with components needed for analysis with matrix-assisted laser desorption ionization time-of-flight mass spectrometry (MALDI-TOF MS) analysis. The cDNA present in the reaction is then quantified by analyzing the ratios of the peak areas in the mass spectrum generated. For further details see, e.g. Ding and Cantor, Proc. Natl. Acad. Sci. USA 100:3059-3064 (2003).

### d. Other PCR-based Methods

Further PCR-based techniques include, for example, differential display (Liang and Pardee, Science 257:967-971 (1992)); amplified fragment length polymorphism (iAFLP) (Kawamoto et al., Genome Res. 12:1305-1312 (1999)); BeadArray™ technology (Illumina, San Diego, CA; Oliphant et al., Discovery of Markers for Disease (Supplement to Biotechniques), June 2002; Ferguson et al., Analytical Chemistry 72:5618 (2000)); BeadsArray for Detection of Gene Expression (BADGE), using the commercially available Luminex100 LabMAP system and multiple color-coded microspheres (Luminex Corp., Austin, TX) in a rapid assay for gene expression (Yang et al., Genome Res. 11:1888-1898 (2001)); and high coverage expression profiling (HiCEP) analysis (Fukumura et al., Nucl. Acids. Res. 31(16) e94 (2003)).

### 3. Microarrays

Differential gene expression can also be identified, or confirmed using the microarray technique. Thus, the expression profile of breast cancer-associated genes can be measured in either fresh or paraffin-embedded tumor tissue, using microarray technology. In this method, polynucleotide sequences of interest (including cDNAs and oligonucleotides) are plated, or arrayed, on a microchip substrate. The arrayed sequences are then hybridized with specific DNA probes from cells or tissues of interest. Just as in the RT-PCR method, the source of mRNA typically is total RNA isolated from human tumors or tumor cell lines, and corresponding normal tissues or cell lines. Thus RNA can be isolated from a variety of primary tumors or tumor cell lines. If the source of mRNA is a primary tumor, mRNA can be extracted, for example, from frozen or archived paraffin-embedded and fixed (e.g. formalin-fixed) tissue samples, which are routinely prepared and preserved in everyday clinical practice.

In a specific embodiment of the microarray technique, PCR amplified inserts of cDNA clones are applied to a substrate in a dense array. Preferably at least 10,000 nucleotide sequences are applied to the substrate. The microarrayed genes, immobilized on the microchip at 10,000 elements each, are suitable for hybridization under stringent conditions. Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After stringent washing to remove non-specifically bound probes, the chip is scanned by confocal laser microscopy or by another detection method, such as a CCD camera. Quantitation of hybridization of each arrayed element allows for assessment of corresponding mRNA abundance. With dual color fluorescence, separately labeled cDNA probes generated from two sources of RNA are hybridized pairwise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately twofold differences in the expression levels (Schena et al., Proc. Natl. Acad. Sci. USA 93(2):106-149 (1996)). Microarray analysis can be performed by commercially available equipment, following manufacturer's protocols, such as by using the Affymetrix GenChip technology, or Agilent's microarray technology.

The development of microarray methods for large-scale analysis of gene expression makes it possible to search systematically for molecular markers of cancer classification and outcome prediction in a variety of tumor types.

### 4. Gene Expression Analysis by Nucleic Acid Sequencing

Nucleic acid sequencing technologies are suitable methods for analysis of gene expression. The principle underlying these methods is that the number of times a cDNA sequence is detected in a sample is directly related to the relative expression of the mRNA corresponding to that sequence. These methods are sometimes referred to by the term Digital Gene Expression (DGE) to reflect the discrete numeric property of the resulting data. Early methods applying this principle were Serial Analysis of Gene Expression (SAGE) and Massively Parallel Signature Sequencing (MPSS). See, e.g., S. Brenner, et al., Nature Biotechnology 18(6):630-634 (2000). More recently, the advent of "next-generation" sequencing technologies has made DGE simpler, higher throughput, and more affordable. As a result, more laboratories are able to utilize DGE to screen the expression of more genes in more individual patient samples than previously possible. See, e.g., J. Marioni, Genome Research 18(9):1509-1517 (2008); R. Morin, Genome Research 18(4):610-621 (2008); A. Mortazavi, Nature Methods 5(7):621-628 (2008); N. Cloonan, Nature Methods 5(7):613-619 (2008).

### 5. Isolating RNA from Body Fluids

Methods of isolating RNA for expression analysis from blood, plasma and serum (See for example, Tsui NB et al. (2002) 48,1647-53 and references cited therein) and from urine (See for example, Boom R et al. (1990) J Clin Microbiol. 28, 495-503 and reference cited therein) have been described.

### 6. Immunohistochemistry

Immunohistochemistry methods are also suitable for detecting the expression levels of the prognostic marker of the present invention. Thus, antibodies or antisera, preferably polyclonal antisera, and most preferably monoclonal antibodies specific for each marker are used to detect expression. The antibodies can be detected by direct labeling of the antibodies themselves, for example, with radioactive labels, fluorescent labels, hapten labels such as, biotin, or an enzyme such as horse radish peroxidase or alkaline phosphatase. Alternatively, unlabeled primary antibody is used in conjunction with a labeled secondary antibody, comprising antisera, polyclonal antisera or a monoclonal antibody specific for the primary antibody. Immunohistochemistry protocols and kits are well known in the art and are commercially available.

### 7. Proteomics

The term "proteome" is defined as the totality of the proteins present in a sample (e.g. tissue, organism, or cell culture) at a certain point of time. Proteomics includes, among other things, study of the global changes of protein expression in a sample (also referred to as "expression proteomics"). Proteomics typically includes the following steps: (1) separation of individual proteins in a sample by 2-D gel electrophoresis (2-D PAGE); (2) identification of the individual proteins recovered from the gel, e.g. my mass spectrometry or N-terminal sequencing, and (3) analysis of the data using bioinformatics. Proteomics methods are valuable supplements to other methods of gene expression profiling, and can be used, alone or in combination with other methods, to detect the products of the prognostic marker of the present invention.

### 8. General Description of the mRNA Isolation, Purification, and Amplification

The steps of a representative protocol for profiling gene expression using fixed, paraffin-embedded tissues as the RNA source, including mRNA isolation, purification, primer extension and amplification are provided in various published journal articles (for example: T.E. Godfrey et al,. J. Molec. Diagnostics 2: 84-91 [2000]; K. Specht et al., Am. J. Pathol. 158: 419-29 [2001]). Briefly, a representative process starts with cutting about 10 µm thick sections of paraffin-embedded tumor tissue samples. The RNA is then extracted, and protein and DNA are removed. After analysis of the RNA concentration, RNA repair and/or amplification steps may be included, if necessary, and RNA is reverse transcribed using gene specific primers followed by RT-PCR. Finally, the data are analyzed to identify the best treatment option(s) available to the patient on the basis of the characteristic gene expression pattern identified in the tumor sample examined, dependent on the predicted likelihood of cancer recurrence.

### 9. Normalization

The expression data used in the methods disclosed herein can be normalized. Normalization refers to a process to correct for (normalize away), for example, differences in the amount of RNA assayed and variability in the quality of the RNA used, to remove unwanted sources of systematic variation in Cₜ measurements, and the like. With respect to RT-PCR experiments involving archived fixed paraffin embedded tissue samples, sources of systematic variation are known to include the degree of RNA degradation relative to the age of the patient sample and the type of fixative used to preserve the sample. Other sources of systematic variation are attributable to laboratory processing conditions.

Assays can provide for normalization by incorporating the expression of certain normalizing genes, which genes do not significantly differ in expression levels under the relevant conditions. Exemplary normalization genes include housekeeping genes such as PGK1 and UBB. (See, e.g., E. Eisenberg, et al., Trends in Genetics 19(7):362-365 (2003).) Normalization can be based on the mean or median signal (C_{T}) of all of the assayed genes or a large subset thereof (global normalization approach). In general, the normalizing genes, also referred to as reference genes should be genes that are known not to exhibit significantly different expression in colorectal cancer as compared to non-cancerous colorectal tissue, and are not significantly affected by various sample and process conditions, thus provide for normalizing away extraneous effects.

Unless noted otherwise, normalized expression levels for each mRNA/tested tumor/patient will be expressed as a percentage of the expression level measured in the reference set. A reference set of a sufficiently high number (e.g. 40) of tumors yields a distribution of normalized levels of each mRNA species. The level measured in a particular tumor sample to be analyzed falls at some percentile within this range, which can be determined by methods well known in the art.

In exemplary embodiments, one or more of the following genes are used as references by which the expression data is normalized: AAMP, ARF1, EEF1A1, ESD, GPS1, H3F3A, HNRPC, RPL13A, RPL41, RPS23, RPS27, SDHA, TCEA1, UBB, YWHAZ, B-actin, GUS, GAPDH, RPLPO, and TFRC. For example, the calibrated weighted average Cₜ measurements for each of the prognostic genes may be normalized relative to the mean of at least three reference genes, at least four reference genes, or at least five reference genes.

Those skilled in the art will recognize that normalization may be achieved in numerous ways, and the techniques described above are intended only to be exemplary, not exhaustive.

### REPORTING RESULTS

The methods of the present disclosure are suited for the preparation of reports summarizing the expected or predicted clinical outcome resulting from the methods of the present disclosure. A "report," as described herein, is an electronic or tangible document that includes report elements that provide information of interest relating to a likelihood assessment or a risk assessment and its results. A subject report includes at least a likelihood assessment or a risk assessment, e.g., an indication as to the risk of recurrence of breast cancer, including local recurrence and metastasis of breast cancer. A subject report can include an assessment or estimate of one or more of disease-free survival, recurrence-free survival, metastasis-free survival, and overall survival. A subject report can be completely or partially electronically generated, e.g., presented on an electronic display (e.g., computer monitor). A report can further include one or more of: 1) information regarding the testing facility; 2) service provider information; 3) patient data; 4) sample data; 5) an interpretive report, which can include various information including: a) indication; b) test data, where test data can include a normalized level of one or more genes of interest, and 6) other features.

The present disclosure thus provides for methods of creating reports and the reports resulting therefrom. The report may include a summary of the expression levels of the RNA transcripts, or the expression products of such RNA transcripts, for certain genes in the cells obtained from the patient's tumor. The report can include information relating to prognostic covariates of the patient. The report may include an estimate that the patient has an increased risk of recurrence. That estimate may be in the form of a score or patient stratifier scheme (e.g., low, intermediate, or high risk of recurrence). The report may include information relevant to assist with decisions about the appropriate surgery (e.g., partial or total mastectomy) or treatment for the patient.

Thus, in some embodiments, the methods of the present disclosure further include generating a report that includes information regarding the patient's likely clinical outcome, e.g. risk of recurrence. For example, the methods disclosed herein can further include a step of generating or outputting a report providing the results of a subject risk assessment, which report can be provided in the form of an electronic medium (e.g., an electronic display on a computer monitor), or in the form of a tangible medium (e.g., a report printed on paper or other tangible medium).

A report that includes information regarding the patient's likely prognosis (e.g., the likelihood that a patient having breast cancer will have a good prognosis or positive clinical outcome in response to surgery and/or treatment) is provided to a user. An assessment as to the likelihood is referred to below as a "risk report" or, simply, "risk score." A person or entity that prepares a report ("report generator") may also perform the likelihood assessment. The report generator may also perform one or more of sample gathering, sample processing, and data generation, e.g., the report generator may also perform one or more of: a) sample gathering; b) sample processing; c) measuring a level of a risk gene; d) measuring a level of a reference gene; and e) determining a normalized level of a risk gene. Alternatively, an entity other than the report generator can perform one or more sample gathering, sample processing, and data generation.

For clarity, it should be noted that the term "user," which is used interchangeably with "client," is meant to refer to a person or entity to whom a report is transmitted, and may be the same person or entity who does one or more of the following: a) collects a sample; b) processes a sample; c) provides a sample or a processed sample; and d) generates data (e.g., level of a risk gene; level of a reference gene product(s); normalized level of a risk gene ("prognosis gene") for use in the likelihood assessment. In some cases, the person(s) or entity(ies) who provides sample collection and/or sample processing and/or data generation, and the person who receives the results and/or report may be different persons, but are both referred to as "users" or "clients" herein to avoid confusion. In certain embodiments, e.g., where the methods are completely executed on a single computer, the user or client provides for data input and review of data output. A "user" can be a health professional (e.g., a clinician, a laboratory technician, a physician (e.g., an oncologist, surgeon, pathologist), etc.).

In embodiments where the user only executes a portion of the method, the individual who, after computerized data processing according to the methods of the present disclosure, reviews data output (e.g., results prior to release to provide a complete report, a complete, or reviews an "incomplete" report and provides for manual intervention and completion of an interpretive report) is referred to herein as a "reviewer." The reviewer may be located at a location remote to the user (e.g., at a service provided separate from a healthcare facility where a user may be located).

Where government regulations or other restrictions apply (e.g., requirements by health, malpractice, or liability insurance), all results, whether generated wholly or partially electronically, are subjected to a quality control routine prior to release to the user.

### CLINICAL UTILITY

The gene expression assay and information provided by the practice of the methods disclosed herein facilitates physicians in making more well-informed treatment decisions, and to customize the treatment of cancer to the needs of individual patients, thereby maximizing the benefit of treatment and minimizing the exposure of patients to unnecessary treatments which may provide little or no significant benefits and often carry serious risks due to toxic side-effects.

Single or multi-analyte gene expression tests can be used measure the expression level of one or more genes involved in each of several relevant physiologic processes or component cellular characteristics. The expression level(s) may be used to calculate such a quantitative score, and such score may be arranged in subgroups (e.g., tertiles) wherein all patients in a given range are classified as belonging to a risk category (e.g., low, intermediate, or high). The grouping of genes may be performed at least in part based on knowledge of the contribution of the genes according to physiologic functions or component cellular characteristics, such as in the groups discussed above.

The utility of a gene marker in predicting cancer may not be unique to that marker. An alternative marker having an expression pattern that is parallel to that of a selected marker gene may be substituted for, or used in addition to, a test marker. Due to the co-expression of such genes, substitution of expression level values should have little impact on the overall prognostic utility of the test. The closely similar expression patterns of two genes may result from involvement of both genes in the same process and/or being under common regulatory control in colon tumor cells. The present disclosure thus contemplates the use of such co-expressed genes or gene sets as substitutes for, or in addition to, prognostic methods of the present disclosure.

The molecular assay and associated information provided by the methods disclosed herein for predicting the clinical outcome in cancer, e.g. breast cancer, have utility in many areas, including in the development and appropriate use of drugs to treat cancer, to stratify cancer patients for inclusion in (or exclusion from) clinical studies, to assist patients and physicians in making treatment decisions, provide economic benefits by targeting treatment based on personalized genomic profile, and the like. For example, the recurrence score may be used on samples collected from patients in a clinical trial and the results of the test used in conjunction with patient outcomes in order to determine whether subgroups of patients are more or less likely to demonstrate an absolute benefit from a new drug than the whole group or other subgroups. Further, such methods can be used to identify from clinical data subsets of patients who are expected to benefit from adjuvant therapy. Additionally, a patient is more likely to be included in a clinical trial if the results of the test indicate a higher likelihood that the patient will have a poor clinical outcome if treated with surgery alone and a patient is less likely to be included in a clinical trial if the results of the test indicate a lower likelihood that the patient will have a poor clinical outcome if treated with surgery alone.

### STATISTICAL ANALYSIS OF GENE EXPRESSION LEVELS

One skilled in the art will recognize that there are many statistical methods that may be used to determine whether there is a significant relationship between an outcome of interest (e.g., likelihood of survival, likelihood of response to chemotherapy) and expression levels of a marker gene as described here. This relationship can be presented as a continuous recurrence score (RS), or patients may stratified into risk groups (e.g., low, intermediate, high). For example, a Cox proportional hazards regression model may fit to a particular clinical endpoint (e.g., RFS, DFS, OS). One assumption of the Cox proportional hazards regression model is the proportional hazards assumption, i.e. the assumption that effect parameters multiply the underlying hazard.

### Coexpression Analysis

The present disclosure provides genes that co-express with particular prognostic and/or predictive gene that has been identified as having a significant correlation to recurrence and/or treatment benefit. To perform particular biological processes, genes often work together in a concerted way, i.e. they are co-expressed. Co-expressed gene significant in the amplified RNA studies described above. The meta-analysis included both fixed-effect and random-effect models, which are further described in L. Hedges and J. Vevea, Psychological Methods 3 (4): 486-504 (1998) and K. Sidik and J. Jonkman, Statistics in Medicine 26:1964-1981 (2006). The results of the validation for all genes identified as having a stastistically significant association with breast cancer clinical outcome are described in Table 13. In those tables, "Est" designates an estimated coefficient of a covariate (gene expression); "SE" is standard error; "t" is the t-score for this estimate (i.e., Est/SE); and "fe" is the fixed estimate of effect from the meta analysis. Several of gene families with significant statistical association with clinical outcome (including metabolic, proliferation, immune, and stromal group genes) in breast cancer were confirmed using the SIB dataset. For example, Table 14 contains analysis of genes included in the metabolic group and Table 15 the stromal group.

### EXAMPLE 4:

A co-expression analysis was conducted using microarray data from six (6) breast cancer data sets. The "processed" expression values are taken from the GEO website, however, further processing was necessary. If the expression values are RMA, they are median normalized on the sample level. If the expression values are MAS5.0, they are: (1) changed to 10 if they are <10; (2) log base e transformed; and (3) median normalized on the sample level.

Generating Correlation Pairs: A rank matrix was generated by arranging the expression values for each sample in decreasing order. Then a correlation matrix was created by calculating the Spearman correlation values for every pair of probe IDs. Pairs of probes which had a Spearman value ≥ 0.7 were considered co-expressed. Redundant or overlapping correlation pairs in multiple datasets were identified. For each correlation matrix generated from an array dataset, pairs of significant probes that occur in >1 dataset were identified. This served to filter "non-significant" pairs from the analysis as well as provide extra evidence for "significant" pairs with their presence in multiple datasets. Depending on the number of datasets included in each tissue specific analysis, only pairs which occur in a minimum # or % of datasets were included.

Co-expression cliques were generated using the Bron-Kerbosch algorithm for maximal clique finding in an undirected graph. The algorithm generates three sets of nodes: *compsub*, *candidates*, and *not. Compsub* contains the set of nodes to be extended or shrunk by one depending on its traversal direction on the tree search. *Candidates* consists of all the above; c) an output device, connected to the computing environment, to provide information to a user (e.g., medical personnel); and d) an algorithm executed by the central computing environment (e.g., a processor), where the algorithm is executed based on the data received by the input device, and wherein the algorithm calculates a, risk, risk score, or treatment group classification, gene co-expression analysis, thresholding, or other functions described herein. The methods provided by the present invention may also be automated in whole or in part.

### Manual and Computer-Assisted Methods and Products

The methods and systems described herein can be implemented in numerous ways. In one embodiment of particular interest, the methods involve use of a communications infrastructure, for example the Internet. Several embodiments are discussed below. It is also to be understood that the present disclosure may be implemented in various forms of hardware, software, firmware, processors, or a combination thereof. The methods and systems described herein can be implemented as a combination of hardware and software. The software can be implemented as an application program tangibly embodied on a program storage device, or different portions of the software implemented in the user's computing environment (e.g., as an applet) and on the reviewer's computing environment, where the reviewer may be located at a remote site associated (e.g., at a service provider's facility).

For example, during or after data input by the user, portions of the data processing can be performed in the user-side computing environment. For example, the user-side computing environment can be programmed to provide for defined test codes to denote a likelihood "risk score," where the score is transmitted as processed or partially processed responses to the reviewer's computing environment in the form of test code for subsequent execution of one or more algorithms to provide a results and/or generate a report in the reviewer's computing environment. The risk score can be a numerical score (representative of a numerical value, e.g. likelihood of recurrence based on validation study population) or a non-numerical score representative of a numerical value or range of numerical values (e.g., low, intermediate, or high).

The application program for executing the algorithms described herein may be uploaded to, and executed by, a machine comprising any suitable architecture. In general, the machine involves a computer platform having hardware such as one or more central processing units (CPU), a random access memory (RAM), and input/output (I/O) interface(s). The computer platform also includes an operating system and microinstruction code. The various processes and functions described herein may either be part of the microinstruction code or part of the application program (or a combination thereof) that is executed via the operating system. In addition, various other peripheral devices may be connected to the computer platform such as an additional data storage device and a printing device.

As a computer system, the system generally includes a processor unit. The processor unit operates to receive information, which can include test data (e.g., level of a risk gene, level of a reference gene product(s); normalized level of a gene; and may also include other data such as patient data. This information received can be stored at least temporarily in a database, and data analyzed to generate a report as described above.

Part or all of the input and output data can also be sent electronically; certain output data (e.g., reports) can be sent electronically or telephonically (e.g., by facsimile, e.g., using devices such as fax back). Exemplary output receiving devices can include a display element, a printer, a facsimile device and the like. Electronic forms of transmission and/or display can include email, interactive television, and the like. In an embodiment of particular interest, all or a portion of the input data and/or all or a portion of the output data (e.g., usually at least the final report) are maintained on a web server for access, preferably confidential access, with typical browsers. The data may be accessed or sent to health professionals as desired. The input and output data, including all or a portion of the final report, can be used to populate a patient's medical record which may exist in a confidential database at the healthcare facility.

A system for use in the methods described herein generally includes at least one computer processor (e.g., where the method is carried out in its entirety at a single site) or at least two networked computer processors (e.g., where data is to be input by a user (also referred to herein as a "client") and transmitted to a remote site to a second computer processor for analysis, where the first and second computer processors are connected by a network, e.g., via an intranet or internet). The system can also include a user component(s) for input; and a reviewer component(s) for review of data, generated reports, and manual intervention. Additional components of the system can include a server component(s); and a database(s) for storing data (e.g., as in a database of report elements, e.g., interpretive report elements, or a relational database (RDB) which can include data input by the user and data output. The computer processors can be processors that are typically found in personal desktop computers (e.g., IBM, Dell, Macintosh), portable computers, mainframes, minicomputers, or other computing devices.

The networked client/server architecture can be selected as desired, and can be, for example, a classic two or three tier client server model. A relational database management system (RDMS), either as part of an application server component or as a separate component (RDB machine) provides the interface to the database.

In one example, the architecture is provided as a database-centric client/server architecture, in which the client application generally requests services from the application server which makes requests to the database (or the database server) to populate the report with the various report elements as required, particularly the interpretive report elements, especially the interpretation text and alerts. The server(s) (e.g., either as part of the application server machine or a separate RDB/relational database machine) responds to the client's requests.

The input client components can be complete, stand-alone personal computers offering a full range of power and features to run applications. The client component usually operates under any desired operating system and includes a communication element (e.g., a modem or other hardware for connecting to a network), one or more input devices (e.g., a keyboard, mouse, keypad, or other device used to transfer information or commands), a storage element (e.g., a hard drive or other computer-readable, computer-writable storage medium), and a display element (e.g., a monitor, television, LCD, LED, or other display device that conveys information to the user). The user enters input commands into the computer processor through an input device. Generally, the user interface is a graphical user interface (GUI) written for web browser applications.

The server component(s) can be a personal computer, a minicomputer, or a mainframe and offers data management, information sharing between clients, network administration and security. The application and any databases used can be on the same or different servers.

Other computing arrangements for the client and server(s), including processing on a single machine such as a mainframe, a collection of machines, or other suitable configuration are contemplated. In general, the client and server machines work together to accomplish the processing of the present disclosure.

Where used, the database(s) is usually connected to the database server component and can be any device that will hold data. For example, the database can be a any magnetic or optical storing device for a computer (e.g., CDROM, internal hard drive, tape drive). The database can be located remote to the server component (with access via a network, modem, etc.) or locally to the server component.

Where used in the system and methods, the database can be a relational database that is organized and accessed according to relationships between data items. The relational database is generally composed of a plurality of tables (entities). The rows of a table represent records (collections of information about separate items) and the columns represent fields (particular attributes of a record). In its simplest conception, the relational database is a collection of data entries that "relate" to each other through at least one common field.

Additional workstations equipped with computers and printers may be used at point of service to enter data and, in some embodiments, generate appropriate reports, if desired. The computer(s) can have a shortcut (e.g., on the desktop) to launch the application to facilitate initiation of data entry, transmission, analysis, report receipt, etc. as desired.

### Computer-readable storage media

The present disclosure also contemplates a computer-readable storage medium (e.g. CD-ROM, memory key, flash memory card, diskette, etc.) having stored thereon a program which, when executed in a computing environment, provides for implementation of algorithms to carry out all or a portion of the results of a response likelihood assessment as described herein. Where the computer-readable medium contains a complete program for carrying out the methods described herein, the program includes program instructions for collecting, analyzing and generating output, and generally includes computer readable code devices for interacting with a user as described herein, processing that data in conjunction with analytical information, and generating unique printed or electronic media for that user.

Where the storage medium provides a program that provides for implementation of a portion of the methods described herein (e.g., the user-side aspect of the methods (e.g., data input, report receipt capabilities, etc.)), the program provides for transmission of data input by the user (e.g., via the internet, via an intranet, etc.) to a computing environment at a remote site. Processing or completion of processing of the data is carried out at the remote site to generate a report. After review of the report, and completion of any needed manual intervention, to provide a complete report, the complete report is then transmitted back to the user as an electronic document or printed document (e.g., fax or mailed paper report). The storage medium containing a program according to the present disclosure can be packaged with instructions (e.g., for program installation, use, etc.) recorded on a suitable substrate or a web address where such instructions may be obtained. The computer-readable storage medium can also be provided in combination with one or more reagents for carrying out response likelihood assessment (e.g., primers, probes, arrays, or other such kit components).

All aspects of the present invention may also be practiced such that a limited number of additional genes that are co-expressed with the disclosed genes, for example as evidenced by statistically meaningful Pearson and/or Spearman correlation coefficients, are included in a prognostic or predictive test in addition to and/or in place of disclosed genes.

Having described the invention, the same will be more readily understood through reference to the following Examples, which are provided by way of illustration, and are not intended to limit the invention in any way.

### EXAMPLE 1:

The study included breast cancer tumor samples obtained from 136 patients diagnosed with breast cancer ("Providence study"). Biostatistical modeling studies of prototypical data sets demonstrated that amplified RNA is a useful substrate for biomarker identification studies. This was verified in this study by including known breast cancer biomarkers along with candidate prognostic genesin the tissues samples. The known biomarkers were shown to be associated with clinical outcome in amplified RNA based on the criteria outlined in this protocol.

### Study design

Refer to the original Providence Phase II study protocol for biopsy specimen information. The study looked at the statistical association between clinical outcome and 384candidate biomarkers tested in amplified samples derived from 25 ng of mRNA that was extracted from fixed, paraffin-embedded tissue samples obtained from 136 of the original Providence Phase II study samples. The expression level of the candidate genes was normalized using reference genes. Several reference genes were analyzed in this study: AAMP, ARF1, EEF1A1, ESD, GPS1, H3F3A, HNRPC, RPL13A, RPL41, RPS23, RPS27, SDHA, TCEA1, UBB, YWHAZ, B-actin, GUS, GAPDH, RPLPO, and TFRC.

The 136 samples were split into 3 automated RT plates each with 2X 48 samples and 40 samples and 3 RT positive and negative controls. Quantitative PCR assays were performed in 384 wells without replicate using the QuantiTect Probe PCR Master Mix® (Qiagen). Plates were analyzed on the Light Cycler® 480 and, after data quality control, all samples from the RT plate 3 were repeated and new RT-PCR data was generated. The data was normalized by subtracting the median crossing point (C_{P}) (point at which detection rises above background signal) for five reference genes from the C_{P} value for each individual candidate gene. This normalization is performed on each sample resulting in final data that has been adjusted for differences in overall sample C_{P}. This data set was used for the final data analysis.

### Data Analysis

For each gene, a standard z test was run.. (S. Darby, J. Reissland, Journal of the Royal Statistical Society 144(3):298-331 (1981)). This returns a z score (measure of distance in standard deviations of a sample from the mean), p value, and residuals along with other statistics and parameters from the model. If the z score is negative, expression is positively correlated with a good prognosis; if positive, expression is negatively correlated to a good prognosis. Using the p values, a q value was created using a library q value. The poorly correlated and weakly expressed genes were excluded from the calculation of the distribution used for the q values. For each gene, Cox Proportional Hazard Model test was run checking survival time matched with the event vector against gene expression. This returned a hazard ratio (HR) estimating the effect of expression of each gene (individually) on the risk of a cancer-related event. The resulting data is provided in Tables 1-6. A HR < 1 indicates that expression of that gene is positively associated with a good prognosis, while a HR > 1 indicates that expression of that gene is negatively associated with a good prognosis.

### EXAMPLE 2:

### Study design

Amplified samples were derived from 25 ng of mRNA that was extracted from fixed, paraffin-embedded tissue samples obtained from 78 evaluable cases from a Phase II breast cancer study conducted at Rush University Medical Center. Three of the samples failed to provide sufficient amplified RNA at 25 ng, so amplification was repeated a second time with 50 ng of RNA. The study also analyzed several reference genes for use in normalization: AAMP, ARF1, EEF1A1, ESD, GPS1, H3F3A, HNRPC, RPL13A, RPL41, RPS23, RPS27, SDHA, TCEA1, UBB, YWHAZ, Beta-actin, RPLPO, TFRC, GUS, and GAPDH.

Assays were performed in 384 wells without replicate using the QuantiTect Probe PCR Master Mix. Plates were analyzed on the Light Cycler 480 instruments. This data set was used for the final data analysis. The data was normalized by subtracting the median C_{P} for five reference genes from the C_{P} value for each individual candidate gene. This normalization was performed on each sample resulting in final data that was adjusted for differences in overall sample C_{P}.

### Data analysis

There were 34 samples with average CP values above 35. However, none of the samples were excluded from analysis because they were deemed to have sufficient valuable information to remain in the study. Principal Component Analysis (PCA) was used to determine whether there was a plate effect causing variation across the different RT plates. The first principal component correlated well with the median expression values, indicating that expression level accounted for most of the variation between samples. Also, there were no unexpected variations between plates.

### Data for Other Variables

Group - The patients were divided into two groups (cancer/non-cancer). There was little difference between the two in overall gene expression as the difference between median CP value in each group was minimal (0.7).

Sample Age - The samples varied widely in their overall gene expression but there was a trend toward lower C_{P} values as they decreased in age.

Instrument - The overall sample gene expression from instrument to instrument was consistent. One instrument showed a slightly higher median C_{P} compared to the other three, but it was well within the acceptable variation.

RT Plate - The overall sample gene expression between RT plates was also very consistent. The median C_{P} for each of the 3 RT plates (2 automated RT plates and 1 manual plate containing repeated samples) were all within 1 C_{P} of each other.

### Univariate Analyses for Genes Significantly Different Between Study Groups

The genes were analyzed using the z-test and Cox Proportional Hazard Model, as described in Example 1. The resulting data can be seen in Tables 7-12.

### EXAMPLE 3:

The statistical correlations between clinical outcome and expression levels of the genes identified in Examples 1 and 2 were validated in breast cancer gene expression datasets maintained by the Swiss Institute of Bioinformatics (SIB). Further information concerning the SIB database, study datasets, and processing methods, is providing in P. Wirapati, et al., Breast Cancer Research 10(4):R65 (2008). Univariate Cox proportional hazards analyses were performed to confirm the relationship between clinical outcome (DFS, MFS, OS) of breast cancer patients and expression levels of the genes identified as groups identified for a disease process like cancer can serve as biomarkers for disease progression and response to treatment. Such co-expressed genes can be assayed in lieu of, or in addition to, assaying of the prognostic and/or predictive gene with which they are co-expressed.

One skilled in the art will recognize that many co-expression analysis methods now known or later developed will fall within the scope of the present claims. These methods may incorporate, for example, correlation coefficients, co-expression network analysis, clique analysis, etc., and may be based on expression data from RT-PCR, microarrays, sequencing, and other similar technologies. For example, gene expression clusters can be identified using pair-wise analysis of correlation based on Pearson or Spearman correlation coefficients. (See, e.g., Pearson K. and Lee A., Biometrika 2, 357 (1902); C. Spearman, Amer. J. Psychol 15:72-101 (1904); J. Myers, A. Well, Research Design and Statistical Analysis, p. 508 (2nd Ed., 2003).) In general, a correlation coefficient of equal to or greater than 0.3 is considered to be statistically significant in a sample size of at least 20. (See, e.g., G. Norman, D. Streiner, Biostatistics: The Bare Essentials, 137-138 (3rd Ed. 2007).) In one embodiment disclosed herein, co-expressed genes were identified using a Spearman correlation value of at least 0.7.

### Computer program

The values from the assays described above, such as expression data, recurrence score, treatment score and/or benefit score, can be calculated and stored manually. Alternatively, the above-described steps can be completely or partially performed by a computer program product. The present disclosure thus provides a computer program product including a computer readable storage medium having a computer program stored on it. The program can, when read by a computer, execute relevant calculations based on values obtained from analysis of one or more biological sample from an individual (e.g., gene expression levels, normalization, thresholding, and conversion of values from assays to a score and/or graphical depiction of likelihood of recurrence/response to chemotherapy, gene co-expression or clique analysis, and the like). The computer program product has stored therein a computer program for performing the calculation.

The present disclosure provides systems for executing the program described above, which system generally includes: a) a central computing environment; b) an input device, operatively connected to the computing environment, to receive patient data, wherein the patient data can include, for example, expression level or other value obtained from an assay using a biological sample from the patient, or microarray data, as described in detail nodes eligible to be added to *compsub. Not* contains the set of nodes that have been added to *compsub* and are now excluded from extension. The algorithm consists of five steps: selection of a candidate; adding the candidate node to *compsub;* creating new sets *candidates* and *not* from the old sets by removing all points not connected to the candidate node; recursively calling the extension operator on the **new** *candidates* and *not* sets; and upon return, remove the candidate node from *compsub* and place in the **old** *not* set.

There was a depth-first search with pruning, and the selection of candidate nodes had an effect on the run time of the algorithm. By selecting nodes in decreasing order of frequency in the pairs, the run time was optimized. Also, recursive algorithms generally cannot be implemented in a multi-threaded manner, but was multi-threaded the extension operator of the first recursive level. Since the data between the threads were independent because they were at the top-level of the recursive tree, they were run in parallel.

Clique Mapping and Normalization: Since the members of the co-expression pairs and cliques are at the probe level, one must map the probe IDs to genes (or Refseqs) before they can be analyzed. The Affymetrix gene map information was used to map every probe ID to a gene name. Probes may map to multiple genes, and genes may be represented by multiple probes. The data for each clique is validated by manually calculating the correlation values for each pair from a single clique.

The results of this co-expression analysis are set forth in Tables 16-18.

**TABLE A**

| Gene | Sequence ID | Official Symbol | F Primer Seq | SEQ ID NO: | R Primer Seq | SEQ ID NO: | Probe Seq | SEQ ID NO: | Target Seq Length | Amplicon Sequence | SEQ ID NO: |
|---|---|---|---|---|---|---|---|---|---|---|---|
| A-Catenin | NM_001903.1 | CTNNAI | | 1 | | 385 | | 769 | 78 | | 1153 |
| AAMP | NM_001087.3 | AAMP | | 2 | | 386 | | 770 | 66 | | 1154 |
| ABCB1 | NM_000927.2 | ABCB1 | | 3 | | 387 | | 771 | 77 | | 1155 |
| ABCC10 | NM_033450.2 | ABCC10 | | 4 | | 388 | | 772 | 68 | | 1156 |
| ABCC5 | NM_005688.1 | ABCC5 | | 5 | | 389 | | 773 | 76 | | 1157 |
| ABR | NM_001092.3 | ABR | | 6 | | 390 | | 774 | 67 | | 1158 |
| ACTR2 | NM_005722.2 | ACTR2 | | 7 | | 391 | | 775 | 66 | | 1159 |
| ACVR2B | NM_001106.2 | ACVR2B | | 8 | | 392 | | 776 | 74 | | 1160 |
| AD024 | NM_020675.3 | SPC25 | | 9 | | 393 | | 777 | 74 | | 1161 |
| ADAM12 | NM_021641.2 | ADAM12 | | 10 | | 394 | | 778 | 66 | | 1162 |
| ADAM17 | NM_003183.3 | ADAM17 | | 11 | | 395 | | 779 | 73 | | 1163 |
| ADAM23 | NM_003812.1 | ADAM23 | | 12 | | 396 | | 780 | 62 | | 1164 |
| ADAMTS8 | NM_007037.2 | ADAMTS8 | | 13 | | 397 | | 781 | 72 | | 1165 |
| ADM | NM_001124.1 | ADM | | 14 | | 398 | | 782 | 75 | | 1166 |
| AES | NM_001130.4 | AES | | 15 | | 399 | | 783 | 78 | | 1167 |
| AGR2 | NM_006408.2 | AGR2 | | 16 | | 400 | | 784 | 70 | | 1168 |
| AK055699 | NM_194317 | LYPD6 | | 17 | | 401 | | 785 | 78 | | 1169 |
| AKR7A3 | NM_012067.2 | AKR7A3 | | 18 | | 402 | | 786 | 67 | | 1170 |
| AKT3 | NM_005465.1 | AKT3 | | 19 | | 403 | | 787 | 75 | | 1171 |
| ALCAM | NM_001627.1 | ALCAM | | 20 | | 404 | | 788 | 66 | | 1172 |
| ALDH4 | NM_003748.2 | ALDH4A1 | | 21 | | 405 | | 789 | 68 | | 1173 |
| ANGPT2 | NM_001147.1 | ANGPT2 | | 22 | | 406 | | 790 | 69 | | 1174 |
| ANXA2 | NM_004039.1 | ANXA2 | | 23 | | 407 | | 791 | 71 | | 1175 |
| AP-1 (JUN official) | NM_002228.2 | JUN | | 24 | | 408 | | 792 | 81 | | 1176 |
| APEX-1 | NM_001641.2 | APEX1 | | 25 | | 409 | | 793 | 68 | | 1177 |
| APOD | NM_001647.1 | APOD | | 26 | | 410 | | 794 | 67 | | 1178 |
| ARF1 | NM_001658.2 | ARF1 | | 27 | | 411 | | 795 | 64 | | 1179 |
| ARHI | NM_004675.1 | DIRAS3 | | 28 | | 412 | | 796 | 67 | | 1180 |
| ARNT2 | NM_014862.3 | ARNT2 | | 29 | | 413 | | 797 | 68 | | 1181 |
| ARSD | NM_001669.1 | ARSD | | 30 | | 414 | | 798 | 79 | | 1182 |
| AURKB | NM_004217.1 | AURKB | | 31 | | 415 | | 799 | 67 | | 1183 |
| B-actin | NM_001101.2 | ACTB | | 32 | | 416 | | 800 | 66 | | 1184 |
| B-Catenin | NM_001904.1 | CTNNB1 | | 33 | | 417 | | 801 | 80 | | 1185 |
| BAD | NM_032989.1 | BAD | | 34 | | 418 | | 802 | 73 | | 1186 |
| BAG1 | NM_004323.2 | BAG1 | | 35 | | 419 | | 803 | 81 | | 1187 |
| BAG4 | NM_004874.2 | BAG4 | | 36 | | 420 | | 804 | 76 | | 1188 |
| BASE | NM_173859.1 | | | 37 | | 421 | | 805 | 72 | | 1189 |
| Bax | NM_004324.1 | BAX | | 38 | | 422 | | 806 | 70 | | 1190 |
| BBC3 | NM_014417.1 | BBC3 | | 39 | | 423 | | 807 | 83 | | 1191 |
| BCAR1 | NM_014567.1 | BCAR1 | | 40 | | 424 | | 808 | 65 | | 1192 |
| BCAR3 | NM_003567.1 | BCAR3 | | 41 | | 425 | | 809 | 75 | | 1193 |
| BCAS1 | NM_003657.1 | BCAS1 | | 42 | | 426 | | 810 | 73 | | 1194 |
| Bcl2 | NM_000633.1 | BCL2 | | 43 | | 427 | | 811 | 73 | | 1195 |
| BCL2L12 | NM_138639.1 | BCL2L12 | | 44 | | 428 | | 812 | 73 | | 1196 |
| BGN | NM_001711.3 | BGN | | 45 | | 429 | | 813 | 66 | | 1197 |
| BIK | NM_001197.3 | BIK | | 46 | | 430 | | 814 | 70 | | 1198 |
| BNIP3 | NM_004052.2 | BNIP3 | | 47 | | 431 | | 815 | 68 | | 1199 |
| BSG | NM_001728.2 | BSG | | 48 | | 432 | | 816 | 66 | | 1200 |
| BTRC | NM_033637.2 | BTRC | | 49 | | 433 | | 817 | 63 | | 1201 |
| BUB1 | NM_004336.1 | BUB1 | | 50 | | 434 | | 818 | 68 | | 1202 |
| BUB1B | NM_001211.3 | BUB1B | | 51 | | 435 | | 819 | 82 | | 1203 |
| BUB3 | NM_004725.1 | BUB3 | | 52 | | 436 | | 820 | 73 | | 1204 |
| c-kit | NM_000222.1 | KIT | | 53 | | 437 | | 821 | 75 | | 1205 |
| C10orf116 | NM_006829.2 | C10orf116 | | 54 | | 438 | | 822 | 67 | | 1206 |
| C17orf37 | NM_032339.3 | C17orf37 | | 55 | | 439 | | 823 | 67 | | 1207 |
| C20 orf1 | NM_012112 | TPX2 | | 56 | | 440 | | 824 | 65 | | 1208 |
| C6orf66 | NM_014165.1 | NDUFAF4 | | 57 | | 441 | | 825 | 70 | | 1209 |
| C8orf4 | NM_020130.2 | C8orf4 | | 58 | | 442 | | 826 | 67 | | 1210 |
| CACNA2D 2 | NM_006030.1 | CACNA2D 2 | | 59 | | 443 | | 827 | 67 | | 1211 |
| CAT | NM_001752.1 | CAT | | 60 | | 444 | | 828 | 78 | | 1212 |
| CAV1 | NM_001753.3 | CAV1 | | 61 | | 445 | | 829 | 74 | | 1213 |
| CBX5 | NM_012117.1 | CBX5 | | 62 | | 446 | | 830 | 78 | | 1214 |
| CCL19 | NM_006274.2 | CCL19 | | 63 | | 447 | | 831 | 78 | | 1215 |
| CCL3 | NM_002983.1 | CCL3 | | 64 | | 448 | | 832 | 77 | | 1216 |
| CCL5 | NM_002985.2 | CCL5 | | 65 | | 449 | | 833 | 65 | | 1217 |
| CCNB1 | NM_031966.1 | CCNB1 | | 66 | | 450 | | 834 | 84 | | 1218 |
| CCND3 | NM_001760.2 | CCND3 | | 67 | | 451 | | 835 | 76 | | 1219 |
| CCNE2 variant 1 | NM_057749var 1 | CCNE2 | | 68 | | 452 | | 836 | 85 | | 1220 |
| CCR5 | NM_000579.1 | CCR5 | | 69 | | 453 | | 837 | 67 | | 1221 |
| CCR7 | NM_001838.2 | CCR7 | | 70 | | 454 | | 838 | 64 | | 1222 |
| CD1A | NM_001763.1 | CD1A | | 71 | | 455 | | 839 | 78 | | 1223 |
| CD24 | NM_013230.1 | CD24 | | 72 | | 456 | | 840 | 77 | | 1224 |
| CD4 | NM_000616.2 | CD4 | | 73 | | 457 | | 841 | 67 | | 1225 |
| CD44E | X55150 | | | 74 | | 458 | | 842 | 90 | | 1226 |
| CD44s | M59040.1 | | | 75 | | 459 | | 843 | 78 | | 1227 |
| CD44v6 | AJ251595v6 | | | 76 | | 460 | | 844 | 78 | | 1228 |
| CD68 | NM_001251.1 | CD68 | | 77 | | 461 | | 845 | 74 | | 1229 |
| CD82 | NM_002231.2 | CD82 | | 78 | | 462 | | 846 | 84 | | 1230 |
| CDC20 | NM_001255.1 | CDC20 | | 79 | | 463 | | 847 | 68 | | 1231 |
| cdc25A | NM_001789.1 | CDC25A | | 80 | | 464 | | 848 | 71 | | 1232 |
| CDC25C | NM_001790.2 | CDC25C | | 81 | | 465 | | 49 | 67 | | 1233 |
| CDC4 | NM_018315.2 | FBXW7 | | 82 | | 466 | | 850 | 77 | | 1234 |
| CDC42BP A | NM_003607.2 | CDC42BPA | | 83 | | 467 | | 851 | 67 | | 1235 |
| CDC42EP4 | NM_012121.4 | CDC42EP4 | | 84 | | 468 | | 852 | 67 | | 1236 |
| CDH11 | NM_001797.2 | CDH11 | | 85 | | 469 | | 853 | 70 | | 1237 |
| CDH3 | NM_001793.3 | CDH3 | | 86 | | 470 | | 854 | 71 | | 1238 |
| CDK4 | NM_000075.2 | CDK4 | | 87 | | 471 | | 855 | 66 | | 1239 |
| CDK5 | NM_004935.2 | CDK5 | | 88 | | 472 | | 856 | 67 | | 1240 |
| CDKN3 | NM_005192.2 | CDKN3 | | 89 | | 473 | | 857 | 70 | | 1241 |
| CEACAM1 | NM_001712.2 | CEACAM1 | | 90 | | 474 | | 858 | 71 | | 1242 |
| CEBPA | NM_004364.2 | CEBPA | | 91 | | 475 | | 859 | 66 | | 1243 |
| CEGP1 | NM_020974.1 | SCUBE2 | | 92 | | 476 | | 860 | 77 | | 1244 |
| CENPA | NM_001809.2 | CENPA | | 93 | | 477 | | 861 | 63 | | 1245 |
| CGA (CHGA official) | NM_001275.2 | CHGA | | 94 | | 478 | | 862 | 76 | | 1246 |
| CGalpha | NM_000735.2 | CGA | | 95 | | 479 | | 863 | 69 | | 1247 |
| CGB | NM_000737.2 | CGB | | 96 | | 480 | | 864 | 80 | | 1248 |
| CHAF1B | NM_005441.1 | CHAF1B | | 97 | | 481 | | 865 | 72 | | 1249 |
| CHFR | NM_018223.1 | CHFR | | 98 | | 482 | | 866 | 76 | | 1250 |
| CHI3L1 | NM_001276.1 | CHI3L1 | | 99 | | 483 | | 867 | 66 | | 1251 |
| CKS2 | NM_001827.1 | CKS2 | | 100 | | 484 | CTGCGCCCGCTCTTCGCG | 868 | 62 | | 1252 |
| Claudin 4 | NM_001305.2 | CLDN4 | | 101 | | 485 | | 869 | 72 | | 1253 |
| CLIC1 | NM_001288.3 | CLIC1 | | 102 | | 486 | | 870 | 68 | | 1254 |
| CLU | NM_001831.1 | CLU | | 103 | | 487 | | 871 | 76 | | 1255 |
| CNOT2 | NM_014515.3 | CNOT2 | | 104 | | 488 | | 872 | 67 | | 1256 |
| COL1A1 | NM_000088.2 | COL1A1 | | 105 | | 489 | | 873 | 68 | | 1257 |
| COL1A2 | NM_000089.2 | COL1A2 | | 106 | | 490 | | 874 | 80 | | 1258 |
| COMT | NM_000754.2 | COMT | | 107 | | 491 | | 875 | 67 | | 1259 |
| Contig 51037 | NM_198477 | CXCL17 | | 108 | | 492 | | 876 | 81 | | 1260 |
| COPS3 | NM_003653.2 | COPS3 | | 109 | | 493 | | 877 | 72 | | 1261 |
| CRYAB | NM_001885.1 | CRYAB | | 110 | | 494 | | 878 | 69 | | 1262 |
| CRYZ | NM_001889.2 | CRYZ | | 111 | | 495 | | 879 | 78 | | 1263 |
| CSF1 isoC | NM_172211.1 | CSF1 | | 112 | | 496 | | 880 | 68 | | 1264 |
| CSF1 | NM_000757.3 | CSF1 | | 113 | | 497 | | 881 | 74 | | 1265 |
| CSF1R | NM_005211.1 | CSF1R | | 114 | | 498 | | 882 | 80 | | 1266 |
| CSF2RA | NM_006140.3 | CSF2RA | | 115 | | 499 | | 883 | 67 | | 1267 |
| CSK (SRC) | NM_004383.1 | CSK | | 116 | | 500 | | 884 | 64 | | 1268 |
| CTGF | NM_001901.1 | CTGF | | 117 | | 501 | | 885 | 76 | | 1269 |
| CTHRC1 | NM_138455.2 | CTHRC1 | | 118 | | 502 | | 886 | 67 | | 1270 |
| CTSD | NM_001909.1 | CTSD | | 119 | | 503 | | 887 | 80 | | 1271 |
| CTSL2 | NM_001333.2 | CTSL2 | | 120 | | 504 | | 888 | 67 | | 1272 |
| CTSL2int2 | NM_001333.2in t2 | | | 121 | | 505 | | 889 | 79 | | 1273 |
| CXCL10 | NM_001565.1 | CXCL10 | | 122 | | 506 | | 890 | 68 | | 1274 |
| CXCL12 | NM_000609.3 | CXCL12 | | 123 | | 507 | | 891 | 67 | | 1275 |
| CXCL14 | NM_004887.3 | CXCL14 | | 124 | | 508 | | 892 | 74 | | 1276 |
| CXCR4 | NM_003467.1 | CXCR4 | | 125 | | 509 | | 893 | 72 | | 1277 |
| CYP17A1 | NM_000102.2 | CYP17A1 | | 126 | | 510 | | 894 | 76 | | 1278 |
| CYP19A1 | NM_000103.2 | CYP19A1 | | 127 | | 511 | | 895 | 70 | | 1279 |
| CYP1B1 | NM_000104.2 | CYP1B1 | | 128 | | 512 | | 896 | 71 | | 1280 |
| CYR61 | NM_001554.3 | CYR61 | | 129 | | 513 | | 897 | 76 | | 1281 |
| DAB2 | NM_001343.1 | DAB2 | | 130 | | 514 | | 898 | 67 | | 1282 |
| DCC | NM_005215.1 | DCC | | 131 | | 515 | | 899 | 75 | | 1283 |
| DCC_exons 18-23 | X76132_18-23 | | | 132 | | 516 | | 900 | 66 | | 1284 |
| DCC_exons 6-7 | X76132_6-7 | | | 133 | | 517 | | 901 | 74 | | 1285 |
| DCK | NM_000788.1 | DCK | | 134 | | 518 | | 902 | 110 | | 1286 |
| DICER1 | NM_177438.1 | DICER1 | | 135 | | 519 | | 903 | 68 | | 1287 |
| DLC1 | NM_006094.3 | DLC1 | | 136 | | 520 | | 904 | 68 | | 1288 |
| DLL4 | NM_019074.2 | DLL4 | | 137 | | 521 | | 905 | 67 | | 1289 |
| DR5 | NM_003842.2 | TNFRSF10 B | | 138 | | 522 | | 906 | 84 | | 1290 |
| DSP | NM_004415.1 | DSP | | 139 | | 523 | | 907 | 73 | | 1291 |
| DTYMK | NM_012145.1 | DTYMK | | 140 | | 524 | | 908 | 78 | | 1292 |
| DUSP1 | NM_004417.2 | DUSP1 | | 141 | | 525 | | 909 | 76 | | 1293 |
| DUSP4 | NM_001394.4 | DUSP4 | | 142 | | 526 | | 910 | 68 | | 1294 |
| E2F1 | NM_005225.1 | E2F1 | | 143 | | 527 | | 911 | 75 | | 1295 |
| EBRP | AF243433.1 | | | 144 | | 528 | | 912 | 76 | | 1296 |
| EDN1 endothelin | NM_001955.1 | EDN1 | | 145 | | 529 | | 913 | 73 | | 1297 |
| EDN2 | NM_001956.2 | EDN2 | | 146 | | 530 | | 914 | 79 | | 1298 |
| EDNRA | NM_001957.1 | EDNRA | | 147 | | 531 | | 915 | 76 | | 1299 |
| EDNRB | NM_000115.1 | EDNRB | | 148 | | 532 | | 916 | 72 | | 1300 |
| EEF1A1 | NM_001402.5 | EEF1A1 | | 149 | | 533 | | 917 | 67 | | 1301 |
| EEF1A2 | NM_001958.2 | EEF1A2 | | 150 | | 534 | | 918 | 66 | | 1302 |
| EFP | NM_005082.2 | TRIM25 | | 151 | | 535 | | 919 | 74 | | 1303 |
| EGR1 | NM_001964.2 | EGR1 | | 152 | | 536 | | 920 | 76 | | 1304 |
| EGR3 | NM_004430.2 | EGR3 | | 153 | | 537 | | 921 | 78 | | 1305 |
| EIF4EBP1 | NM_004095.2 | EIF4EBP1 | | 154 | | 538 | | 922 | 66 | | 1306 |
| ELF3 | NM_004433.2 | ELF3 | | 155 | | 539 | | 923 | 71 | | 1307 |
| EMP1 | NM_001423.1 | EMP1 | | 156 | | 540 | | 924 | 75 | | 1308 |
| ENO1 | NM_001428.2 | ENO1 | | 157 | | 541 | | 925 | 68 | | 1309 |
| EP300 | NM_001429.1 | EP300 | | 158 | | 542 | | 926 | 75 | | 1310 |
| EpCAM | NM_002354.1 | EPCAM | | 159 | | 543 | | 927 | 75 | | 1311 |
| EPHA2 | NM_004431.2 | EPHA2 | | 160 | | 544 | | 928 | 72 | | 1312 |
| EPHB2 | NM_004442.4 | EPHB2 | | 161 | | 545 | | 929 | 66 | | 1313 |
| EPHB4 | NM_004444.3 | EPHB4 | | 162 | | 546 | | 930 | 77 | | 1314 |
| ER2 | NM_001437.1 | ESR2 | | 163 | | 547 | | 931 | 76 | | 1315 |
| ERBB4 | NM_005235.1 | ERBB4 | | 164 | | 548 | | 932 | 86 | | 1316 |
| ERCC1 | NM_001983.1 | ERCC1 | | 165 | | 549 | | 933 | 67 | | 1317 |
| ERG | NM_004449.3 | ERG | | 166 | | 550 | | 934 | 70 | | 1318 |
| ERRa | NM_004451.3 | ESRRA | | 167 | | 551 | | 935 | 67 | | 1319 |
| ESD | NM_001984.1 | ESD | | 168 | | 552 | | 936 | 66 | | 1320 |
| ESPL1 | NM_012291.1 | ESPL1 | | 169 | | 553 | | 937 | 70 | | 1321 |
| ESRRG | NM_001438.1 | ESRRG | | 170 | | 554 | | 938 | 67 | | 1322 |
| EstR1 | NM_000125.1 | ESR1 | | 171 | | 555 | | 939 | 68 | | 1323 |
| ETV5 | NM_004454.1 | ETV5 | | 172 | | 556 | | 940 | 67 | | 1324 |
| EZH2 | NM_004456.3 | EZH2 | | 173 | | 557 | | 941 | 78 | | 1325 |
| F3 | NM_001993.2 | F3 | | 174 | | 558 | | 942 | 73 | | 1326 |
| FAP | NM_004460.2 | FAP | | 175 | | 559 | | 943 | 66 | | 1327 |
| FASN | NM_004104.4 | FASN | | 176 | | 560 | | 944 | 66 | | 1328 |
| FGFR2 isoform 1 | NM_000141.2 | FGFR2 | | 177 | | 561 | | 945 | 80 | | 1329 |
| FGFR4 | NM_002011.3 | FGFR4 | | 178 | | 562 | | 946 | 81 | | 1330 |
| FHIT | NM_002012.1 | FHIT | | 179 | | 563 | | 947 | 67 | | 1331 |
| FLOT2 | NM_004475.1 | FLOT2 | | 180 | | 564 | | 948 | 66 | | 1332 |
| FN1 | NM_002026.2 | FN1 | | 181 | | 565 | | 949 | 69 | | 1333 |
| FOS | NM_005252.2 | FOS | | 182 | | 566 | | 950 | 67 | | 1334 |
| FOXC2 | NM_005251.1 | FOXC2 | | 183 | | 567 | | 951 | 66 | | 1335 |
| FOXO3A | NM_001455.1 | FOXO3 | | 184 | | 568 | | 952 | 83 | | 1336 |
| FOXP1 | NM_032682.3 | FOXP1 | | 185 | | 569 | | 953 | 70 | | 1337 |
| FOXP3 | NM_014009.2 | FOXP3 | | 186 | | 570 | | 954 | 66 | | 1338 |
| FSCN1 | NM_003088.1 | FSCN1 | | 187 | | 571 | | 955 | 74 | | 1339 |
| FUS | NM_004960.1 | FUS | | 188 | | 572 | | 956 | 80 | | 1340 |
| FYN | NM_002037.3 | FYN | | 189 | | 573 | | 957 | 69 | | 1341 |
| G-Catenin | NM_002230.1 | JUP | | 190 | | 574 | | 958 | 68 | | 1342 |
| GAB2 | NM_012296.2 | GAB2 | | 191 | | 575 | | 959 | 74 | | 1343 |
| GADD45 | NM_001924.2 | GADD45A | | 192 | | 576 | | 960 | 73 | | 1344 |
| GADD45B | NM_015675.1 | GADD45B | | 193 | | 577 | | 961 | 70 | | 1345 |
| GAPDH | NM_002046.2 | GAPDH | | 194 | | 578 | | 962 | 74 | | 1346 |
| GATA3 | NM_00205 1.1 | GATA3 | | 195 | | 579 | | 963 | 75 | | 1347 |
| GBP1 | NM_002053.1 | GBP1 | | 196 | | 580 | | 964 | 73 | | 1348 |
| GBP2 | NM_004120.2 | GBP2 | | 197 | | 581 | | 965 | 83 | | 1349 |
| GCLM | NM_002061.1 | GCLM | | 198 | | 582 | | 966 | 85 | | 1350 |
| GDF15 | NM_004864.1 | GDF15 | | 199 | | 583 | | 967 | 72 | | 1351 |
| GH1 | NM_000515.3 | GH1 | | 200 | | 584 | | 968 | 66 | | 1352 |
| GJA1 | NM_000165.2 | GJA1 | | 201 | | 585 | | 969 | 68 | | 1353 |
| GJB2 | NM_004004.3 | GJB2 | | 202 | | 586 | | 970 | 74 | | 1354 |
| GMNN | NM_015895.3 | GMNN | | 203 | | 587 | | 971 | 67 | | 1355 |
| GNAZ | NM_002073.2 | GNAZ | | 204 | | 588 | | 972 | 68 | | 1356 |
| GPR30 | NM_001505.1 | GPER | | 205 | | 589 | | 973 | 70 | | 1357 |
| GPS1 | NM_004127.4 | GPS1 | | 206 | | 590 | | 974 | 66 | | 1358 |
| GPX1 | NM_000581.2 | GPX1 | | 207 | | 591 | | 975 | 67 | | 1359 |
| GPX2 | NM_002083.1 | GPX2 | | 208 | | 592 | | 976 | 75 | | 1360 |
| GPX4 | NM_002085.1 | GPX4 | | 209 | | 593 | | 977 | 66 | | 1361 |
| GRB7 | NM_005310.1 | GRB7 | | 210 | | 594 | | 978 | 67 | | 1362 |
| GREB1 variant a | NM_014668.2 | GREB1 | | 211 | | 595 | | 979 | 71 | | 1363 |
| GREB1 variant b | NM_033090.1 | GREB1 | | 212 | | 596 | | 980 | 73 | | 1364 |
| GREB1 variant c | NM_148903.1 | GREB1 | | 213 | | 597 | | 981 | 64 | | 1365 |
| GRN | NM_002087.1 | GRN | | 214 | | 598 | | 982 | 72 | | 1366 |
| GSTM1 | NM_000561.1 | GSTM1 | | 215 | | 599 | | 983 | 86 | | 1367 |
| GSTM2 gene | NM_000848gen e | | | 216 | | 600 | | 984 | 71 | | 1368 |
| GSTM2 | NM_000848.2 | GSTM2 | | 217 | | 601 | | 985 | 68 | | 1369 |
| GSTM3 | NM_000849.3 | GSTM3 | | 218 | | 602 | | 986 | 76 | | 1370 |
| GSTT1 | NM_000853.1 | GSTT1 | | 219 | | 603 | | 987 | 66 | | 1371 |
| GUS | NM_000181.1 | GUSB | | 220 | | 604 | | 988 | 73 | | 1372 |
| H3F3A | NM_002107.3 | H3F3A | | 221 | | 605 | | 989 | 70 | | 1373 |
| HDAC1 | NM_004964.2 | HDAC1 | | 222 | | 606 | | 990 | 74 | | 1374 |
| HDAC6 | NM_006044.2 | HDAC6 | | 223 | | 607 | | 991 | 66 | | 1375 |
| HER2 | NM_004448.1 | ERBB2 | | 224 | | 608 | | 992 | 70 | | 1376 |
| HES1 | NM_005524.2 | HES1 | | 225 | | 609 | | 993 | 68 | | 1377 |
| HGFAC | NM_001528.2 | HGFAC | | 226 | | 610 | | 994 | 72 | | 1378 |
| HLA-DPB1 | NM_002121.4 | HLA-DPB1 | | 227 | | 611 | | 995 | 73 | | 1379 |
| HMGB 1 | NM_002128.3 | HMGB 1 | | 228 | | 612 | | 996 | 71 | | 1380 |
| HNF3A | NM_004496.1 | FOXA1 | | 229 | | 613 | | 997 | 73 | | 1381 |
| HNRPAB | NM_004499.3 | HNRNPAB | | 230 | | 614 | | 998 | 84 | | 1382 |
| HNRPC | NM_004500.3 | HNRNPC | | 231 | | 615 | | 999 | 68 | | 1383 |
| HoxA1 | NM_005522.3 | HOXA1 | | 232 | | 616 | | 1000 | 69 | | 1384 |
| HoxA5 | NM_019102.2 | HOXA5 | | 233 | | 617 | | 1001 | 78 | | 1385 |
| HOXB13 | NM_006361.2 | HOXB13 | | 234 | | 618 | | 1002 | 71 | | 1386 |
| HOXB7 | NM_004502.2 | HOXB7 | | 235 | | 619 | | 1003 | 68 | | 1387 |
| HSD17B1 | NM_000413.1 | HSD17B1 | | 236 | | 620 | | 1004 | 78 | | 1388 |
| HSD17B2 | NM_002153.1 | HSD17B2 | | 237 | | 621 | | 1005 | 68 | | 1389 |
| HSHIN1 | NM_017493.3 | OTUD4 | | 238 | | 622 | | 1006 | 77 | | 1390 |
| HSPA1A | NM_005345.4 | HSPA1A | | 239 | | 623 | | 1007 | 70 | | 1391 |
| HSPA1B | NM_005346.3 | HSPA1B | | 240 | | 624 | | 1008 | 63 | | 1392 |
| HSPA4 | NM_002154.3 | HSPA4 | | 241 | | 625 | | 1009 | 72 | | 1393 |
| HSPA5 | NM_005347.2 | HSPA5 | | 242 | | 626 | | 1010 | 84 | | 1394 |
| HSPA8 | NM_006597.3 | HSPA8 | | 243 | | 627 | | 1011 | 73 | | 1395 |
| HSPB1 | NM_001540.2 | HSPB1 | | 244 | | 628 | | 1012 | 84 | | 1396 |
| IBSP | NM_004967.2 | IBSP | | 245 | | 629 | | 1013 | 83 | | 1397 |
| ICAM1 | NM_000201.1 | ICAM1 | | 246 | | 630 | | 1014 | 68 | | 1398 |
| ID1 | NM_002165.1 | ID1 | | 247 | | 631 | | 1015 | 70 | | 1399 |
| ID4 | NM_001546.2 | ID4 | | 248 | | 632 | | 1016 | 83 | | 1400 |
| IDH2 | NM_002168.2 | IDH2 | | 249 | | 633 | | 1017 | 74 | | 1401 |
| IGF1R | NM_000875.2 | IGF1R | | 250 | | 634 | | 1018 | 83 | | 1402 |
| IGF2 | NM_000612.2 | IGF2 | | 251 | | 635 | | 1019 | 72 | | 1403 |
| IGFBP6 | NM_002178.1 | IGFBP6 | | 252 | | 636 | | 1020 | 77 | | 1404 |
| IGFBP7 | NM_001553.1 | IGFBP7 | | 253 | | 637 | | 1021 | 68 | | 1405 |
| IKBKE | NM_014002.2 | IKBKE | | 254 | | 638 | | 1022 | 66 | | 1406 |
| IL-8 | NM_000584.2 | IL8 | | 255 | | 639 | | 1023 | 70 | | 1407 |
| IL10 | NM_000572.1 | IL10 | | 256 | | 640 | | 1024 | 79 | | 1408 |
| IL11 | NM_000641.2 | IL11 | | 257 | | 641 | | 1025 | 66 | | 1409 |
| IL17RB | NM_018725.2 | IL17RB | | 258 | | 642 | | 1026 | 76 | | 1410 |
| IL6ST | NM_002184.2 | IL6ST | | 259 | | 643 | | 1027 | 74 | | 1411 |
| ING1 | NM_005537.2 | ING1 | | 260 | | 644 | | 1028 | 66 | | 1412 |
| INHBA | NM_002192.1 | INHBA | | 261 | | 645 | | 1029 | 72 | | 1413 |
| IRF1 | NM_002198.1 | IRF1 | | 262 | | 646 | | 1030 | 69 | | 1414 |
| IRS1 | NM_005544.1 | IRS1 | | 263 | | 647 | | 1031 | 74 | | 1415 |
| ITGA3 | NM_002204.1 | ITGA3 | | 264 | | 648 | | 1032 | 77 | | 1416 |
| ITGA4 | NM_000885.2 | ITGA4 | | 265 | | 649 | | 1033 | 66 | | 1417 |
| ITGA5 | NM_002205.1 | ITGA5 | | 266 | | 650 | | 1034 | 75 | | 1418 |
| ETGA6 | NM_000210.1 | ETGA6 | | 267 | | 651 | | 1035 | 69 | | 1419 |
| ITGAV | NM_002210.2 | ITGAV | | 268 | | 652 | | 1036 | 79 | | 1420 |
| ITGB1 | NM_002211.2 | ITGB1 | | 269 | | 653 | | 1037 | 74 | | 1421 |
| ITGB3 | NM_000212.2 | ITGB3 | | 270 | | 654 | | 1038 | 78 | | 1422 |
| ITGB4 | NM_000213.2 | ITGB4 | | 271 | | 655 | | 1039 | 66 | | 1423 |
| ITGB5 | NM_002213.3 | ITGB5 | | 272 | | 656 | | 1040 | 71 | | 1424 |
| JAG1 | NM_000214.1 | JAG1 | | 273 | | 657 | | 1041 | 69 | | 1425 |
| JUNB | NM_002229.2 | JUNB | | 274 | | 658 | | 1042 | 70 | | 1426 |
| Ki-67 | NM_002417.1 | MKI67 | | 275 | | 659 | | 1043 | 80 | | 1427 |
| KIAA0555 | NM_014790.3 | JAKMIP2 | | 276 | | 660 | | 1044 | 67 | | 1428 |
| KIAA1199 | NM_018689.1 | KIAA1199 | | 277 | | 661 | | 1045 | 66 | | 1429 |
| KIF14 | NM_014875.1 | KIF14 | | 278 | | 662 | | 1046 | 69 | | 1430 |
| KIF20A | NM_005733.1 | KIF20A | | 279 | | 663 | | 1047 | 67 | | 1431 |
| KIF2C | NM_006845.2 | KIF2C | | 280 | | 664 | | 1048 | 73 | | 1432 |
| KLK11 | NM_006853.1 | KLK11 | | 281 | | 665 | | 1049 | 66 | | 1433 |
| KLK6 | NM_002774.2 | KLK6 | | 282 | | 666 | | 1050 | 78 | | 1434 |
| KLRC1 | NM_002259.3 | KLRC1 | | 283 | | 667 | | 1051 | 67 | | 1435 |
| KNSL2 | BC000712.1 | | | 284 | | 668 | | 1052 | 77 | | 1436 |
| KNTC2 | NM_006101.1 | NDC80 | | 285 | | 669 | | 1053 | 71 | | 1437 |
| KPNA2 | NM_002266.1 | KPNA2 | | 286 | | 670 | | 1054 | 67 | | 1438 |
| L1CAM | NM_000425.2 | L1CAM | | 287 | | 671 | | 1055 | 66 | | 1439 |
| LAMA3 | NM_000227.2 | LAMA3 | | 288 | | 672 | | 1056 | 73 | | 1440 |
| LAMA5 | NM_005560.3 | LAMA5 | | 289 | | 673 | | 1057 | 67 | | 1441 |
| LAMB1 | NM_002291.1 | LAMB1 | | 290 | | 674 | | 1058 | 66 | | 1442 |
| LAMB3 | NM_000228.1 | LAMB3 | | 291 | | 675 | | 1059 | 67 | | 1443 |
| LAMC2 | NM_005562.1 | LAMC2 | | 292 | | 676 | | 1060 | 80 | | 1444 |
| LAPTM4B | NM_018407.4 | LAPTM4B | | 293 | | 677 | | 1061 | 67 | | 1445 |
| LGALS3 | NM_002306.1 | LGALS3 | | 294 | | 678 | | 1062 | 69 | | 1446 |
| LIMK1 | NM_016735.1 | | | 295 | | 679 | | 1063 | 67 | | 1447 |
| LIMS 1 | NM_004987.3 | LIMS1 | | 296 | | 680 | | 1064 | 71 | | 1448 |
| LMNB1 | NM_005573.1 | LMNB1 | | 297 | | 681 | | 1065 | 66 | | 1449 |
| LOX | NM_002317.3 | LOX | | 298 | | 682 | | 1066 | 66 | | 1450 |
| LRIG1 | NM_015541.1 | | | 299 | | 683 | | 1067 | 67 | | 1451 |
| LSM1 | NM_014462.1 | LSM1 | | 300 | | 684 | | 1068 | 66 | | 1452 |
| LTBP1 | NM_ 206943.1 | LTBP1 | | 301 | | 685 | | 1069 | 67 | | 1453 |
| LYRIC | NM_178812.2 | MTDH | | 302 | | 686 | | 1070 | 67 | | 1454 |
| MAD1L1 | NM_003550.1 | MAD1L1 | | 303 | | 687 | | 1071 | 67 | | 1455 |
| MCM2 | NM_004526.1 | MCM2 | | 304 | | 688 | | 1072 | 75 | | 1456 |
| MELK | NM_014791.1 | MELK | | 305 | | 689 | | 1073 | 70 | | 1457 |
| MGMT | NM_002412.1 | MGMT | | 306 | | 690 | | 1074 | 69 | | 1458 |
| mGST1 | NM_020300.2 | MGST1 | | 307 | | 691 | | 1075 | 79 | | 1459 |
| MMP1 | NM_002421.2 | MMP1 | | 308 | | 692 | | 1076 | 72 | | 1460 |
| MMP12 | NM_002426.1 | MMP12 | | 309 | | 693 | | 1077 | 78 | | 1461 |
| MMP2 | NM_004530.1 | MMP2 | | 310 | | 694 | | 1078 | 86 | | 1462 |
| MMP7 | NM_002423.2 | MMP7 | | 311 | | 695 | | 1079 | 79 | | 1463 |
| MMP8 | NM_002424.1 | MMP8 | | 312 | | 696 | | 1080 | 79 | | 1464 |
| MMTV-like env | AF346816.1 | | | 313 | | 697 | | 1081 | 72 | | 1465 |
| MNAT1 | NM_002431.1 | MNAT1 | | 314 | | 698 | | 1082 | 75 | | 1466 |
| MRP1 | NM_004996.2 | ABCC1 | | 315 | | 699 | | 1083 | 79 | | 1467 |
| MRP3 | NM_003786.2 | ABCC3 | | 316 | | 700 | | 1084 | 91 | | 1468 |
| MS4A1 | NM_021950.2 | MS4A1 | | 317 | | 701 | | 1085 | 70 | | 1469 |
| MSH2 | NM_00025 1.1 | MSH2 | | 318 | | 702 | | 1086 | 73 | | 1470 |
| MTA3 | XM_038567 | | | 319 | | 703 | | 1087 | 69 | | 1471 |
| MX1 | NM_002462.2 | MX1 | | 320 | | 704 | | 1088 | 78 | | 1472 |
| MYBL2 | NM_002466.1 | MYBL2 | | 321 | | 705 | | 1089 | 74 | | 1473 |
| NAT1 | NM_000662.4 | NAT1 | | 322 | | 706 | | 1090 | 75 | | 1474 |
| NAT2 | NM_000015.1 | NAT2 | | 323 | | 707 | | 1091 | 73 | | 1475 |
| NRG1 | NM_013957.1 | NRG1 | | 324 | | 708 | | 1092 | 83 | | 1476 |
| OPN, osteopontin | NM_000582.1 | SPP1 | | 325 | | 709 | | 1093 | 80 | | 1477 |
| p16-INK4 | L27211.1 | | | 326 | | 710 | | 1094 | 76 | | 1478 |
| PAI1 | NM_000602.1 | SERPINE1 | | 327 | | 711 | | 1095 | 81 | | 1479 |
| PGF | NM_002632.4 | PGF | | 328 | | 712 | | 1096 | 71 | | 1480 |
| PR | NM_000926.2 | PGR | | 329 | | 713 | | 1097 | 85 | | 1481 |
| PRDX1 | NM_002574.2 | PRDX1 | | 330 | | 714 | | 1098 | 67 | | 1482 |
| PTEN | NM_000314.1 | PTEN | | 331 | | 715 | | 1099 | 81 | | 1483 |
| PTP4A3 | NM_007079.2 | PTP4A3 | | 332 | | 716 | | 1100 | 70 | | 1484 |
| RhoB | NM_004040.2 | RHOB | | 333 | | 717 | | 1101 | 67 | | 1485 |
| RPL13A | NM_012423.2 | RPL13A | | 334 | | 718 | | 1102 | 68 | | 1486 |
| RPL41 | NM_021104.1 | RPL41 | | 335 | | 719 | | 1103 | 66 | | 1487 |
| RPLPO | NM_001002.2 | RPLP0 | | 336 | | 720 | | 1104 | 75 | | 1488 |
| RPS23 | NM_001025.1 | RPS23 | | 337 | | 721 | | 1105 | 67 | | 1489 |
| RPS27 | NM_001030.3 | RPS27 | | 338 | | 722 | | 1106 | 80 | | 1490 |
| RRM1 | NM_001033.1 | RRM1 | | 339 | | 723 | | 1107 | 66 | | 1491 |
| RRM2 | NM_001034.1 | RRM2 | | 340 | | 724 | | 1108 | 71 | | 1492 |
| RUNX1 | NM_001754.2 | RUNX1 | | 341 | | 725 | | 1109 | 69 | | 1493 |
| S100A10 | NM_002966.1 | S100A10 | | 342 | | 726 | | 1110 | 77 | | 1494 |
| S100A2 | NM_005978.2 | S100A2 | | 343 | | 727 | | 1111 | 73 | | 1495 |
| S100A4 | NM_002961.2 | S100A4 | | 344 | | 728 | | 1112 | 70 | | 1496 |
| S100A7 | NM_002963.2 | S100A7 | | 345 | | 729 | | 1113 | 75 | | 1497 |
| S100A8 | NM_002964.3 | S100A8 | | 346 | | 730 | | 1114 | 76 | | 1498 |
| S100A9 | NM_002965.3 | S100A9 | | 347 | | 731 | | 1115 | 67 | | 1499 |
| S100B | NM_006272.1 | S100B | | 348 | | 732 | | 1116 | 70 | | 1500 |
| S100G | NM_004057.2 | S100G | | 349 | | 733 | | 1117 | 67 | | 1501 |
| S100P | NM_005980.2 | S100P | | 350 | | 734 | | 1118 | 67 | | 1502 |
| SDHA | NM_004168.1 | SDHA | | 351 | | 735 | | 1119 | 67 | | 1503 |
| SEMA3F | NM_004186.1 | SEMA3F | | 352 | | 736 | | 1120 | 86 | | 1504 |
| SFRP2 | NM_003013.2 | SFRP2 | | 353 | | 737 | | 1121 | 66 | | 1505 |
| SIR2 | NM_012238.3 | SIRT1 | | 354 | | 738 | | 1122 | 72 | | 1506 |
| SKIL | NM_005414.2 | SKIL | | 355 | | 739 | | 1123 | 66 | | 1507 |
| SKP2 | NM_005983.2 | SKP2 | | 356 | | 740 | | 1124 | 71 | | 1508 |
| SLPI | NM_003064.2 | SLPI | | 357 | | 741 | | 1125 | 74 | | 1509 |
| SNAI1 | NM_005985.2 | SNAI1 | | 358 | | 742 | | 1126 | 69 | | 1510 |
| STK15 | NM_003600.1 | AURKA | | 359 | | 743 | | 1127 | 69 | | 1511 |
| STMN1 | NM_005563.2 | STMN1 | | 360 | | 744 | | 1128 | 71 | | 1512 |
| STMY3 | NM_005940.2 | MMP11 | | 361 | | 745 | | 1129 | 90 | | 1513 |
| SURV | NM_001168.1 | BIRC5 | | 362 | | 746 | | 1130 | 80 | | 1514 |
| SYK | NM_003177.1 | SYK | | 363 | | 747 | | 1131 | 85 | | 1515 |
| TAGLN | NM_003186.2 | TAGLN | | 364 | | 748 | | 1132 | 73 | | 1516 |
| TCEA1 | NM_201437.1 | TCEA1 | | 365 | | 749 | | 1133 | 72 | | 1517 |
| TFRC | NM_003234.1 | TFRC | | 366 | | 750 | | 1134 | 68 | | 1518 |
| TGFB2 | NM_003238.1 | TGFB2 | | 367 | | 751 | | 1135 | 75 | | 1519 |
| TGFB3 | NM_003239.1 | TGFB3 | | 368 | | 752 | | 1136 | 65 | | 1520 |
| TGFBR2 | NM_003242.2 | TGFBR2 | | 369 | | 753 | | 1137 | 66 | | 1521 |
| TIMP3 | NM_000362.2 | TIMP3 | | 370 | | 754 | | 1138 | 67 | | 1522 |
| TNFRSF11 A | NM_003839.2 | TNFRSF11 A | | 371 | | 755 | | 1139 | 67 | | 1523 |
| TNFRSF11 B | NM_002546.2 | TNFRSF11 B | | 372 | | 756 | | 1140 | 67 | | 1524 |
| TNFSF11 | NM_003701.2 | TNFSF11 | | 373 | | 757 | | 1141 | 71 | | 1525 |
| TWIST1 | NM_000474.2 | TWIST1 | | 374 | | 758 | | 1142 | 64 | | 1526 |
| UBB | NM_018955.1 | UBB | | 375 | | 759 | | 1143 | 522 | | 1527 |
| VCAM1 | NM_001078.2 | VCAM1 | | 376 | | 760 | | 1144 | 89 | | 1528 |
| VIM | NM_003380.1 | VIM | | 377 | | 761 | | 1145 | 72 | | 1529 |
| VTN | NM_000638.2 | VTN | | 378 | | 762 | | 1146 | 67 | | 1530 |
| WAVE3 | NM_006646.4 | WASF3 | | 379 | | 763 | | 1147 | 68 | | 1531 |
| WISP1 | NM_003882.2 | WISP1 | | 380 | | 764 | | 1148 | 75 | | 1532 |
| Wnt-5a | NM_003392.2 | WNT5A | | 381 | | 765 | | 1149 | 75 | | 1533 |
| Wnt-5b | NM_032642.2 | WNT5B | | 382 | | 766 | | 1150 | 79 | | 1534 |
| WWOX | NM_016373.1 | WWOX | | 383 | | 767 | | 1151 | 74 | | 1535 |
| YWHAZ | NM_003406.2 | YWHAZ | | 384 | | 768 | | 1152 | 81 | | 1536 |

**Table 1: Cox proportional hazards for Prognostic Genes that are positively associated with good prognosis for breast cancer (Providence study)**

| **Table 1** | | | |
|---|---|---|---|
| **Gene_all** | **z (Coef)** | **HR** | **p (Wald)** |
| GSTM2 | -4.306 | 0.525 | 0.000 |
| IL6ST | -3.730 | 0.522 | 0.000 |
| CEGP1 | -3.712 | 0.756 | 0.000 |
| Bcl2 | -3.664 | 0.555 | 0.000 |
| GSTM1 | -3.573 | 0.679 | 0.000 |
| ERBB4 | -3.504 | 0.767 | 0.000 |
| GADD45 | -3.495 | 0.601 | 0.000 |
| PR | -3.474 | 0.759 | 0.001 |
| GPR30 | -3.348 | 0.660 | 0.001 |
| CAV1 | -3.344 | 0.649 | 0.001 |
| C10orf116 | -3.194 | 0.681 | 0.001 |
| DR5 | -3.102 | 0.543 | 0.002 |
| DICER1 | -3.097 | 0.296 | 0.002 |
| EstR1 | -2.983 | 0.825 | 0.003 |
| BTRC | -2.976 | 0.639 | 0.003 |
| GSTM3 | -2.931 | 0.722 | 0.003 |
| GATA3 | -2.874 | 0.745 | 0.004 |
| DLC1 | -2.858 | 0.564 | 0.004 |
| CXCL14 | -2.804 | 0.693 | 0.005 |
| IL17RB | -2.796 | 0.744 | 0.005 |
| C8orf4 | -2.786 | 0.699 | 0.005 |
| FOXO3A | -2.786 | 0.617 | 0.005 |
| TNFRSF11B | -2.690 | 0.739 | 0.007 |
| BAG1 | -2.675 | 0.451 | 0.008 |
| SNAI1 | -2.632 | 0.692 | 0.009 |
| TGFB3 | -2.617 | 0.623 | 0.009 |
| NAT1 | -2.576 | 0.820 | 0.010 |
| FUS | -2.543 | 0.376 | 0.011 |
| F3 | -2.527 | 0.705 | 0.012 |
| GSTM2 gene | -2.461 | 0.668 | 0.014 |
| EPHB2 | -2.451 | 0.708 | 0.014 |
| LAMA3 | -2.448 | 0.778 | 0.014 |
| BAD | -2.425 | 0.506 | 0.015 |
| IGF1R | -2.378 | 0.712 | 0.017 |
| RUNX1 | -2.356 | 0.511 | 0.018 |
| ESRRG | -2.289 | 0.825 | 0.022 |
| HSHIN1 | -2.275 | 0.371 | 0.023 |
| CXCL12 | -2.151 | 0.623 | 0.031 |
| IGFBP7 | -2.137 | 0.489 | 0.033 |
| SKIL | -2.121 | 0.593 | 0.034 |
| PTEN | -2.110 | 0.449 | 0.035 |
| AKT3 | -2.104 | 0.665 | 0.035 |
| MGMT | -2.060 | 0.571 | 0.039 |
| LRIG1 | -2.054 | 0.649 | 0.040 |
| S100B | -2.024 | 0.798 | 0.043 |
| GREB1 variant a | -1.996 | 0.833 | 0.046 |
| CSF1 | -1.976 | 0.624 | 0.048 |
| ABR | -1.973 | 0.575 | 0.048 |
| AK055699 | -1.972 | 0.790 | 0.049 |

**Table 2: Cox proportional hazards for Prognostic Genes that are negatively associated with good prognosis for breast cancer (Providence study)**

| **Table 2** | | | |
|---|---|---|---|
| **Gene_all** | **z (Coef)** | **HR** | **p (Wald)** |
| S100A7 | 1.965 | 1.100 | 0.049 |
| MCM2 | 1.999 | 1.424 | 0.046 |
| Contig 51037 | 2.063 | 1.185 | 0.039 |
| S100P | 2.066 | 1.170 | 0.039 |
| ACTR2 | 2.119 | 2.553 | 0.034 |
| MYBL2 | 2.158 | 1.295 | 0.031 |
| DUSP1 | 2.166 | 1.330 | 0.030 |
| HOXB13 | 2.192 | 1.206 | 0.028 |
| SURV | 2.216 | 1.329 | 0.027 |
| MELK | 2.234 | 1.336 | 0.026 |
| HSPA8 | 2.240 | 2.651 | 0.025 |
| cdc25A | 2.314 | 1.478 | 0.021 |
| C20_orf1 | 2.336 | 1.497 | 0.019 |
| LMNB1 | 2.387 | 1.682 | 0.017 |
| S100A9 | 2.412 | 1.185 | 0.016 |
| CENPA | 2.419 | 1.366 | 0.016 |
| CDC25C | 2.437 | 1.384 | 0.015 |
| GAPDH | 2.498 | 1.936 | 0.012 |
| KNTC2 | 2.512 | 1.450 | 0.012 |
| PRDX1 | 2.540 | 2.131 | 0.011 |
| RRM2 | 2.547 | 1.439 | 0.011 |
| ADM | 2.590 | 1.445 | 0.010 |
| ARF1 | 2.634 | 2.973 | 0.008 |
| E2F1 | 2.716 | 1.486 | 0.007 |
| TFRC | 2.720 | 1.915 | 0.007 |
| STK15 | 2.870 | 1.860 | 0.004 |
| LAPTM4B | 2.880 | 1.538 | 0.004 |
| EpCAM | 2.909 | 1.919 | 0.004 |
| ENO1 | 2.958 | 2.232 | 0.003 |
| CCNB1 | 3.003 | 1.738 | 0.003 |
| BUB1 | 3.018 | 1.590 | 0.003 |
| Claudin 4 | 3.034 | 2.151 | 0.002 |
| CDC20 | 3.056 | 1.555 | 0.002 |
| Ki-67 | 3.329 | 1.717 | 0.001 |
| KPNA2 | 3.523 | 1.722 | 0.000 |
| IDH2 | 3.994 | 1.638 | 0.000 |

**Table 3: Cox proportional hazards for Prognostic Genes that are positively associated with good prognosis for ER-negative (ER0) breast cancer (Providence study)**

| **Gene_ER0** | **HR** | **z (Coef)** | **p (Wald)** |
|---|---|---|---|
| SYK | 0.185 | -2.991 | 0.003 |
| Wnt-5a | 0.443 | -2.842 | 0.005 |
| WISP1 | 0.455 | -2.659 | 0.008 |
| CYR61 | 0.405 | -2.484 | 0.013 |
| GADD45 | 0.520 | -2.474 | 0.013 |
| TAGLN | 0.364 | -2.376 | 0.018 |
| TGFB3 | 0.465 | -2.356 | 0.018 |
| INHBA | 0.610 | -2.255 | 0.024 |
| CDH11 | 0.584 | -2.253 | 0.024 |
| CHAF1B | 0.551 | -2.113 | 0.035 |
| ITGAV | 0.192 | -2.101 | 0.036 |
| SNAI1 | 0.655 | -2.077 | 0.038 |
| IL11 | 0.624 | -2.026 | 0.043 |
| KIAA1199 | 0.692 | -2.005 | 0.045 |
| TNFRSF11B | 0.659 | -1.989 | 0.047 |

**Table 4: Cox proportional hazards for Prognostic Genes that are negatively associated with good prognosis for ER-negative (ER0) breast cancer (Providence study)**

| **Gene_ER0** | **HR** | **z (Coef)** | **p (Wald)** |
|---|---|---|---|
| RPL41 | 3.547 | 2.062 | 0.039 |
| Claudin 4 | 2.883 | 2.117 | 0.034 |
| LYRIC | 4.029 | 2.364 | 0.018 |
| TFRC | 3.223 | 2.596 | 0.009 |
| VTN | 2.484 | 3.205 | 0.001 |

**Table 5: Cox proportional hazards for Prognostic Genes that are positively associated with good prognosis for ER-positive (ER1) breast cancer (Providence study)**

| **Gene_ER1** | **HR** | **z (Coef)** | **p (Wald)** |
|---|---|---|---|
| DR5 | 0.428 | -3.478 | 0.001 |
| GSTM2 | 0.526 | -3.173 | 0.002 |
| HSHIN1 | 0.175 | -3.031 | 0.002 |
| ESRRG | 0.736 | -3.028 | 0.003 |
| VTN | 0.622 | -2.935 | 0.003 |
| Bcl2 | 0.469 | -2.833 | 0.005 |
| ERBB4 | 0.705 | -2.802 | 0.005 |
| GPR30 | 0.625 | -2.794 | 0.005 |
| BAG1 | 0.339 | -2.733 | 0.006 |
| CAV1 | 0.635 | -2.644 | 0.008 |
| IL6ST | 0.503 | -2.551 | 0.011 |
| C10orf116 | 0.679 | -2.497 | 0.013 |
| FOXO3A | 0.607 | -2.473 | 0.013 |
| DICER1 | 0.311 | -2.354 | 0.019 |
| GADD45 | 0.645 | -2.338 | 0.019 |
| CSF1 | 0.500 | -2.312 | 0.021 |
| F3 | 0.677 | -2.300 | 0.021 |
| GBP2 | 0.604 | -2.294 | 0.022 |
| APEX-1 | 0.234 | -2.253 | 0.024 |
| FUS | 0.322 | -2.252 | 0.024 |
| BBC3 | 0.581 | -2.248 | 0.025 |
| GSTM3 | 0.737 | -2.203 | 0.028 |
| ITGA4 | 0.620 | -2.161 | 0.031 |
| EPHB2 | 0.685 | -2.128 | 0.033 |
| IRF1 | 0.708 | -2.105 | 0.035 |
| CRYZ | 0.593 | -2.103 | 0.035 |
| CCL19 | 0.773 | -2.076 | 0.038 |
| SKIL | 0.540 | -2.019 | 0.043 |
| MRP1 | 0.515 | -1.964 | 0.050 |

**Table 6: Cox proportional hazards for Prognostic Genes that are negatively associated with good prognosis for ER-positive (ER1) breast cancer (Providence study)**

| **Gene_ER1** | **HR** | **z (Coef)** | **p (Wald)** |
|---|---|---|---|
| CTHRC1 | 2.083 | 1.958 | 0.050 |
| RRM2 | 1.450 | 1.978 | 0.048 |
| BUB1 | 1.467 | 1.988 | 0.047 |
| LMNB1 | 1.764 | 2.009 | 0.045 |
| SURV | 1.380 | 2.013 | 0.044 |
| EpCAM | 1.966 | 2.076 | 0.038 |
| CDC20 | 1.504 | 2.081 | 0.037 |
| GAPDH | 2.405 | 2.126 | 0.033 |
| STK15 | 1.796 | 2.178 | 0.029 |
| HSPA8 | 3.095 | 2.215 | 0.027 |
| LAPTM4B | 1.503 | 2.278 | 0.023 |
| MCM2 | 1.872 | 2.370 | 0.018 |
| CDC25C | 1.485 | 2.423 | 0.015 |
| ADM | 1.695 | 2.486 | 0.013 |
| MMP1 | 1.365 | 2.522 | 0.012 |
| CCNB1 | 1.893 | 2.646 | 0.008 |
| Ki-67 | 1.697 | 2.649 | 0.008 |
| E2F1 | 1.662 | 2.689 | 0.007 |
| KPNA2 | 1.683 | 2.701 | 0.007 |
| DUSP1 | 1.573 | 2.824 | 0.005 |
| GDF15 | 1.440 | 2.896 | 0.004 |

**Table 7: Cox proportional hazards for Prognostic Genes that are positively associated with good prognosis for breast cancer (Rush study)**

| **Gene_all** | **z (Coef)** | **HR** | **p (Wald)** |
|---|---|---|---|
| GSTM2 | -3.275 | 0.752 | 0.001 |
| GSTM1 | -2.946 | 0.772 | 0.003 |
| C8orf4 | -2.639 | 0.793 | 0.008 |
| ELF3 | -2.478 | 0.769 | 0.013 |
| RUNX1 | -2.388 | 0.609 | 0.017 |
| IL6ST | -2.350 | 0.738 | 0.019 |
| AAMP | -2.325 | 0.715 | 0.020 |
| PR | -2.266 | 0.887 | 0.023 |
| FHIT | -2.193 | 0.790 | 0.028 |
| CD44v6 | -2.191 | 0.754 | 0.028 |
| GREB1 variant c | -2.120 | 0.874 | 0.034 |
| ADAM17 | -2.101 | 0.686 | 0.036 |
| EstR1 | -2.084 | 0.919 | 0.037 |
| NAT1 | -2.081 | 0.878 | 0.037 |
| TNFRSF11B | -2.074 | 0.843 | 0.038 |
| ITGB4 | -2.006 | 0.740 | 0.045 |
| CSF1 | -1.963 | 0.750 | 0.050 |

**Table 8: Cox proportional hazards for Prognostic Genes that are negatively associated with good prognosis for breast cancer (Rush study)**

| **Gene_all** | **z (Coef)** | **HR** | **p (Wald)** |
|---|---|---|---|
| STK15 | 1.968 | 1.298 | 0.049 |
| TFRC | 2.049 | 1.399 | 0.040 |
| ITGB1 | 2.071 | 1.812 | 0.038 |
| ITGAV | 2.081 | 1.922 | 0.037 |
| MYBL2 | 2.089 | 1.205 | 0.037 |
| MRP3 | 2.092 | 1.165 | 0.036 |
| SKP2 | 2.143 | 1.379 | 0.032 |
| LMNB1 | 2.155 | 1.357 | 0.031 |
| ALCAM | 2.234 | 1.282 | 0.025 |
| COMT | 2.271 | 1.412 | 0.023 |
| CDC20 | 2.300 | 1.253 | 0.021 |
| GAPDH | 2.307 | 1.572 | 0.021 |
| GRB7 | 2.340 | 1.205 | 0.019 |
| S100A9 | 2.374 | 1.120 | 0.018 |
| S100A7 | 2.374 | 1.092 | 0.018 |
| HER2 | 2.425 | 1.210 | 0.015 |
| ACTR2 | 2.499 | 1.788 | 0.012 |
| S100A8 | 2.745 | 1.144 | 0.006 |
| ENO1 | 2.752 | 1.687 | 0.006 |
| MMP1 | 2.758 | 1.212 | 0.006 |
| LAPTM4B | 2.775 | 1.375 | 0.006 |
| FGFR4 | 3.005 | 1.215 | 0.003 |
| C17orf37 | 3.260 | 1.387 | 0.001 |

**Table 9: Cox proportional hazards for Prognostic Genes that are positively associated with good prognosis for ER-negative (ER0) breast cancer (Rush study)**

| **Gene_ER0** | **z (Coef)** | **HR** | **p (Wald)** |
|---|---|---|---|
| SEMA3F | -2.465 | 0.503 | 0.014 |
| LAMA3 | -2.461 | 0.519 | 0.014 |
| CD44E | -2.418 | 0.719 | 0.016 |
| AD024 | -2.256 | 0.617 | 0.024 |
| LAMB3 | -2.237 | 0.690 | 0.025 |
| Ki-67 | -2.209 | 0.650 | 0.027 |
| MMP7 | -2.208 | 0.768 | 0.027 |
| GREB1 variant c | -2.019 | 0.693 | 0.044 |
| ITGB4 | -1.996 | 0.657 | 0.046 |
| CRYZ | -1.976 | 0.662 | 0.048 |
| CD44s | -1.967 | 0.650 | 0.049 |

**Table 10: Cox proportional hazards for Prognostic Genes that are negatively associated with good prognosis for ER-negative (ER0) breast cancer (Rush study)**

| **Gene_ER0** | **z (Coef)** | **HR** | **p (Wald)** |
|---|---|---|---|
| S100A8 | 1.972 | 1.212 | 0.049 |
| EEF1A2 | 2.031 | 1.195 | 0.042 |
| TAGLN | 2.072 | 2.027 | 0.038 |
| GRB7 | 2.086 | 1.231 | 0.037 |
| HER2 | 2.124 | 1.232 | 0.034 |
| ITGAV | 2.217 | 3.258 | 0.027 |
| CDH11 | 2.237 | 2.728 | 0.025 |
| COL1A1 | 2.279 | 2.141 | 0.023 |
| C17orf37 | 2.319 | 1.329 | 0.020 |
| COL1A2 | 2.336 | 2.577 | 0.020 |
| ITGB5 | 2.375 | 3.236 | 0.018 |
| ITGA5 | 2.422 | 2.680 | 0.015 |
| RPL41 | 2.428 | 6.665 | 0.015 |
| ALCAM | 2.470 | 1.414 | 0.013 |
| CTHRC1 | 2.687 | 3.454 | 0.007 |
| PTEN | 2.692 | 8.706 | 0.007 |
| FN1 | 2.833 | 2.206 | 0.005 |

**Table 11: Cox proportional hazards for Prognostic Genes that are positively associated with good prognosis for ER-positive (ER1) breast cancer (Rush study)**

| **Gene_ER1** | **z (Coef)** | **HR** | **p (Wald)** |
|---|---|---|---|
| GSTM1 | -3.938 | 0.628 | 0.000 |
| HNF3A | -3.220 | 0.500 | 0.001 |
| EstR1 | -3.165 | 0.643 | 0.002 |
| Bcl2 | -2.964 | 0.583 | 0.003 |
| GATA3 | -2.641 | 0.624 | 0.008 |
| ELF3 | -2.579 | 0.741 | 0.010 |
| C8orf4 | -2.451 | 0.730 | 0.014 |
| GSTM2 | -2.416 | 0.774 | 0.016 |
| PR | -2.416 | 0.833 | 0.016 |
| RUNX1 | -2.355 | 0.537 | 0.019 |
| CSF1 | -2.261 | 0.662 | 0.024 |
| IL6ST | -2.239 | 0.627 | 0.025 |
| AAMP | -2.046 | 0.704 | 0.041 |
| TNFRSF11B | -2.028 | 0.806 | 0.043 |
| NAT1 | -2.025 | 0.833 | 0.043 |
| ADAM17 | -1.981 | 0.642 | 0.048 |

**Table 12: Cox proportional hazards for Prognostic Genes that are negatively associated with good prognosis for ER-positive (ER1) breast cancer (Rush study)**

| **Gene_ER1** | **z (Coef)** | **HR** | **p (Wald)** |
|---|---|---|---|
| HSPA1B | 1.966 | 1.382 | 0.049 |
| AD024 | 1.967 | 1.266 | 0.049 |
| FGFR4 | 1.991 | 1.175 | 0.047 |
| CDK4 | 2.014 | 1.576 | 0.044 |
| ITGB1 | 2.021 | 2.163 | 0.043 |
| EPHB2 | 2.121 | 1.342 | 0.034 |
| LYRIC | 2.139 | 1.583 | 0.032 |
| MYBL2 | 2.174 | 1.273 | 0.030 |
| PGF | 2.176 | 1.439 | 0.030 |
| EZH2 | 2.199 | 1.390 | 0.028 |
| HSPA1A | 2.209 | 1.452 | 0.027 |
| RPLPO | 2.273 | 2.824 | 0.023 |
| LMNB1 | 2.322 | 1.529 | 0.020 |
| IL-8 | 2.404 | 1.166 | 0.016 |
| C6orf66 | 2.468 | 1.803 | 0.014 |
| GAPDH | 2.489 | 1.950 | 0.013 |
| P16-INK4 | 2.490 | 1.541 | 0.013 |
| CLIC1 | 2.557 | 2.745 | 0.011 |
| ENO1 | 2.719 | 2.455 | 0.007 |
| ACTR2 | 2.878 | 2.543 | 0.004 |
| CDC20 | 2.931 | 1.452 | 0.003 |
| SKP2 | 2.952 | 1.916 | 0.003 |
| LAPTM4B | 3.124 | 1.558 | 0.002 |

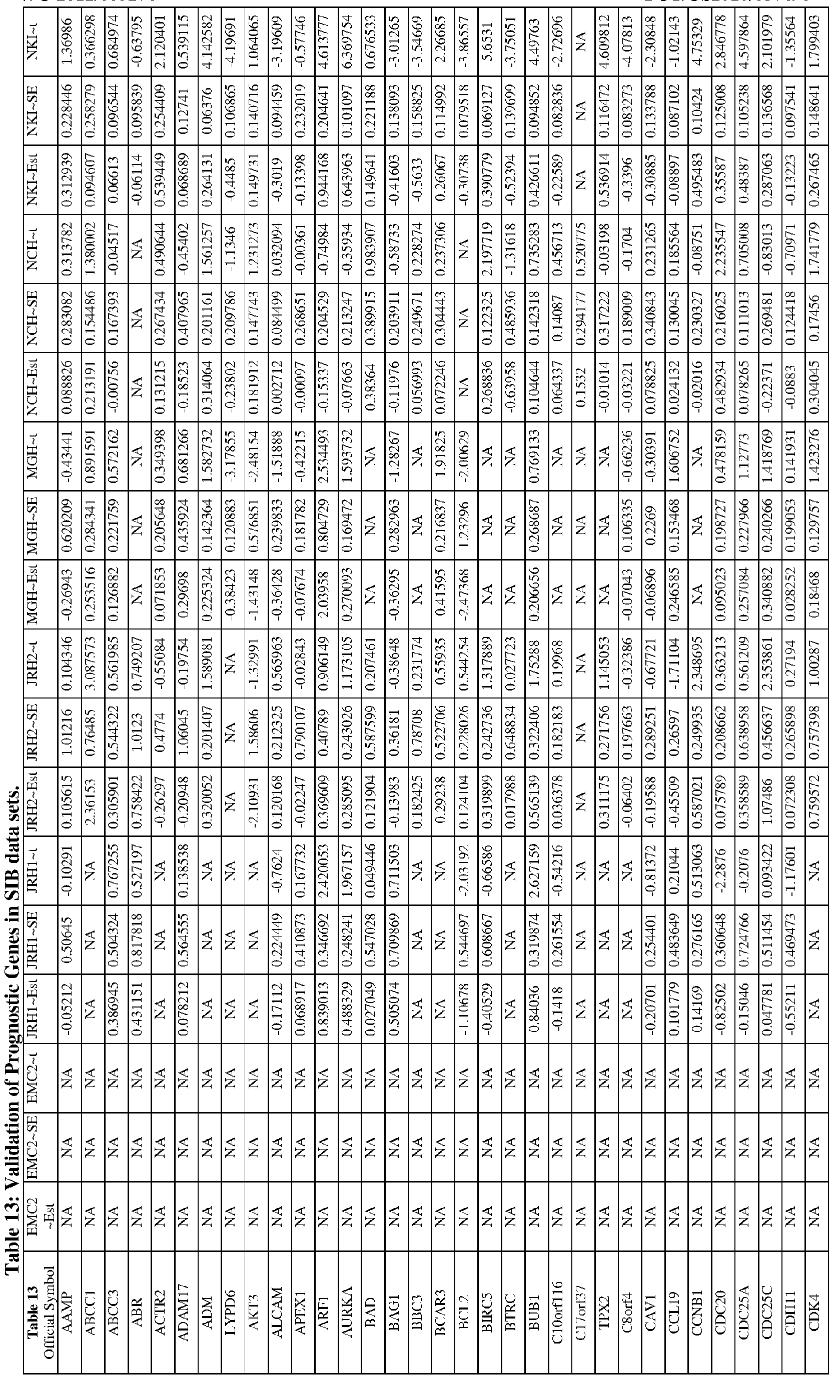

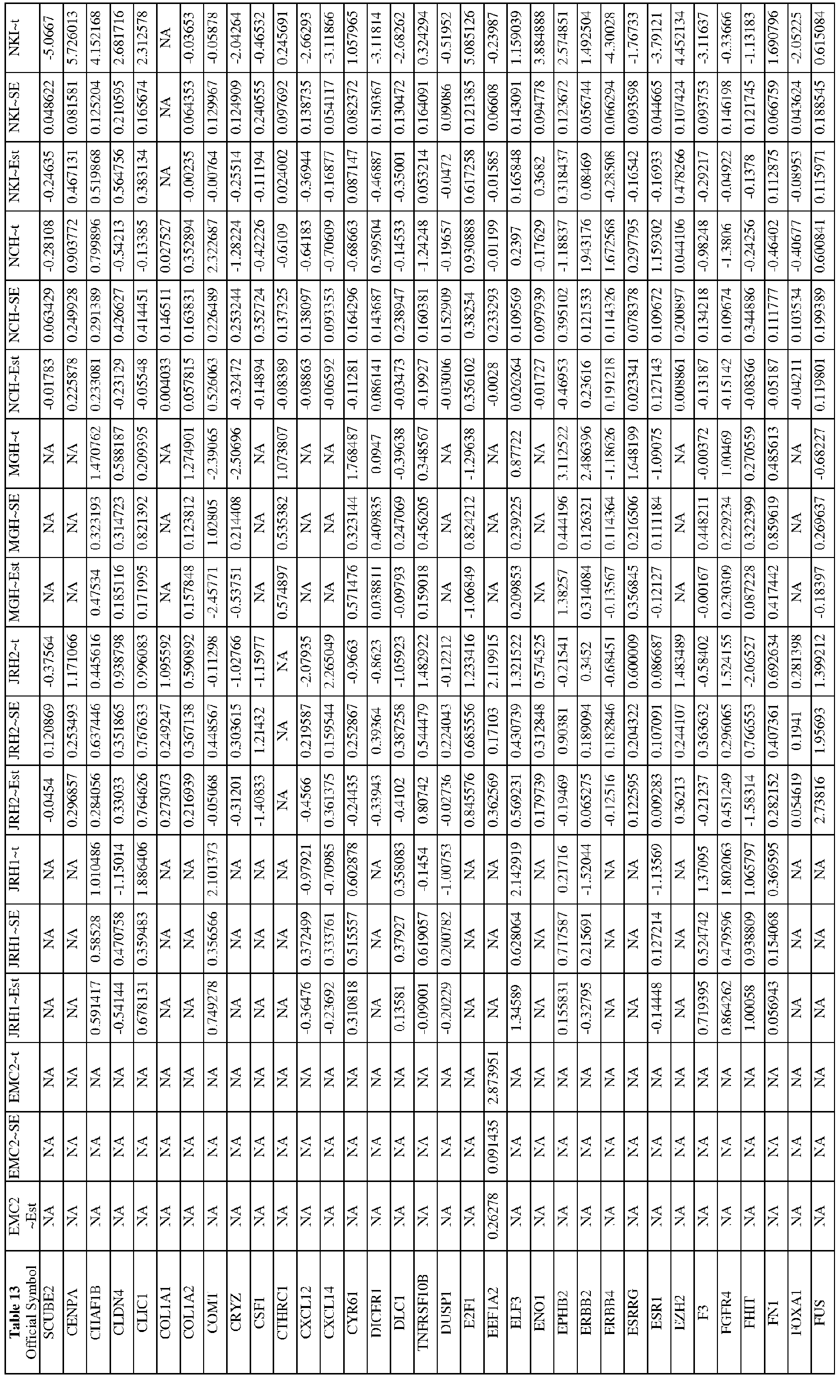

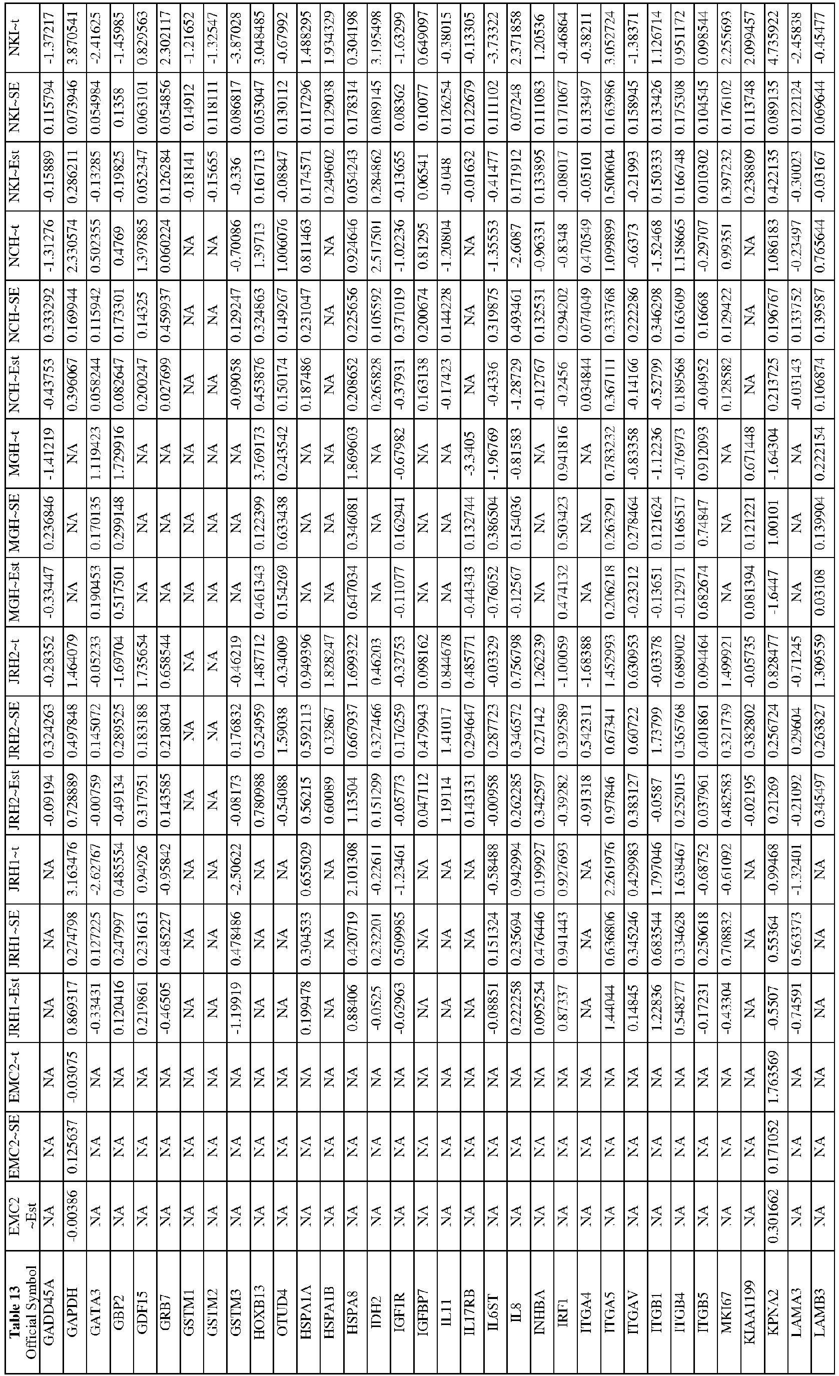

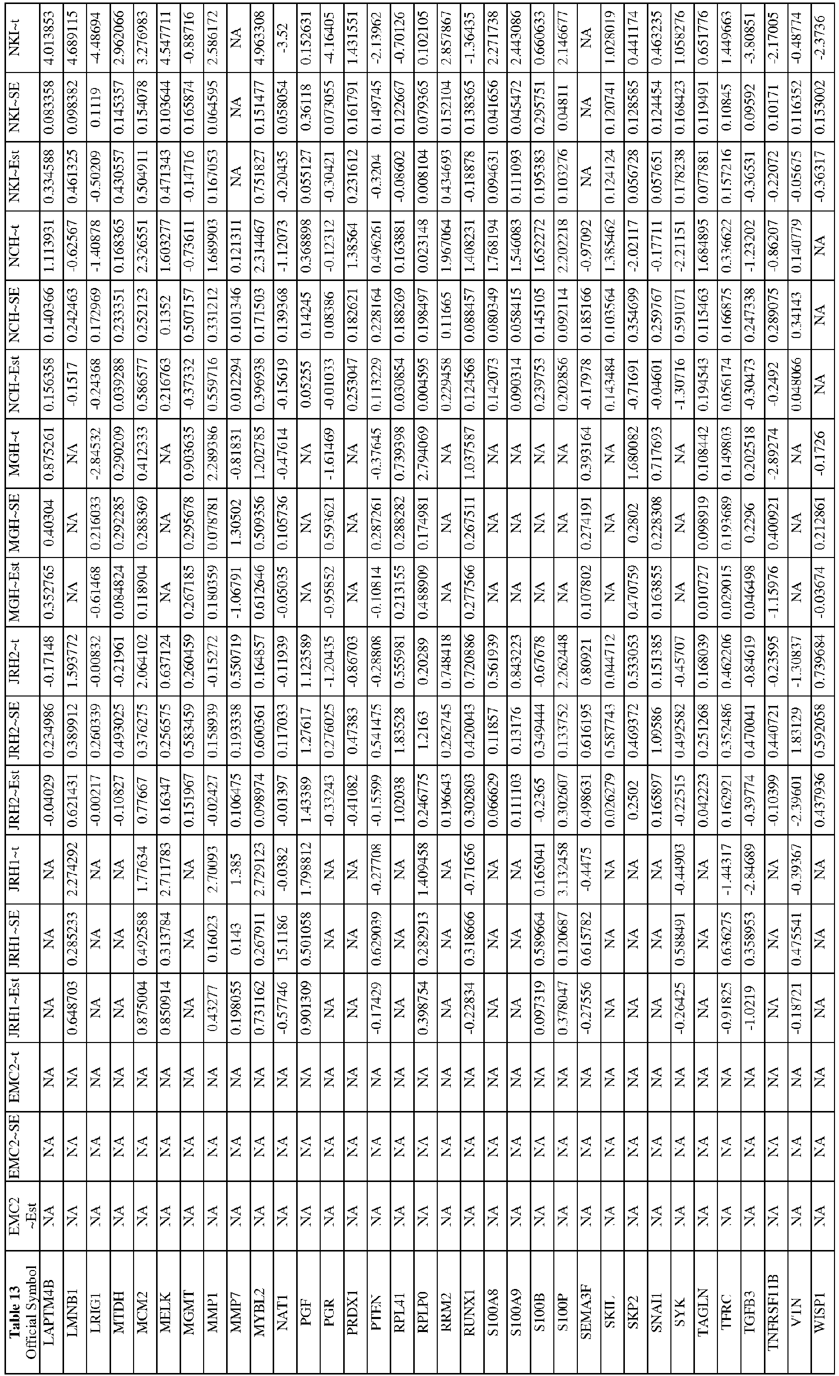

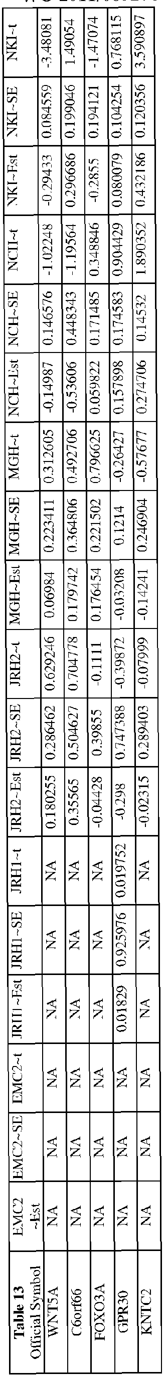

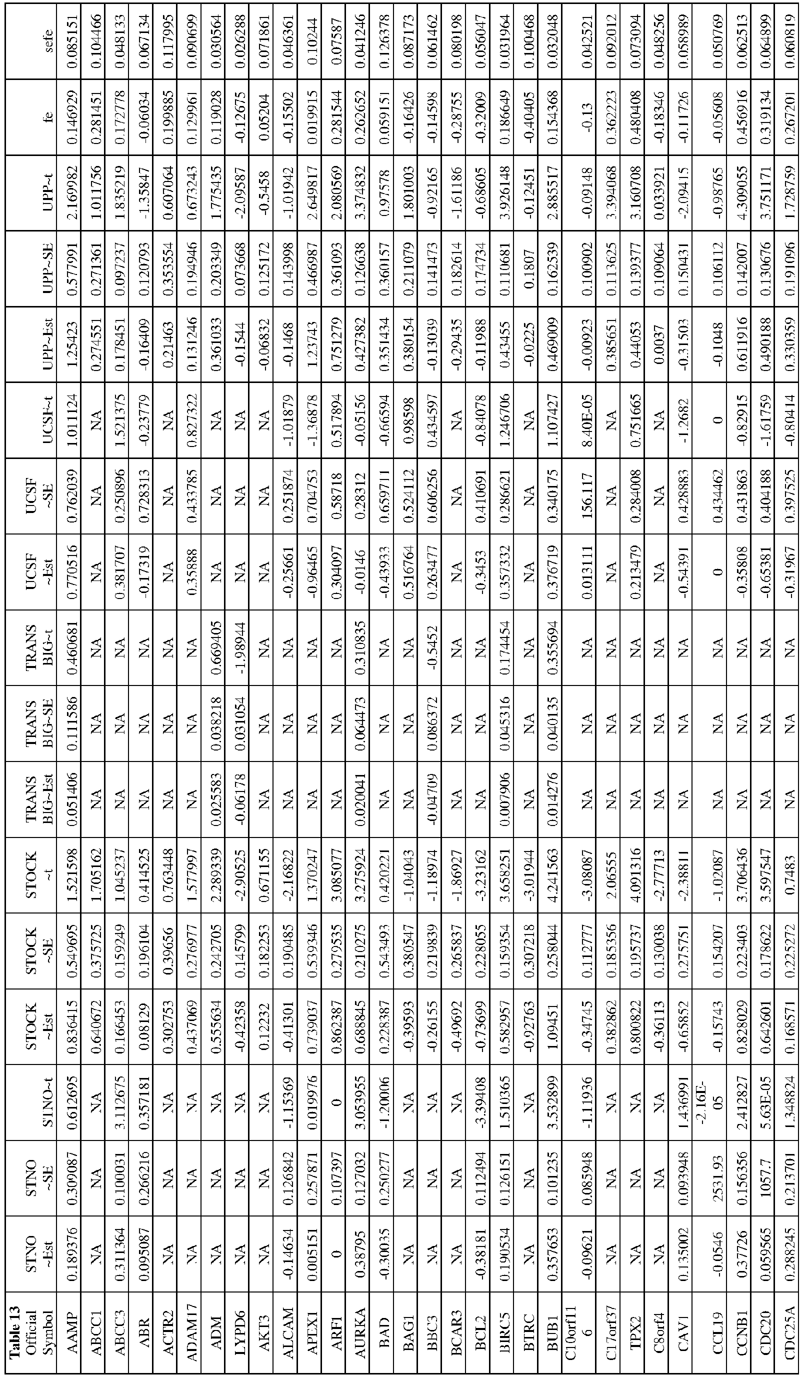

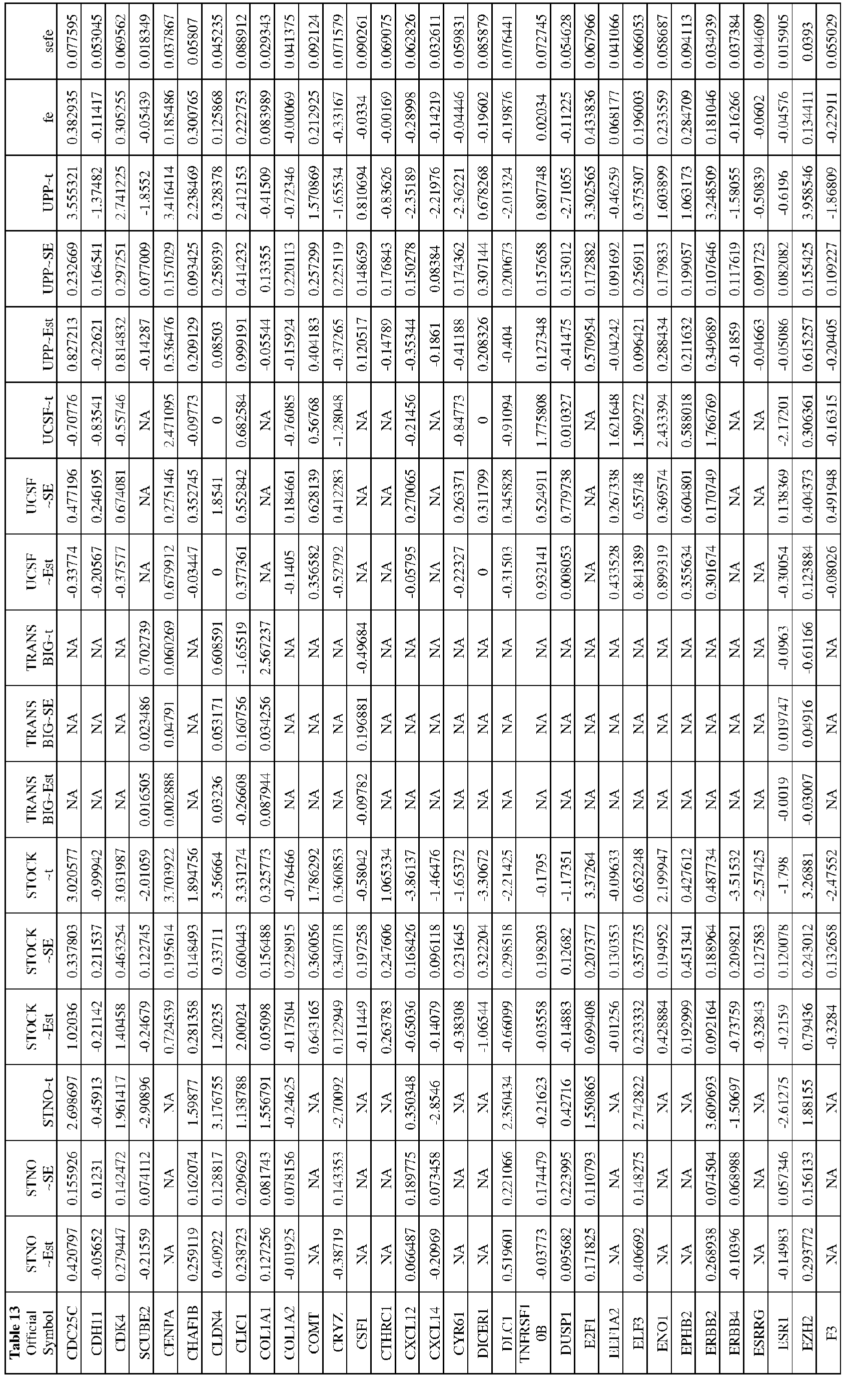

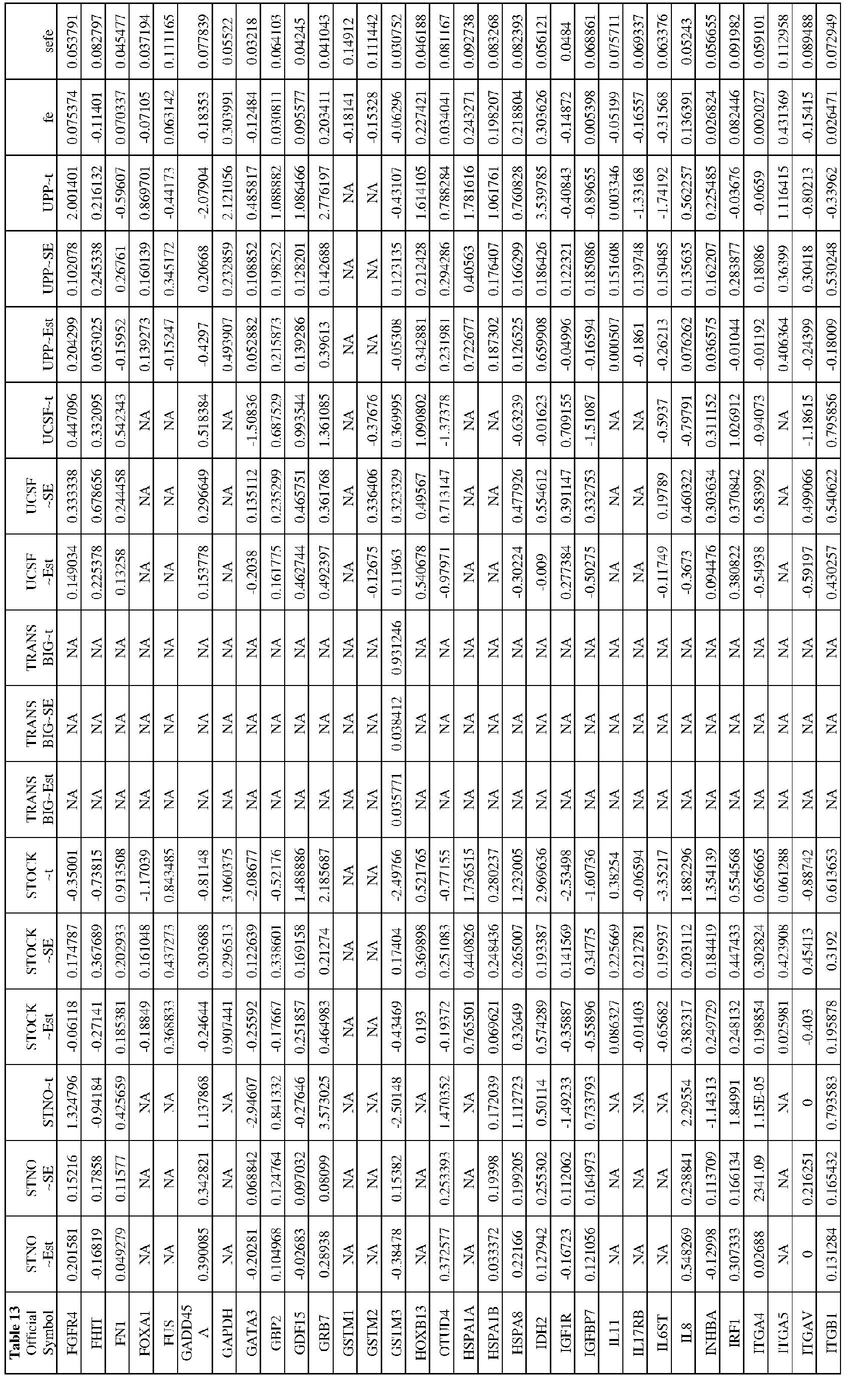

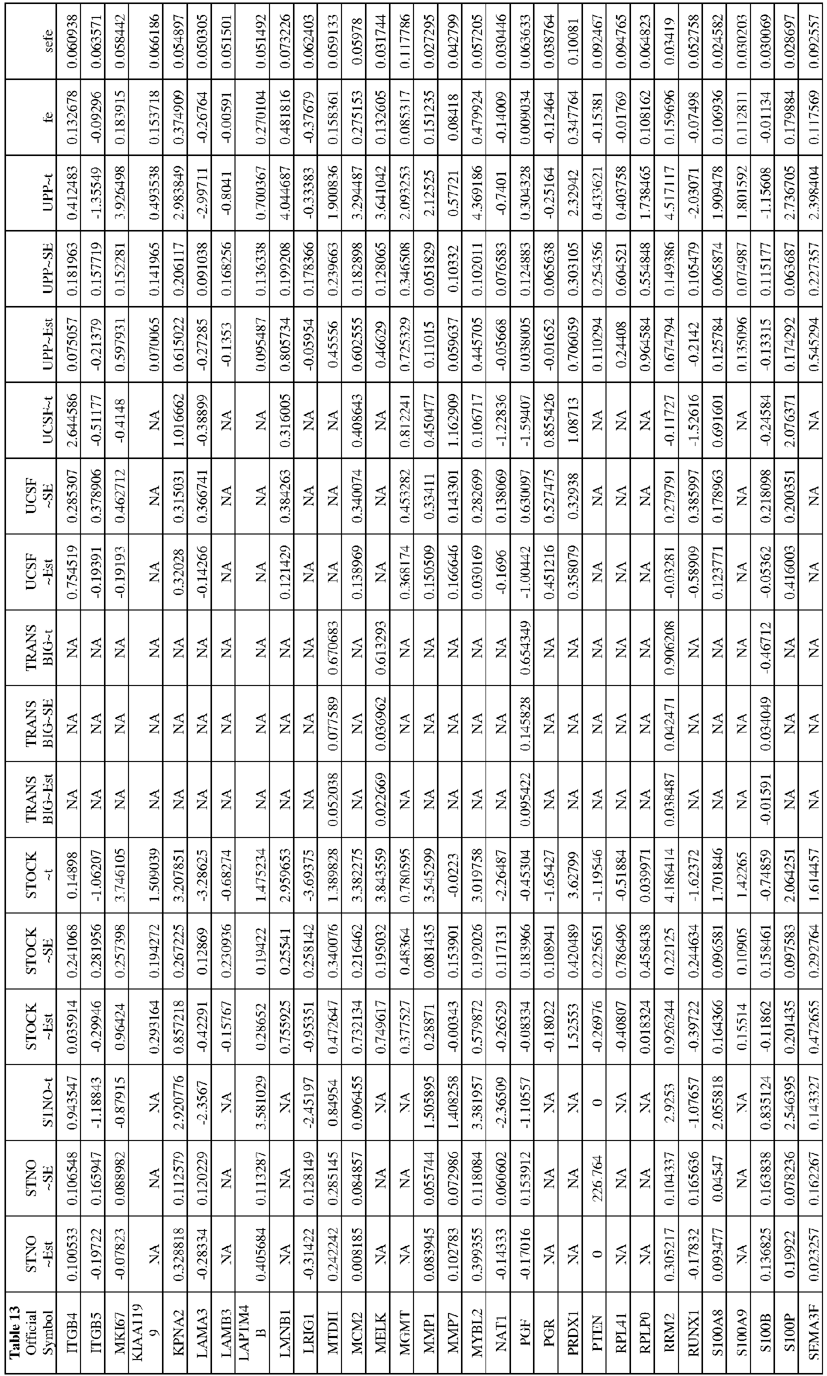

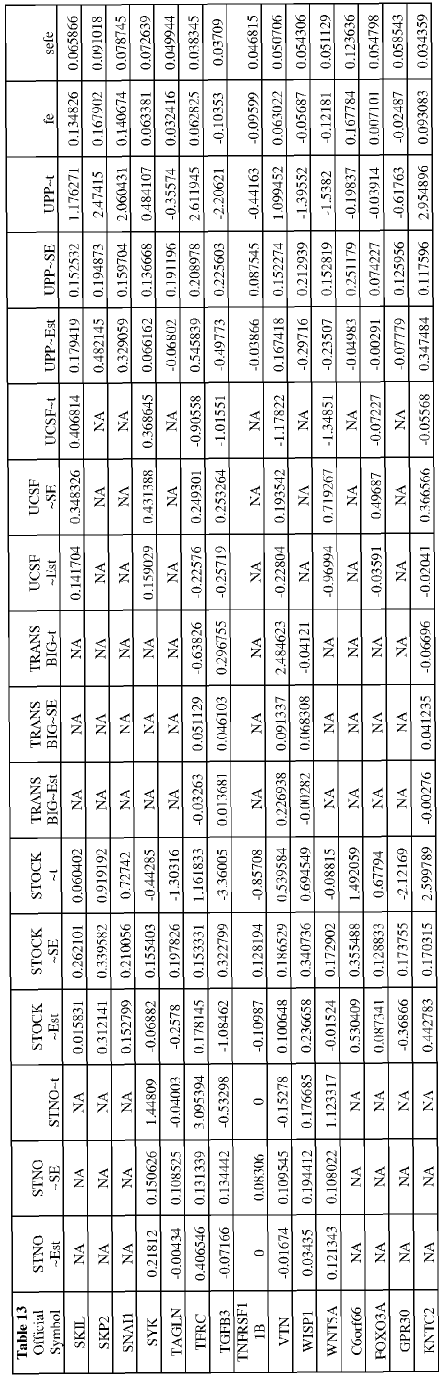

**Table 14: Validation of Transferrin Receptor Group genes in SIB data sets.**

| | **Genes** | | | | | | |
|---|---|---|---|---|---|---|---|
| **Study data set** | TFRC | ENO1 | IDH2 | ARF1 | CLDN4 | PRDX1 | GBP1 |
| EMC2∼Est | NA | NA | NA | NA | NA | NA | NA |
| EMC2∼SE | NA | NA | NA | NA | NA | NA | NA |
| EMC2∼t | NA | NA | NA | NA | NA | NA | NA |
| JRH1∼Est | -0.91825 | NA | -0.0525 | 0.839013 | -0.54144 | NA | 0.137268 |
| JRH1∼SE | 0.636275 | NA | 0.232201 | 0.346692 | 0.470758 | NA | 0.159849 |
| JRH1∼t | -1.44317 | NA | -0.22611 | 2.420053 | -1.15014 | NA | 0.858735 |
| JRH2∼Est | 0.162921 | 0.179739 | 0.151299 | 0.369609 | 0.33033 | -0.41082 | -0.07418 |
| JRH2∼SE | 0.352486 | 0.312848 | 0.327466 | 0.40789 | 0.351865 | 0.47383 | 0.198642 |
| JRH2∼t | 0.462206 | 0.574525 | 0.46203 | 0.906149 | 0.938798 | -0.86703 | -0.37345 |
| MGH∼Est | 0.029015 | NA | NA | 2.03958 | 0.185116 | NA | 0.15434 |
| MGH∼SE | 0.193689 | NA | NA | 0.804729 | 0.314723 | NA | 0.188083 |
| MGH∼t | 0.149803 | NA | NA | 2.534493 | 0.588187 | NA | 0.820595 |
| NCH∼Est | 0.056174 | -0.01727 | 0.265828 | -0.15337 | -0.23129 | 0.253047 | 0.095457 |
| NCH∼SE | 0.166875 | 0.097939 | 0.105592 | 0.204529 | 0.426627 | 0.182621 | 0.1323 |
| NCH∼t | 0.336622 | -0.17629 | 2.517501 | -0.74984 | -0.54213 | 1.38564 | 0.721522 |
| NKI∼Est | 0.157216 | 0.3682 | 0.284862 | 0.944168 | 0.564756 | 0.231612 | 0.13712 |
| NKI∼SE | 0.10845 | 0.094778 | 0.089145 | 0.204641 | 0.210595 | 0.161791 | 0.075391 |
| NKI∼t | 1.449663 | 3.884888 | 3.195498 | 4.613777 | 2.681716 | 1.431551 | 1.818777 |
| STNO∼Est | 0.406546 | NA | 0.127942 | 0 | 0.40922 | NA | 0.298139 |
| STNO∼SE | 0.131339 | NA | 0.255302 | 0.107397 | 0.128817 | NA | 0.113901 |
| STNO∼t | 3.095394 | NA | 0.50114 | 0 | 3.176755 | NA | 2.617528 |
| STOCK∼Est | 0.178145 | 0.428884 | 0.574289 | 0.862387 | 1.20235 | 1.52553 | 0.068821 |
| STOCK-SE | 0.153331 | 0.194952 | 0.193387 | 0.279535 | 0.33711 | 0.420489 | 0.183692 |
| STOCK∼t | 1.161833 | 2.199947 | 2.969636 | 3.085077 | 3.56664 | 3.62799 | 0.374652 |
| TRANSBIG∼Est | -0.03263 | NA | NA | NA | 0.03236 | NA | NA |
| TRANSBIG∼SE | 0.051129 | NA | NA | NA | 0.053171 | NA | NA |
| TRANSBIG∼t | -0.63826 | NA | NA | NA | 0.608591 | NA | NA |
| UCSF∼Est | -0.22576 | 0.899319 | -0.009 | 0.304097 | 0 | 0.358079 | -0.43879 |
| UCSF∼SE | 0.249301 | 0.369574 | 0.554612 | 0.58718 | 1.8541 | 0.32938 | 0.874728 |
| UCSF∼t | -0.90558 | 2.433394 | -0.01623 | 0.517894 | 0 | 1.08713 | -0.50163 |
| UPP∼Est | 0.545839 | 0.288434 | 0.659908 | 0.751279 | 0.08503 | 0.706059 | 0.119778 |
| UPP∼SE | 0.208978 | 0.179833 | 0.186426 | 0.361093 | 0.258939 | 0.303105 | 0.117879 |
| UPP∼t | 2.611945 | 1.603899 | 3.539785 | 2.080569 | 0.328378 | 2.32942 | 1.01611 |
| Fe | 0.062825 | 0.233559 | 0.303626 | 0.281544 | 0.125868 | 0.347764 | 0.139381 |
| Sefe | 0.038345 | 0.058687 | 0.056121 | 0.07587 | 0.045235 | 0.10081 | 0.044464 |

**Table 15: Validation of Stromal Group genes in SIB data sets.**

| Gene | CXCL14 | TNFRSF11B | CXCL12 | C10orf116 | RUNX1 | GSTM2 | TGFB3 | BCAR3 | CAV1 | DLC1 | TNFRSF10B | F3 | DICER1 |
|---|---|---|---|---|---|---|---|---|---|---|---|---|---|
| EMC2∼Est | NA | NA | NA | NA | NA | NA | NA | NA | NA | NA | NA | NA | NA |
| EMC2∼SE | NA | NA | NA | NA | NA | NA | NA | NA | NA | NA | NA | NA | NA |
| EMC2∼t | NA | NA | NA | NA | NA | NA | NA | NA | NA | NA | NA | NA | NA |
| JRH1∼Est | -0.23692 | NA | -0.36476 | -0.1418 | -0.22834 | NA | -1.0219 | NA | -0.20701 | 0.13581 | -0.09001 | 0.719395 | NA |
| JRH1∼SE | 0.333761 | NA | 0.372499 | 0.261554 | 0.318666 | NA | 0.358953 | NA | 0.254401 | 0.37927 | 0.619057 | 0.524742 | NA |
| JRH1∼t | -0.70985 | NA | -0.97921 | -0.54216 | -0.71656 | NA | -2.84689 | NA | -0.81372 | 0.358083 | -0.1454 | 1.37095 | NA |
| JRH2∼Est | 0.361375 | -0.10399 | -0.4566 | 0.036378 | 0.302803 | NA | -0.39774 | -0.29238 | -0.19588 | -0.4102 | 0.80742 | -0.21237 | -0.33943 |
| JRH2∼SE | 0.159544 | 0.440721 | 0.219587 | 0.182183 | 0.420043 | NA | 0.470041 | 0.522706 | 0.289251 | 0.387258 | 0.544479 | 0.363632 | 0.39364 |
| JRH2∼t | 2.265049 | -0.23595 | -2.07935 | 0.19968 | 0.720886 | NA | -0.84619 | -0.55935 | -0.67721 | -1.05923 | 1.482922 | -0.58402 | -0.8623 |
| MGH∼Est | NA | -1.15976 | NA | NA | 0.277566 | NA | 0.046498 | -0.41595 | -0.06896 | -0.09793 | 0.159018 | -0.00167 | 0.038811 |
| MGH∼SE | NA | 0.400921 | NA | NA | 0.267511 | NA | 0.2296 | 0.216837 | 0.2269 | 0.247069 | 0.456205 | 0.448211 | 0.409835 |
| MGH∼t | NA | -2.89274 | NA | NA | 1.037587 | NA | 0.202518 | -1.91825 | -0.30391 | -0.39638 | 0.348567 | -0.00372 | 0.0947 |
| NCH∼Est | -0.06592 | -0.2492 | -0.08863 | 0.064337 | 0.124568 | NA | -0.30473 | 0.072246 | 0.078825 | -0.03473 | -0.19927 | -0.13187 | 0.086141 |
| NCH∼SE | 0.093353 | 0.289075 | 0.138097 | 0.14087 | 0.088457 | NA | 0.247338 | 0.304443 | 0.340843 | 0.238947 | 0.160381 | 0.134218 | 0.143687 |
| NCH∼t | -0.70609 | -0.86207 | -0.64183 | 0.456713 | 1.408231 | NA | -1.23202 | 0.237306 | 0.231265 | -0.14533 | -1.24248 | -0.98248 | 0.599504 |
| NKI∼Est | -0.16877 | -0.22072 | -0.36944 | -0.22589 | -0.18878 | -0.15655 | -0.36531 | -0.26067 | -0.30885 | -0.35001 | 0.053214 | -0.29217 | -0.46887 |
| NKI∼SE | 0.054117 | 0.10171 | 0.138735 | 0.082836 | 0.138365 | 0.118111 | 0.09592 | 0.114992 | 0.133788 | 0.130472 | 0.164091 | 0.093753 | 0.150367 |
| NKI∼t | -3.11866 | -2.17005 | -2.66293 | -2.72696 | -1.36435 | -1.32547 | -3.80851 | -2.26685 | -2.30848 | -2.68262 | 0.324294 | -3.11637 | -3.11814 |
| STNO∼Est | -0.20969 | 0 | 0.066487 | -0.09621 | -0.17832 | NA | -0.07166 | NA | 0.135002 | 0.519601 | -0.03773 | NA | NA |
| STNO∼SE | 0.073458 | 0.08306 | 0.189775 | 0.085948 | 0.165636 | NA | 0.134442 | NA | 0.093948 | 0.221066 | 0.174479 | NA | NA |
| STNO∼t | -2.8546 | 0 | 0.350348 | -1.11936 | -1.07657 | NA | -0.53298 | NA | 1.436991 | 2.350434 | -0.21623 | NA | NA |
| STOCK∼Est | -0.14079 | -0.10987 | -0.65036 | -0.34745 | -0.39722 | NA | -1.08462 | -0.49692 | -0.65852 | -0.66099 | -0.03558 | -0.3284 | -1.06544 |
| STOCK∼SE | 0.096118 | 0.128194 | 0.168426 | 0.112777 | 0.244634 | NA | 0.322799 | 0.265837 | 0.275751 | 0.298518 | 0.198203 | 0.132658 | 0.322204 |
| STOCK∼t | -1.46476 | -0.85708 | -3.86137 | -3.08087 | -1.62372 | NA | -3.36005 | -1.86927 | -2.38811 | -2.21425 | -0.1795 | -2.47552 | -3.30672 |

| Gene | CXXL14 | TNFRSF11B | CXCL12 | C10orf116 | RUNX1 | GSTM2 | TGFB3 | BCAR3 | CAV1 | DLC1 | TNFRSF10B | F3 | DICER1 |
|---|---|---|---|---|---|---|---|---|---|---|---|---|---|
| TRANSBIG∼Est | NA | NA | NA | NA | NA | NA | 0.013681 | NA | NA | NA | NA | NA | N/A |
| TRANSBIG∼SE | NA | NA | NA | NA | NA | NA | 0.046103 | NA | NA | NA | NA | NA | N/A |
| TRANSBIG∼t | NA | NA | NA | NA | NA | NA | 0.296755 | NA | NA | NA | NA | NA | N/A |
| UCSF∼Est | NA | NA | -0.05795 | 0.013111 | -0.58909 | -0.12675 | -0.25719 | NA | -0.54391 | -0.31503 | 0.932141 | -0.08026 | 0 |
| UCSF-SE | NA | NA | 0.270065 | 156.117 | 0.385997 | 0.336406 | 0.253264 | NA | 0.428883 | 0.345828 | 0.524911 | 0.491948 | 0.311799 |
| UCSF∼t | NA | NA | -0.21456 | 8.40E-05 | -1.52616 | -0.37676 | -1.01551 | NA | -1.2682 | -0.91094 | 1.775808 | -0.16315 | 0 |
| UPP∼Est | -0.1861 | -0.03866 | -0.35344 | -0.00923 | -0.2142 | NA | -0.49773 | -0.29435 | -0.31503 | -0.404 | 0.127348 | -0.20405 | 0.208326 |
| UPP∼SE | 0.08384 | 0.087545 | 0.150278 | 0.100902 | 0.105479 | NA | 0.225603 | 0.182614 | 0.150431 | 0.200673 | 0.157658 | 0.109227 | 0.307144 |
| UPP∼t | -2.21976 | -0.44163 | -2.35189 | -0.09148 | -2.03071 | NA | -2.20621 | -1.61186 | -2.09415 | -2.01324 | 0.807748 | -1.86809 | 0.678268 |
| Fe | -0.14219 | -0.09599 | -0.28998 | -0.13 | -0.07498 | -0.15328 | -0.10353 | -0.28755 | -0.11726 | -0.19876 | 0.02034 | -0.22911 | -0.19602 |
| Sefe | 0.032611 | 0.046815 | 0.062826 | 0.042521 | 0.052758 | 0.111442 | 0.03709 | 0.080198 | 0.058989 | 0.076441 | 0.072745 | 0.055029 | 0.085879 |

**Table 16: Genes that co-express with Prognostic genes in ER+ breast cancer tumors (Spearman corr. coef. ≥ 0.7)**

| **Prognostic Gene** | **Table 16** | | | | |
|---|---|---|---|---|---|
| | **Co-expressed Genes** | | | | |
| INHBA | AEBP1 | CDH11 | COL10A1 | COL11A1 | COL1A2 |
| | COL5A1 | COL5A2 | COL8A2 | ENTPD4 | LOXL2 |
| | LRRC15 | MMP11 | NOX4 | PLAU | THBS2 |
| | THY1 | VCAN | | | |
| CAV1 | ANK2 | ANXA1 | AQP1 | C10orf56 | CAV2 |
| | CFH | COL14A1 | CRYAB | CXCL12 | DAB2 |
| | DCN | ECM2 | FHL1 | FLRT2 | GNG11 |
| | GSN | IGF1 | JAM2 | LDB2 | NDN |
| | NRN1 | PCSK5 | PLSCR4 | PROS1 | TGFBR2 |
| NAT1 | PSD3 | | | | |
| GSTM1 | GSTM2 | | | | |
| GSTM2 | GSTM1 | | | | |
| ITGA4 | ARHGAP15 | ARHGAP25 | CCL5 | CD3D | CD48 |
| | CD53 | CORO1A | EVI2B | FGL2 | GIMAP4 |
| | IRF8 | LCK | PTPRC | TFEC | TRAC |
| | TRAF3IP3 | TRBC1 | EVI2A | FLI1 | GPR65 |
| | IL2RB | LCP2 | LOC100133233 | MNDA | PLAC8 |
| | PLEK | TNFAIP8 | | | |
| CCL19 | ARHGAP15 | ARHGAP25 | CCL5 | CCR2 | CCR7 |
| | CD2 | CD247 | CD3D | CD3E | CD48 |
| | CD53 | FLJ78302 | GPR171 | IL10RA | IL7R |
| | IRF8 | LAMP3 | LCK | LTB | PLAC8 |
| | PRKCB1 | PTPRC | PTPRCAP | SASH3 | SPOCK2 |
| | TRA@ | TRBC1 | TRD@ | PPP1R16B | TRAC |
| CDH11 | TAGLN | ADAM12 | AEBP1 | ANGPTL2 | ASPN |
| | BGN | BICC1 | C10orf56 | C1R | C1S |
| | C20orf39 | CALD1 | COL10A1 | COL11A1 | COL1A1 |
| | COL1A2 | COL3A1 | COL5A1 | COL5A2 | COL6A1 |
| | COL6A2 | COL6A3 | COL8A2 | COMP | COPZ2 |
| | CRISPLD2 | CTSK | DACT1 | DCN | DPYSL3 |
| | ECM2 | EFEMP2 | ENTPD4 | FAP | FBLN1 |
| | FBLN2 | FBN1 | FERMT2 | FLRT2 | FN1 |
| | FSTL1 | GAS1 | GLT8D2 | HEPH | HTRA1 |
| | ISLR | ITGBL1 | JAM3 | KDELC1 | LAMA4 |
| | LAMB1 | LOC100133502 | LOX | LOXL2 | LRRC15 |
| | LRRC17 | LUM | MFAP2 | MFAP5 | MMP2 |
| | MRC2 | MXRA5 | MXRA8 | MYL9 | NDN |
| | NID1 | NID2 | NINJ2 | NOX4 | OLFML2B |
| | OMD | PALLD | PCOLCE | PDGFRA | PDGFRB |
| | PDGFRL | POSTN | PRKCDBP | PRKD1 | PTRF |
| | RARRES2 | RCN3 | SERPINF1 | SERPINH1 | SFRP4 |
| | SNAI2 | SPARC | SPOCK1 | SPON1 | SRPX2 |
| | SSPN | TCF4 | THBS2 | THY1 | TNFAIP6 |
| | VCAN | WWTR1 | ZEB1 | ZFPM2 | INHBA |
| | PLS3 | SEC23A | WISP1 | | |
| TAGLN | CDH11 | ADAM12 | AEBP1 | ANGPTL2 | ASPN |
| | BGN | BICC1 | C10orf56 | C1R | C1S |
| | C20orf39 | CALD1 | COL10A1 | COL11A1 | COL1A1 |
| | COL1A2 | COL3A1 | COL5A1 | COL5A2 | COL6A1 |
| | COL6A2 | COL6A3 | COL8A2 | COMP | COPZ2 |
| | CRISPLD2 | CTSK | DACT1 | DCN | DPYSL3 |
| | ECM2 | EFEMP2 | ENTPD4 | FAP | FBLN1 |
| | FBLN2 | FBN1 | FERMT2 | FLRT2 | FN1 |
| | FSTL1 | GAS1 | GLT8D2 | HEPH | HTRA1 |
| | ISLR | ITGBL1 | JAM3 | KDELC1 | LAMA4 |
| | LAMB1 | LOC100133502 | LOX | LOXL2 | LRRC15 |
| | LRRC17 | LUM | MFAP2 | MFAP5 | MMP2 |
| | MRC2 | MXRA5 | MXRA8 | MYL9 | NDN |
| | NID1 | NID2 | NINJ2 | NOX4 | OLFML2B |
| | OMD | PALLD | PCOLCE | PDGFRA | PDGFRB |
| | PDGFRL | POSTN | PRKCDBP | PRKD1 | PTRF |
| | RARRES2 | RCN3 | SERPINF1 | SERPINH1 | SFRP4 |
| | SNAI2 | SPARC | SPOCK1 | SPON1 | SRPX2 |
| | SSPN | TCF4 | THBS2 | THY1 | TNFAIP6 |
| | VCAN | WWTR1 | ZEB1 | ZFPM2 | ACTA2 |
| | CNN1 | DZIP1 | EMILIN1 | | |
| ENO1 | ATP5J2 | C10orf10 | CLDN15 | CNGB1 | DET1 |
| | EIF3CL | HS2ST1 | IGHG4 | KIAA0195 | KIR2DS5 |
| | PARP6 | PRH1 | RAD1 | RIN3 | RPL10 |
| | SGCG | SLC16A2 | SLC9A3R1 | SYNPO2L | THBS1 |
| | ZNF230 | | | | |
| IDH2 | AEBP1 | HIST1H2BN | PCDHAC1 | | |
| ARF1 | CRIM1 | | | | |
| DICER1 | ADM | LOC100133583 | | | |
| AKT3 | AKAP12 | ECM2 | FERMT2 | FLRT2 | JAM3 |
| | LOC100133502 | PROS1 | TCF4 | WWTR1 | ZEB1 |
| CXCL12 | ANXA1 | C1R | C1S | CAV1 | DCN |
| | FLRT2 | SRPX | | | |
| CYR61 | CTGF | | | | |
| IGFBP7 | VIM | | | | |
| KIAA1199 | COL11A1 | PLAU | | | |
| SPC25 | ASPM | BUB1 | BUB1B | CCNA2 | CCNE2 |
| | CDC2 | CDC25C | CENPA | CEP55 | FANCI |
| | GINS1 | HJURP | KIAA0101 | KIF11 | KIF14 |
| | KIF15 | KIF18A | KIF20A | KIF4A | MAD2L1 |
| | MELK | NCAPG | NEK2 | NUSAP1 | PRC1 |
| | STIL | ZWINT | | | |
| WISP1 | CDH11 | COL5A2 | | | |

**Table 17: Genes that co-express with Prognostic Genes in ER- breast cancer tumors (Spearman corr. coef. ≥ 0.7)**

| **Prognostic Gene** | **Table 17** | | | | |
|---|---|---|---|---|---|
| | **Co-expressed Genes** | | | | |
| IRF1 | APOL6 | CXCL10 | GABBR1 | GBP1 | HCP5 |
| | HLA-E | HLA-F | HLA-G | HLA-J | INDO |
| | PSMB8 | PSMB9 | STAT1 | TAP1 | UBD |
| | UBE2L6 | WARS | APOBEC3F | APOBEC3G | APOL1 |
| | APOL3 | ARHGAP25 | BTN3A1 | BTN3A2 | BTN3A3 |
| | C1QB | CCL5 | CD2 | CD38 | CD40 |
| | CD53 | CD74 | CD86 | CSF2RB | CTSS |
| | CYBB | FGL2 | GIMAP5 | GZMA | hCG_1998957 |
| | HCLS1 | HLA-C | HLA-DMA | HLA-DMB | HLA-DPA1 |
| | HLA-DQB1 | HLA-DQB2 | HLA-DRA | HLA-DRB1 | HLA-DRB2 |
| | HLA-DRB3 | HLA-DRB4 | HLA-DRB5 | HLA-DRB6 | IL10RA |
| | IL2RB | LAP3 | LAPTM5 | LOC100133484 | LOC100133583 |
| | LOC100133661 | LOC100133811 | LOC730415 | NKG7 | PLEK |
| | PSMB10 | PTPRC | RNASE2 | SLAMF8 | TFEC |
| | TNFRSF1B | TRA@ | TRAC | TRAJ17 | TRAV20 |
| | ZNF749 | | | | |
| CDH11 | ADAM12 | AEBP1 | ANGPTL2 | ASPN | CFH |
| | CFHR1 | COL10A1 | COL11A1 | COL1A1 | COL1A2 |
| | COL3A1 | COL5A1 | COL5A2 | COL6A3 | CRISPLD2 |
| | CTSK | DACT1 | DCN | FAP | FBN1 |
| | FN1 | HTRA1 | LOX | LRRC15 | LUM |
| | NID2 | PCOLCE | PDGFRB | POSTN | SERPINF1 |
| | SPARC | THBS2 | THY1 | VCAN | DAB2 |
| | GLT8D2 | ITGB5 | JAM3 | LOC100133502 | MMP2 |
| | PRSS23 | TIMP3 | ZEB1 | | |
| CCL19 | ITGA4 | ADAM28 | AIF1 | APOBEC3F | APOBEC3G |
| | APOL3 | ARHGAP15 | ARHGAP25 | CASP1 | CCDC69 |
| | CCR2 | CCR7 | CD2 | CD247 | CD27 |
| | CD37 | CD3D | CD3G | CD48 | CD52 |
| | CD53 | CD74 | CD86 | CD8A | CLEC4A |
| | CORO1A | CTSS | CXCL13 | DOCK10 | EVI2A |
| | EVI2B | FGL2 | FLJ78302 (CCR2) | FYB | GIMAP4 |
| | GIMAP5 | GIMAP6 | GMFG | GPR171 | GPR18 |
| | GPR65 | GZMA | GZMB | GZMK | hCG_1998957 |
| | HCLS1 | HLA-DMA | HLA-DMB | HLA-DPA1 | HLA-DQA1 |
| | HLA-DQA2 | HLA-DQB1 | HLA-DQB2 | HLA-DRB1 | HLA-DRB2 |
| | HLA-DRB3 | HLA-DRB4 | HLA-DRB5 | HLA-E | IGHM |
| | IGSF6 | IL10RA | IL2RG | IL7R | IRF8 |
| | KLRB1 | KLRK1 | LAPTM5 | LAT2 | LCK |
| | LCP2 | LOC100133484 | LOC100133583 | LOC100133661 | LOC100133811 |
| | LOC730415 | LPXN | LRMP | LST1 | LTB |
| | LY96 | LYZ | MFNG | MNDA | MS4A4A |
| | NCKAP1L | PLAC8 | PLEK | PRKCB1 | PSCDBP |
| | PTPRC | PTPRCAP | RAC2 | RNASE2 | RNASE6 |
| | SAMHD1 | SAMSN1 | SASH3 | SELL | SELPLG |
| | SLA | SLAMF1 | SLC7A7 | SP140 | SRGN |
| | TCL1A | TFEC | TNFAIP8 | TNFRSF1B | TRA@ |
| | TRAC | TRAJ17 | TRAT1 | TRAV20 | TRBC1 |
| | TYROBP | ZNF749 | ITM2A | LTB | P2RY13 |
| | PRKCB1 | PTPRCAP | SELL | TRBC1 | |
| ITGA4 | CCL19 | ADAM28 | AIF1 | APOBEC3F | APOBEC3G |
| | APOL3 | ARHGAP15 | ARHGAP25 | CASP1 | CCDC69 |
| | CCR2 | CCR7 | CD2 | CD247 | CD27 |
| | CD37 | CD3D | CD3G | CD48 | CD52 |
| | CD53 | CD74 | CD86 | CD8A | CLEC4A |
| | CORO1A | CTSS | CXCL13 | DOCK10 | EVI2A |
| | EVI2B | FGL2 | FLJ78302 (CCR2) | FYB | GIMAP4 |
| | GIMAP5 | GIMAP6 | GMFG | GPR171 | GPR18 |
| | GPR65 | GZMA | GZMB | GZMK | hCG_1998957 |
| | HCLS1 | HLA-DMA | HLA-DMB | HLA-DPA1 | HLA-DQA1 |
| | HLA-DQA2 | HLA-DQB1 | HLA-DQB2 | HLA-DRB1 | HLA-DRB2 |
| | HLA-DRB3 | HLA-DRB4 | HLA-DRB5 | HLA-E | IGHM |
| | IGSF6 | IL10RA | IL2RG | IL7R | IRF8 |
| | KLRB1 | KLRK1 | LAPTM5 | LAT2 | LCK |
| | LCP2 | LOC100133484 | LOC100133583 | LOC100133661 | LOC100133811 |
| | LOC730415 | LPXN | LRMP | LST1 | LTB |
| | LY96 | LYZ | MFNG | MNDA | MS4A4A |
| | NCKAP1L | PLAC8 | PLEK | PRKCB1 | PSCDBP |
| | PTPRC | PTPRCAP | RAC2 | RNASE2 | RNASE6 |
| | SAMHD1 | SAMSN1 | SASH3 | SELL | SELPLG |
| | SLA | SLAMF1 | SLC7A7 | SP140 | SRGN |
| | TCL1A | TFEC | TNFAIP8 | TNFRSF1B | TRA@ |
| | TRAC | TRAJ17 | TRAT1 | TRAV20 | TRBC1 |
| | TYROBP | ZNF749 | MARCH1 | C17orf60 | CSF1R |
| | FLI1 | FLJ78302 | FYN | IKZF1 | INPP5D |
| | NCF4 | NR3C1 | P2RY13 | PLXNC1 | PSCD4 |
| | PTPN22 | SERPINB9 | SLCO2B1 | VAMP3 | WIPF1 |
| IDH2 | AEBP1 | DSG3 | HIST1H2BN | PCDHAC1 | |
| ARF1 | FABP5L2 | FLNB | IL1RN | PAX6 | |
| DICER1 | ARS2 | IGHA1 | VDAC3 | | |
| TFRC | RGS20 | | | | |
| ADAM17 | TFDP3 | GPR107 | | | |
| CAV1 | CAV2 | CXCL12 | IGF1 | | |
| CYR61 | CTGF | | | | |
| ESR1 | CBLN1 | SLC45A2 | | | |
| GSTM1 | GSTM2 | | | | |
| GSTM2 | GSTM1 | | | | |
| IL11 | FAM135A | | | | |
| IL6ST | P2RY5 | | | | |
| IGFBP7 | SPARCL1 | TMEM204 | | | |
| INHBA | COL10A1 | FN1 | SULF1 | | |
| SPC25 | KIF4A | KIF20A | NCAPG | | |
| TAGLN | ACTA2 | MYL9 | NNMT | PTRF | |
| TGFB3 | GALNT10 | HTRA1 | LIMA1 | | |
| TNFRSF10B | BIN3 | | | | |
| FOXA1 | CLCA2 | TFAP2B | AGR2 | MLPH | SPDEF |
| CXCL12 | DCN | CAV1 | IGF1 | CFH | |
| GBP2 | APOL1 | APOL3 | CD2 | CTSS | CXCL9 |
| | CXCR6 | GBP1 | GZMA | HLA-DMA | HLA-DMB |
| | IL2RB | PTPRC | TRBC1 | | |

**Table 18: Genes that co-express with Prognostic Genes in all breast cancer tumors (Spearman corr. coef. ≥ 0.7)**

| **Prognostic Gene** | **Table 18** | | | | |
|---|---|---|---|---|---|
| | **Co-expressed Genes** | | | | |
| S100A8 | S100A9 | | | | |
| S100A9 | S100A8 | | | | |
| MKI67 | BIRC5 | KIF20A | MCM10 | | |
| MTDH | ARMC1 | AZIN1 | ENY2 | MTERFD1 | POLR2K |
| | PTDSS1 | RAD54B | SLC25A32 | TMEM70 | UBE2V2 |
| GSTM1 | GSTM2 | | | | |
| GSTM2 | GSTM1 | | | | |
| CXCL12 | AKAP12 | DCN | F13A1 | | |
| TGFB3 | C10orf56 | JAM3 | | | |
| TAGLN | ACTA2 | CALD1 | COPZ2 | FERMT2 | HEPH |
| | MYL9 | NNMT | PTRF | TPM2 | |
| PGF | ALMS1 | ATP8B1 | CEP27 | DBT | FAM128B |
| | FBXW12 | FGFR1 | FLJ12151 | FLJ42627 | GTF2H3 |
| | HCG2P7 | KIAA0894 | KLHL24 | LOC152719 | PDE4C |
| | PODNL1 | POLR1B | PRDX2 | PRR11 | RIOK3 |
| | RP5-886K2.1 | SLC35E1 | SPN | USP34 | ZC3H7B |
| | ZNF160 | ZNF611 | | | |
| CCL19 | ARHGAP15 | ARHGAP25 | CCL5 | CCR2 | CCR7 |
| | CD2 | CD37 | CD3D | CD48 | CD52 |
| | CSF2RB | FLJ78302 | GIMAP5 | GIMAP6 | GPR171 |
| | GZMK | IGHM | IRF8 | LCK | LTB |
| | PLAC8 | PRKCB1 | PTGDS | PTPRC | PTPRCAP |
| | SASH3 | TNFRSF1B | TRA@ | TRAC | TRAJ17 |
| | TRAV20 | TRBC1 | | | |
| IRF1 | ITGA4 | MARCH1 | AIF1 | APOBEC3F | APOBEC3G |
| | APOL1 | APOL3 | ARHGAP15 | ARHGAP25 | BTN3A2 |
| | BTN3A3 | CASP1 | CCL4 | CCL5 | CD2 |
| | CD37 | CD3D | CD48 | CD53 | CD69 |
| | CD8A | CORO1A | CSF2RB | CST7 | CYBB |
| | EVI2A | EVI2B | FGL2 | FLI1 | GBP1 |
| | GIMAP4 | GIMAP5 | GIMAP6 | GMFG | GPR65 |
| | GZMA | GZMK | hCG_1998957 | HCLS1 | HLA-DMA |
| | HLA-DMB | HLA-DPA1 | HLA-DQB1 | HLA-DQB2 | HLA-DRA |
| | HLA-DRB1 | HLA-DRB2 | HLA-DRB3 | HLA-DRB4 | HLA-DRB5 |
| | HLA-E | HLA-F | IGSF6 | IL10RA | IL2RB |
| | IRF8 | KLRK1 | LCK | LCP2 | LOC100133583 |
| | LOC100133661 | LOC100133811 | LST1 | LTB | LY86 |
| | MFNG | MNDA | NKG7 | PLEK | PRKCB1 |
| | PSCDBP | PSMB10 | PSMB8 | PSMB9 | PTPRC |
| | PTPRCAP | RAC2 | RNASE2 | RNASE6 | SAMSN1 |
| | SLA | SRGN | TAP1 | TFEC | TNFAIP3 |
| | TNFRSF1B | TRA@ | TRAC | TRAJ17 | TRAV20 |
| | TRBC1 | TRIM22 | ZNF749 | | |
| ITGA4 | IRF1 | MARCH1 | AIF1 | APOBEC3F | APOBEC3G |
| | APOL1 | APOL3 | ARHGAP15 | ARHGAP25 | BTN3A2 |
| | BTN3A3 | CASP1 | CCL4 | CCL5 | CD2 |
| | CD37 | CD3D | CD48 | CD53 | CD69 |
| | CD8A | CORO1A | CSF2RB | CST7 | CYBB |
| | EVI2A | EVI2B | FGL2 | FLI1 | GBP1 |
| | GIMAP4 | GIMAP5 | GIMAP6 | GMFG | GPR65 |
| | GZMA | GZMK | hCG_1998957 | HCLS1 | HLA-DMA |
| | HLA-DMB | HLA-DPA1 | HLA-DQB1 | HLA-DQB2 | HLA-DRA |
| | HLA-DRB1 | HLA-DRB2 | HLA-DRB3 | HLA-DRB4 | HLA-DRB5 |
| | HLA-E | HLA-F | IGSF6 | IL10RA | IL2RB |
| | IRF8 | KLRK1 | LCK | LCP2 | LOC100133583 |
| | LOC100133661 | LOC100133811 | LST1 | LTB | LY86 |
| | MFNG | MNDA | NKG7 | PLEK | PRKCB1 |
| | PSCDBP | PSMB10 | PSMB8 | PSMB9 | PTPRC |
| | PTPRCAP | RAC2 | RNASE2 | RNASE6 | SAMSN1 |
| | SLA | SRGN | TAP1 | TFEC | TNFAIP3 |
| | TNFRSF1B | TRA@ | TRAC | TRAJ17 | TRAV20 |
| | TRBC1 | TRIM22 | ZNF749 | CTSS | |
| SPC25 | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP 13 | TTK | TYMS | UBE2C |
| | UBE2S | AURKA | BIRC5 | BUB1 | CCNB1 |
| | CENPA | KPNA2 | LMNB1 | MCM2 | MELK |
| | NDC80 | TPX2 | | | |
| AURKA | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP 13 | TTK | TYMS | UBE2C |
| | UBE2S | SPC25 | BIRC5 | BUB1 | CCNB1 |
| | CENPA | KPNA2 | LMNB1 | MCM2 | MELK |
| | NDC80 | TPX2 | PSMA7 | CSE1L | |
| BIRC5 | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP 13 | TTK | TYMS | UBE2C |
| | UBE2S | AURKA | SPC25 | BUB1 | CCNB1 |
| | CENPA | KPNA2 | LMNB1 | MCM2 | MELK |
| | NDC80 | TPX2 | MKI67 | | |
| BUB1 | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP 13 | TTK | TYMS | UBE2C |
| | UBE2S | AURKA | BIRC5 | SPC25 | CCNB1 |
| | CENPA | KPNA2 | LMNB1 | MCM2 | MELK |
| | NDC80 | TPX2 | | | |
| CCNB1 | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP 13 | TTK | TYMS | UBE2C |
| | UBE2S | AURKA | BIRC5 | BUB1 | SPC25 |
| | CENPA | KPNA2 | LMNB1 | MCM2 | MELK |
| | NDC80 | TPX2 | | | |
| CENPA | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP 13 | TTK | TYMS | UBE2C |
| | UBE2S | AURKA | BIRC5 | BUB1 | CCNB1 |
| | SPC25 | KPNA2 | LMNB1 | MCM2 | MELK |
| | NDC80 | TPX2 | | | |
| KPNA2 | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP 13 | TTK | TYMS | UBE2C |
| | UBE2S | AURKA | BIRC5 | BUB1 | CCNB1 |
| | CENPA | SPC25 | LMNB1 | MCM2 | MELK |
| | NDC80 | TPX2 | NOL11 | PSMD12 | |
| LMNB1 | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP 13 | TTK | TYMS | UBE2C |
| | UBE2S | AURKA | BIRC5 | BUB1 | CCNB1 |
| | CENPA | KPNA2 | SPC25 | MCM2 | MELK |
| | NDC80 | TPX2 | | | |
| MCM2 | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP 13 | TTK | TYMS | UBE2C |
| | UBE2S | AURKA | BIRC5 | BUB1 | CCNB1 |
| | CENPA | KPNA2 | LMNB1 | SPC25 | MELK |
| | NDC80 | TPX2 | | | |
| MELK | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP 13 | TTK | TYMS | UBE2C |
| | UBE2S | AURKA | BIRC5 | BUB1 | CCNB1 |
| | CENPA | KPNA2 | LMNB1 | MCM2 | SPC25 |
| | NDC80 | TPX2 | | | |
| NDC80 | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP 13 | TTK | TYMS | UBE2C |
| | UBE2S | AURKA | BIRC5 | BUB1 | CCNB1 |
| | CENPA | KPNA2 | LMNB1 | MCM2 | MELK |
| | SPC25 | TPX2 | | | |
| TPX2 | ASPM | ATAD2 | AURKB | BUB1B | C12orf48 |
| | CCNA2 | CCNE1 | CCNE2 | CDC2 | CDC45L |
| | CDC6 | CDCA3 | CDCA8 | CDKN3 | CENPE |
| | CENPF | CENPN | CEP55 | CHEK1 | CKS1B |
| | CKS2 | DBF4 | DEPDC1 | DLG7 | DNAJC9 |
| | DONSON | E2F8 | ECT2 | ERCC6L | FAM64A |
| | FBXO5 | FEN1 | FOXM1 | GINS1 | GTSE1 |
| | H2AFZ | HJURP | HMMR | KIF11 | KIF14 |
| | KIF15 | KIF18A | KIF20A | KIF23 | KIF2C |
| | KIF4A | KIFC1 | MAD2L1 | MCM10 | MCM6 |
| | NCAPG | NEK2 | NUSAP1 | OIP5 | PBK |
| | PLK4 | PRC1 | PTTG1 | RACGAP1 | RAD51AP1 |
| | RFC4 | SMC2 | STIL | STMN1 | TACC3 |
| | TOP2A | TRIP 13 | TTK | TYMS | UBE2C |
| | UBE2S | AURKA | BIRC5 | BUB1 | CCNB1 |
| | CENPA | KPNA2 | LMNB1 | MCM2 | MELK |
| | NDC80 | SPC25 | | | |
| CDH11 | INHBA | WISP1 | COL1A1 | COL1A2 | FN1 |
| | ADAM12 | AEBP1 | ANGPTL2 | ASPN | BGN |
| | BNC2 | C1QTNF3 | COL10A1 | COL11A1 | COL3A1 |
| | COL5A1 | COL5A2 | COL5A3 | COL6A3 | COMP |
| | CRISPLD2 | CTSK | DACT1 | DCN | DKK3 |
| | DPYSL3 | EFEMP2 | EMILIN1 | FAP | FBN1 |
| | FSTL1 | GLT8D2 | HEG1 | HTRA1 | ITGBL1 |
| | JAM3 | KIAA1462 | LAMA4 | LOX | LOXL1 |
| | LRP1 | LRRC15 | LRRC17 | LRRC32 | LUM |
| | MFAP5 | MICAL2 | MMP11 | MMP2 | MXRA5 |
| | MXRA8 | NID2 | NOX4 | OLFML2B | PCOLCE |
| | PDGFRB | PLAU | POSTN | SERPINF1 | SPARC |
| | SPOCK1 | SPON1 | SRPX2 | SULF1 | TCF4 |
| | THBS2 | THY1 | VCAN | ZEB1 | |
| INHBA | CDH11 | WISP1 | COL1A1 | COL1A2 | FN1 |
| | ADAM12 | AEBP1 | ANGPTL2 | ASPN | BGN |
| | BNC2 | C1QTNF3 | COL10A1 | COL11A1 | COL3A1 |
| | COL5A1 | COL5A2 | COL5A3 | COL6A3 | COMP |
| | CRISPLD2 | CTSK | DACT1 | DCN | DKK3 |
| | DPYSL3 | EFEMP2 | EMILIN1 | FAP | FBN1 |
| | FSTL1 | GLT8D2 | HEG1 | HTRA1 | ITGBL1 |
| | JAM3 | KIAA1462 | LAMA4 | LOX | LOXL1 |
| | LRP1 | LRRC15 | LRRC17 | LRRC32 | LUM |
| | MFAP5 | MICAL2 | MMP11 | MMP2 | MXRA5 |
| | MXRA8 | NID2 | NOX4 | OLFML2B | PCOLCE |
| | PDGFRB | PLAU | POSTN | SERPINF1 | SPARC |
| | SPOCK1 | SPON1 | SRPX2 | SULF1 | TCF4 |
| | THBS2 | THY1 | VCAN | ZEB1 | |
| WISP1 | INHBA | CDH11 | COL1A1 | COL1A2 | FN1 |
| | ADAM12 | AEBP1 | ANGPTL2 | ASPN | BGN |
| | BNC2 | C1QTNF3 | COL10A1 | COL11A1 | COL3A1 |
| | COL5A1 | COL5A2 | COL5A3 | COL6A3 | COMP |
| | CRISPLD2 | CTSK | DACT1 | DCN | DKK3 |
| | DPYSL3 | EFEMP2 | EMILIN1 | FAP | FBN1 |
| | FSTL1 | GLT8D2 | HEG1 | HTRA1 | ITGBL1 |
| | JAM3 | KIAA1462 | LAMA4 | LOX | LOXL1 |
| | LRP1 | LRRC15 | LRRC17 | LRRC32 | LUM |
| | MFAP5 | MICAL2 | MMP11 | MMP2 | MXRA5 |
| | MXRA8 | NID2 | NOX4 | OLFML2B | PCOLCE |
| | PDGFRB | PLAU | POSTN | SERPINF1 | SPARC |
| | SPOCK1 | SPON1 | SRPX2 | SULF1 | TCF4 |
| | THBS2 | THY1 | VCAN | ZEB1 | |
| COL1A1 | INHBA | WISP1 | CDH11 | COL1A2 | FN1 |
| | ADAM12 | AEBP1 | ANGPTL2 | ASPN | BGN |
| | BNC2 | C1QTNF3 | COL10A1 | COL11A1 | COL3A1 |
| | COL5A1 | COL5A2 | COL5A3 | COL6A3 | COMP |
| | CRISPLD2 | CTSK | DACT1 | DCN | DKK3 |
| | DPYSL3 | EFEMP2 | EMILIN1 | FAP | FBN1 |
| | FSTL1 | GLT8D2 | HEG1 | HTRA1 | ITGBL1 |
| | JAM3 | KIAA1462 | LAMA4 | LOX | LOXL1 |
| | LRP1 | LRRC15 | LRRC17 | LRRC32 | LUM |
| | MFAP5 | MICAL2 | MMP11 | MMP2 | MXRA5 |
| | MXRA8 | NID2 | NOX4 | OLFML2B | PCOLCE |
| | PDGFRB | PLAU | POSTN | SERPINF1 | SPARC |
| | SPOCK1 | SPON1 | SRPX2 | SULF1 | TCF4 |
| | THBS2 | THY1 | VCAN | ZEB1 | |
| COL1A2 | INHBA | WISP1 | COL1A1 | CDH11 | FN1 |
| | ADAM12 | AEBP1 | ANGPTL2 | ASPN | BGN |
| | BNC2 | C1QTNF3 | COL10A1 | COL11A1 | COL3A1 |
| | COL5A1 | COL5A2 | COL5A3 | COL6A3 | COMP |
| | CRISPLD2 | CTSK | DACT1 | DCN | DKK3 |
| | DPYSL3 | EFEMP2 | EMILIN1 | FAP | FBN1 |
| | FSTL1 | GLT8D2 | HEG1 | HTRA1 | ITGBL1 |
| | JAM3 | KIAA1462 | LAMA4 | LOX | LOXL1 |
| | LRP1 | LRRC15 | LRRC17 | LRRC32 | LUM |
| | MFAP5 | MICAL2 | MMP11 | MMP2 | MXRA5 |
| | MXRA8 | NID2 | NOX4 | OLFML2B | PCOLCE |
| | PDGFRB | PLAU | POSTN | SERPINF1 | SPARC |
| | SPOCK1 | SPON1 | SRPX2 | SULF1 | TCF4 |
| | THBS2 | THY1 | VCAN | ZEB1 | |
| FN1 | INHBA | WISP1 | COL1A1 | COL1A2 | CDH11 |
| | ADAM12 | AEBP1 | ANGPTL2 | ASPN | BGN |
| | BNC2 | C1QTNF3 | COL10A1 | COL11A1 | COL3A1 |
| | COL5A1 | COL5A2 | COL5A3 | COL6A3 | COMP |
| | CRISPLD2 | CTSK | DACT1 | DCN | DKK3 |
| | DPYSL3 | EFEMP2 | EMILIN1 | FAP | FBN1 |
| | FSTL1 | GLT8D2 | HEG1 | HTRA1 | ITGBL1 |
| | JAM3 | KIAA1462 | LAMA4 | LOX | LOXL1 |
| | LRP1 | LRRC15 | LRRC17 | LRRC32 | LUM |
| | MFAP5 | MICAL2 | MMP11 | MMP2 | MXRA5 |
| | MXRA8 | NID2 | NOX4 | OLFML2B | PCOLCE |
| | PDGFRB | PLAU | POSTN | SERPINF1 | SPARC |
| | SPOCK1 | SPON1 | SRPX2 | SULF1 | TCF4 |
| | THBS2 | THY1 | VCAN | ZEB1 | |

## Claims

1. A method for predicting the clinical outcome of a patient diagnosed with cancer comprising:
(a) obtaining an expression level of an expression product of at least one prognostic gene from a tissue sample obtained from a tumour of the patient, wherein the at least one prognostic gene is IL6ST;
(b) normalizing the expression level of the expression product of the at least one prognostics gene to obtain a normalized expression level;
(c) expressing the normalized expression level as an expression value compared to an expression level from a tumour reference set; and
(d) calculating a risk score based on the normalized expression value, wherein increased expression of IL6ST is positively correlated with good prognosis.

2. A method as claimed in claim 1, further comprising: generating a report based on the risk score.

3. A method as claimed in claim 1, wherein the patient is a human patient.

4. A method as claimed in claim 1, wherein the tumour is a breast cancer tumour.

5. A method as claimed in claim 1, wherein the tissue sample is a fixed paraffin-embedded tissue.

6. A method as claimed in claim 1, wherein the expression level is obtained using a PCR-based method.

7. A method as claimed in claim 1, wherein an expression level is obtained from at least two genes in any of the stromal, metabolic, immune, proliferation, or metabolic groups, or their gene products.

8. A method as claimed in claim 1, wherein an expression level is obtained from at least four genes in any two of the stromal, metabolic, immune, proliferation, or metabolic groups, or their gene products.

9. A method as claimed in claim 1, further comprising obtaining an expression level of at least one co-expressed gene from those listed in Table 18.

10. A method for classifying a cancer patient according to prognosis, comprising the steps of:
(a) receiving a first data structure comprising the respective levels of an expression product of each of at least three different prognostic genes listed in any of Tables 1-12 in a tissue sample obtained from tumour in the patient, wherein one of the prognostic genes is IL6ST;
(b) normalizing the at least three expression values to obtain normalized expression values;
(c) determining the similarity of the normalized expression values of each of the at least three prognostic genes to respective control levels of expression of the at least three prognostic genes obtained from a second data structure to obtain a patient similarity value, wherein the second data structure is based on levels of expression from a plurality of cancer tumours;
(d) comparing the patient similarity value to a selected threshold value of similarity of the respective normalized expression values of each of the at least three prognostic genes to the respective control levels of expression of the at least three prognostic genes; and
(e) classifying the patient as having a first prognosis if the patient similarity value exceeds the threshold similarity value, and a second prognosis if the patient similarity value does not exceed the threshold similarity value.

11. A computer program comprising computer code means for performing steps (b) to (d) of a method as claimed in any of claims 1 to 10,
wherein the expression level of an expression product of at least one prognostic gene has been obtained from a tissue sample obtained from a tumour of the patient, wherein the at least one prognostic gene is IL6ST
wherein said program is run on a computer.

12. A computer program as claimed in claim 11 embodied on a computer-readable medium.

## Patentansprüche

1. Verfahren zum Prognostizieren des klinischen Outcomes eines Patienten mit diagnostiziertem Krebs, das umfasst:
(a) Erhalten einer Expressionshöhe eines Expressionsprodukts zumindest eines Prognosegens aus einer Gewebeprobe, die aus einem Tumor des Patienten erhalten wurde, wobei das zumindest eine Prognosegen IL6ST ist;
(b) Normalisieren der Expressionshöhe des Expressionsprodukts des zumindest einen Prognosegens, um eine normalisierte Expressionshöhe zu erhalten;
(c) Ausdrücken der normalisierten Expressionshöhe als Expressionswerts im Vergleich zu einer Expressionshöhe aus einem Tumorreferenzsatz; und
(d) Berechnen einer Risikobewertung auf Basis des normalisierten Expressionswerts, wobei eine erhöhte Expression von IL6ST positiv mit einer guten Prognose korreliert ist.

2. Verfahren nach Anspruch 1, das ferner umfasst: Erstellen eines Berichts auf Basis der Risikobewertung.

3. Verfahren nach Anspruch 1, wobei der Patient ein humaner Patient ist.

4. Verfahren nach Anspruch 1, wobei der Tumor ein Brustkrebstumor ist.

5. Verfahren nach Anspruch 1, wobei die Gewebeprobe ein fixiertes in Paraffin eingebettetes Gewebe ist.

6. Verfahren nach Anspruch 1, wobei die Expressionshöhe unter Verwendung eines PCR-basierten Verfahrens erhalten wird.

7. Verfahren nach Anspruch 1, wobei eine Expressionshöhe aus zumindest zwei Genen in einem beliebigen der Stroma-, Stoffwechsel-, Immun-, Proliferations- oder Stoffwechselgruppen oder deren Genprodukte erhalten wird.

8. Verfahren nach Anspruch 1, wobei eine Expressionshöhe aus zumindest vier Genen in beliebigen zwei der Stroma-, Stoffwechsel-, Immun-, Proliferations- oder Stoffwechselgruppen oder deren Genprodukte erhalten wird.

9. Verfahren nach Anspruch 1, das ferner das Erhalten einer Expressionshöhe zumindest eines coexprimierten Gens aus den in Tabelle 18 Aufgelisteten umfasst.

10. Verfahren zum Klassifizieren eines Krebspatienten gemäß einer Prognose, das die Schritte umfasst:
(a) Empfangen einer ersten Datenstruktur, die die jeweiligen Höhen eines Expressionsprodukts jedes von zumindest drei unterschiedlichen Prognosegenen, die in einer beliebigen der Tabelle 1 bis 12 aufgelistet sind, in einer Gewebeprobe, die aus einem Tumor im Patienten erhalten wurde, umfasst, wobei eines der Prognosegene IL6ST ist;
(b) Normalisieren der zumindest drei Expressionswerte, um normalisierte Expressionswerte zu erhalten;
(c) Bestimmen der Ähnlichkeit der normalisierten Expressionswerte jedes der zumindest drei Prognosegene in Bezug auf jeweilige Kontrollhöhen der Expression der zumindest drei Prognosegene, die aus einer zweiten Datenstruktur erhalten werden, um einen Patientenähnlichkeitswert zu erhalten, wobei die zweite Datenstruktur auf Expressionshöhen aus einer Mehrzahl von Krebstumoren basiert;
(d) Vergleichen des Patientenähnlichkeitswerts mit einem ausgewählten Ähnlichkeitsschwellenwert der jeweiligen normalisierten Expressionswerte jedes der zumindest drei Prognosegene mit den jeweiligen Kontrollhöhen der Expression der zumindest drei Prognosegene; und
(e) Klassifizieren des Patienten als eine erste Prognose habend, wenn der Patientähnlichkeitswert den Ähnlichkeitsschwellenwert überschreitet, und als eine zweite Prognose habend, wenn der Patientähnlichkeitswert den Ähnlichkeitsschwellenwert nicht überschreitet.

11. Computerprogramm, das ein Computercodemittel umfasst, um Schritte (b) bis (d) eines Verfahrens nach einem der Ansprüche 1 bis 10 durchzuführen,
wobei die Expressionshöhe eines Expressionsprodukts zumindest eines Prognosegens aus einer Gewebeprobe erhalten wurde, die aus einem Tumor des Patienten erhalten worden war, wobei das zumindest eine Prognosegen IL6ST ist;
wobei das Programm auf einem Computer ausgeführt wird.

12. Computerprogramm nach Anspruch 11, das auf einem computerlesbaren Medium umgesetzt ist

## Revendications

1. Méthode de prédiction du résultat clinique d'un patient chez qui un cancer a été diagnostiqué, comprenant :
(a) obtenir un taux d'expression d'un produit d'expression d'au moins un gène de pronostic provenant d'un échantillon de tissu obtenu à partir d'une tumeur du patient, le au moins un gène de pronostic étant IL6ST ;
(b) normaliser le taux d'expression du produit d'expression dudit au moins un gène de pronostic pour obtenir un taux d'expression normalisé ;
(c) exprimer le taux d'expression normalisé comme valeur d'expression par comparaison avec un taux d'expression provenant d'un ensemble de référence de tumeur ; et
(d) calculer un score de risque sur la base de la valeur d'expression normalisée, une expression accrue de IL6ST étant corrélée de façon positive avec un bon pronostic.

2. Méthode selon la revendication 1, comprenant en outre : générer un rapport sur la base du score de risque.

3. Méthode selon la revendication 1, dans laquelle le patient est un patient humain.

4. Méthode selon la revendication 1, dans laquelle la tumeur est une tumeur de cancer du sein.

5. Méthode selon la revendication 1, dans laquelle l'échantillon de tissu est un tissu fixé, inclus dans la paraffine.

6. Méthode selon la revendication 1, dans laquelle le taux d'expression est obtenu à l'aide d'une méthode à base de PCR.

7. Méthode selon la revendication 1, dans laquelle un taux d'expression est obtenu à partir d'au moins deux gènes dans l'un quelconque des groupes stromal, métabolique, immun, de prolifération ou métabolique, ou leurs produits de gène.

8. Méthode selon la revendication 1, dans laquelle un taux d'expression est obtenu à partir d'au moins quatre gènes dans deux quelconques des groupes stromal, métabolique, immun, de prolifération ou métabolique, ou leurs produits de gène.

9. Méthode selon la revendication 1, comprenant en outre l'obtention d'un taux d'expression d'au moins un gène co-exprimé parmi ceux énumérés dans le Tableau 18.

10. Méthode de classification d'un patient atteint d'un cancer conformément au pronostic, comprenant les étapes consistant à :
(a) recevoir une première structure de données comprenant les taux respectifs d'un produit d'expression de chacun d'au moins trois gènes de pronostic différents énumérés dans l'un quelconque des Tableaux 1 à 12 dans un échantillon de tissu obtenu à partir d'une tumeur chez le patient, l'un des gènes de pronostic étant IL6ST ;
(b) normaliser lesdites au moins trois valeurs d'expression pour obtenir des valeurs d'expression normalisées ;
(c) déterminer la similarité des valeurs d'expression normalisées de chacun desdits au moins trois gènes de pronostic avec des taux d'expression témoins respectifs desdits au moins trois gènes de pronostic obtenus à partir d'une seconde structure de données pour obtenir une valeur de similarité de patient, la seconde structure de données étant basée sur des taux d'expression provenant d'une pluralité de tumeurs cancéreuses ;
(d) comparer la valeur de similarité de patient avec une valeur seuil sélectionnée de similarité des valeurs d'expression normalisées respectives de chacun desdits au moins trois gènes de pronostic avec les taux d'expression témoins respectifs desdits au moins trois gènes de pronostic ; et
(e) classer le patient comme ayant un premier pronostic si la valeur de similarité de patient dépasse la valeur de similarité de seuil, et un second pronostic si la valeur de similarité de patient ne dépasse pas la valeur de similarité de seuil.

11. Programme d'ordinateur comprenant des moyens de codage informatique pour effectuer les étapes (b) à (d) d'une méthode selon l'une quelconque des revendications 1 à 10,
dans lequel le taux d'expression d'un produit d'expression d'au moins un gène de pronostic a été obtenu à partir d'un échantillon de tissu obtenu à partir d'une tumeur du patient, ledit au moins un gène de pronostic étant IL6ST
ledit programme étant exécuté sur un ordinateur.

12. Programme d'ordinateur selon la revendication 11 intégré dans un support pouvant être lu par un ordinateur.
